# EUROPÄISCHE PATENTSCHRIFT

(11) **EP 0 217 256 B1**
(45) Veröffentlichungstag und Bekanntmachung des Hinweises auf die Patenterteilung: **06.03.1991**
(21) Anmeldenummer: 86113021.9
(22) Anmeldetag: 22.09.1986
(51) Int. Cl.: D06L 3/12, C07D 221/14, C07D 401/14, C07D 405/14, C07D 409/14, C07D 413/14, C07D 471/06, C07D 519/00, C08G 73/10, D21H 21/00

(54) **Verfahren zur Fluoreszenzlöschung und neue kationische Naphthalin-peri-dicarbonsäureimid-Derivate**
Method for quenching fluorescence, and cationic derivatives of naphthalene-peri-dicarboxamide
Procédé pour éteindre la fluorescence et dérivés cationiques de la naphtaline-péri-dicarboxamide

(30) Priorität: 04.10.1985 DE 3535496
(43) Veröffentlichungstag der Anmeldung: 08.04.1987
(73) Patentinhaber: BAYER AG, 51368 Leverkusen (DE)
(72) Erfinder: Harnisch, Horst, Dr., D-5203 Much (DE)

(56) Entgegenhaltungen:
- EP-A- 0 028 777
- FR-A- 2 172 402
- GB-A- 1 221 888
- GB-A- 1 342 350
- US-A- 3 639 642
- US-A- 4 098 954
- Römpps Chemie-Lexikon, achte Auflage 1985, Franckh'sche Verlagshandlung, Stuttgart, Seite 3047
- D.J.Cram et al.:"Organic Chemistry, zweite Ausgabe, 1964, McGraw-Hill Book Company, New York, San Francisco, Seite 773
- A.F.Holleman "Lehrbuch der Chemie, zweiter Teil, "Organische Chemie", 27. u. 28. Auflage,1951, Walter de Gruyter & Co., Berlin, Seite 418

## Beschreibung

Gegenstand der Erfindung ist ein Verfahren zur Löschung der durch anionische optische Aufheller erzeugten Fluoreszenz durch Einwirkung praktisch farbloser, wasserlöslicher, kationischer Verbindungen auf die genannten Aufheller, das dadurch gekennzeichnet ist, daß die Verbindungen die allgemeine Formel 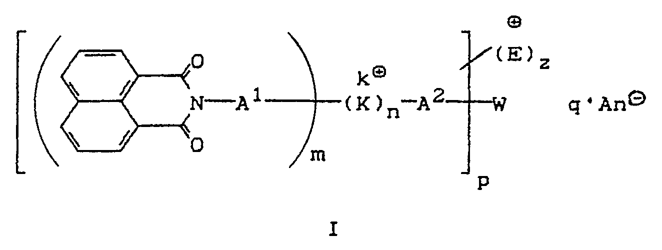 besitzen, worin
A¹, A² und W für ein Brückenglied oder eine Einfachbindung,
W auch für Wasserstoff,
_{E}^{⊕} für eine endständige Ammonium- oder Sulfoniumgruppe
^{⊕}_{K} für eine zweibindige Ammonium- oder Sulfoniumgruppe,
An für ein Anion,
m und k für 1 oder 2,
z für 0, 1, 2, 3 oder 4,
n für 0 oder 1 und
p für 1, 2 oder 3 stehen,
q der Summe der freien kationischen Ladungen entspricht, wobei n + z * 0 ist,

worin
das Naphthalinringsystem in der zweiten peri-Stellung auch durch -CH₂-CH₂-, -CO-O-CO- oder -CO-NH-CO- substituiert sein kann,
das Naphthalinringsystem, A¹ , A², W,-^{⊕}_{E} und -^{⊕}ₖ - sowie das zweite peri-Dicarbonsäureimid-Stickstoffatom durch nichtionische Reste substituiert sein können, und die gesamte angegebene Struktur auch wiederkehrende Einheit einer hochmolekularen Verbindung sein kann.

Unter cyclischen Ammonium- und Sulfoniumgruppen werden ^{⊕}_{N}-bzw, -haltige Ringe oder Ringsysteme als ganze verstanden. Diese können gesättigt, teilgesättigt oder quasiaromatisch sein, wobei die gesättigten und quasiaromatischen bevorzugt sind.

Geeignete Ammonium- und Sulfoniumgruppen sind beispielsweise:

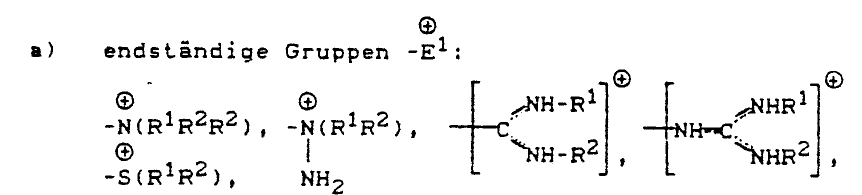

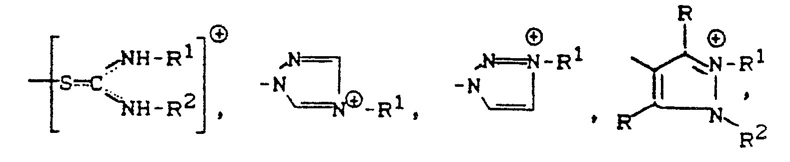

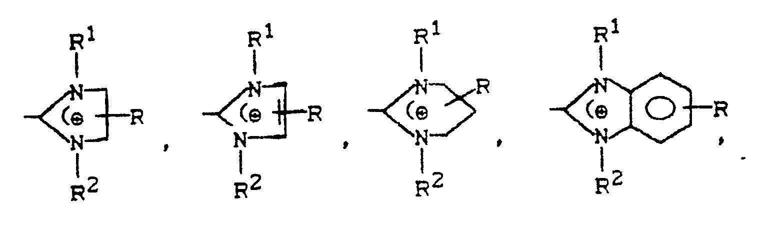

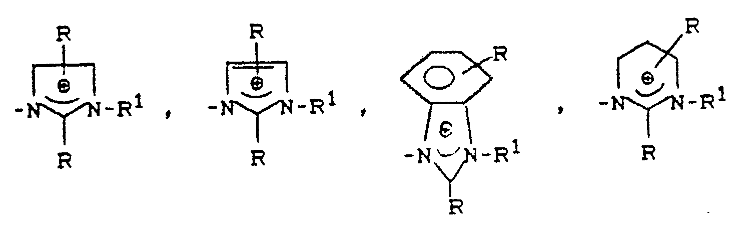

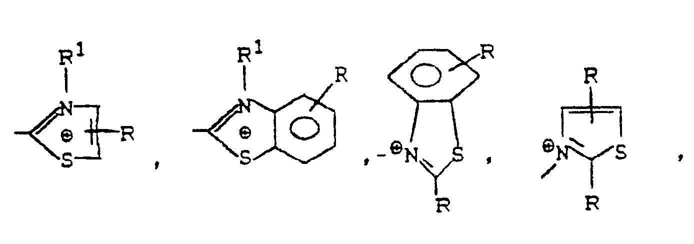

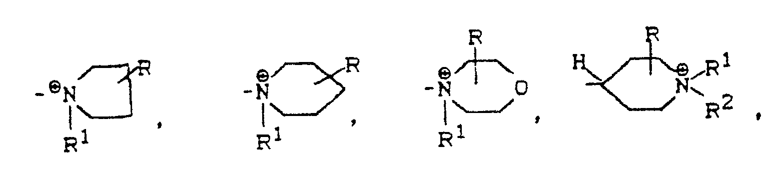

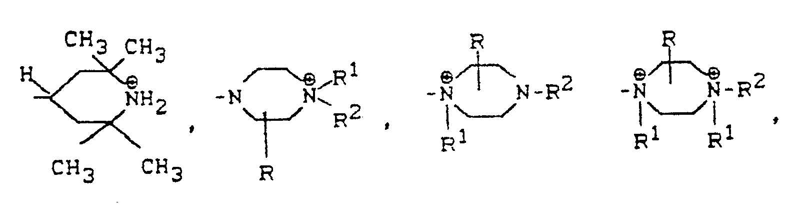

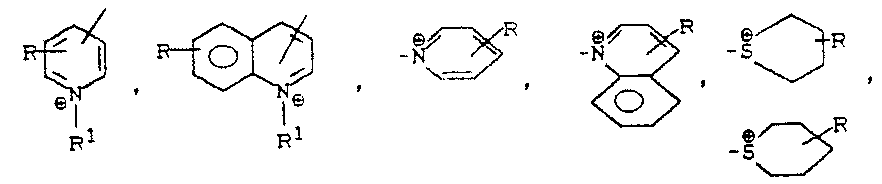

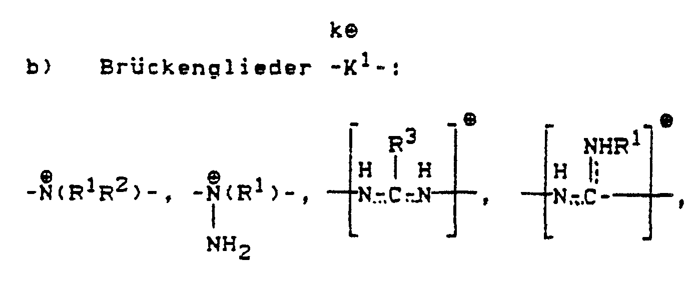

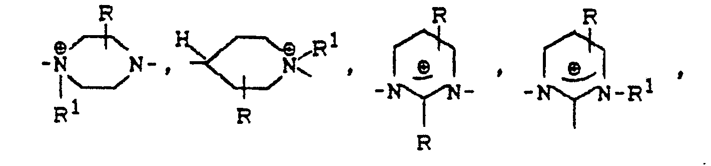

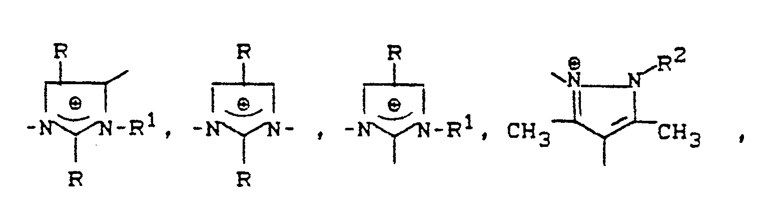

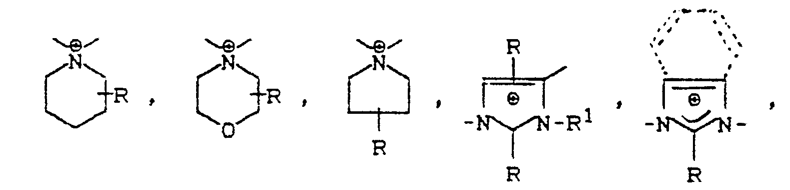

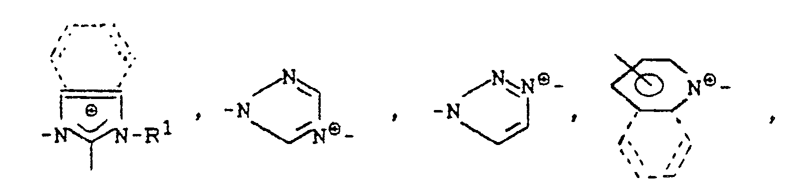

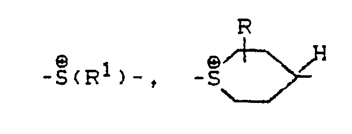

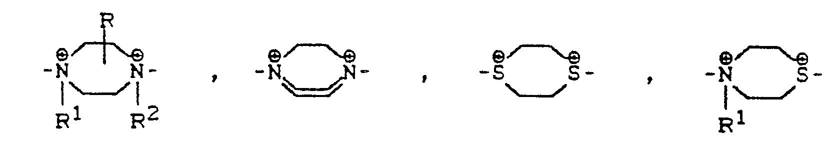

In den Formeln bedeuten:
R Wasserstoff oder 1 bis 4 Methylgruppen,
R¹ und R² Wasserstoff, C₁-C₄-Alkyl, das durch OH-, NH₂, C₁-C₄-Alkoxy, Halogen, CONH₂, CN, COOH, C₁-C₄-Alkoxycarbonyl oder C₁-C₄-Alkylcarbonyl substituiert sein kann, C₂-C₄-Alkenyl, Phenyl-C₁-C₃- alkyl, Phenylaminocarbonylmethyl, Benzoylmethyl oder Benzimidazolyl-(2)-methyl, die durch Nitro, Cyan, C₁-C₄-Alkyl, C₁-C₄-Alkoxy, Chlor oder Brom kernsubstituiert sein können,
R² außerdem Cyclohexyl oder Phenyl, das durch Nitro, C₁-C₄-Alkyl, C₁-C₄-Alkoxy, Chlor oder Brom substituiert sein kann, und
R³ Wasserstoff, C₁-C₄-Alkyl, SH oder NH₂.

Bevorzugte acyclische Ammoniumgruppen entsprechen den Formeln -N (R¹R²R²) und -N (R¹R²)-, insbesondere -N(CH₃)₂-R¹ und -N (CH₃)₂-.

Bevorzugte cyclische Ammoniumgrupen sind Piperazinium-, Piperidinium-, Morpholinium-, Pyrrolidinium-, Imidazolium-, Pyrazolium- und Pyridinium-Reste. Von besonderer Bedeutung sind die Piperazinium- und Pyridinium-Gruppe.

Als Anionen An> kommen die üblichen farblosen anorganischen oder organischen vasserlöslichmachenden Anionen in Betracht wie Chlorid, Bromid, Jodid, Chlorozinkat, Tetrafluoroborat, Sulfat, Hydrogensulfat, Methosulfat, Ethosulfat, Benzolsul fonat, p-Toluolsulfonat, Methylsulfonat, Amidosulfonat, Nitrat, Hydrogenphosphat, Methylphosphonat, Methylphosphonatmonomethylester, Acetat, Lactat, Formiat, Maleat, Succinat, Citrat, Tartrat und Oxalat.

Beispiele für anionische Substituenten sind die Carbonsäure- und Sulfonsäuregruppe.

Als Beispiele für nichtionische Substituenten im Rahmen von Verbindung (I) seien genannt:
Halogen wie Chlor und Brom, Hydroxy, Cyan, Alkyl, Alkoxy, Aralkoxy, Aryloxy, Cyclohexyloxy, Alkylcarbonyloxy, Aroyloxy, Arylaminocarbonyl, Alkoxycarbonyl, Alkylcarbonyl, Alkylsulfonyl, Arylsulfonyl, gegebenenfalls mono- oder dialkyliertes oder ringgeschlossenes Carbamoyl oder Sulfamoyl, als Substituenten für Ringe außerdem Alkyl, Aralkyl und Aryl sowie (außer für II und III) auch Nitro.

Generell wird im Rahmen der Erfindung vorzugsweise unter Alkyl ein Rest mit 1 bis 4 C-Atomen, unter Alkenyl, Allyl oder Methallyl, unter Aryl, Phenyl oder Naphthyl und unter Aralkyl, Phenyl-C₁-C₃-alkyl verstanden.

Geeignete nitrogruppenfreie peri-Dicarbonsäureimid-Reste gehören der Naphthalimid-Reihe (Formel II) oder der Naphthalin-1,4,5,8-tetracarbonsäurdiimid-Reihe (Formel 111) an:

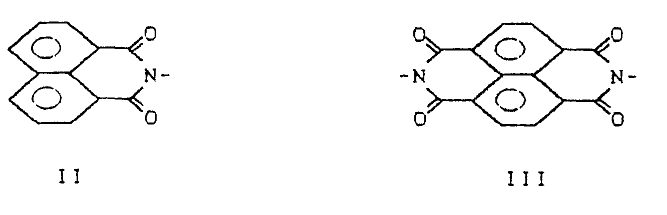

Die Kohlenstoffatome von (II) und (III) können durch nichtionische, anionische oder kationische Reste substituiert sein.

Die Imidstickstoffatome von (II) und (III), insbesondere eines der Imidstickstoffatome von (III), können auch nichtionische Substituenten tragen, beispielsweise gegebenenfalls über -O- oder -N(R)-CO- an das Imidstickstoffatom gebundene Alkyl-, Alkenyl-, Aralkyl-, Cycloalkyl-, Aryl- und Hetaryl-Reste, wobei R für H oder CH₃ steht. Bevorzugt sind diese Reste direkt mit dem Imidstickstoffatom verknüpft. Sie können ihrerseits durch nichtionische, anionische oder kationische Reste wie -^{⊕}_{E} substituiert sein.

In (II) stehen die Substituenten bevorzugt in 3-, 4-, 5- und 6-Stellung.

Bevorzugte Substituenten an (II) sind Chlor, Brom und Carboxy in 4- und/oder 5-Position sowie die peri-Dicarbonsäureanhydridgruppe. Von besonderer technischer Bedeutung sind die unsubstituierten Naphthalimidreste.

Geeignete Brückenglieder A¹, A² und W, die die Reste (II) und/oder (III) miteinander oder diese Gruppen mit den Ammonium- und/oder Sulfonium-Resten verbinden oder die auch zwei dieser kationischen Gruppen miteinander verknüpfen, sind solche, die unter Applikationsbedingungen beständig sind, also beispielsweise in Wasser nicht hydrolysiert werden. Sie sind in der Regel zwei- oder dreibindig und bestehen aus einem oder mehreren acyclischen und/oder cyclischen nichtchromophoren Strukturelementen. Als Beispiele seien genannt:
Gegebenenfalls substituierte C₁-C₁₀-Alkylen-, -C₆H₄-CH₂-, Phenylen-, Naphthylen-, Anthracen-, Phenanthren-, Dihydrophenanthren-, Fluorenon-, Hetarylen-, Cyclohexylen-Reste, S- oder 6-gliedrige gesättigte heterocyclische 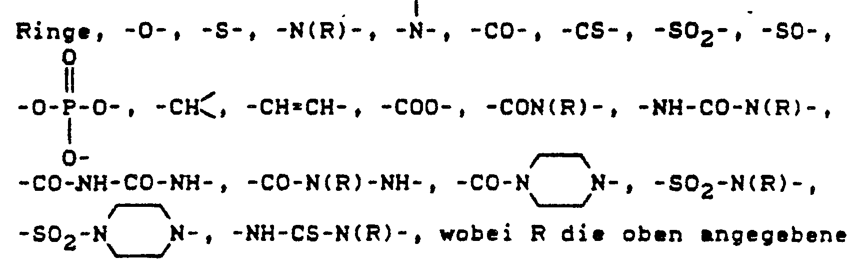 Bedeutung hat. Die genannten Kohlenwasserstoffreste und Heterocyclen können auch zusammen mit 1 bis 3 der übrigen Brückenglieder eine gemeinsame Brücke bilden.

Bevorzugtes Brückenglied A¹ zwischen einem Imidstickstoffatom von (II) oder (III) und einer Ammonium- oder Sulfoniumgruppe ist des Brückenglied A³, des für eine Einfachbindung, 1,3- oder 1,4-C₆H₄-CH₂-, -Xylylen, -Phenylen oder -Cyclohexylen, C₁-C₁₀-Alkylen, das durch -O-, -CO-O-, -CO-N(R)-oder 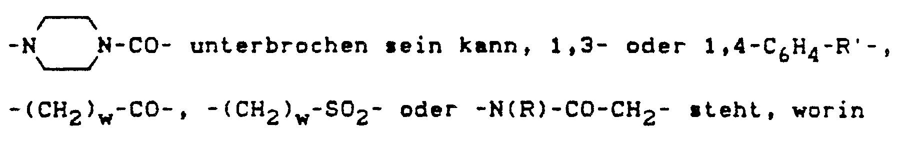
R für Wasserstoff oder Methyl,
R' für -CO-(CH₂)ᵤ-, -S0₂-(CH₂)ᵤ oder -CO-N(R)-C₁-C₅-alkylen,
w für 1, 2 oder 3 und
u für 0, 1, 2 oder 3 stehen.

Besonders geeignete Brückenglieder A³ sind folgende, wobei die gegebenenfalls mit ^{*} gekennzeichnete Seite des Brückengliedes mit dem Imidstickstoffatom verknüpft ist: 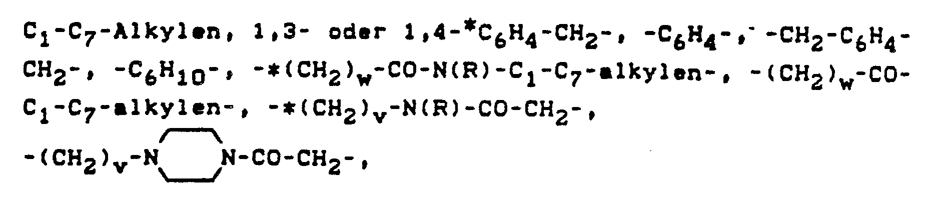 -*C₆H₄-CO-NH-C₁-C₇-alkylen, -*C₆H₄-CO-(CH₂)_{W}-, -*C₆H₄-NH-CO-CH₂-, -*N(R)-CO-CH₂- oder anknüpfend an das nichtkationische N-Atom eines Piperaziniumrestes auch -*C₆H₄-CO-, -*C₆H₄-S0₂-, -^{*}(CH₂)_{w}-CO- oder -*(CH₂)_{w-}SO₂-. Hierin bedeuten R = H oder CH₃; v = 2 oder 3 und w = 1, 2 oder 3.

Die Brückenglieder A¹ entfallen, wenn eine cyclische Ammonium- oder Sulfoniumgruppe über eines ihrer Ring-C-Atome mit dem Imidstickstoff verknüpft ist, beispieleweise in folgender Weise:

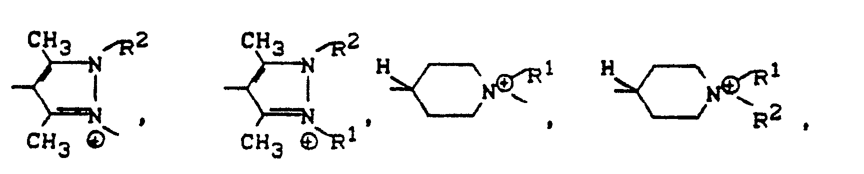

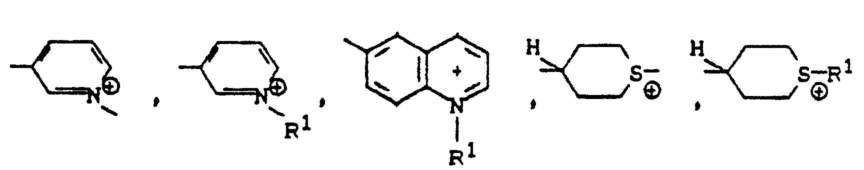

Bevorzugte Brückenglieder -(A²)p-W zwischen den Ammonium- und/oder Sulfoniumgruppen entsprechen der Formel
-(A⁴⁻)p-W¹
   Hierin bedeuten:
   A⁴ = -Z-y-,
   Z = C₁-C₅-Alkylen, -C₆H₄-CH₂-, Xylylen, Phenylen, Cyclohexylen oder eine Einfachbindung,
   Y = -0-, -S-, -N(R)-, -N(R)-NH-, -CO-, -CO-NH-CO-, -CO-NH-CH₂-, S0₂-, -OCO-, -N(R)CO-, -N(R)-NH-CO-, -CON(R)-, -S0₂N(R)-, -COO-, -N(R)-CO-NH-, -N(R)-CH₂-CO-, -N(R)-CH₂-CO-NH- oder eine Einfachbindung,
   W^{l =} -CO- oder p-bindiger Rest aus der Reihe C₁-C₁₀-Alkylen, Benzol, Naphthalin, Anthracen, Phenanthren, 9,10-Dihydrophenanthren, Cyclohexen, Fluoren-9-on (3,6), Thiophen (2,5), Dibenzofuran (3,6), Dibenzothiophen (3,6), Dibenzothiophen-S-dioxid (2,7), 9H-Thioxanthen-S-dioxid (3,6), Carbazol (3,6), 9H-Xanthen-9-on (2,7), 9-Acridon (2,7), 1,3,4-Oxadiazol (2,5), 1,2,4-Oxadiazol (3,5), 1,3,4-Thiadiazol (2,5), s-Triezin (2,4,6), Piperazin (1,4), 1,2-Dihydro-1,2,4,5-tetrazin (3,6), Reste der 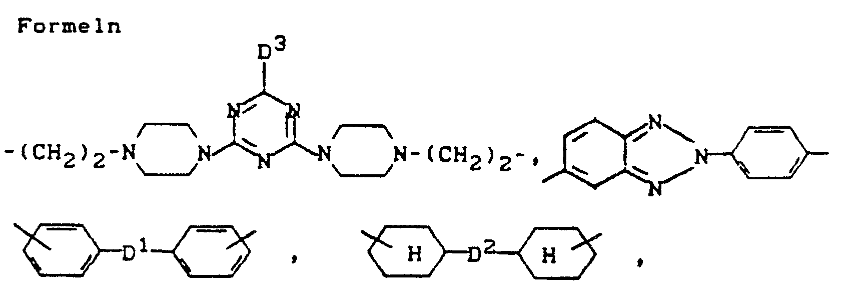 oder eine Einfachbindung,
   D¹, D² = Einfachbindung, gegebenenfalls durch -O-unterbrochener C₁-C₅-Alkylenrest, -O-C₂-C₄-alkylen-O-, -O-, -N(R)-, -CO-, -CO-NH-, -NH-CO-NH-, 1,1-Cyclohexylen oder ein Rest der Formel 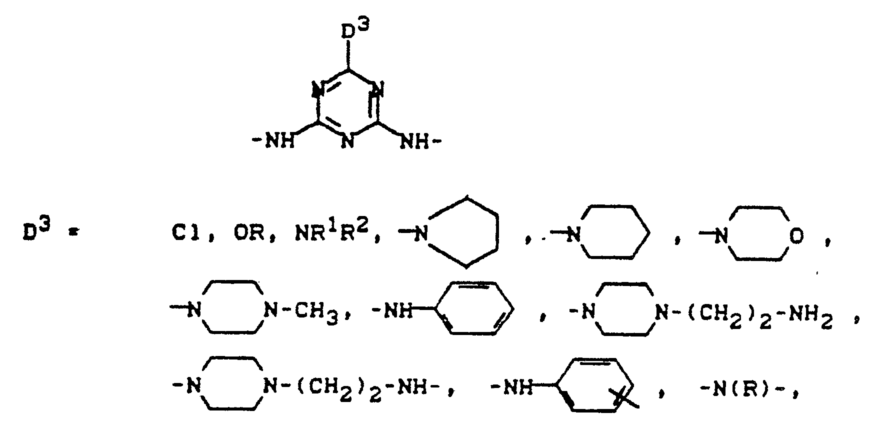
   D¹ eußerdem = -CH(C₆H₅)-, -CH(C₆H₄-)-, -N(C₆H₅)-, -CH=CH-, -S-, -S0₂, m- oder p-Phenylen, Thiophen(2,5), 1,3,4-Oxadiazol(2,5), 1,3,4-Thiadiazol(2,5), Oxazol(2,5), Thiazol(2,5), 1,2-Dihydro-1,2,4,5-tetrazin(3,6) oder 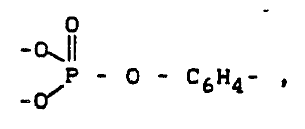
   D² außerdem = -CH(C₆H₁₁)- oder -CH(C₆H₁₀-)-, wobei die unter W¹ genannten Ringe durch C₁-C₄-Alkyl, C₁-C₄-Alkoxy oder Chlor kernsubstituiert sein können,

und worin R, R¹, R² die oben engegebene Bedeutung haben.

Typische Brückenglieder zwischen den Ammonium- und/oder Sulfoniumgruppen sind beispielsweise folgende: 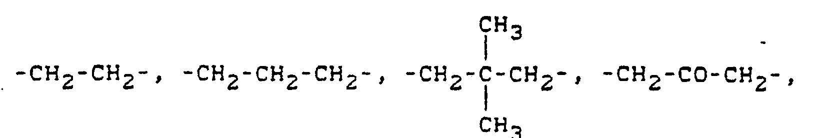 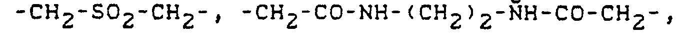 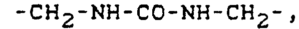 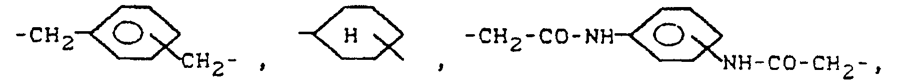 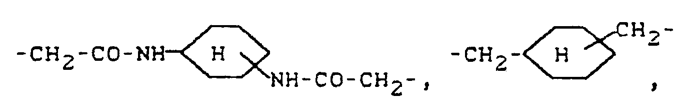 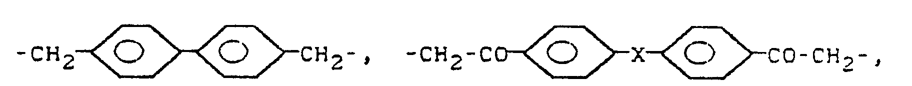 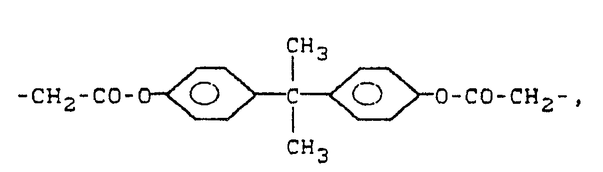 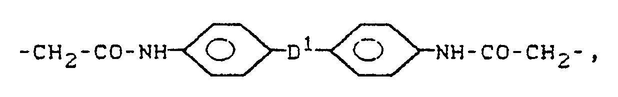 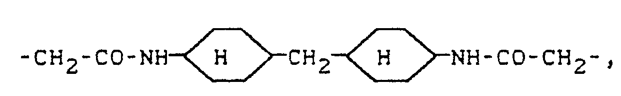 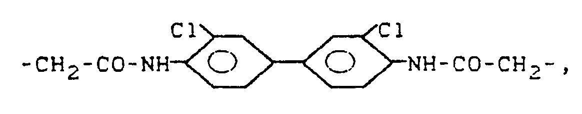 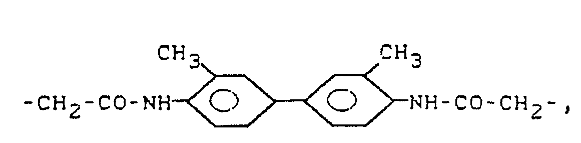 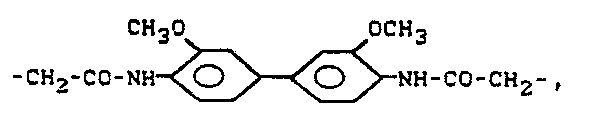 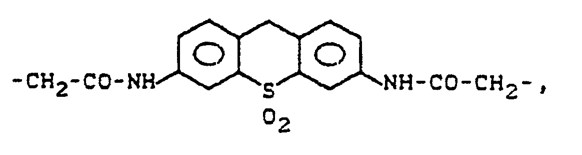 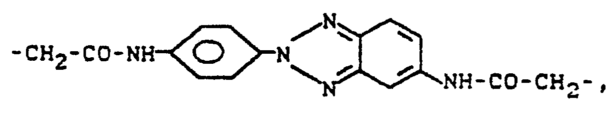 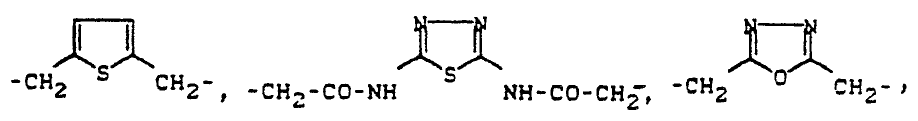 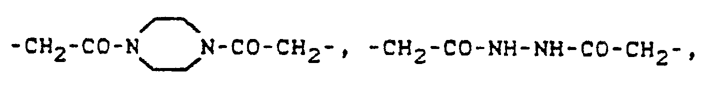 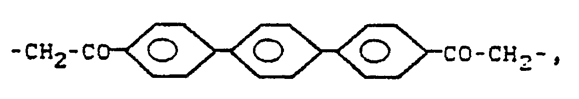 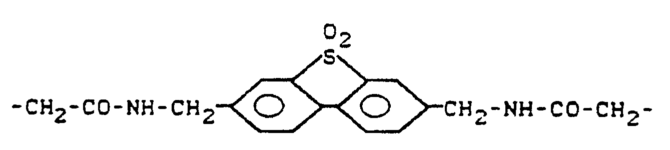 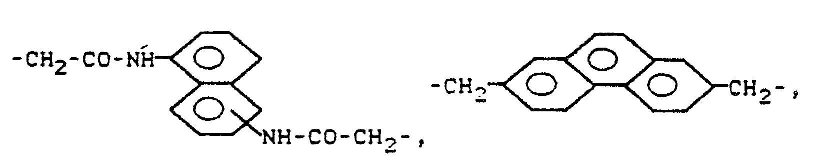 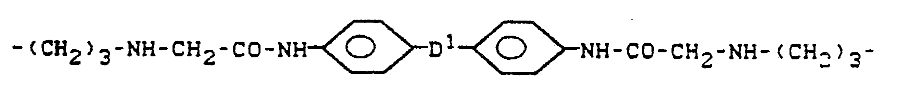 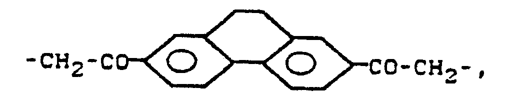 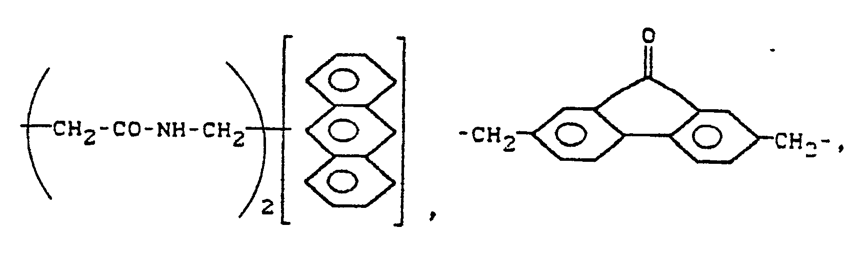 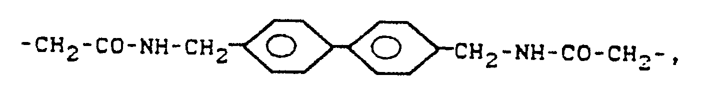 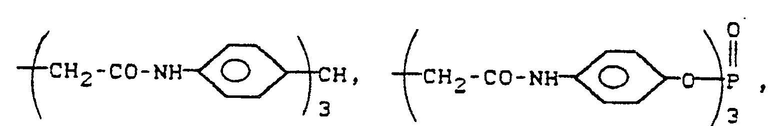 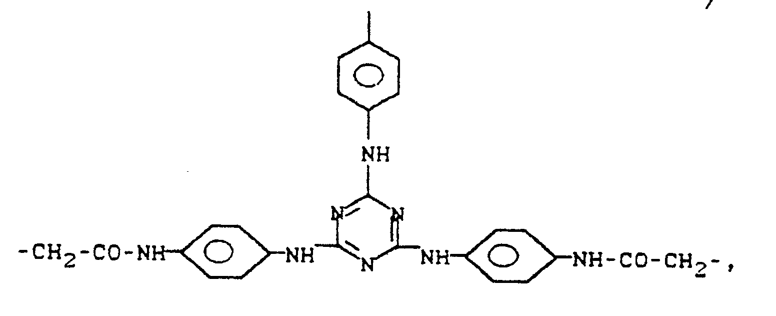 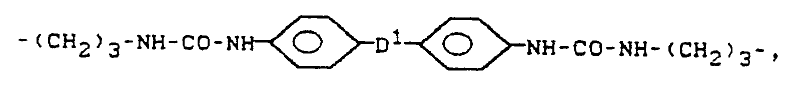 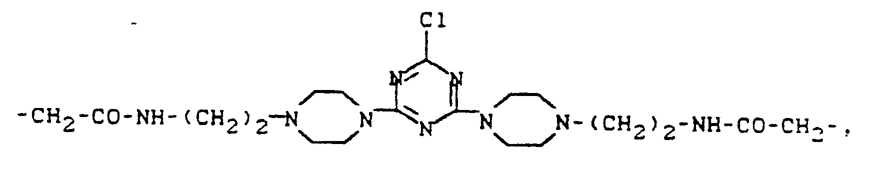 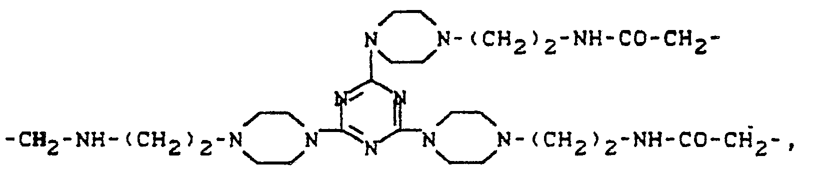 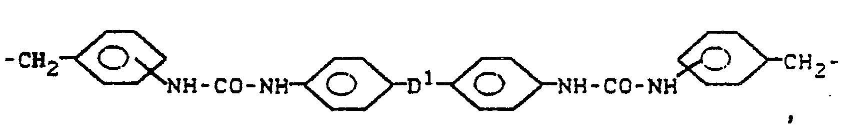 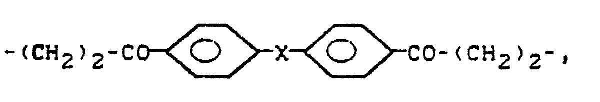 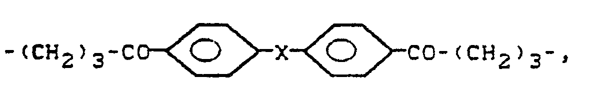 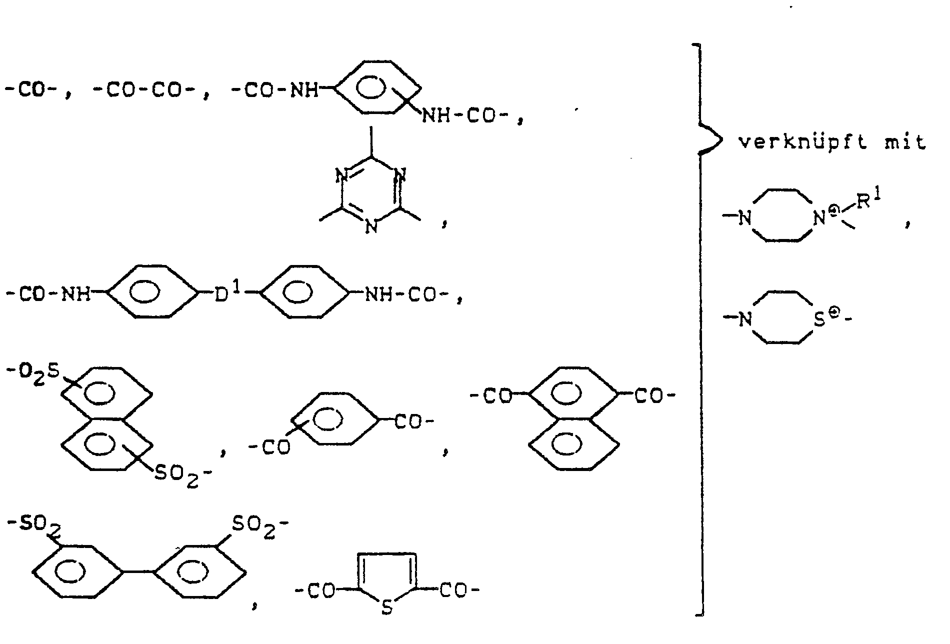 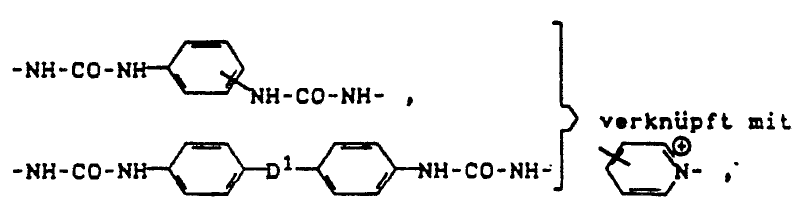 wobei
x = -O-, -S- oder -N(R)- ist und
R und D¹ die oben angegebene Bedeutung haben.

Typische Brückenglieder -A¹ -A²-W-A²-A¹- zwischen Imidstickstoffatomen von 2 Resten der Formel (II) und/oder (III), vorzugsweise zwischen je einem Imidstickstoffatom von 2 Resten der Formel (III) sind beispielsweise: 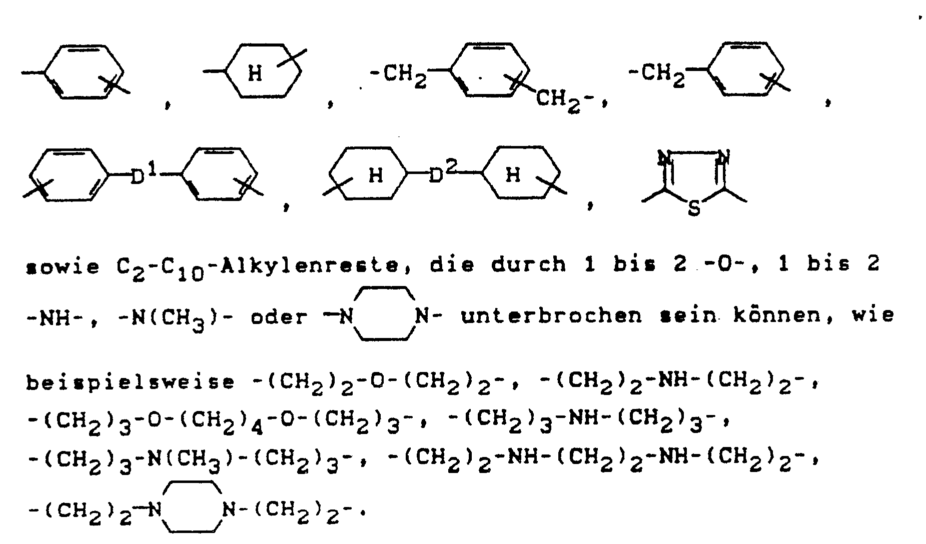
D¹ und D² haben die oben angegebene Bedeutung.

Im Rahmen der Formel entsprechen bevorzugte Verbindungen der Formel 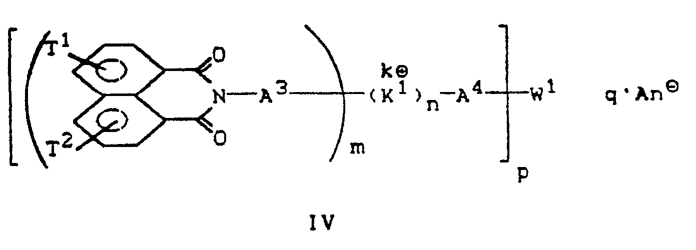 worin
T¹ und T² je für Wasserstoff, Chlor, Brom, Hydroxy, Alkoxy, Benzyloxy, Phenoxy, Cyclohexyloxy, Cyan, Carboxy, Alkoxycarbonyl, Alkylcarbonyl, Alkylcarbonyloxy, Benzoyloxy, Carbamoyl- oder Sulfamoylreste, die durch 1 bis 2 Alkylreste substituiert sein können, Sulfonyl- oder Carbonylpyrrolidino, -piperidino, -morpholino, -N'-alkyl-piperazino, 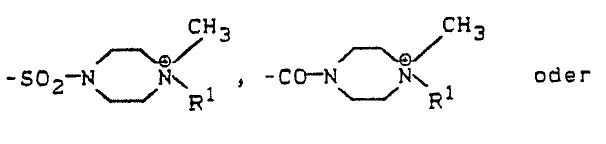 Alkylsulfonyl,
T¹ und T² auch zusammen in peri-Stellung für -CH₂-CH₂-, -CO-O-CO- oder -CO-N(T³)-CO-,
T³ für Wasserstoff, -0-Alkyl, Alkyl, Allyl, Phenyl, Cyclohexyl, Phenyl-C₁-C₃-alkyl, Pyridyl-(2), -(3) oder --(4), Thiazol-2-yl, Benzthiazol-2-yl, 1,2,4-Triazol-3-yl, Pyrazol-5-yl, Imidazol-2-yl, Benzimidazol-2-yl, Pyrimid-2-yl, -N(R)-CO-alkyl, -N(R)-CO-phenyl, -A^{3 ⊕}_{E} oder im Falle m = 1 auch zwei T³⁻Gruppen zusammen für einen zweibindigen Rest -A^{3 ⊕}_{K} ¹-A⁴- oder -A³-^{⊕}_{K} 1-A⁴-W¹-A⁴-^{⊕}_{K} ¹-A³- unter Ausbildung einer hochmolekularen Struktur stehen, worin
die Alkylreste 1 bis 6 C-Atome enthalten und durch OH, Alkoxy, Chlor oder -^{⊕}_{E}¹ substituiert sein können, die Alkoxyreste 1 bis 4 C-Atome enthalten und durch -0- unterbrochen sein können, die Phenyl- und Phenylenreste sowie die Hetarylreste 1 bis 2 Substituenten aus der Reihe Alkyl, Alkoxy, Chlor, Brom, Alkoxycarbonyl, Alkylsulfonyl, Cyan, Carboxy, Carbamoyl, Sulfamoyl, Nitro und -^{⊕}_{E} enthalten können, worin
die Indices m und p nur dann gleichzeitig 1 bedeuten, wenn T¹ und T² zusammen für peri-CO-N(T³)-CO- stehen, worin
n nur dann Null sein kann, wenn mindestens einer der Reste T¹ und T² oder der Rest T³ eine kationische Gruppe -^{⊕}_{K}1- oder -^{⊕}_{E} ¹ enthält,

worin die Gruppierung -^{⊕}_{K}1-A⁴-W¹-A⁴-^{⊕}_{K}¹- insgesamt auch für 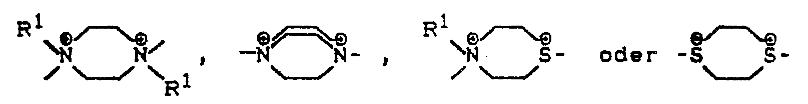 stehen kann
und worin A³, A⁴, -^{⊕}_{K} 1-, -^{⊕}_{E} 1-, W¹, An^{⊖}, R, R¹, m, k, n, p und q die vorstehend genannte Bedeutung besitzen,

Bevorzugt stehen T¹ und T² in den beiden peri-Stellungen des Naphthalinringsystems. Bevorzugte Bedeutungen sind, wenn sie als einwertige Reste vorliegen, Wasserstoff, Chlor, Brom und Carboxy.

Zusammen stehen T¹ und T² bevorzugt für -CO-N(T³)-CO-. Unter den Resten T³ sind diejenigen bevorzugt, die mit einem Kohlenstoffatom an den Imidstickstoff anknüpfen.

Wenn m = 2 und p = 1, können beide Reste A³ voneinander verschieden sein.

Wenn p = ₁, ist der Rest -A⁴-W¹ bevorzugt = R¹ oder R² (als Substituent an ^{⊕}_{K} 1

Im Rahmen der Formel (IV) entspricht ein bevorzugter Verbindungstyp der Formel 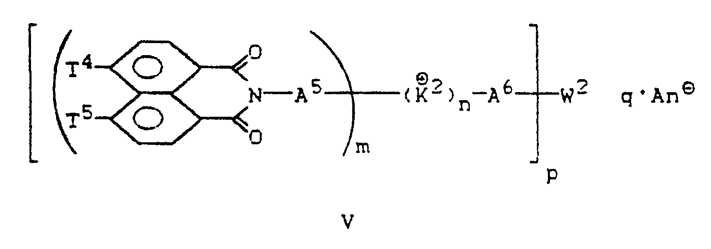 worin
T⁴- und T⁵ für Wasserstoff, Chlor oder Brom, oder
T⁴ und T⁵ zusammen für eine -CO-N(T⁶)-CO-Gruppe,
T⁶ für Wasserstoff, OH, gegebenenfalls durch 1 bis 2 OH-Gruppen, Chlor, C₁-C₄-Alkoxy oder COOH substituiertes C₁-C₆-Alkyl, Allyl, Phenyl oder Benzyl, die durch 1 oder 2 C₁-C₄-Alkyl, Chlor, C₁-C₂-Alkoxy, Brom, Carboxy, Cyan, C₁-C₂-Alkoxycarbonyl, C₁-C₂-Alkylsulfonyl, Carbamoyl, Sulfamoyl substituiert sein können,
ß-Aminoethylphenyl, Pyridyl-(2)- oder -(3)-, Benzthiazol-(2)-yl, 1,2,4-Triazol-3-yl, Cyclohexyl, -A⁵-^{⊕}_{E} 2 oder im Falle m = 1, n = 0 und p = 1 für eine Gruppierung der Formel -A⁵ -^{⊕}_{K} ²⁻A⁶⁻ oder -A^{5-⊕}_{K} ²-A⁶-W²-A⁶-^{⊕}_{K} 2_{-A}5_{-,}
wobei A⁵ und A⁶ bzw. die beiden Reste A⁵ mit 2 verschiedenen 1,4,5,8-Naphthalintetracarbonsäurediimid-Molekülen verbunden sind und somit die gesamte angegebene Struktur zur wiederkehrenden Einheit einer hochmolekularen Verbindung machen,
A⁵ für eine Einfachbindung, C₁-C₅-Alkylen, m- oder p-C₆H₄-CH₂-,
A6 für eine Einfachbindung, -CHz-, -CH₂-CO-O-, -CH₂-CO-N(R)-, -CH₂-S0₂-N(R)-, -(CH₂)₂-CO-N(R)-, -CH₂-CO-NH-CH₂-, C₁-C₅-Alkylen-N(R)-CO-, C₁-C₅-Alkylen-N(R)-SO₂-, C₁-C₅-Alkylen-N(R)-CO-NH-, -CH₂-C₆H₄-NH-C0-, -CH₂-C₆H₄-NH-S0₂-, -CH₂-C₆H₄-NH-CO-NH-, -(CHz)w-CO-, -(CH₂)_{W}-CO-C₆H₄-NH-CO-, -(CH₂)_{w} CO-C₆H₄-NH-SO₂-, -(CH₂)_{w} CO-C₆H₄-NH-CO-NH-, außerdem: -SOz-, -CO- oder -CONH-, wenn 2 2 = Piperazinium und -NH-CO-NH-, wenn ^{⊕}_{K} ² = Pyridinium, 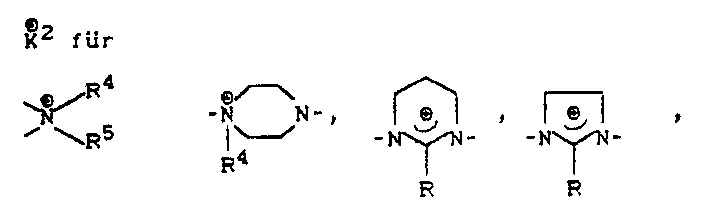 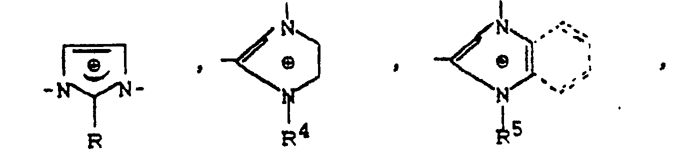 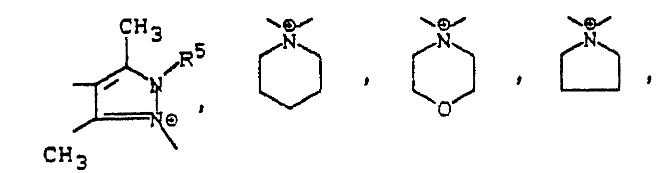 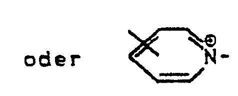 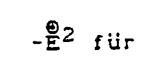 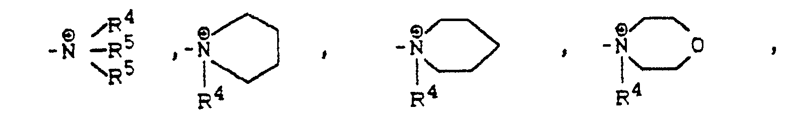 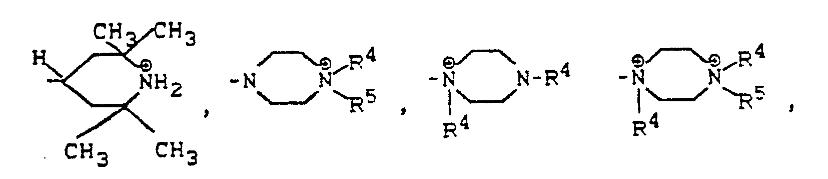 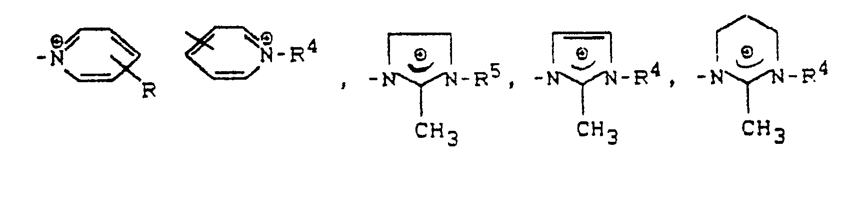 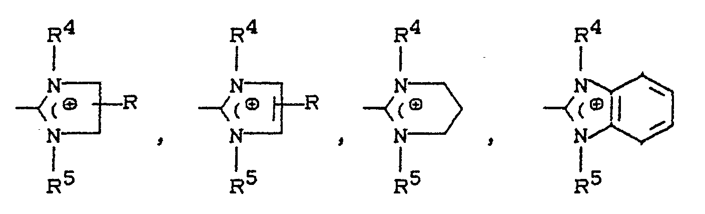 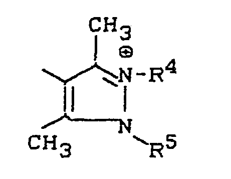
R für Wasserstoff oder 1-4 Methylgruppen,
R⁴ und R⁵ je für C₁-C₄-Alkyl, das durch OH, CONH₂, C,-C_{z}-Alkoxycarbonyl oder C₁-C₂-Alkylcarbonyl substituiert sein kann, Allyl,
R⁴ außerdem für Wasserstoff, Cyclohexyl, Benzyl-, Benzoylmethyl-Reste, die durch 1 oder 2 Reste der Reihe Methyl, Chlor, Methoxy, Ethoxy, Cyan, C₁-C₂-Alkoxycarbonyl oder Nitro kernsubstituiert sein können, oder Benzimidazol-2-ylmethyl,
R⁵ außerdem für Benzyl oder je ein Rest R⁵ auch für Phenyl,
W² für -CO-, einen p-bindigen Benzol-, Naphthalin-, Cyclohexan-, Piperazin-, Thiophen-(2,5)-, 1,3,4-Oxadiazol-(2,5)-, s-Triazin-(2,4 oder 2,4,6)-Rest oder einen Rest der Formeln 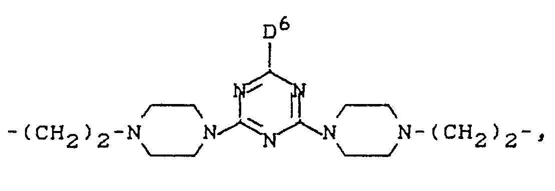 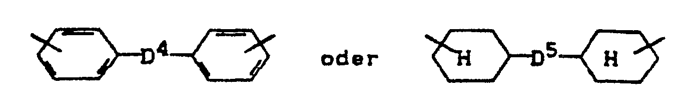 worin im Falle p = 1 eine der angegebenen freien Valenzen durch Wasserstoff abgesättigt ist,
D⁴ und D⁵ je für CH₂, einen gegebenenfalls durch -0- unterbrochenen C₂-C₅-Alkylenrest (insbesondere -CH₂-, -CH(CH₃)-, -C(CH₃)₂ und -CH₂-CH₂-), -O-, -N(R)-, -O-C₂-C₄-alkylen-O- oder einen Rest der Formel 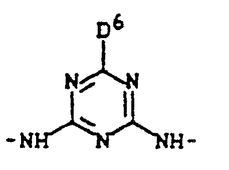 mit D⁶ = Cl, OR, -N(R⁴R⁵), 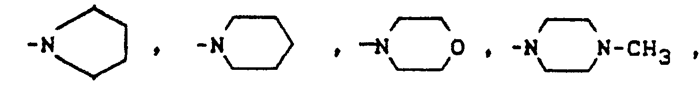 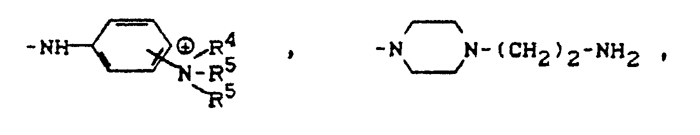 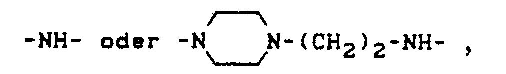 D⁴ außerdem für 1,1-Cyclohexylen, p-Phenylen, -CH(C₆H₅)-, -CH(C₆H₄)-, -S-, -S0₂-, -CO-NH-, -NH-CO-NH-, 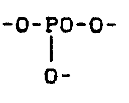 oder eine Einfachbindung stehen, wobei die in D⁴, D⁵ und sonst noch in W² genannten Ringe durch CH₃, CH₃O oder CI subtituiert sein können,

worin im Falle p = 2 die Gruppierung --^{⊕}_{K} ²-A⁶-W²-A⁶-^{⊕}_{K} 2-insgesamt auch für 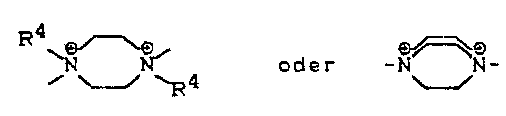 stehen kann,
worin die Indices m und p nur dann gleichzeitig 1 bedeuten, wenn T⁴ und T⁵ eine cyclische peri-Dicarbonsäureimid-Gruppierung bilden,
worin n, p, q, An^{⊖} und R die gleiche Bedeutung wie in Formel IV besitzen,
und worin n nur dann Null sein kann, wenn T⁶ eine kationische Gruppe -^{⊕}_{K} 2- oder -^{⊕}_{K} ² enthält.

Von den Verbindungen (V) sind die Verbindungen 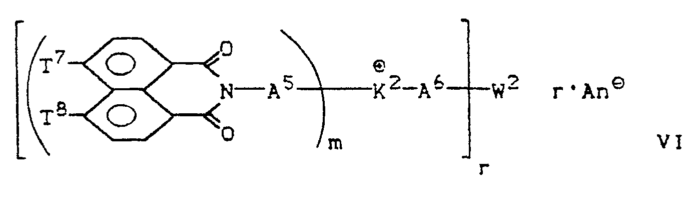 mit T⁷ und T⁸ = Wasserstoff, Chlor oder Brom,
r = 2 oder 3,

worin die Reste A⁶ und - wenn m = 2 - die Reste A⁵ voneinander verschieden sein können, und 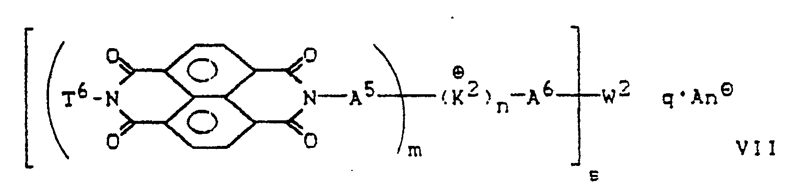 mit s = 1 oder 2
hervorzuheben.

In (VI) stehen T⁷und T⁸ bevorzugt für Wasserstoff, und in Formel (VII) bedeutet bevorzugt m nur dann 2, wenn
s für 1 und
   -A⁶-W² für R⁴ oder R⁵ stehen.

Von den Verbindungen (VI) und (VII) sind besonders zu nennen:
A. VI mit m = 1 und
VII mit m und n = 1. 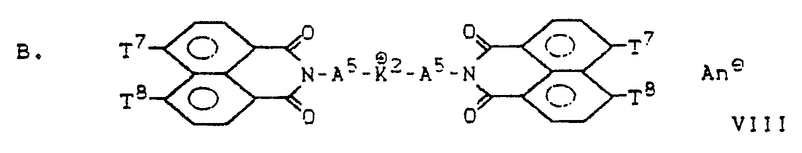 und 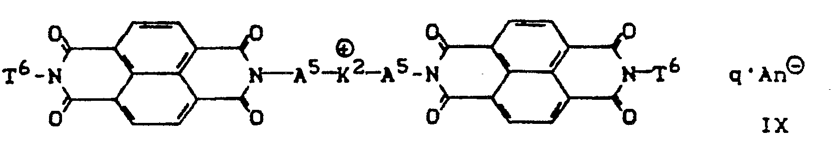 worin die beiden Reste A⁵, T⁶, T⁷, T⁸ jeweils gleich oder verschieden sein können, und T⁷ und T⁸ insbesondere Wasserstoff bedeuten.

Von den Verbindungen (VII) sind außerdem folgende zu nennen: 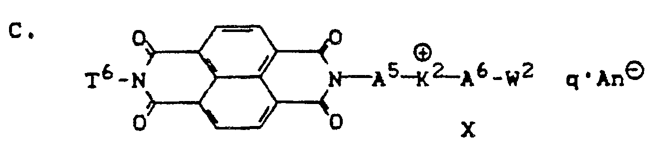 worin W2 einbindig ist und auch für Wasserstoff steht. 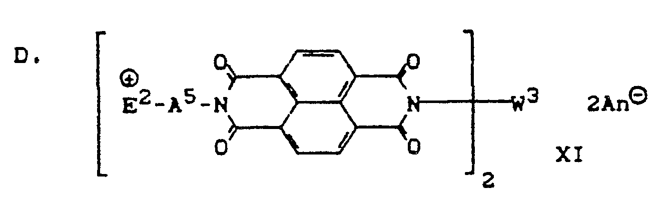 worin
W³ für C₂-C ₁₀-Alkylen, das durch 1 oder 2 -O-, -NH-, 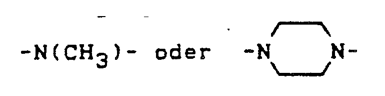 unterbrochen sein kann, 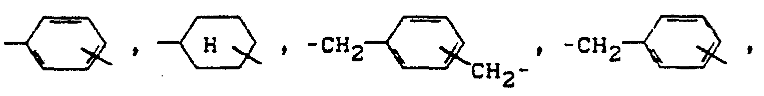 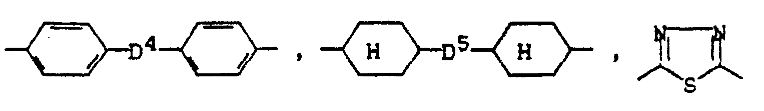 steht, und
D⁴ und D⁵ die vorstehend genannte Bedeutung haben.

### E. Verbindungen mit der wiederkehrenden Einheit

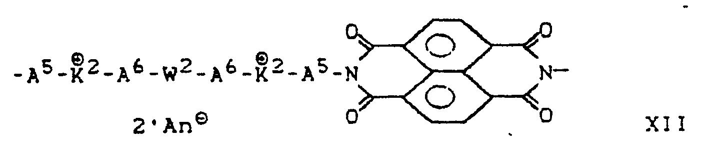

### F. Verbindungen mit der wiederkehrenden Einheit

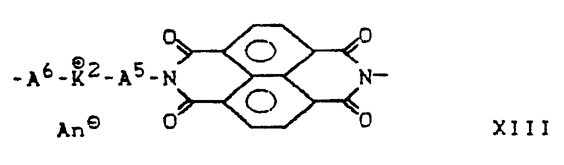

Von den unter A-C genannten Tetracarbonsäurediimid-Derivaten seien die mit T⁶ = -A⁵-^{⊕}_{E} 2 besonders hervorgehoben.

Gegenstand der Erfindung sind auch Verbindungen der allgemeinen Formel (VI), Verbindungen der allgemeinen Formel (VIII) und Verfahren zu ihrer Herstellung.

Verbindungen (VI) werden hergestellt durch Umsetzung
a) von Verbindungen der Formel 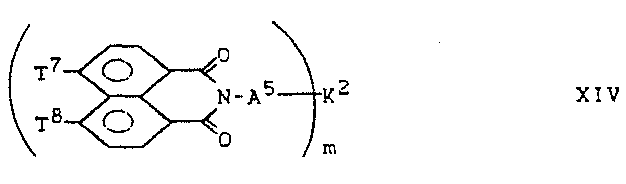 worin
   T⁷, T⁸, A⁵ und m die gleiche Bedeutung wie in Formel VI haben und
      -K² die nichtkationische einbindige Basenvorstufe von -^{⊕}_{K} 2- bedeutet,

      im Molverhältnis r : 1 mit Verbindungen der Formel 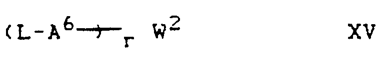 worin
   A⁶, r und W² die gleiche Bedeutung wie in Formel VI haben und
      L eine als Anion abspaltbare Gruppe bedeutet,
b) von Verbindungen der Formel 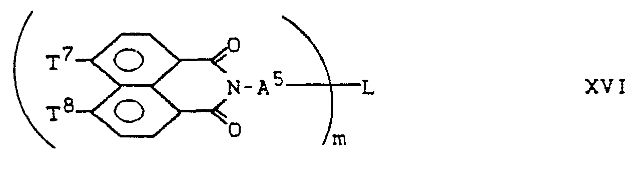 worin
   T⁷, T⁸, A⁵ und m die gleiche Bedeutung wie in Formel VI haben und
   L eine als Anion abspaltbare Gruppe bedeutet,

   im Molverhältnis r : 1 mit Verbindungen der Formel 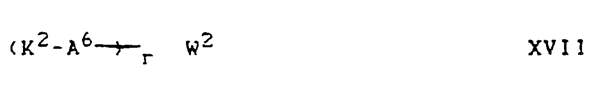 worin
   K²⁻, A⁶, r und W² die oben angegebene Bedeutung haben,
c) von Verbindungen der Formel (XIV) im äquimolaren Verhältnis mit Verbindungen der Formel (XVI),
d) von Verbindungen der Formel 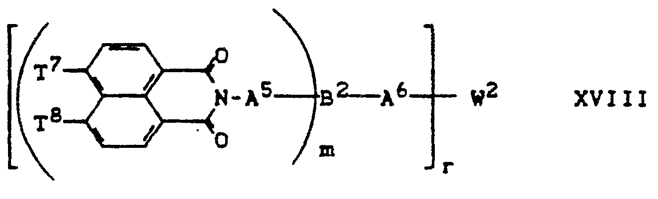 worin 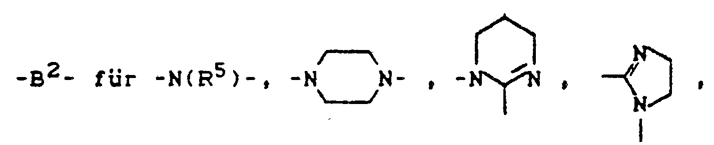 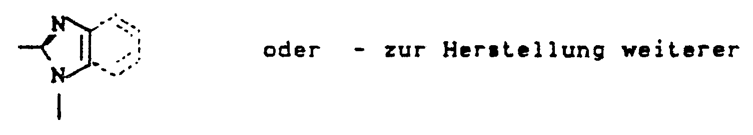 Verbindungen der Formel I - auch für 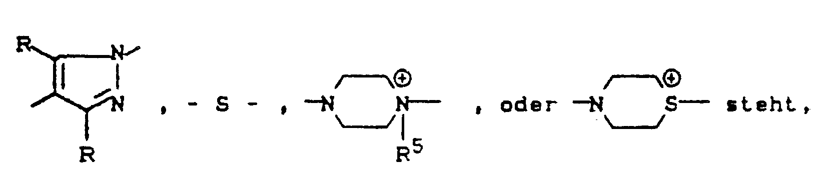 und T⁷, T⁸, A⁵, m, R, R⁵, A⁶, r und W² die gleiche Bedeutung wie in Formel VI haben, im Verhältnis 1:r mit Verbindungen der Formel 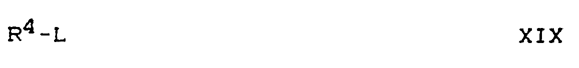 worin
   R⁴ die gleiche Bedeutung wie in Formel VI hat und
   L für eine als Anion abspaltbare Gruppe steht.

Die Reaktion wird zweckmäßig in einem Lösungsmittel wie Dimethylformamid, N-Methylpyrrolidon, Dimethylsulfoxid, Acetonitril, Ethylenglykol, Diethylenglykol, Triethylenglykol, Polyglykolgemischen, vorzugsweise mit einem mittleren Molekulargewicht von ca. 400, Aceton, Methylethylketon, Cyclohexanon, Toluol, Chlorbenzol, o-Dichlorbenzol, Wasser sowie homogenen oder inhomogenen Gemischen von diesen im Temperaturbereich von 20 - -180° C, vorzugsweise 40 - 150 C durchgeführt.

In analoger Weise können auch die übrigen Naphthalimidderivate der Formel I mit n = 1 hergestellt werden.

Geeignete Reste -K² und in der allgemeineren Bedeutung -K¹ oder -K entsprechen den folgenden Formeln:

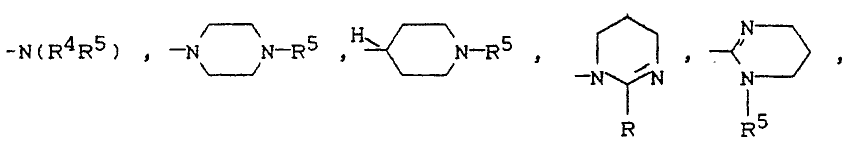

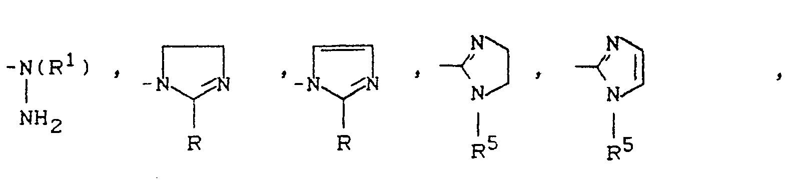

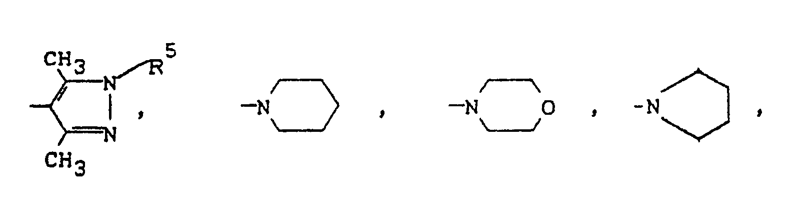

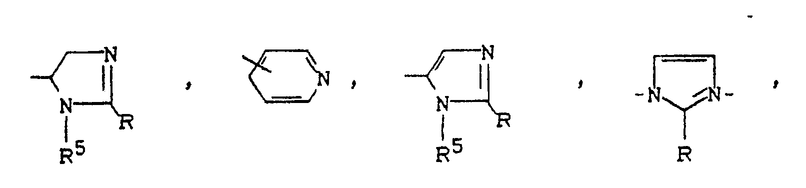

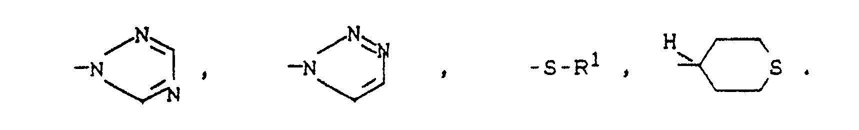

Hierin besitzen R, R⁴ und R⁵ die oben angegebene Bedeutung.

Als Anion abspaltbare Reste L sind die von Alkylierungsmitteln bekannten Abgangsgruppen wie Halogen (Chlor, Brom, Jod) und Arylsulfonsäureester-Reste wie Tosylat oder Benzolsulfonat geeignet.

Die Ausgangsprodukte der Formel (XIV) sind teilweise bekannt, insbesondere als Ausgangsverbindungen für kationische optische Aufheller, wie sie zum Weißtönen von Polyacrylnitrilfasern verwendet werden.

Geeignete Ausgangsverbindungen der Formal XIV sind beispielsweise: Naphthalsäure-N-(3-dimethylaminopropyl)-imid, 4-Chlornaphthalsäure-N-(2-diethylaminoethyl)-imid, 4-Bromnaphthalsäure-N-(α-dimethyla- minobenzyl)-imid, 4,5-Di chlor-naphthalsäure-N-(ß-N'-methyl-piperazinoethyl)-imid,Naphthalsäure-N(methyl- pyridyl-(4))-imid, Naphthalsäure-N-(ß-N'-morpholinoethyl)-imid.

Geeignete Ausgangsverbindungen der Formel XV sind beispielsweise:
1,2-Dichloraceton, 1,4-Bischlormethylbenzol, 1,4-Bisbrommethylbenzol, 2,4-Bischlormethyl-1,5-dimethylbenzol, 1,3,5- Trischlormethylbenzol, 4,4'-Bischlormethylbiphenyl, 1,4-Bischlormethylnaphthalin, 2,6-Bischlormethylnaphthalin, 4,4'-Bischlormethyl-benzophenon, -diphenylsulfon, -diphenylamin, -N-methyl-diphenylamin, -triphenylamin, -diphenylether, -diphenylsulfid, -diphenylmethan, -triphenylmethan, -diphenylpropan-(2), Tris-(4-chlormethyl-phenyl)-methan, 2,5-Bischlormethyl-thiophen, -1,3,4-oxadiazol, -1,3,4-thiadiazol, N,N',N"-Trischlormethyl-s-triazin-2,4,6-trion, N,N',N"-Trismethylol-cyanursäure-p-toluolsulfonsäure-triester, 4,4'-Bischloracetamido-benzoesäureanilid, 4,4'-Bis-(chloracetamidomethyl)-biphenyl (US-PS 4 370 486), 4,4'-Bis-(chloracetamido)-triphenylmethan, -diphenylmethan oder -3,3'-dimethoxy-biphenyl, 4,4',4"-Tris-(chloracetamido)-triphenylmethan, 4,4',4"-Tris-(chloracetamido)-triphenylphosphonat, Bis-(4-chloracetamido- cyclohexyl)-methan, 2,2-Bis-(4-chloracetamidocyclohexyl)-propan-(2), 4,4'-Bis-chloracetyl-diphenylether, 4,4'-Bis-chloracetyldiphenylsulfid, 4,4'-Bis-(chloracetureido)-diphenylmethan, 4,4',4"- Tris(chloracetureido)-triphenylmethan (aus 4,4',4"-Triaminotriphenylmethan und 3 Äquivalenten Chloracetylisocyanat), 3,6-Bischloracetamido-9H-thioxanthen-S-dioxid, 4,4'-Bis-(chloracetoxyphenyl)-propan-(2), 2,4-Bis-(p-chloraceta- midophenylamino)-6-chlor-1,3,5-triazin, 2,4,6-Tris(p-chlor acetamidophenylamino)-1,3,S-triazin, 2,4-Bis-(ß- chloracetamidoethyl-piperazino)-6-morpholino-1,3,5-triazin, N,N'-Bis-chloracetyl-piperazin, N,N'-Bis-(ω-bro- macetophenyl)-harnstoff, N,N'-Bis-(a-chloracetophenyl)-guanidin, N,N'-Bis-chloracetyl-hydrazin.

Geeignete Ausgangsverbindungen der Formel XVI sind beispielsweise:
N-Chloracetamido-naphthalimid, 4-Chlor-N-chloracetamidonaphthalimid, 4,5-Dichlor-N-chloracetamido-naphthalimid, 4-Brom-N-chloracetamido-naphthalimid, N-(2-p-Toluolsulfonyl-oxyethyl)-naphthalimid, N-(2-Bromethyl)-naphthalimid, N-(3-N'-methyl-N'-chloracetamidopropyl)-naphthalimid, N-(p-Chloracetamidophenyl)-naphthalimid, N-(ω-Bromocetophenyl)-naphthalimid.

Die Ausgangsprodukte der Formel XIV und XVI werden vorteilhoft dadurch hergestellt, daß man Anhydride der Formel 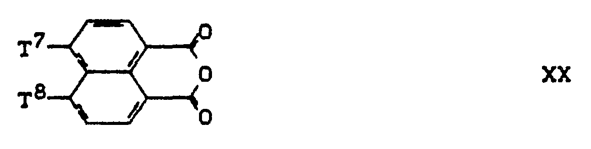 worin T und T⁸ die oben angegebene Bedeutung haben,

im Molverhältnis m:1 unter Abspaltung von m Äquivalenten Wasser mit Verbindungen der Formel 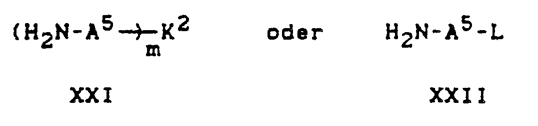 worin A⁵, m, K² und L die oben angegebene Bedeutung haben,
kondensiert.

Geeignete Ausgangsverbindungen der Formel XXI sind beispielsweise:
1-Amino-3-dimethylamino-propan, 1-Amino-3-methylaminopropen, 1-Amino-2-diethylamino-ethan, 1-Amino-3-cyclohexylamino-propan, 1-Amino-2,2-dimethyl-3-dimethylamino-propan, Bis-(3-aminopropyl)-methylamin, Bis-(3-aminopropyl)-amin, Diethylentriamin, Triethylentetramin, 4-Amino-1-diethylamino-n-pentan, Glycincholinester, Cholin-ß-aminoethylether, 2-(2-Amino-ethylamino)-ethanol, 2-[(3-Aminopropyl)-methylamino]-ethanol, N-(3-Aminopropyl)-pyrrolidin, N-(2-Aminoethyl)-morpholin, N-(2-Aminoethyl)-piperidin, N-Methyl-N'-(2-aminoethyl)-piperazin, 1-(2-Aminoethyl)-imidazol, 3- und 4-Amino-N,N-dimethylbenzylamin, 4-Aminomethyl-N,N-dimethylbenzylamin, 4-Aminomethylpyridin, 3-Aminopyridin, 2-Aminomethyl-benzimidazol, 4-Amino-1,3,5-trimethyl-pyrazol, ferner als Ausgangskomponenten für weitere analoge Verbindungen der Formel I auch 1-(3-Aminopropyl)-1,2,3- oder -1,2,4-triazol, Aminoguanidin, 2-Mercaptoethylamin oder 2-Aminobenzthiazol.

Geeignete Ausgangsverbindungen der Formel (XXII) sind beispielsweise 3-Chlorpropylamin, 4-Amino-oichloracetophenon, 3-Aminobenzylchlorid, 3-Chlor-1-amino-propan-2-on.

Die Reaktion wird zweckmäßig in einem der oben angegebenen Lösungsmittel im Temperaturbereich von 60-180 C, vorzugsweise 80-130 C, vorteilhaft in Gegenwart katalytischer Mengen Säure wie Eisessig und vorteilhaft durch azeotropes Abdestillieren des Reaktionswassers durchgeführt.

Die Umsetzung von (XX) mit (XXI) zu (XIV) und von (XIV) mit (XV) oder (XVI) kann in der Regel und vorteilhaft ohne Zwischenisolierung von (XIV) erfolgen.

Zu den Ausgangsverbindungen der Formel (XVII) gelangt man auf einfache Weise entweder dadurch, daß man eine Verbindung der Formel XV im Molverhältnis p:1 mit einer Verbindung K²⁻H, wobei K² die oben angegebene Bedeutung hat, umsetzt und gleichzeitig oder anschließend mit Hilfe von Alkali p Äquivalente H-L abspaltet, oder dadurch, daß man an ein Isocyanat der Formel W²(NCO)p p-fach eine Verbindung der Formel K²⁻Z-N(R)-H oder N-Methylpiperazin, worin K², R und Z die oben angegebene Bedeutung besitzen, addiert.

Die Umsetzung von (XV) mit p Äquivalenten K²⁻H wird zweckmäßig in einem dipolar aprotischen Lösungsmittel wie Dimethylformamid, N-Methyl-pyrrolidon, Dimethylsulfoxid oder auch in Acetonitril, Aceton, Polyethylenglykol, vorzugsweise mit einem mittleren Molekulargewicht von etwa 400, im Temperaturbereich von 15 - 100 C, vorzugsweise 20-60' C durchgeführt.

Für die p-fache Addition von K²⁻Z-N(R)-H oder N-Methylpiperazin an W²(NCO)p kommen vor allem wasserfreie Aromaten wie Toluol, Xylol, Chlorbenzol und o-Dichlorbenzol als Lösungsmittel in Betracht. Man arbeitet vorzugsweise bei Raumtemperatur und kann die Temperatur während der exothermen Reaktion auf 40- 70 C ansteigen lassen.

Geeignete Verbindungen der Formel K²⁻H sind beispielsweise Dimethylamin, Diethylamin, Di-n-butylamin, Methyl-n-butylamin, Dibenzylamin, N-Methyl-benzylamin, N-Methyl-ethanolamin, Pyrrolidin, Piperidin, Morpholin, N-Methyl-piperazin, N-Methyl-imidazol.

Besonders geeignete Isocyanate W(NCO)p sind: Phenylisocyanat, Benzylisocyanat, Phenylen-1,4-diisocyanat, Toluylen-2,4-diisocyanat, Xylylen-1,3-diisocyanat, Naphthylen-1,5-diisocyanat, Bis-(4-isocyanatophenyl)-methan, 2,2-Bis-(4-isocyanatophenyl)-propan, Tris-(4-isocyanatophenyl)-methan, Bis-(4-isocyanatophenyl)-ether.

Verbindungen der Formel K²⁻Z-N(R)-H sind beispielsweise 1-Amino-3-dimethylamino-propan, 1-Amino-2-diethylaminoethan, 4-Amino-N,N-dimethyl-benzylamin, N,N,2,2-Tetramethylpropan-1,3-diamin (=Dimethylamino-neopentamin), N-(2-Aminoethyl)-morpholin, N-(3-Amino-propyl)-piperidin.

Beispiele für Verbindungen der Formel R⁴⁻L sind: Dimethylsulfat, Diethylsulfat, Methylchlorid, Methylbromid, Methyljodid, p-Toluolsulfonsäuremethylester, Ethyljodid, Propylbromid, n-Butylbromid, Ethylenoxid (+Eisessig), Propylenoxid (+Eisessig), Aziridin (+Eisessig), Acrylnitril (+Eisessig), Chloressigsäure, Bromessigsäureethylester, Acrylsäureethylester, Acrylsäuredimethylpropylamid, Chloracetamid, Chloracetonitril, 2-Methoxyethylbromid, 3-Chlorpropylbromid, Allylbromid, Cyclohexylbromid, Benzylchlorid, p-Methylbenzylchlorid, p-Chlorbenzylchlorid, 3,4-Dichlorbenzylchlorid, o-, m-und p-Cyanbenzylchlorid, p-Methoxybenzylchlorid, p-Nitrobenzylchlorid, 1-Phenylethylbromid, 2-Phenylethylchlorid, 3-Phenylpropylchlorid, ω-Chloracetophenon, w-Brom-p-chloracetophenon, ω-Brom-propiophenon, 3-Benzoyl-propylbromid, N-Phenyl-chloracetamid, N-p-Tolyl-chloracetemid, N-(4-Chlorphenyl)-chloracetamid, 2-Chlormethyl-benzimidazol, Bromaceton sowie für R⁴ = H auch Säuren wie HCI, Eisessig oder Milchsäure,

Verbindungen der Formel (I), die endständiges -^{⊕}_{E} 1 enthalten können dadurch hergestellt werden, daß man die entsprechende nichtkationische Basenvorstufe von -^{⊕}_{E} 1, die der Formel -E¹ entspricht, im äquimolaren Verhältnis mit Verbindungen der Formel R^{I-}L, wobei R¹ und L die oben angegebene Bedeutung haben, umsetzt. Die Reaktionsbedingungen sind die gleichen wie für (XVIII) + (XIX). Geeignete Reste -E¹ sind die gleichen wie für -K² genannt werden.

Verbindungen des Naphthalintetracarbonsäurediimid-Typs (VII). insbesondere (X), sind zum Teil bekannt (DE-B 1 225 191, FR-A 1 512 338, SU-A-253 047 und 259 875; Pharm. Chem. J. 5 (1971) 515-519, Khim. Farm. Zh. 8 (1974) 11-13, 12 (1978) 50 und 16 (1982) 801-806). Die übrigen sind in analoger Weise zugänglich. Unsymmetrische Naphthalintetracarbonsäurediimide können auch analog DE-A-3 309 060 hergestellt werden.

Typische anionische optische Aufheller sind solche, die der Stilben-, Distyrylbenzol-, Distyrylbiphenyl-und 1,3-Diphenyl-pyrazolin-Reihe angehören, insbesondere 4,4'-Bistriazinylaminostilben-2,2'-disulfonsäuren, 4,4'-Bistriazolylstilben-2,2'-disulfonsäuren, Naphthotriazolylstilben-mono-oder -disulfonsäuren und 4,4'-Bis-(sulfostyryl)-biphenyl-Typen. Sie sind u.a. von H. Gold in K. Venkataraman, The Chemistry of Synthetic Dyes, Vol. v, p. 535-678, und in Ullmanns Encyclopädie der technischen Chemie (4. Auflage), Band 17, Seite 459 bis 473 beschrieben. Durch die Löschung der blauen Fluoreszenz der anionischen Weißtöner heben die kationichen Naphthalin-peri-dicarbonsäureimid-Verbindungen die Weißtönung auf den behandelten Substraten auf. Das Verfahren kann so durchgeführt werden, daß weißgetönte Substrate wie natürliche, halbsynthetische oder synthetische Substrate, beispielsweise natürliche oder regenerierte Cellulose, Papiermassen oder -blätter, Papierbeschichtungen, Seide, Wolle, Jute, Hanf sowie auch synthetische Polyamide, oder auch Rückstände von Weißtönern mit den Naphthalin-peri-dicarbonsäureimid-Verbindungen behandelt werden.

Das erfindungsgemäße Verfahren kann auch zur Erhaltung heller, klarer Farbtöne auf Cellulosematerialien verwendet werden, wenn die Naphthalin-peri-dicarbonsäureimid-Verbindungen dem Färbebad zugesetzt werden, oder die Cellulosematerialien mit den Verbindungen nach- oder vorbehandelt werden. Auf diese Weise wird der nachteilige Einfluß von Waschmittelweißtönern auf den Farbton vermieden bzw. aufgehoben.

Das erfindungsgemäße Verfahren eignet sich besonders gut zur Herstellung von nicht aufgehelltem Papier unter Verwendung von weißgetöntem Papier als Rohstoff, zum raschen, bequemen Entfernen unerwünschter Restanteile von optisch aufgehellten Papiermassen in Papiermaschinen und zum Unwirksammachen unerwünschter Restanteile von wäßrigen oder organischen, mit Wasser mischbaren Lösungen oder Färbeflotten anionischer Weißtöner in Färbeapparaturen, Färbemaschinen, Vorratsgefäßen und Zuleitungen. Es wird verhindert, daß diese Restanteile bei anschließenden, an sich weißtönerfreien Applikationsverfahren noch unerwünschte Weißtönungen oder Fluoreszenzeffekte bewirken.

Bei der Beschichtung von Papier kann man das kationische Naphthalin-peri-dicarbonsäureimid-Derivat in der Beschichtungsmasse (wie Stärke) verteilen und so durch die Beschichtungsoperation auf die Papieroberfläche aufbringen.

Bei der Herstellung von nichtweißgetöntem Papier aus weißtönerhaltigem Altpapier ziehen die kationischen Naphthalin-peri-dicarbonsäureimid-Derivate nach der Zugabe zur wäßrigen Cellulosefaser-Suspension aufgrund ihrer guten Substantivität rasch auf die Faser auf und bleiben somit bei der Papierblatt-Bildung nicht im Wasser zurück.

In weißgetöntem, fertigen Papier kann man die Weißtönung dadurch aufheben, daß man das Papier mit der wäßrigen Lösung des kationischen Naphthalin-peri-dicarbonsäureimid-Derivates imprägniert. Dieser Effekt kann auch gezielt zur Erzeugung schrift- und bildmäßiger Aufzeichnungen oder Wasserzeichen verwendet werden.

Die Behandlung von Geweben, z.B. Baumwolle, kann nach bekannten Verfahren, z.B. dem Foulard-oder Ausziehverfahren in wäßrigem Medium erfolgen.

Die Wasserlöslichkeit der Naphthalin-peri-dicarbonsäureimid-Verbindungen beträgt in der Regel mindestens 1 g/I bei 20° C. Vorteilhaft bereitet man aus den kristallinen Verbindungen konzentrierte, etwa 10 bis 30 gew.-%ige organische oder organisch-wäßrige stabile Lösungen, die mit Wasser von Raumtemperatur in jedem Verhältnis mischbar sind.

Als Lösungsmittel eignen sich vorteilhaft die gleichen, wie sie für die Bereitung stabiler Flüssigeinstellungen von Kationisch substantiven Farbstoffen bekanntermaßen verwendet werden, beispieleweise Ethylenglykol, Diethylenglykol, Triethylenglykol, 2-Methoxy-propanol, Ethylencarbonat, 1,2-Propylencarbonat, Butyrolacton, Caprolacton, 2-Cyanoethanol, Milchsäure und N-Methylpyrrolidon. Bevorzugt setzt man solchen Lösungen zusätzlich etwa 5 bis 50, vorzugsweise 10 bis 30 Gew.-% einer wasserlöslichen Carbonamid-oder Carbonimidgruppen enthaltenden Verbindung wie Harnstoff, Caprolactam, Pyrrolidon oder Dicyandiamid zu. Weitere übliche Zusätze wie beispielsweise o-Hydroxybiphenyl als Bactericid in 0,3 bis 2 Gew.-% sind ebenfalls vorteilhaft.

Für die Herstellung der Flüssigeinstellung kann man häufig auf eine Zwischenisolierung des Fluoreszenzlöscher-Wirkstoffs in kristalliner Form verzichten, d.h., daß man die Synthese in einem für die Flüssigeinstellung vorgesehenen Lösungsmittel so durchführt, daß man direkt die stabile Lösung des Fluoreszenzlöschers erhält.

Man setzt etwa 1 bis 5, vorzugsweise 1 bis 3 Gew.-Teile pro Gew.-Teil des optischen Aufhellers ein, Die Einsatzmenge bezogen auf trockenes Substrat liegt vorzugsweise zwischen 0,05 und 0,3 Gew.-%. Die Applikation erfolgt beispielsweise bei pH-Werten von 3 bis 9,5, vorzugsweise 4 bis 8,5, bei Temperaturen von 10 bis 100° C, vorzugsweise 18 bis 60⁰ C, und insbesondere bei 20 bis 40 C.

Kationische Fluoreszenzlöscher sind bereits aus der DE-A-1 912 647 und der DE-A-2 448 293 bekannt. Gegenüber diesen Verbindungen zeigen die Verbindungen des erfindungsgemäßen Verfahrens eine gesteigerte und vollständigere fluoreszenslöschende Wirkung.

Die Überlegenheit zeigt sich besonders im neutralen bis schwach alkalischen Anwendungsbereich von pH 7 bis 9,5. Dadurch, daß man mit geringeren Einsatzmengen bereits praktisch eine vollständige Aufhebung des durch anionische Weißtöner erzeugten Weißeffektes erreicht, tritt bei einer gewünschten nochmaligen Weißtönung des Cellulose-Substrates eine wesentlich geringere Beeinträchtigung des neu applizierten anionischen Weißtöners ein, so daß man zur Erzeugung eines bestimmten Weißeffektes mit geringeren Weißtönermengen auskommt.

Die erfindungsgemäß zu verwendenden Fluoreszenzlöscher zeichnen sich außerdem durch eine gute Beständigkeit gegen Hydrolyse- und Lichteinflüsse und eine hohe Substantivität auf Cellulose aus, Sie sind praktisch farblos und erzeugen auf dem aufhellerhaltigen Substrat keine unerwünschten Verfärbungen.

Bemerkenswert ist, daß die fluoreszenzgelöschten optischen Aufheller im Substrat durch Fluoreszenzlöscher des erfindungsgemäßen Verfahrens fixiert werden und damit in noch geringerem Maße als ohne die Behandlung mit dem Fluoreszenzlöscher aus dem Substrat mit Wasser extrahiert werden.

Farblose kationische Naphthalimid-Verbindungen haben bisher nur als optische Aufheller, insbesondere zum Weißtönen von Polyacrylnitrilfasern, Verwendung gefunden. Daher ist es überraschend, daß man mit den konstitutionell sehr ähnlichen kationischen Naphthalin-peri-dicarbonsäureimid-Verbindungen in technisch hervorragend brauchbarer Weise geradezu eine gegenteilige Wirkung, nämlich die Aufhebung von Aufhellerwirkungen durch Fluoreszenzlöschung, erzielen kann.

Kationische Naphthalimidverbindungen und ihre Verwendung zum Weißtönen von Polyacrylnitrilfasern sind z.B. beschrieben von R. Anliker et al., Das Aufhellen von Polyacrylnitrilfasern, in Textilveredlung 11 - (1976), 369 und 370, und A. Dorlars, C-W. Schellhammer, J. Schroeder, Heterocyclen als Bausteine neuer optischer Aufheller, Angew. Chem. 87 (1975) 693.

### Herstellungsbeispiele

### Beispiel 1

282 g Naphthalsäure-N-(3-dimethylaminopropyl)-imid (1 Mol) und 175,5 g 4,4'-Bis-(chloracetamidophenyl)-methan (0,5 Mol) werden in 1330 g Polyglykol (durchschn. Molgew. 400) 3 h auf 100°C erhitzt, wobei sich zunächst eine klare viskose Lösung bildet, aus der sich anschließend ein farbloser kristalliner Niederschlag abscheidet. Nach dem Abkühlen auf Raumtemperatur verdünnt man die Reaktionsmischung unter Rühren mit 3,3 1 Isopropanol, saugt den kristallinen Niederschlag ab, wäscht ihn mit Isopropanol bis zum farblosen Ablauf und trocknet ihn bei 60. C im Vakuum. Man erhält 444 g (97 % der Theorie) Verbindung der Formel

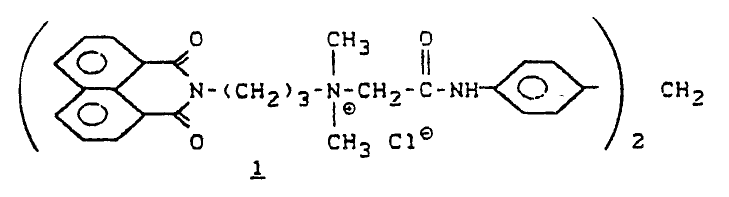

Die Substanz ist dünnschichtchromatographisch praktisch rein. 1 zeigt auf der fluoreszierenden Kieselgelplatte Fluoreszenzlöschung. Rf-Wert: 0,3 (Laufmittel: 45 Vol-% Essigsäurebutylester, 33 Vol-% Eisessig, 9 Vol-% Ameisen-säure und 13 Vol-% Wasser).

### Herstellung einer stabilen Flüssigeinstellung

20 g Verbindung der Formel 1 , 15 g∈-Caprolactam und 65 g Ethylenglykol werden unter Rühren auf 80 C erwärmt, wobei eine klare Lösung entsteht. Diese wird abgekühlt und ist gebrauchsfertig. Der Trübungspunkt liegt unter -5°C. Die Lösung ist bei +5 bis +40 C mindestens 6 Monate lagerstabil. Sie ist mit Wasser in jedem Verhältnis mischbar.

### Herstellung der Ausgangsverbindungen

A. Naphthalsäure-N-(3-dimethylaminopropyl)-imid
   258 g Naphthalsäureanhydrid werden in 1,5 1 Toluol suspendiert, zunächst mit 2 g Eisessig, dann unter Rühren und Sieden am Wasserabscheider tropfenweise mit 133 9 1-Amino-3-dimethylamino-propan versetzt und noch 1 h am Wasserabscheider zum Sieden erhitzt, wobei sich insgesamt ca. 24 ml Wasser abscheiden. Man kühlt auf 90° C ab, fügt 300 ml Wasser hinzu, verrührt die 2-phasige Mischung 30 Min. unter Rückflußsieden, trennt die wäßrige Unterphase aus der heißen Mischung ab, gibt 6 g Tonsil und 12 g Aktivkohle hinzu, entfernt das restliche, noch anhaftende Wasser durch azeotropes Abdestillieren am Wasserabscheider, filtriert heiß von den Klärmitteln und dampft die klare Toluol- Lösung am Rotationsverdampfer zur Trockene, Man erhält 352 9 (96 % der Theorie) Naphthalsäure-N-(3- dimethylaminopropyl)-imid als fast farbloses Kristallpulver. Schmelzpunkt: 117° C, m/e = 282 (M^{⊕}).
B. 4,4'-Bis-(chloracetamidophenyl)-methan
   119 g 4,4'-Diaminodiphenylmethan, (0,6 Mol) werden in 1,5 1 Toluol bei 60° C gelöst, abgekühlt auf Raumtemperatur, tropfenweise unter Rühren mit 147 g (1,3 Mol) Chloracetylchlorid versetzt, wobei man die Temperatur der exothermen Reaktion auf 40 C ansteigen läßt und den entweichenden Chlorwasserstoff in eine alkalische Absorptionsanlage leitet. Während der Reaktion scheidet sich 4,4'-Bis-(chloracetamidophenyl)-methan in Form farbloser Kristalle aus der Lösung aus, Man erhitzt die Suspension 2 h auf 105°C und kühlt auf Raumtemperatur. Der kristalline Niederschlag wird abgesaugt, mit Toluol gewaschen und bei 50 °C im Vakuum getrocknet. Man erhält 208 g (98 % der Theorie) 4,4'-Bis-(chloracetamidophenyl)-methan. Schmelzpunkt: 238 C, m/e = 350 (M^{⊕}, 2CI).

### Eintopf-Methode

Die Verbindung der Formel 1 kann vorteilhaft auch ohne Zwischenisolierung der Ausgangskomponenten gleich in Form einer gebrauchsfertigen stabilen Lösung gewonnen werden, wenn man die toluolischen Suspensionen der unter A. und B. hergestellten, aber nicht isolierten Ausgangsverbindungen vereinigt, anstelle des Polyglykols 1,8 kg Ethylenglykol und 410 g ∈-Caprolactam zusetzt und das Toluol während der 3-stündigen Reaktion bei 100° C unter Zuhilfenahme eines leichten Wasserstrahlvakuums abdestilliert. Man erhält 2785 g ca. 20 %ig. Lösung von 1 . In analoger Weise werden die folgenden Verbindungen hergestellt:

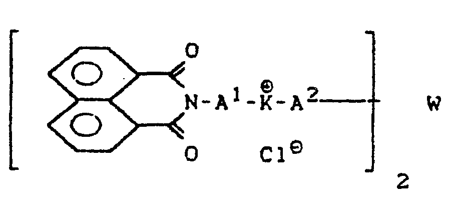

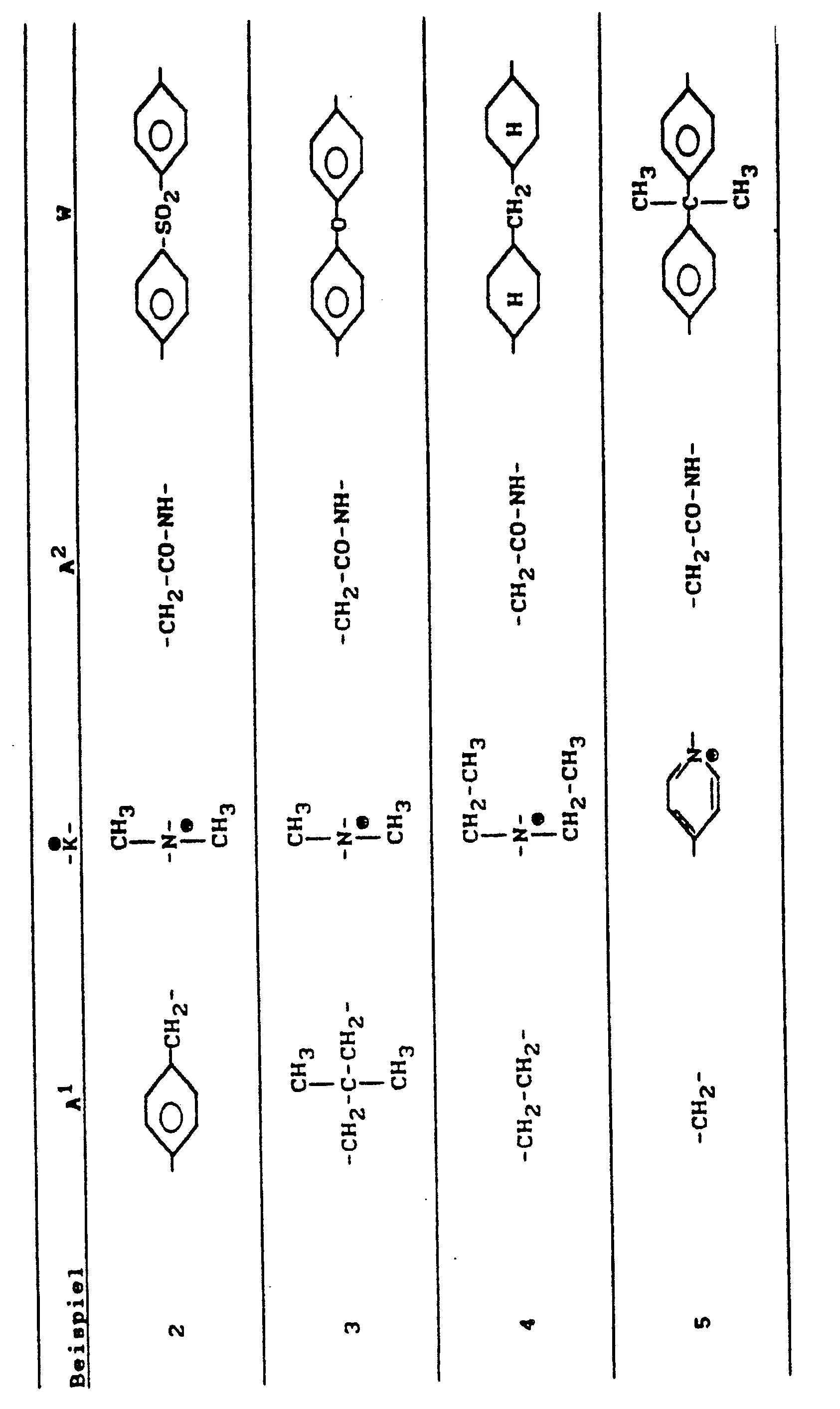

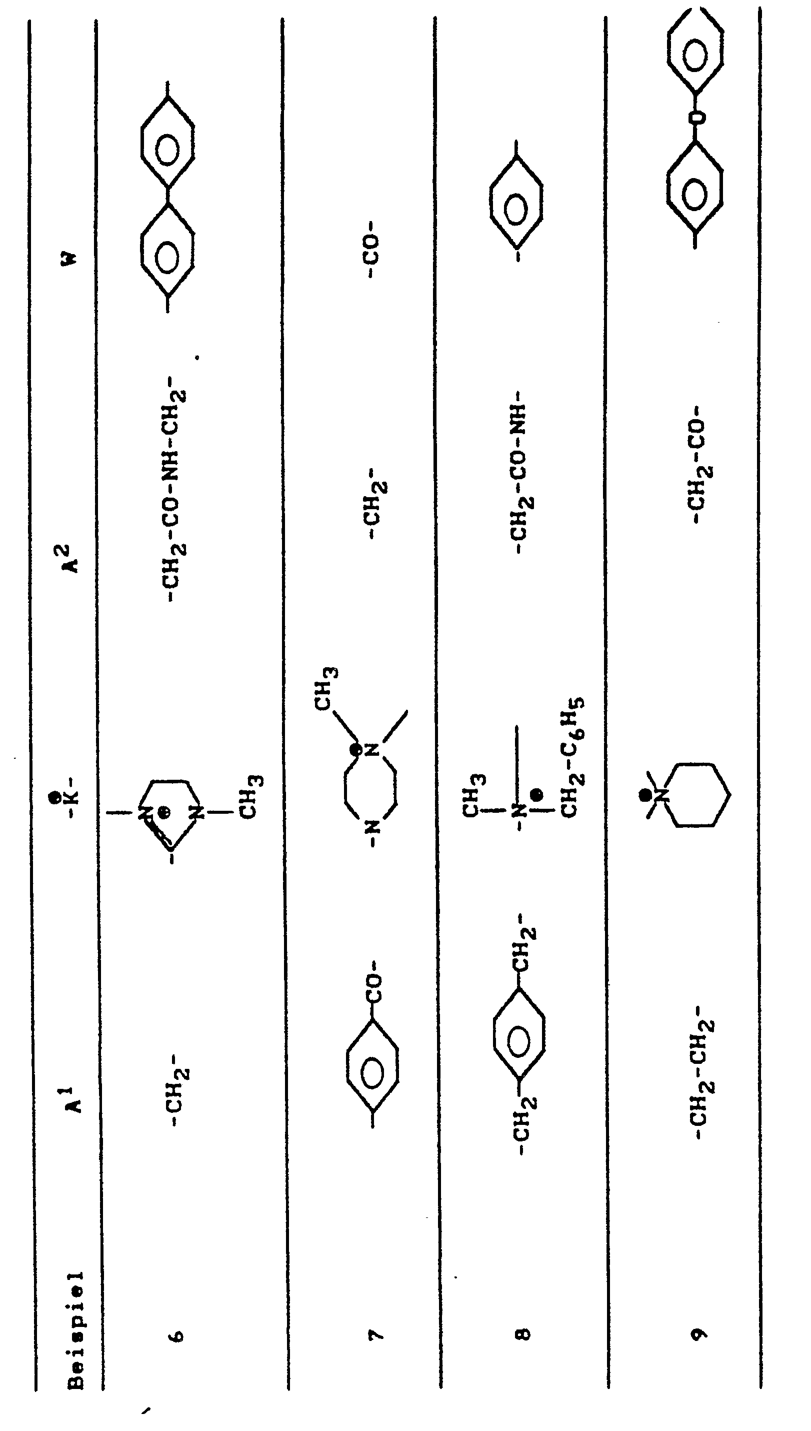

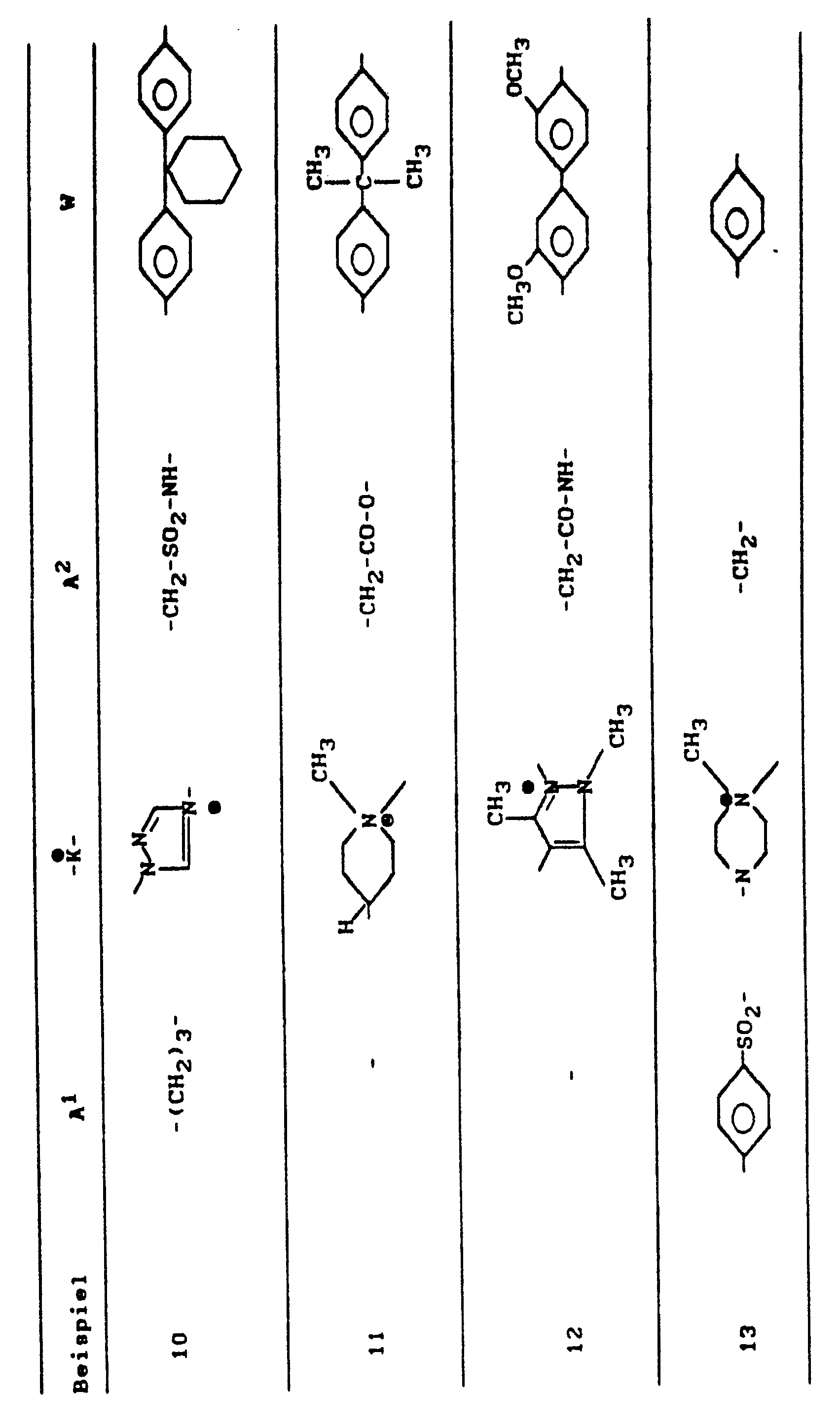

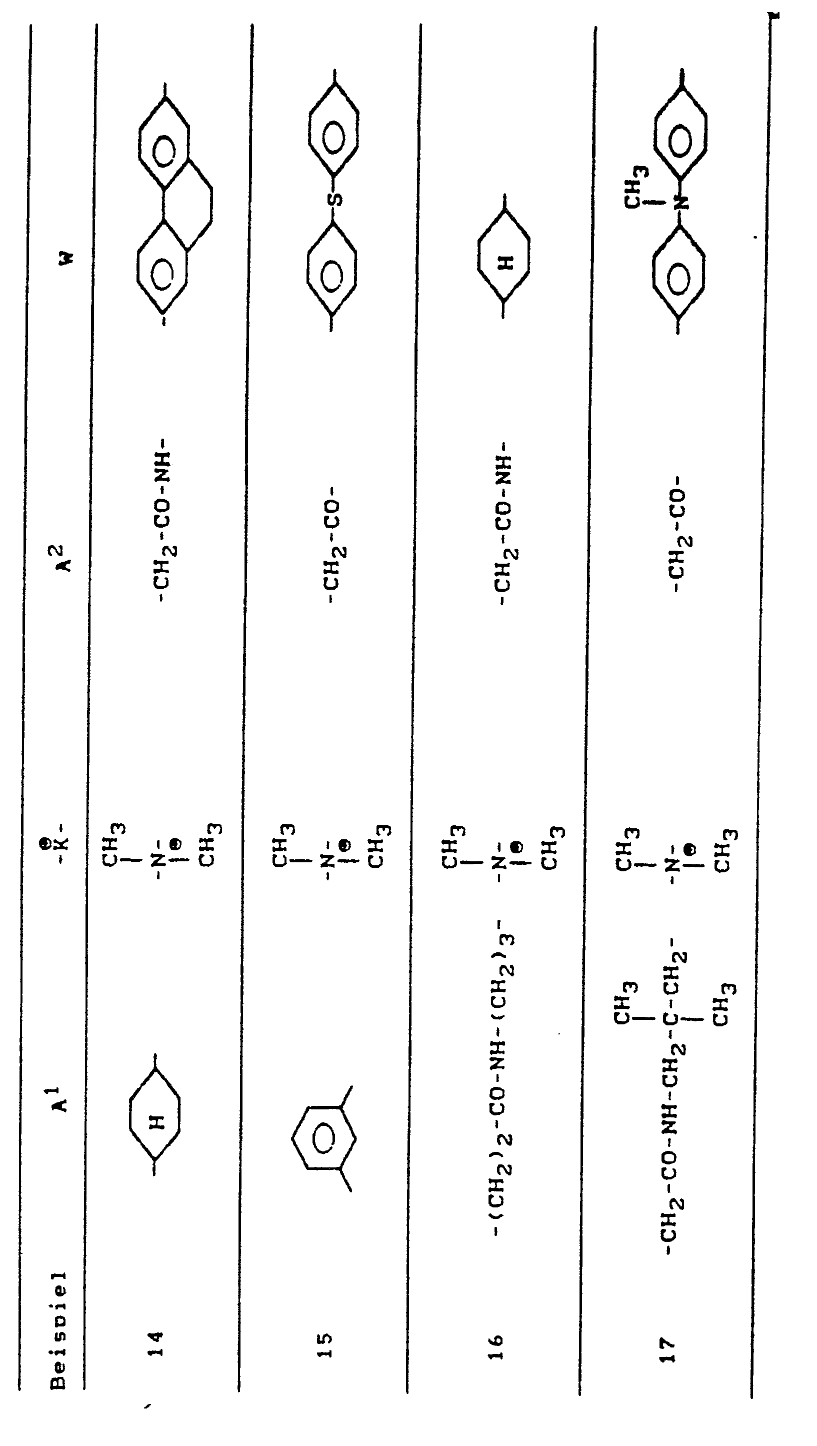

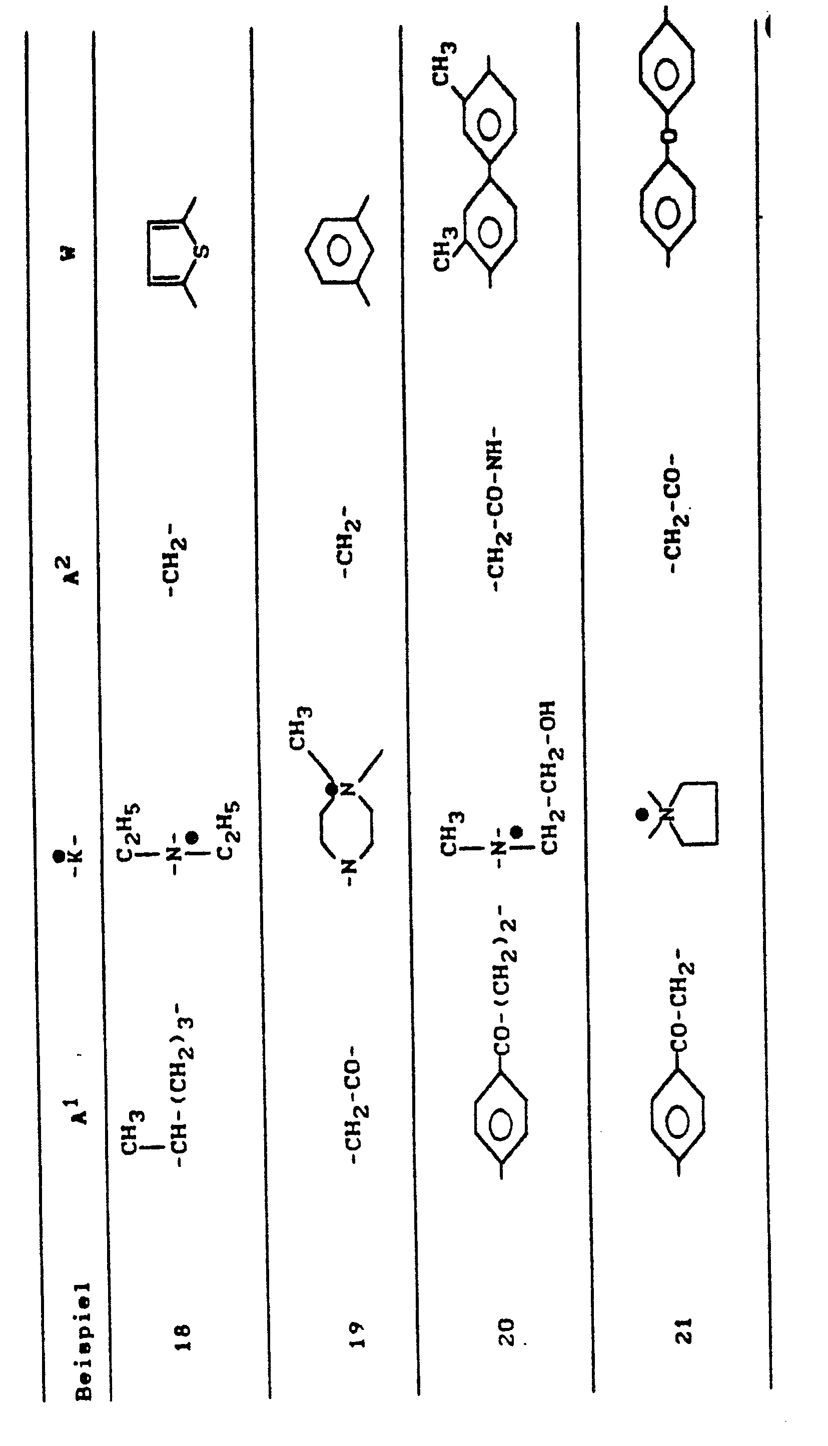

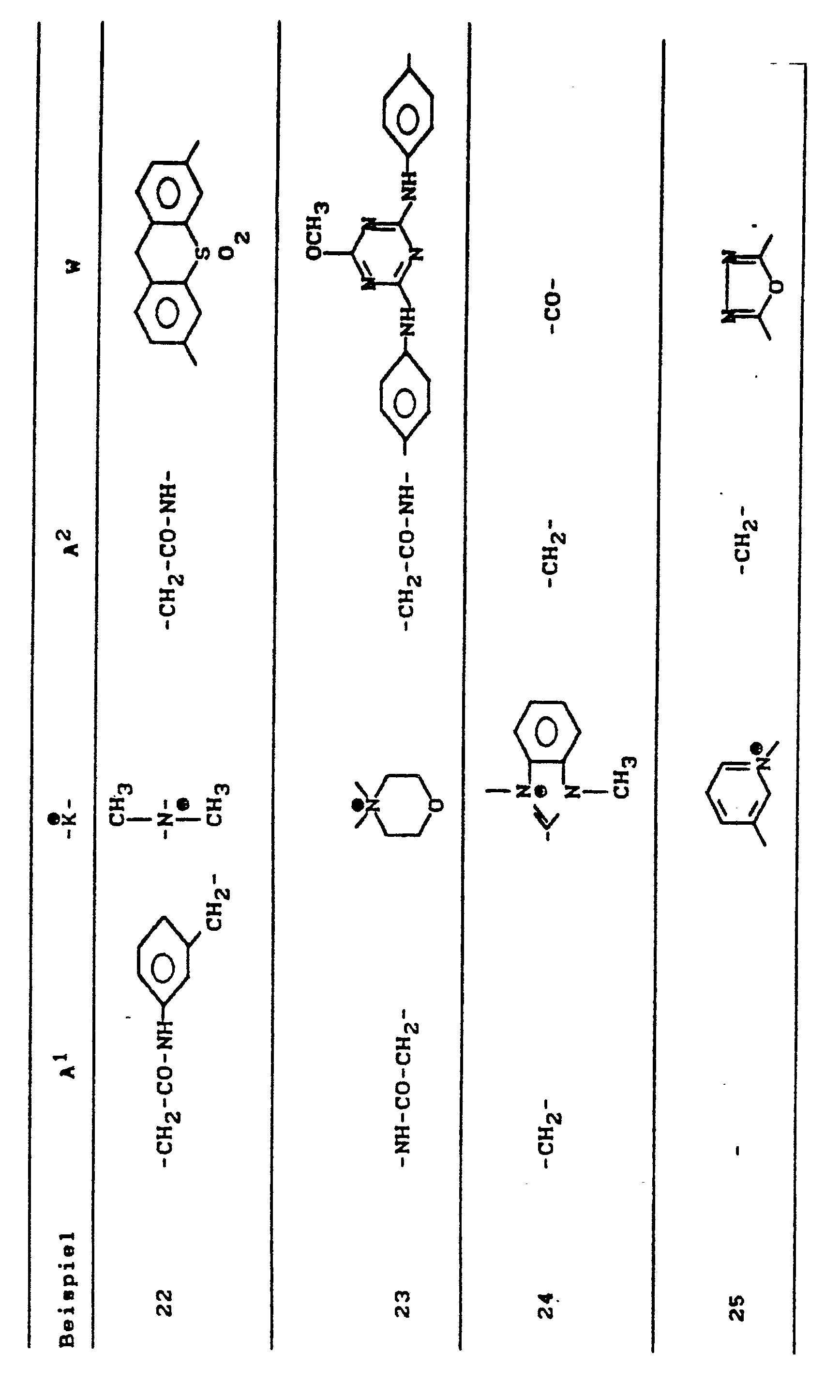

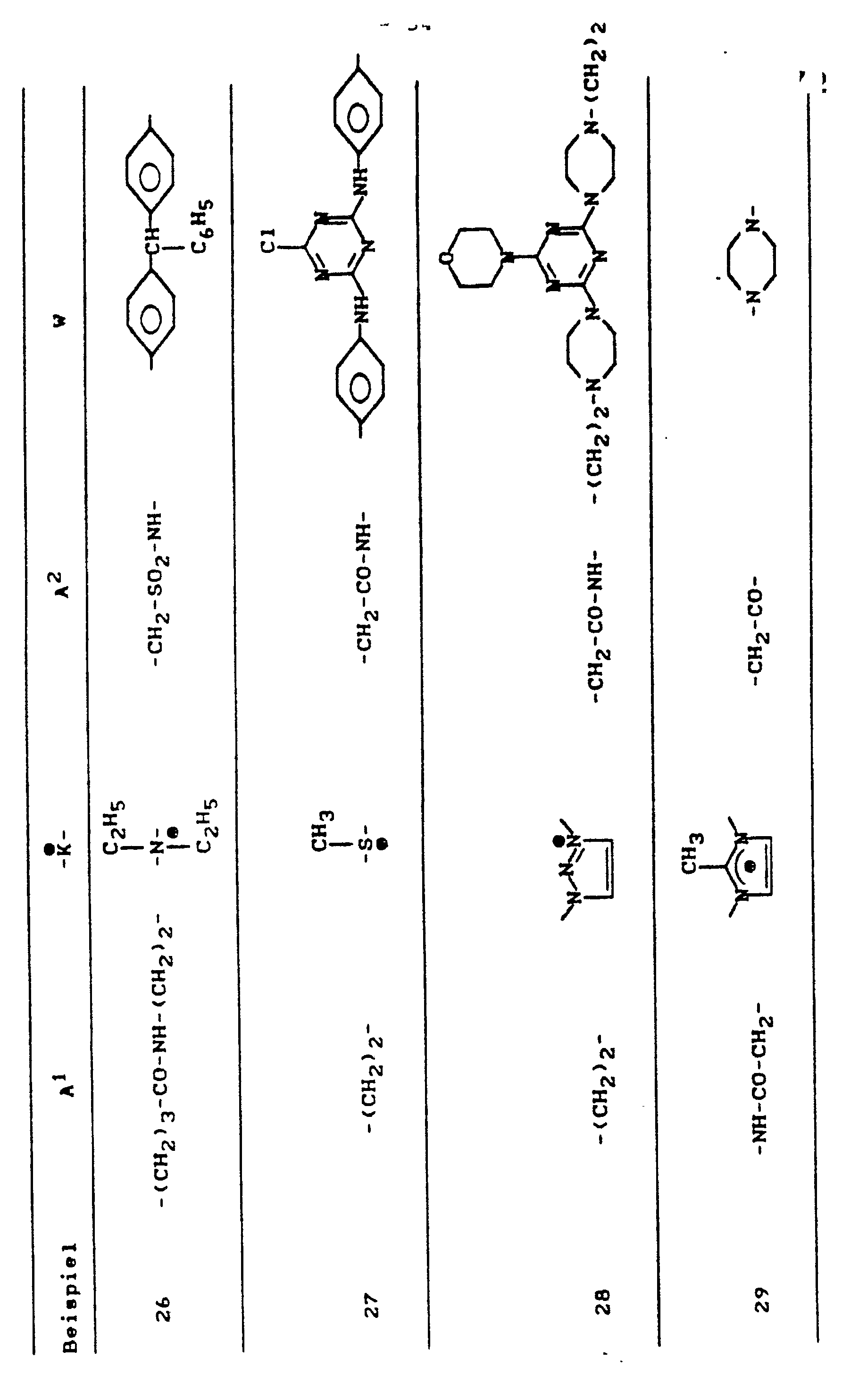

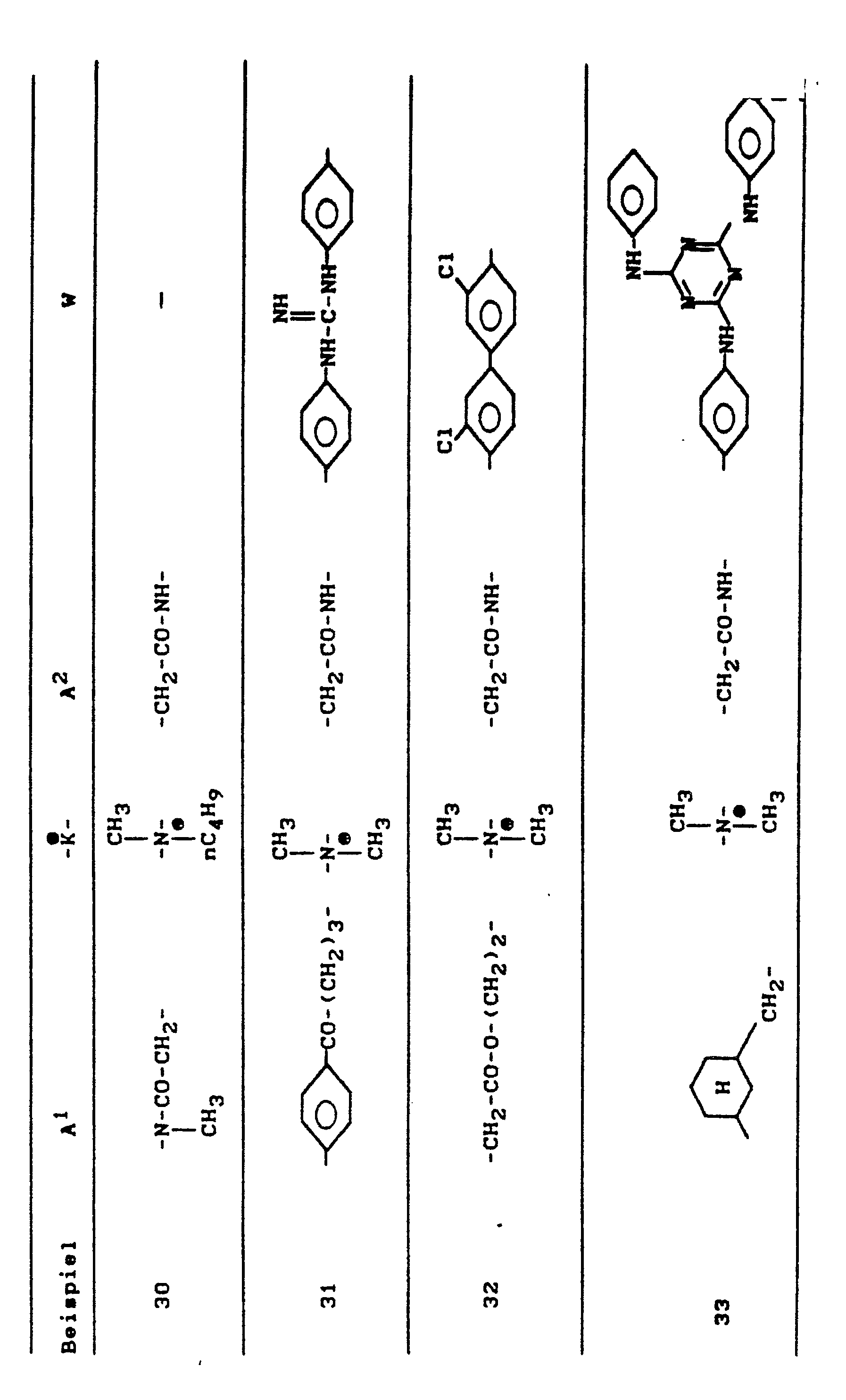

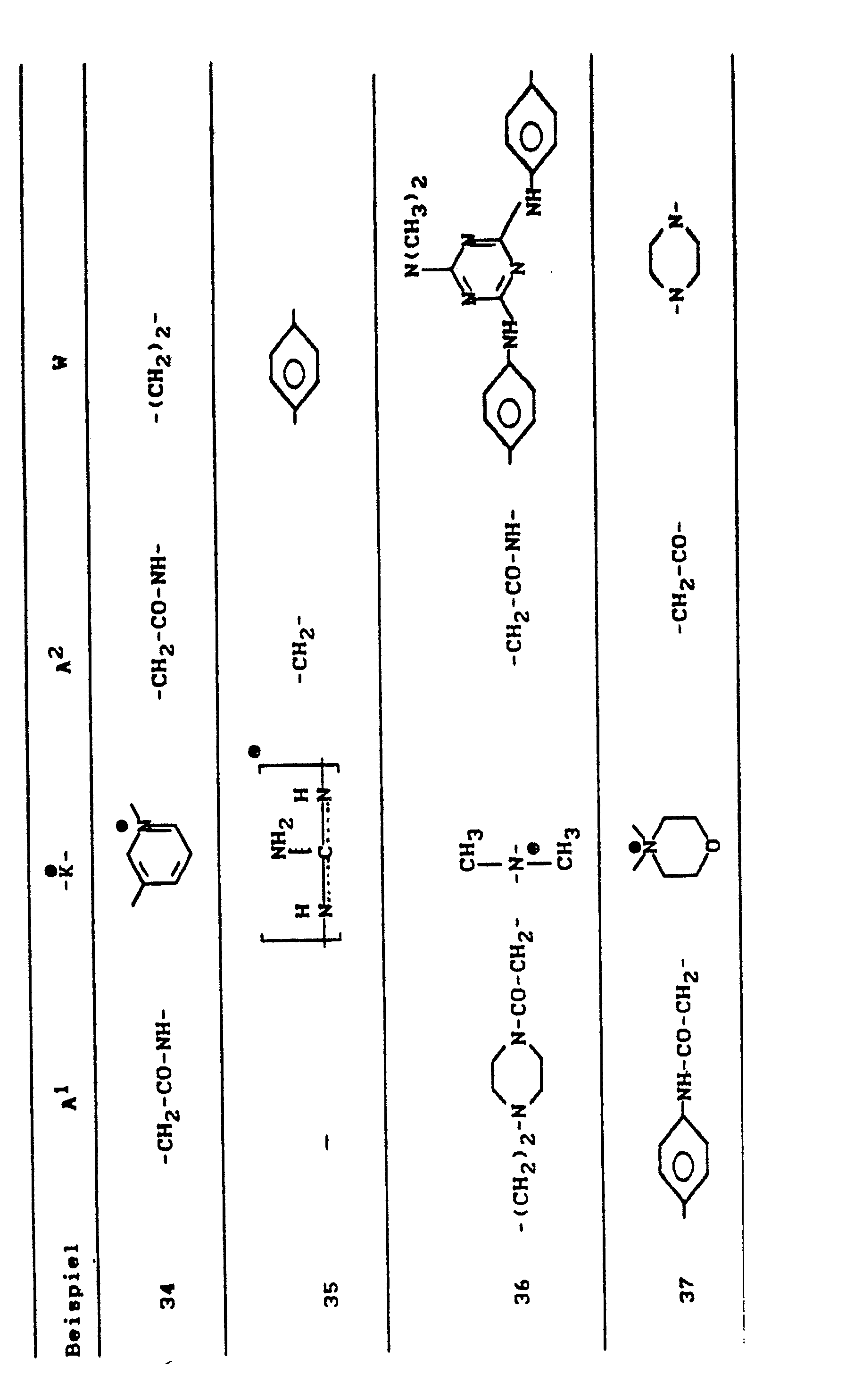

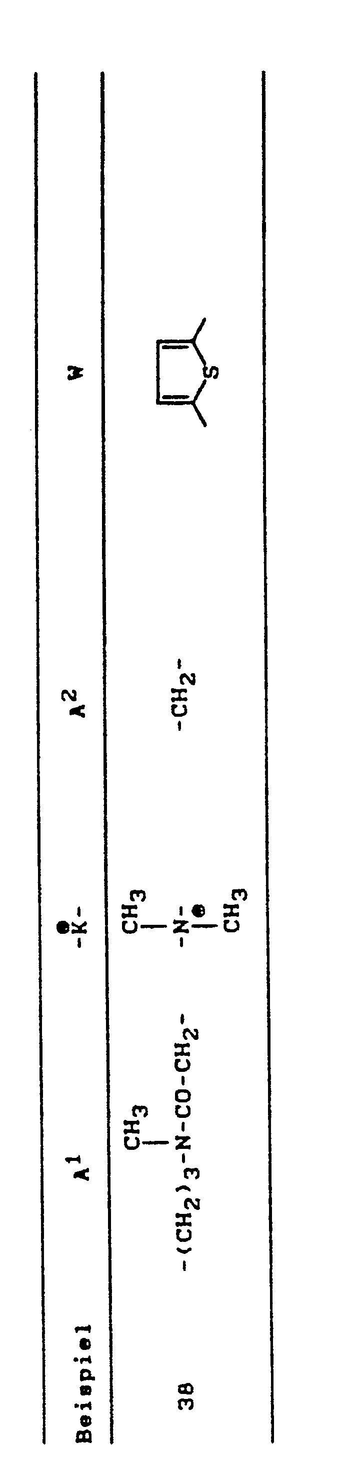

Ebenfalls in analoger Weise werden die folgenden Verbindungen hergestellt:

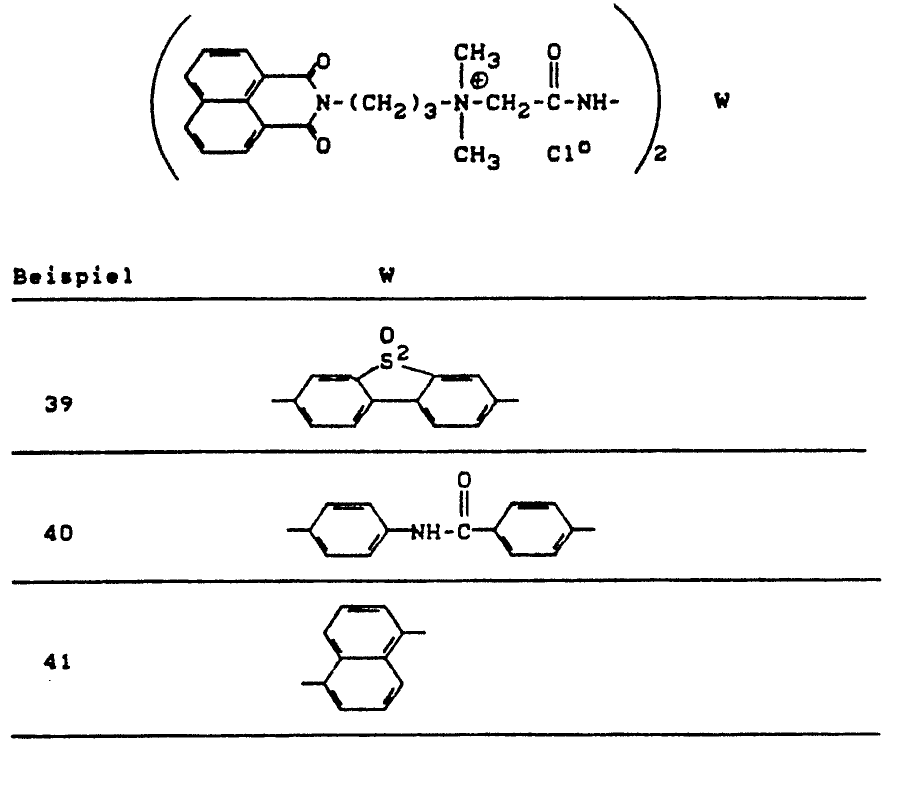

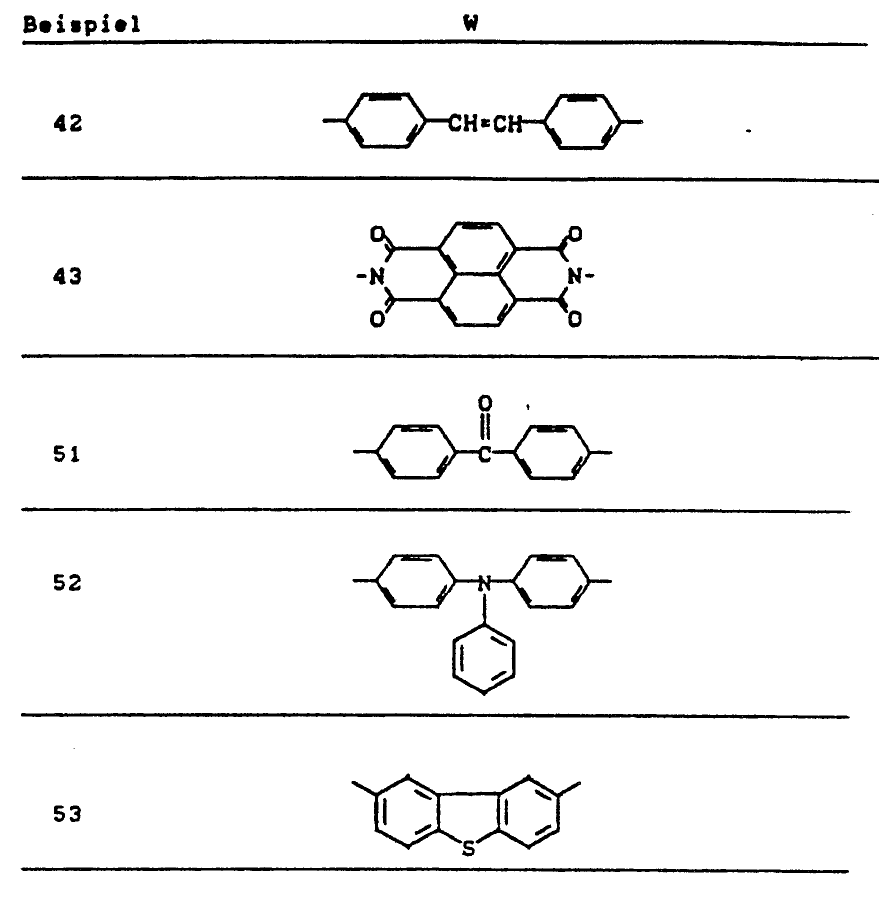

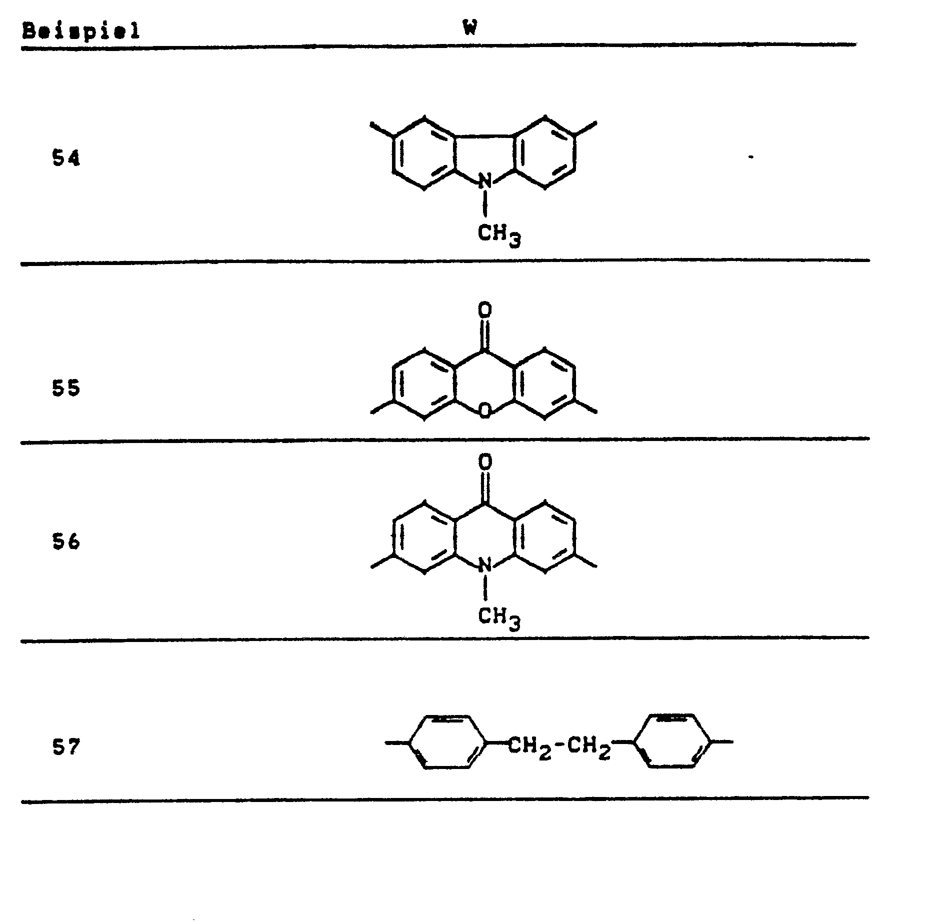

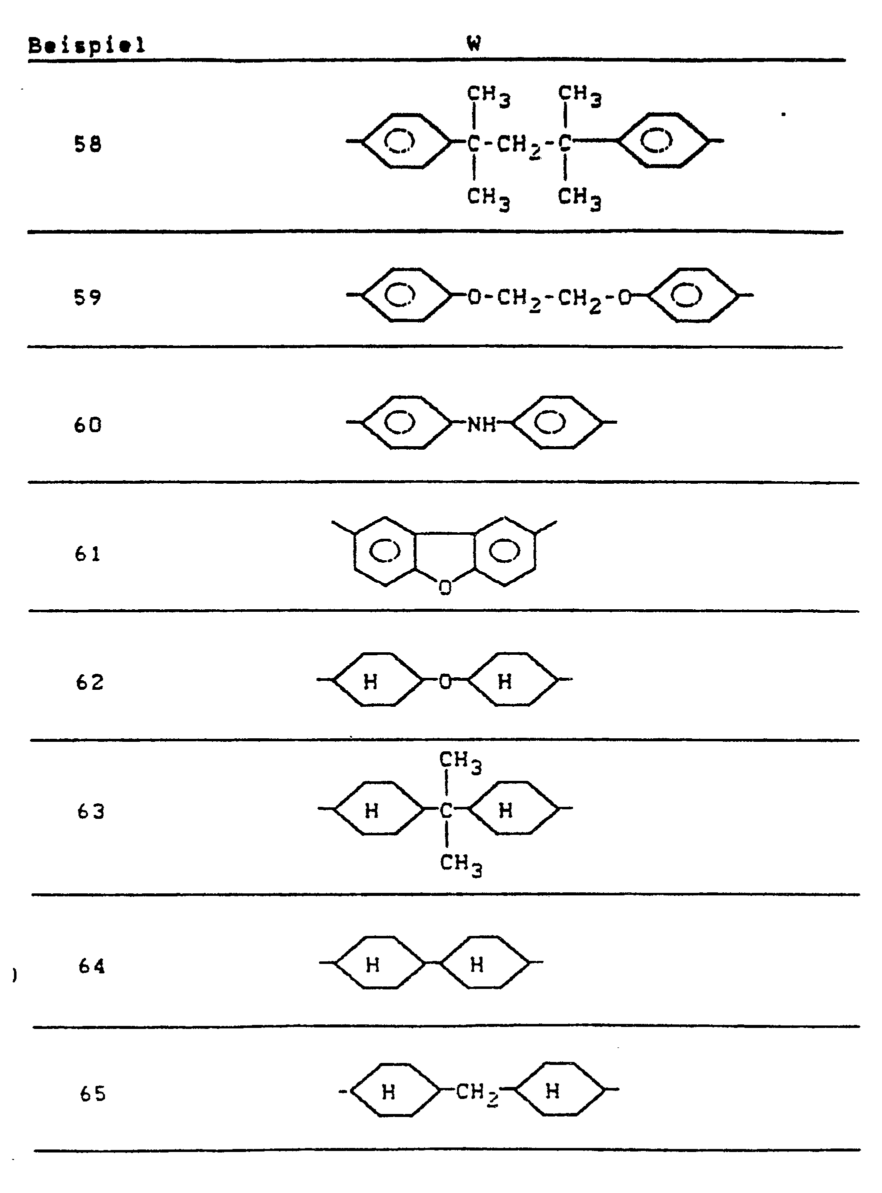

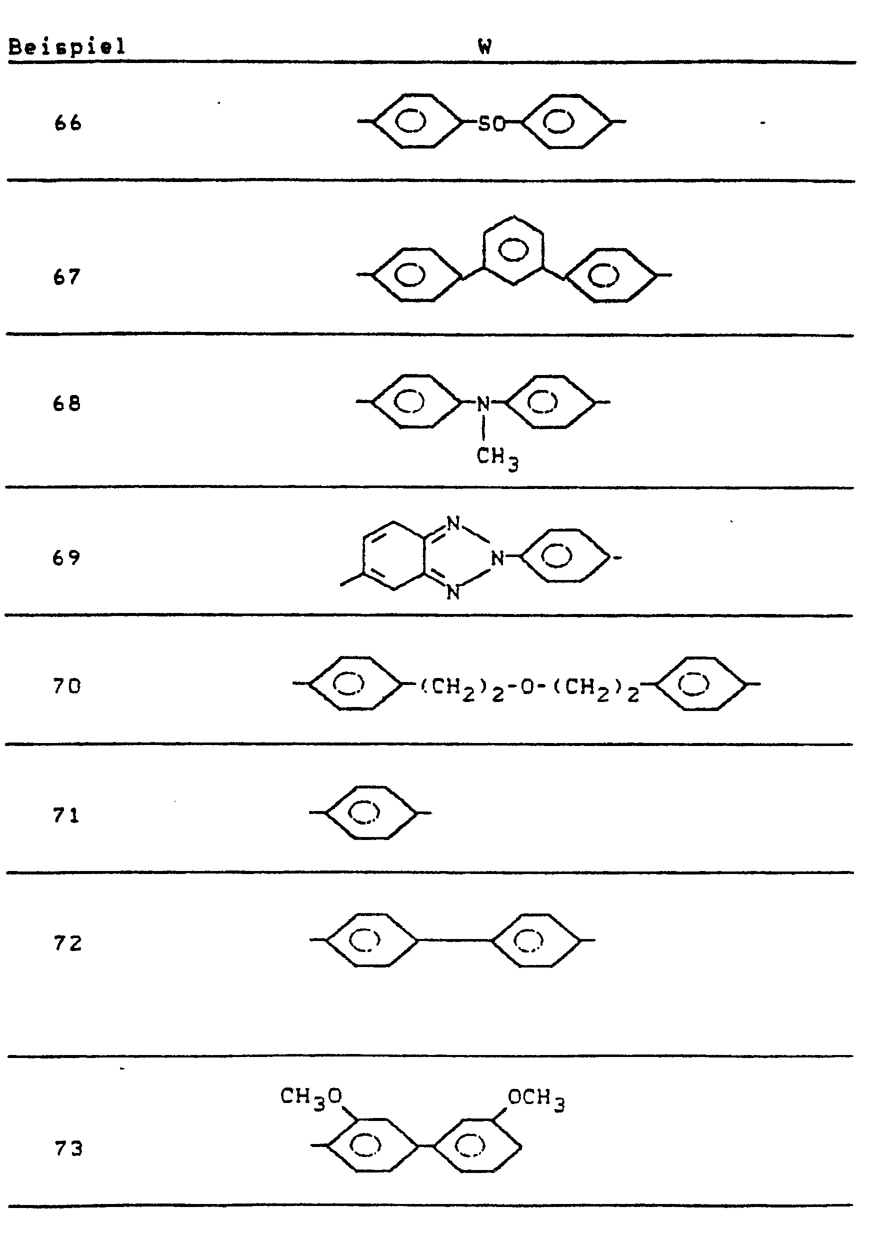

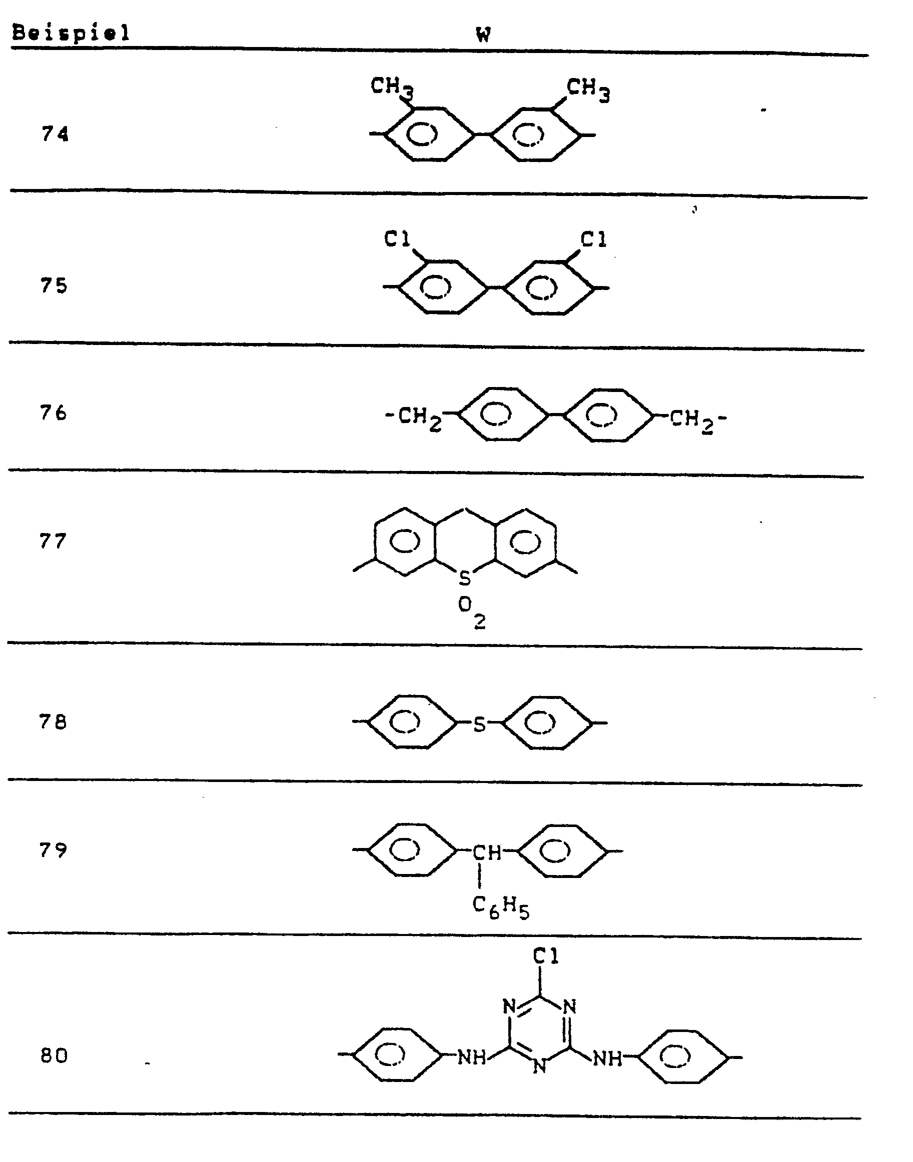

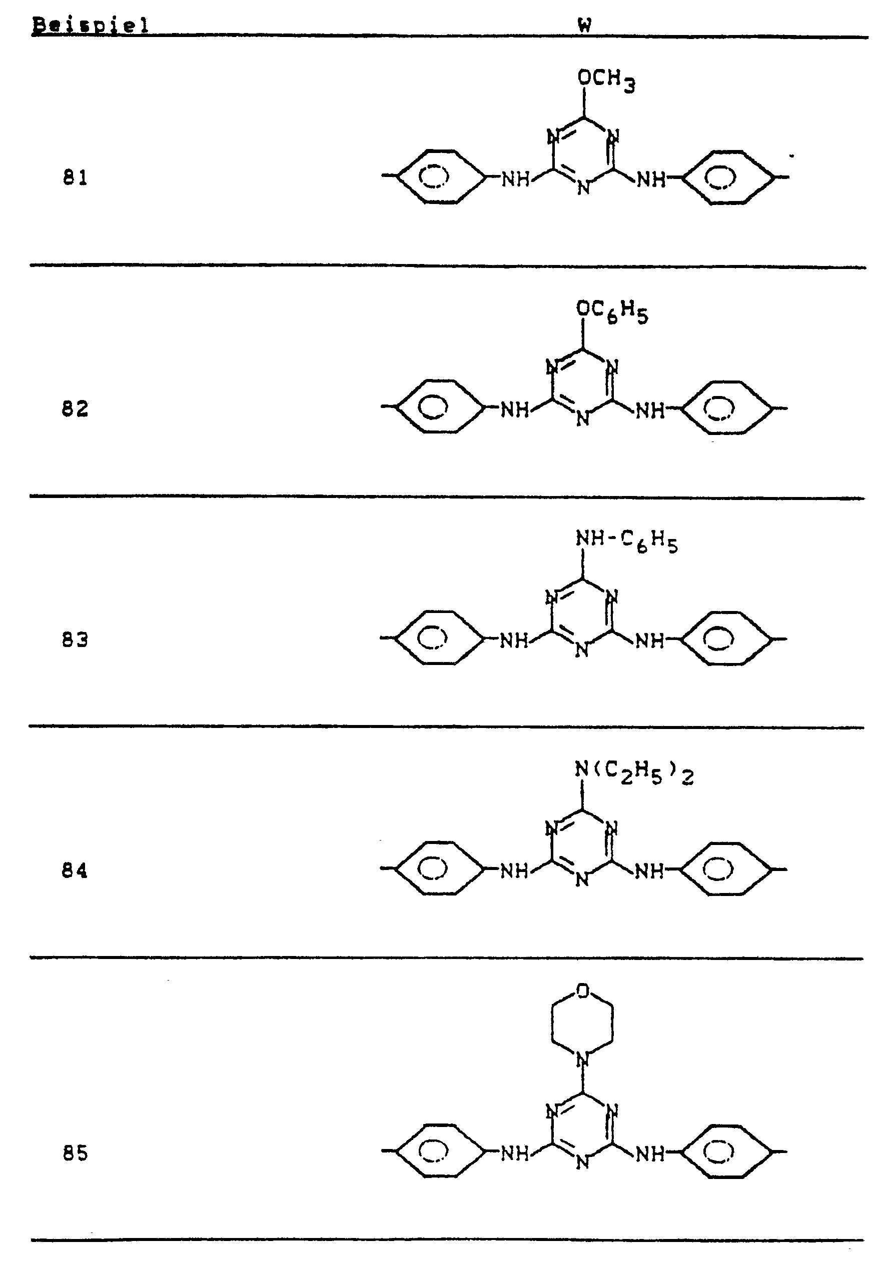

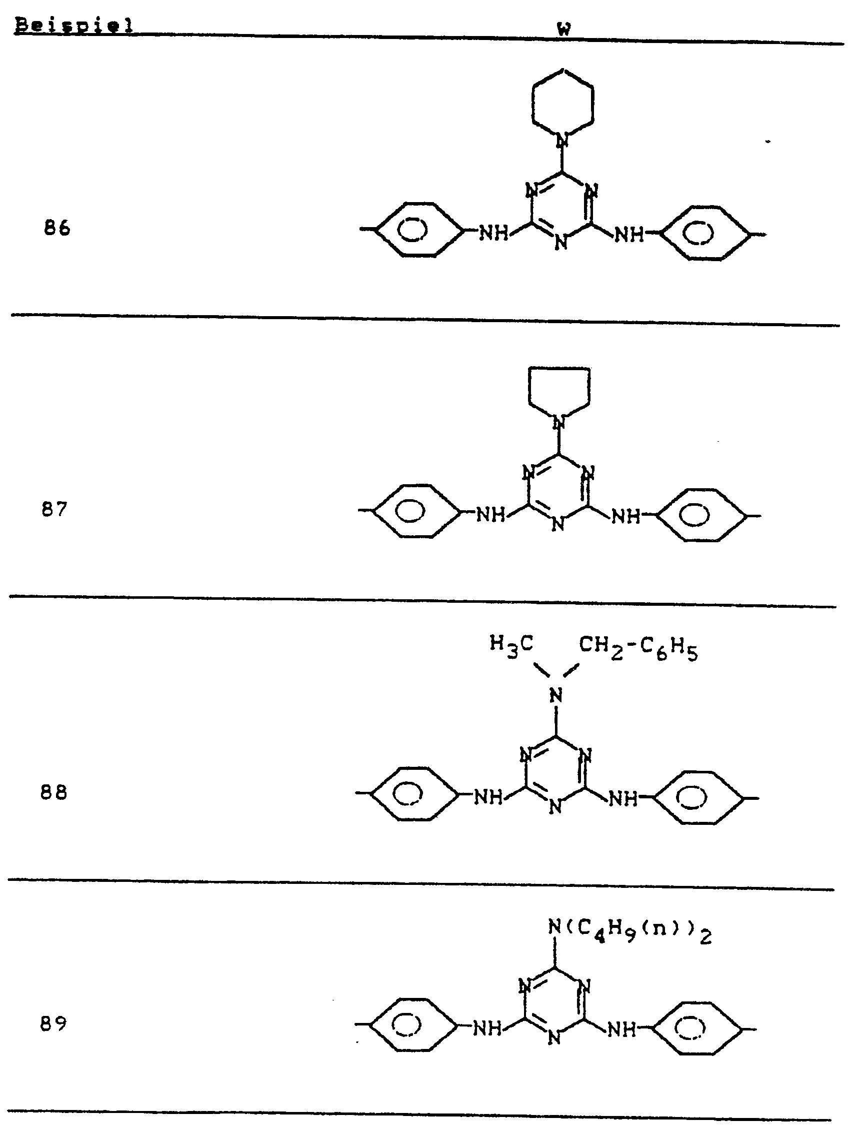

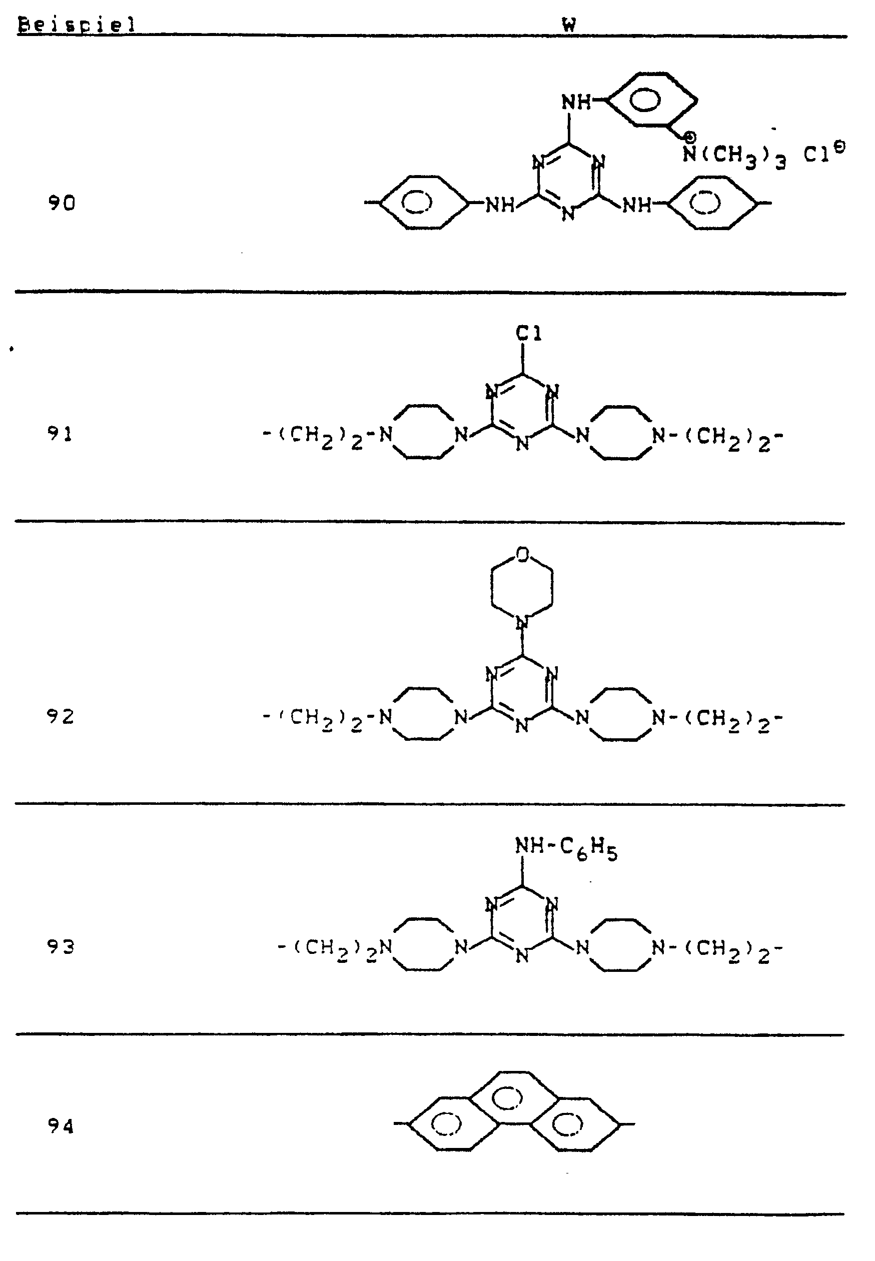

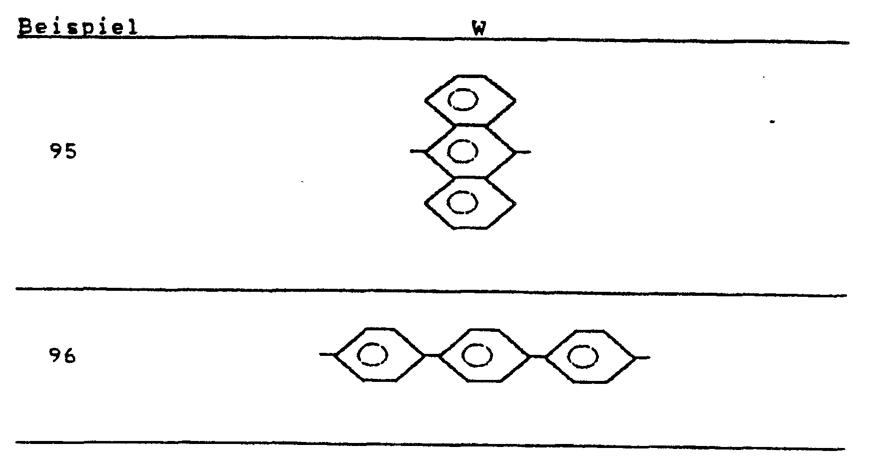

Ebenfalls in analoger Weise werden die folgenden Verbindungen hergestellt:

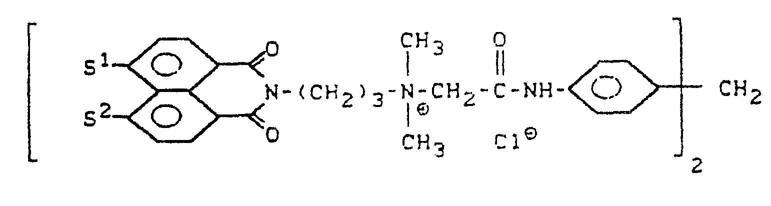

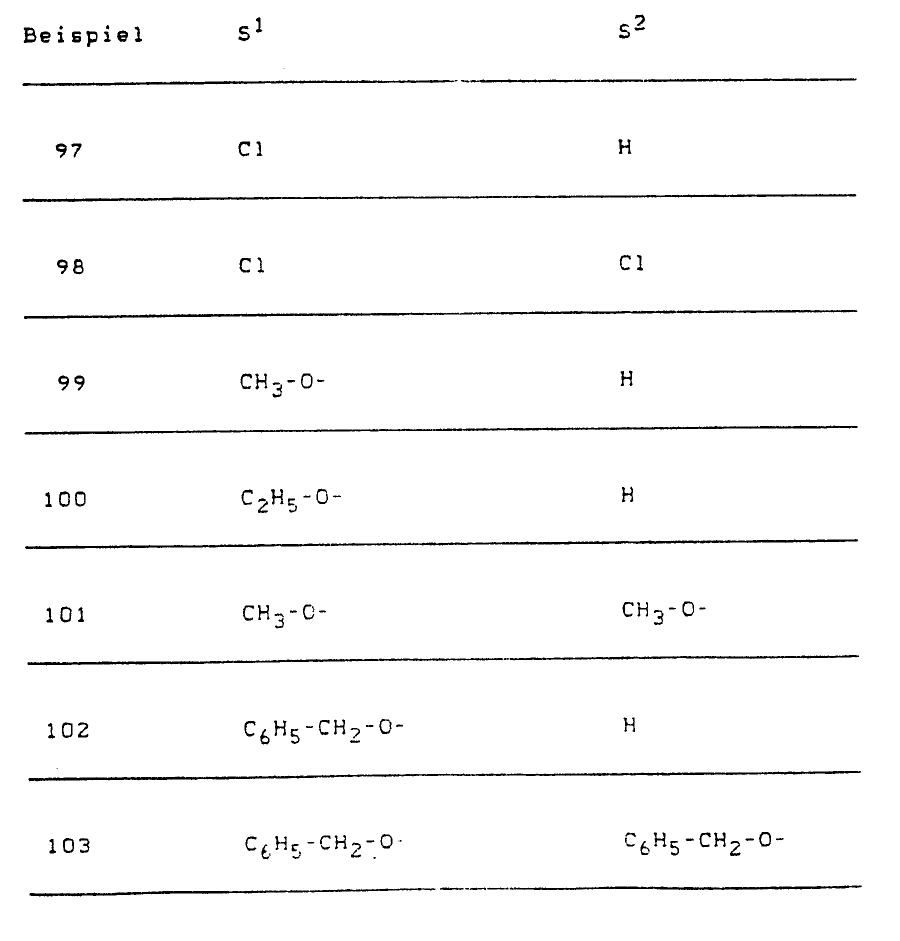

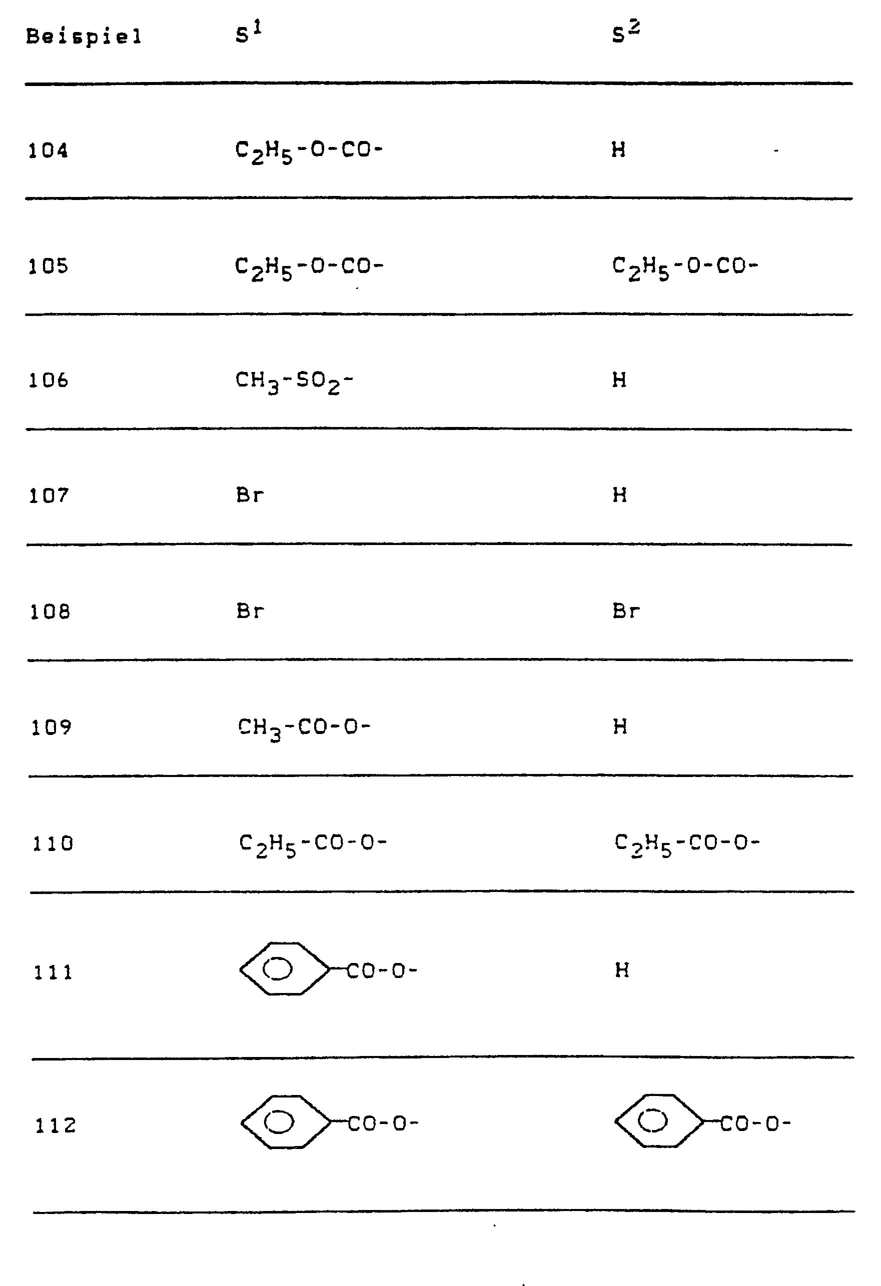

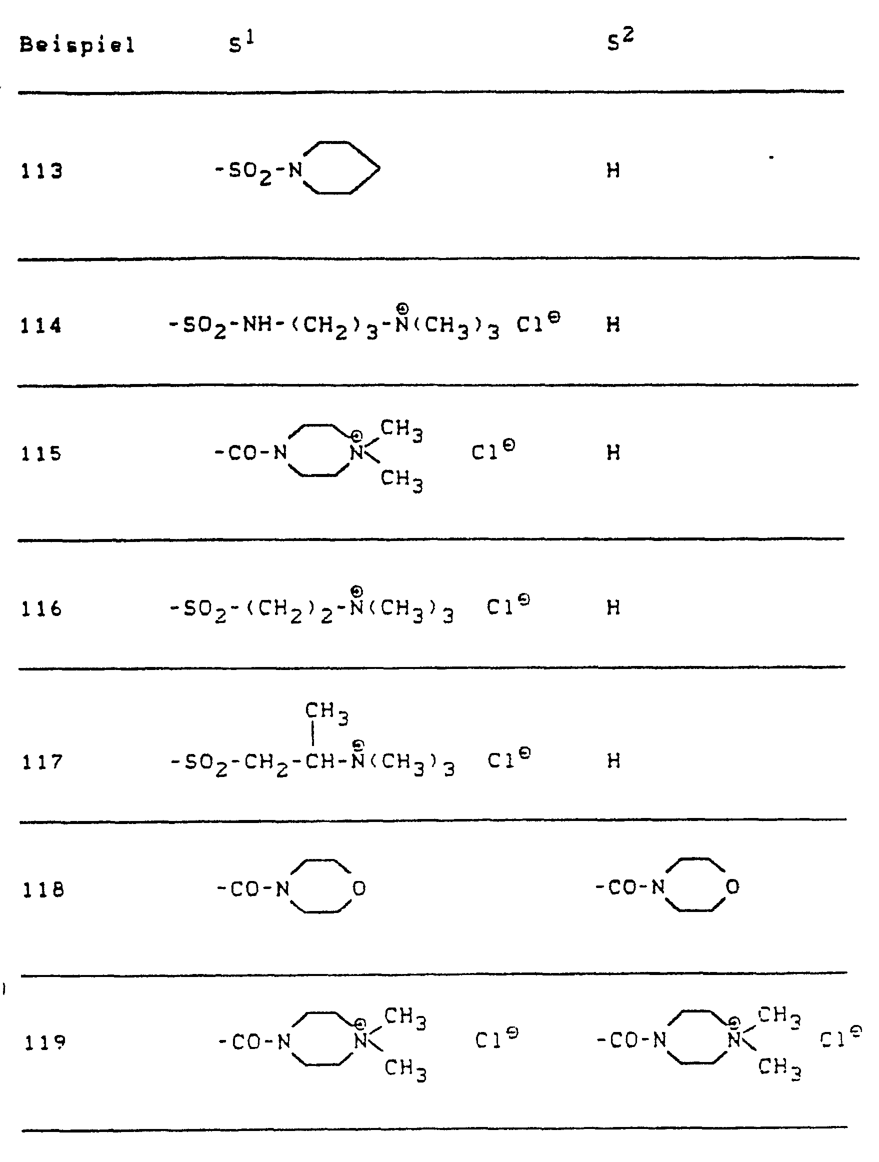

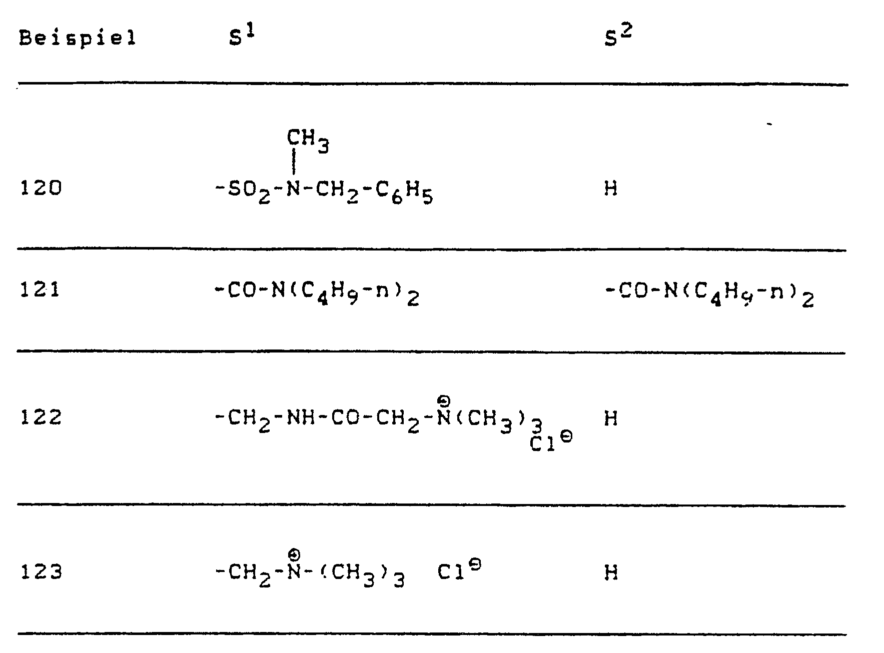

### Beispiel 124

85 g Naphthalsäure-N-(3-dimethylaminopropyl)-imid (0,3 Mol) und 52 g 4,4',4"-Tris-(chloracetamidophenyl)-methan (0,1 Mol) werden in 600 g Polyglykol (durchschnittliches Molgewicht 400) unter Stickstoff 20 h auf 100° C erhitzt, wobei sich zunächst eine klare Lösung bildet, aus der sich anschließend ein farbloser kristalliner Niederschlag abscheidet. Nun fügt man bei 60° C 1,5 I Aceton hinzu und kühlt die Suspension unter Rühren auf Raumtemperatur. Der kristalline Niederschlag wird abgesaugt, mit Aceton gewaschen und bei 60° C im Vakuum getrocknet. Man erhält 135 g (98,5 % der Theorie) Verbindung der Formel

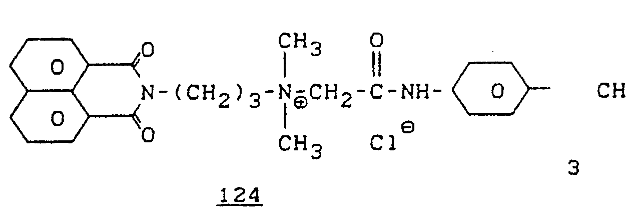

Die Substanz ist dünnschichtchromatographisch praktisch rein (Kieselgel, Laufmittel wie für Verb. 1 ); RF-Wert: 0,12.

4,4',4"-Tris-(chloracetamidophenyl )-methan ist analog der in Beispiel 1B. aufgeführten Bis-Verbindung aus 4,4',4"-Trisaminophenyl-methan und 3,2 Äquivalenten Chloracetylchlorid in siedendem Toluol in 95 %iger Ausbeute zugänglich. Schmelzpunkt: 240° C; m/e = 517 (M^{L} , 3CI).

In analoger Weise wie Verbindung 124 werden die folgenden Verbindungen hergestellt:

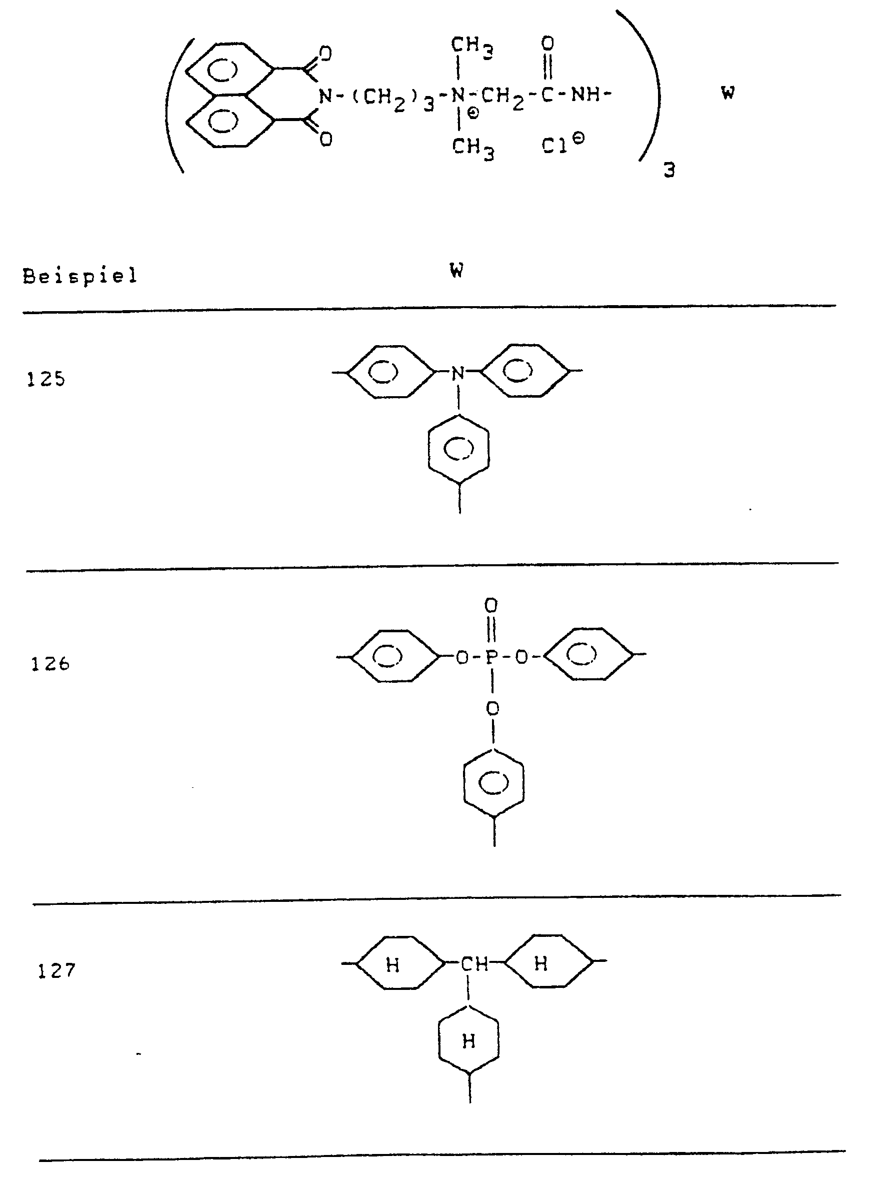

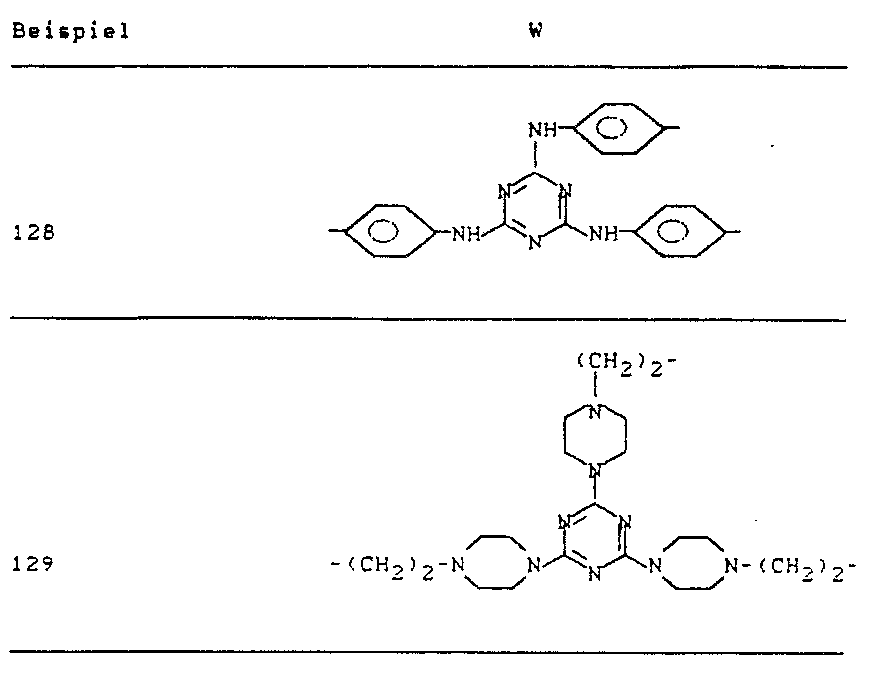

### Beispiel 130

69 g N-(3-N'-Methyl-N'-chloracetamidopropyl)-naphthalimid (0,2 Mol) und 45 g 4,4'-Bis-(3-dimethylaminopropylureido-phenyl)-methan (0,1 Mol) werden in 500 g Polyglykol (durchschnittliches Molgewicht 400) 8 h auf 100° C erhitzt, wobei eine gebrauchsfertige Lösung erhalten wird, die als Wirkstoff 18,6 Gewichtsprozent Fluoreszenzlöscher der folgenden Formel enthält

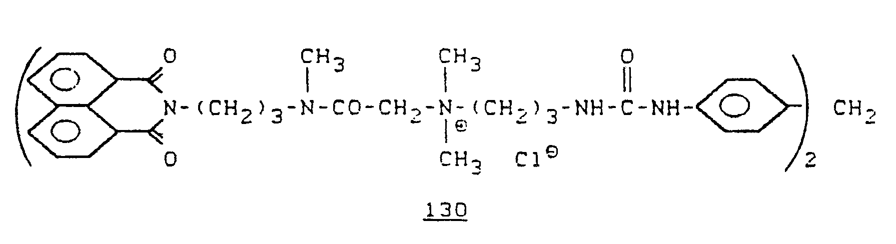

Die Substanz ist dünnschichtchromatographisch annähernd rein. RF-Wert: 0,19 (Laufmittel wie für Verbindung 1 ).

### Herstellung der Ausgangsverbindungen

A. N-(3-N'-Methyl-N'-chloracetamidopropyl)-naphthalimid
   79 g Naphthalsäureanhydrid (0,4 Mol) werden in 500 ml siedendem Toluol unter Zusatz von 1 ml Eisessig analog Beispiel 1A. am Wasserabscheider mit 36 g 1-Amino-3-methylamino-propan (ca. 0,4 Mol) umgesetzt und aufgearbeitet. Nach dem Umkristallisieren aus Methylcyclohexan erhält man 78 g (73 % der Theorie) Naphthalsäure-N-(3-methylaminopropyl)-imid vom Schmelzpunkt 108° C; m/e = 268 (M^{L}). 67 g Naphthalsäure-N-(3-methylaminopropyl)-imid (0,25 Mol) werden in 600 ml Toluol bei 20 - 40° C tropfenweise unter Rühren mit 29 g Chloracetylchlorid (0,257 Mol) versetzt, 4 h auf 105° C erhitzt und auf Raumtemperatur gekühlt. Der kristalline Niederschlag wird abgesaugt, mit Toluol gewaschen und bei 60° C im Vakuum getrocknet. Man erhält 81 g (94 % der Theorie) N-(3-N'-Methyl-N'-chloracet- amidopropyl)-naphthalimid vom Schmelzpunkt 152° C; m/e = 344 (M^{L} , Cl).
B. 4,4'-Bis-(3-dimethylaminopropylureidophenyl)-methan
   25 g Bis-(4-isocyanatophenyl)-methan werden in 250 ml wasserfreiem Accetonitril gelöst, unter Kühlung tropfenweise bei 20 - 30° C mit 20,4 g 1-Amino-3-dimethyl amino-propan versetzt und 20 h bei Raumtemperatur verrührt. Der kristalline Niederschlag wird abgesaugt, mit Acetonitril gewaschen und bei 50° C im Vakuum getrocknet. Man erhält 39,8 9 (88 % der Theorie) 4,4'-Bis-(3-dimethylaminopropylurei- dophenyl)-methan vom Schmelzpunkt 178°C; m/e = 454 (M^{⊕}).

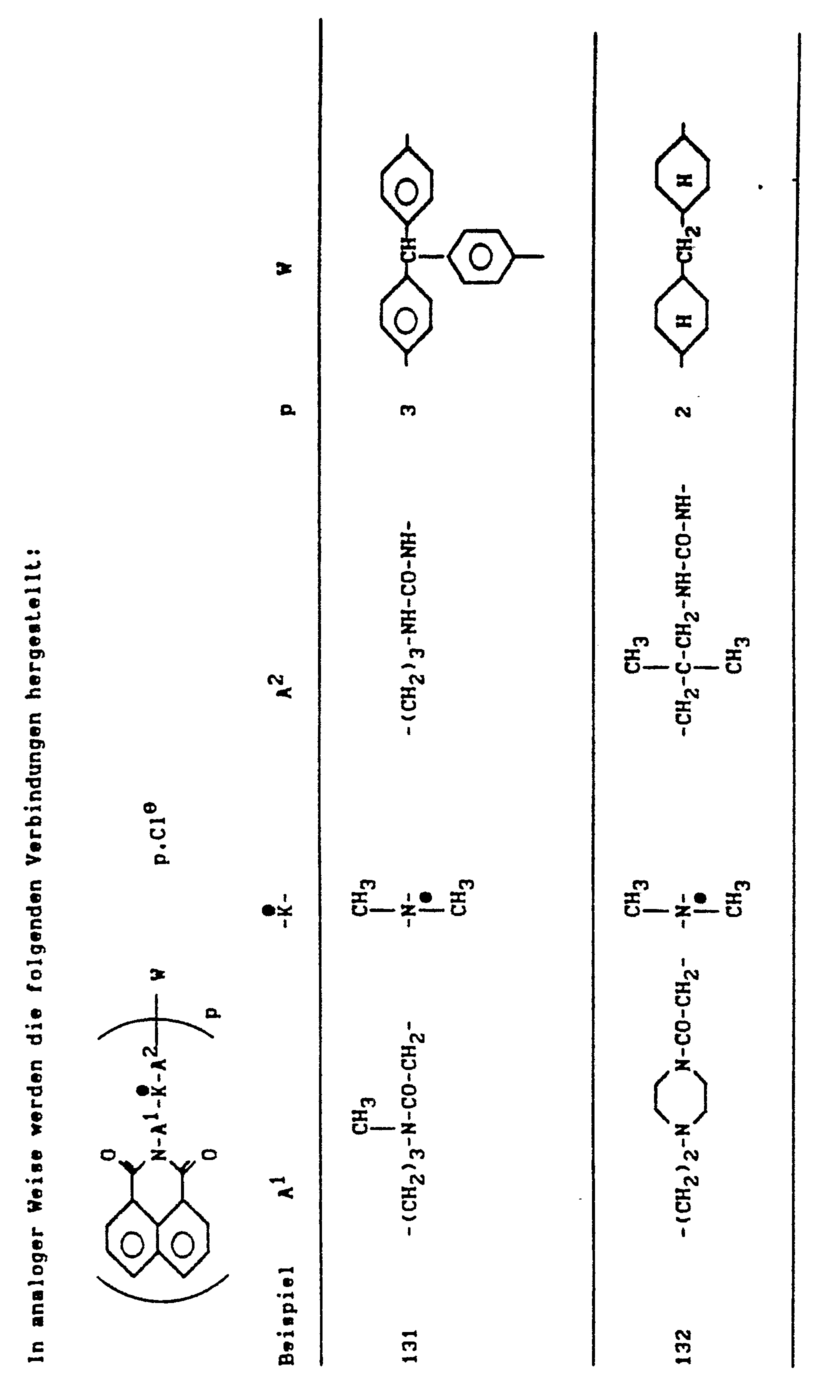

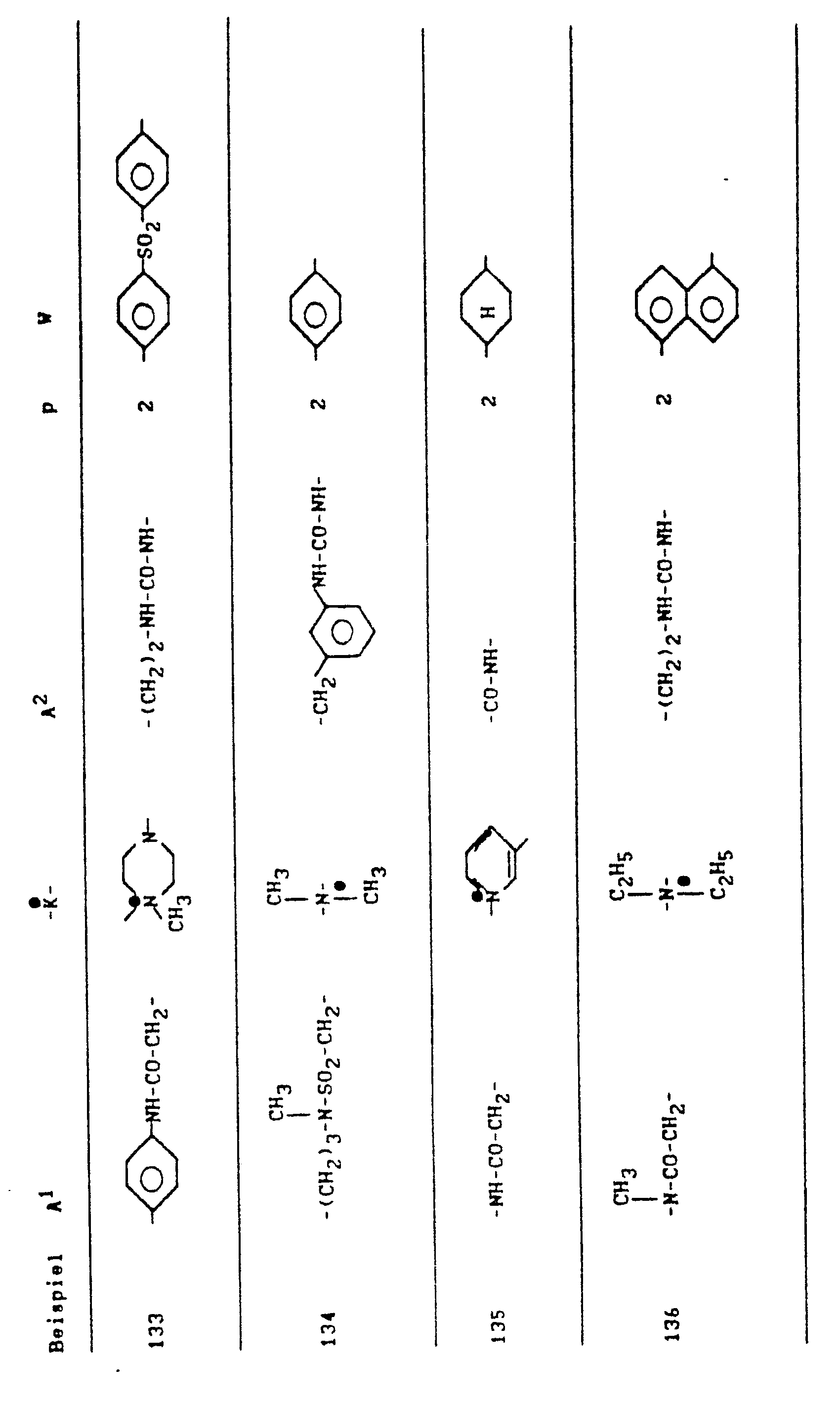

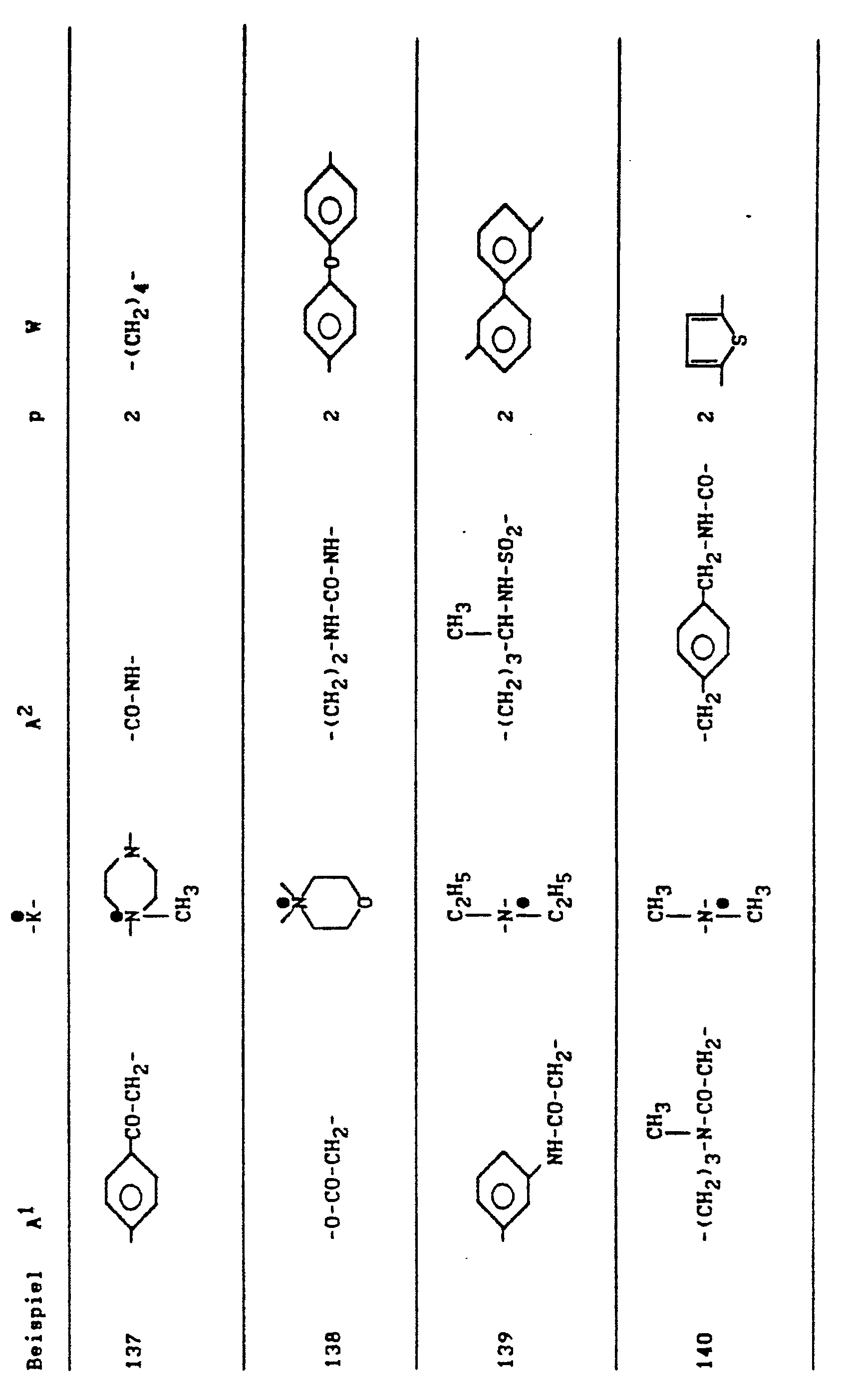

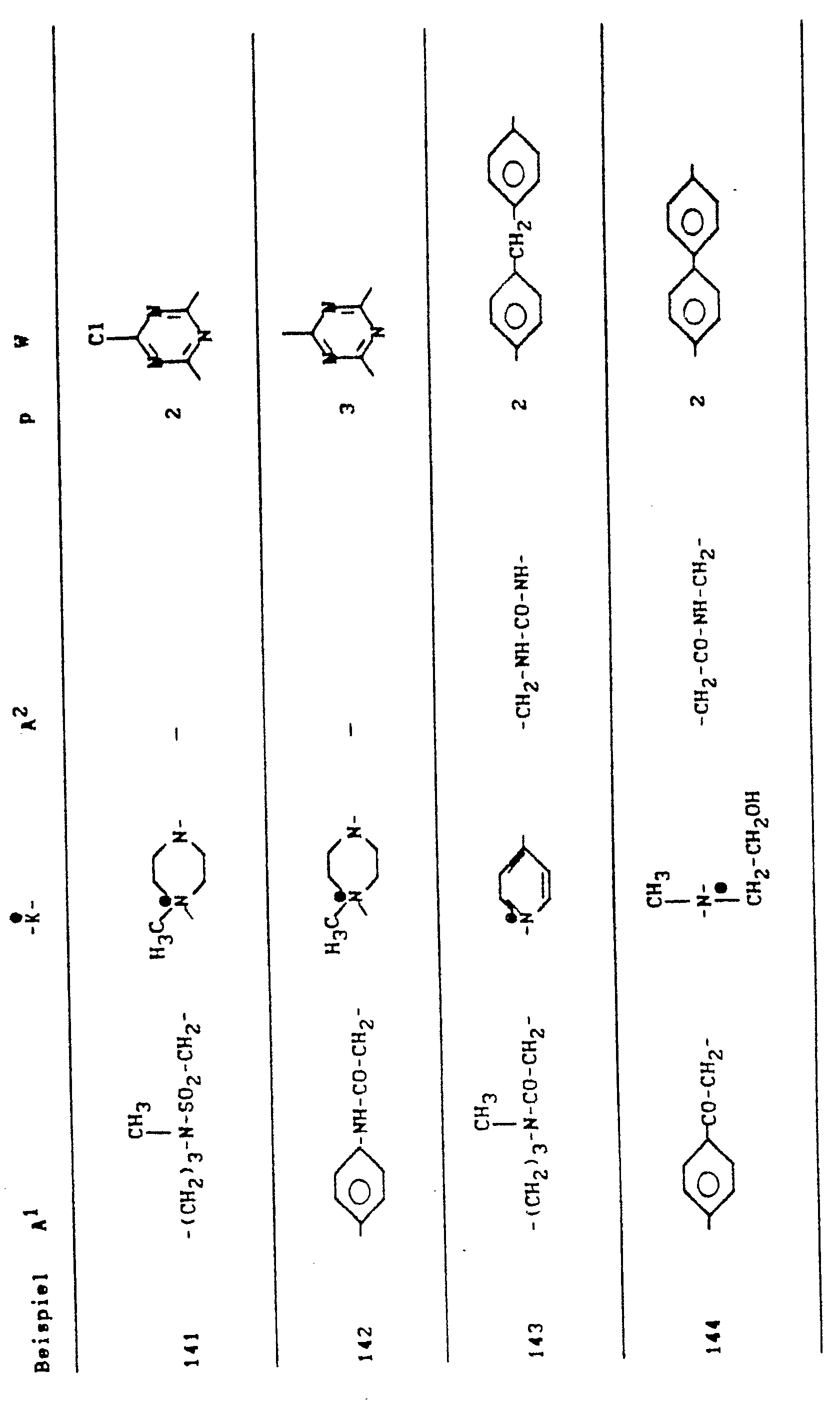

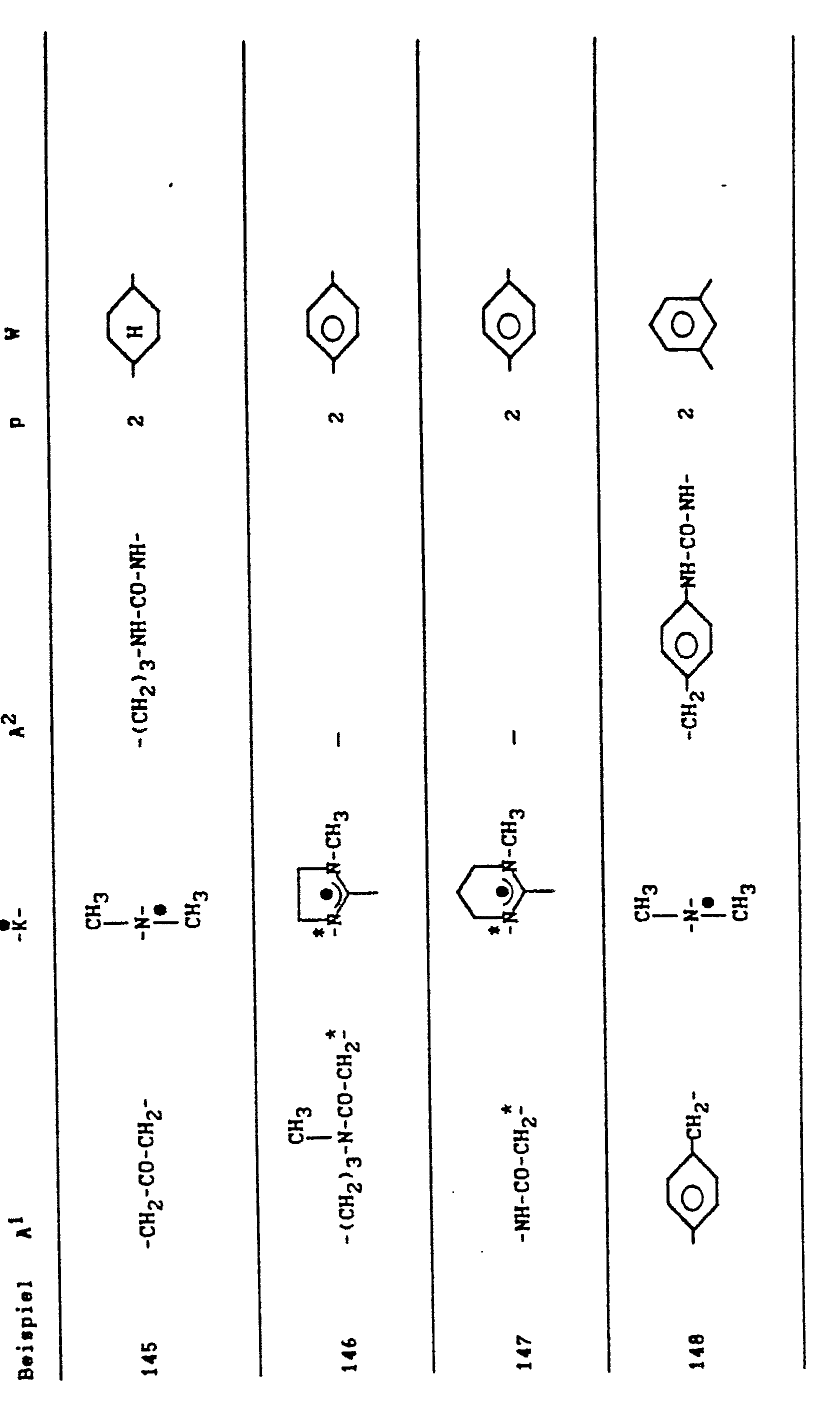

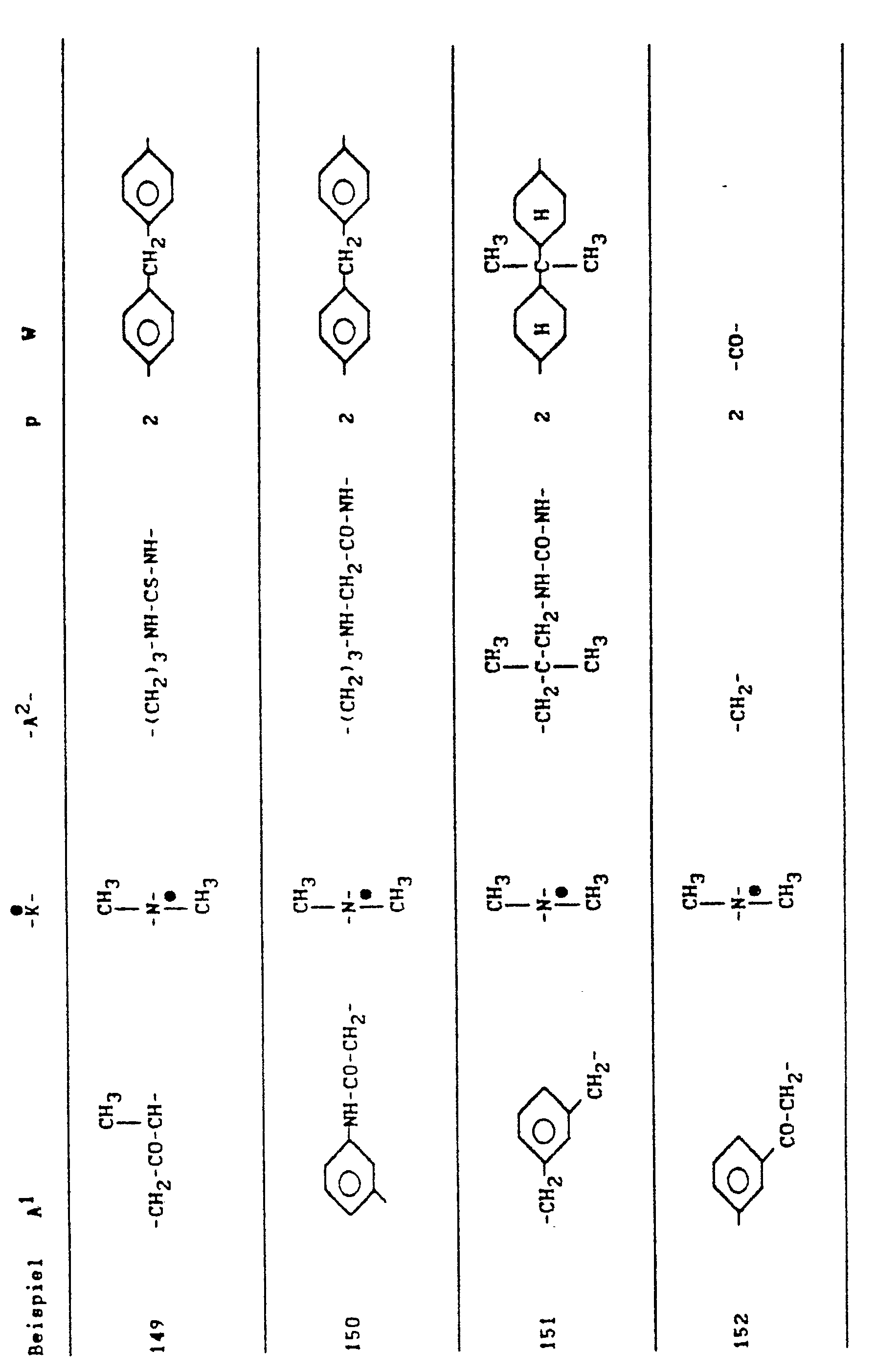

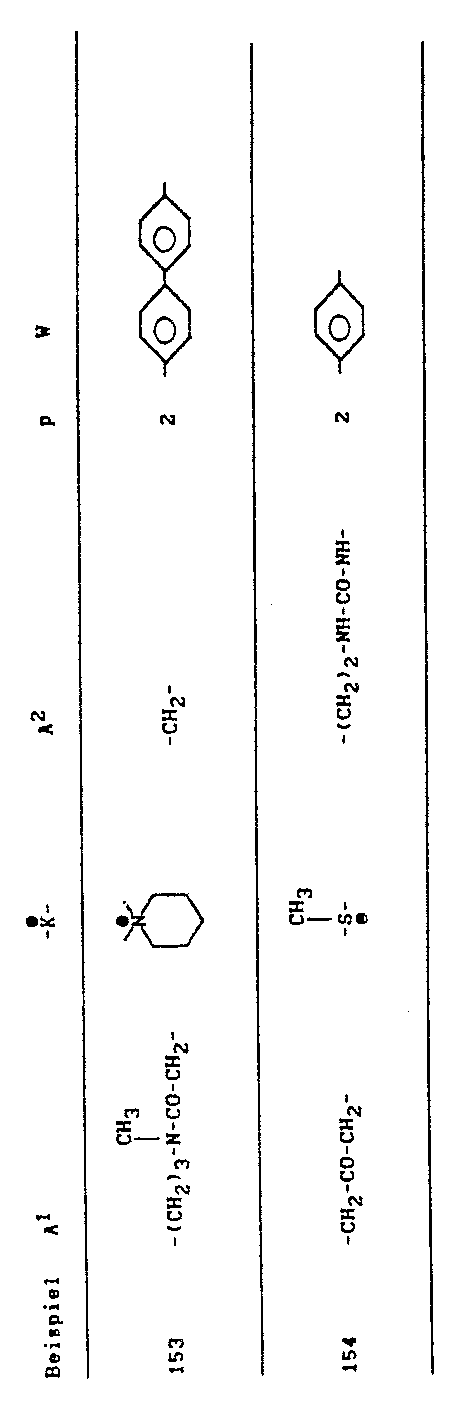

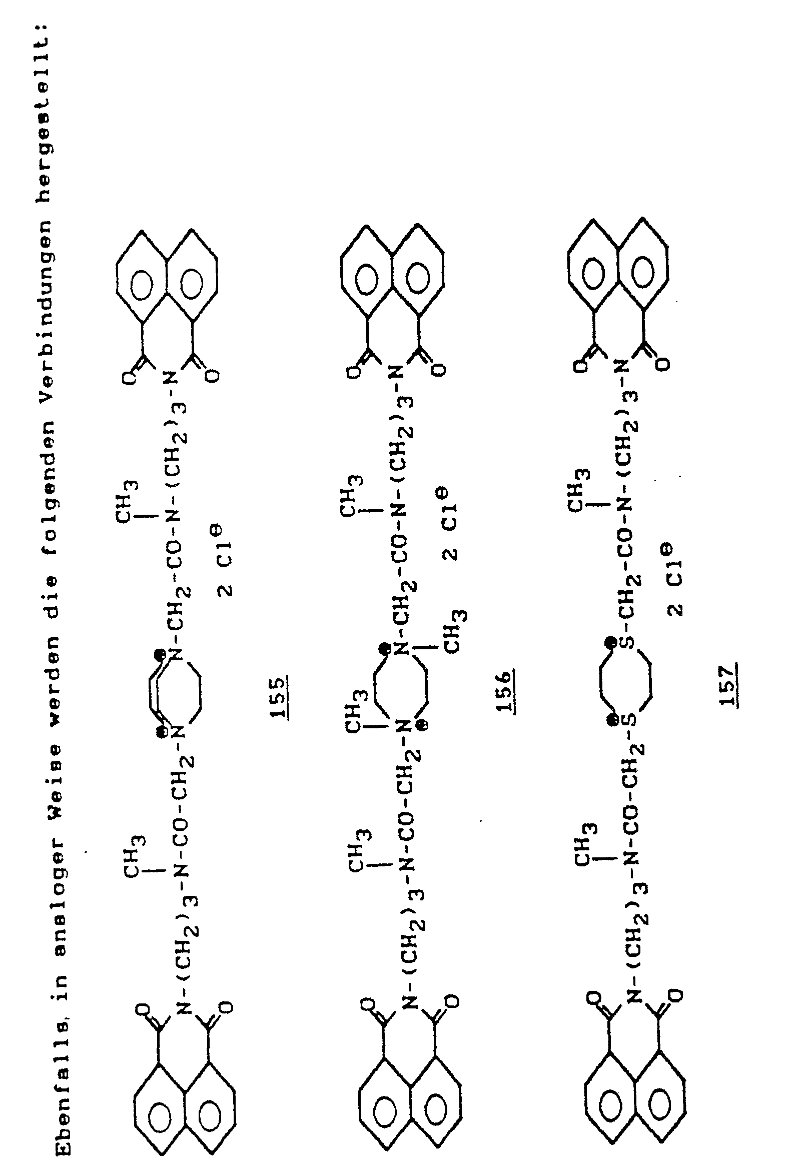

### Beispiel 158

50,5 g Bis-(3-naphthalimido-N-propyl)-methylamin (0,1 Mol) werden in 2 I wasserfreiem Chlorbenzol bei 70 - 75° C tropfenweise unter Rühren mit 10,5 ml Dimethylsulfat versetzt, 6 h bei 70 - 75° C verrührt, abgekühlt auf Raumtemperatur und 30 Min. mit 1,3 I Aceton verrührt. Der farblose kristalline Niederschlag wird abgesaugt, mit Aceton gewaschen und bei 40° C im Vakuum getrocknet. Man erhält 60 g (95 % der Theorie) Verbindung der Formel

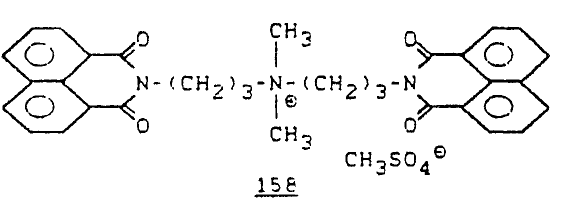

Die Substanz ist dünnschichtchromatographisch praktisch rein (Kieselgel, Laufmittel wie für Verbindung 1 ); RF-Wert: 0,53.

### Herstellung des Ausgangsproduktes

99 g Naphthalsäureanhydrid (0,5 Mol) werden in 1,3 I Toluol suspendiert, zunächst mit 1,5 g Eisessig, dann unter Rühren und Sieden am Wasserabscheider tropfenweise mit 36,3 g Bis-(3-aminopropyl)-methylamin (0,25 Mol) versetzt und noch 5 h am Wasserabscheider zum Sieden erhitzt, wobei sich insgesamt 9 ml Wasser abscheiden. Man fügt 3 g Aktivkohle und 6 g Tonsil hinzu, erhitzt die Suspension 20 Min. am Wasserabscheider zum Sieden, filtriert die heiße Lösung und dampft das Filtrat am Rotationsverdampfer zur Trockene ein. Dar Rückstand wird aus 600 ml Ethylenglykolmonomethylether umkriatallisiert, mit Ethanol gewaschen und bei 60 C im Vakuum getrocknat. Man erhält 88 g (70 % der Theorie) Bis-(3-naphthalimido-N-propyl)-methylamin vom Schmelzpunkt 166° C; m/e = 505 (M^{⊕}).

Analog 158 werden die folgenden Verbindungen hergestellt:

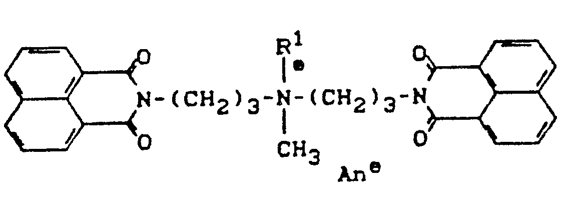

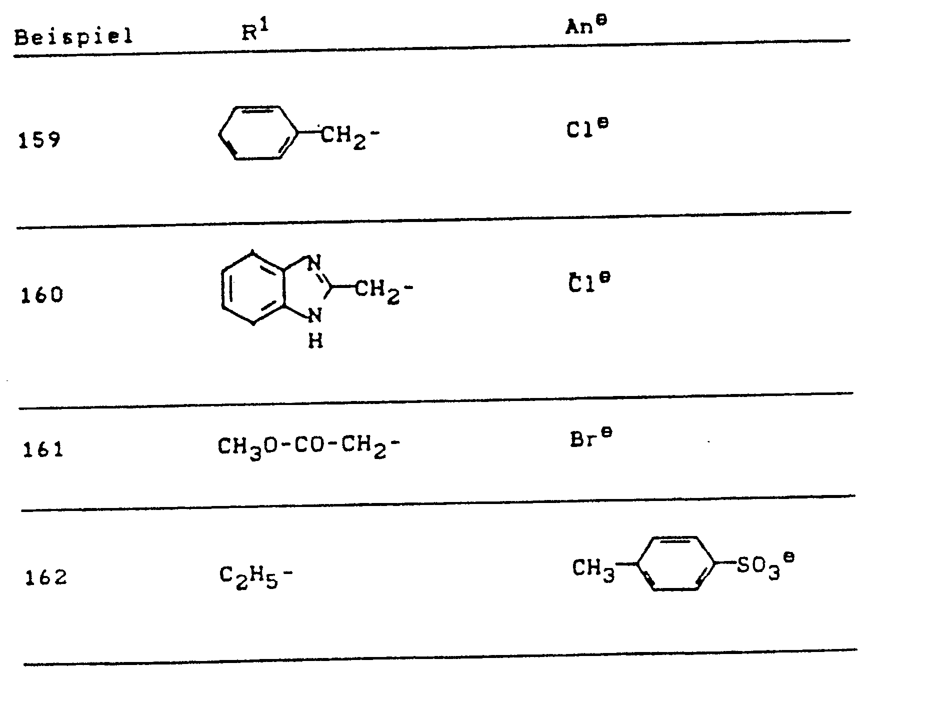

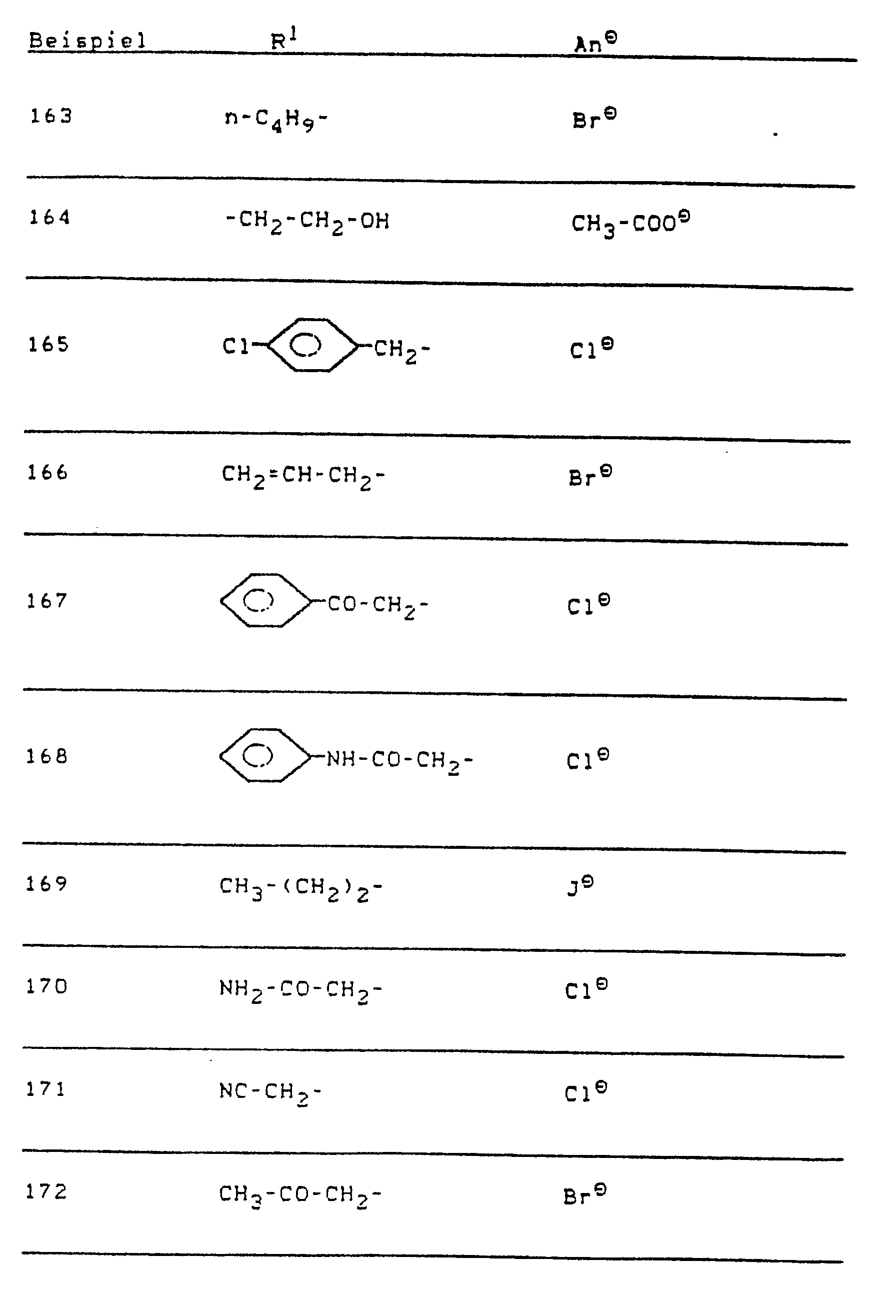

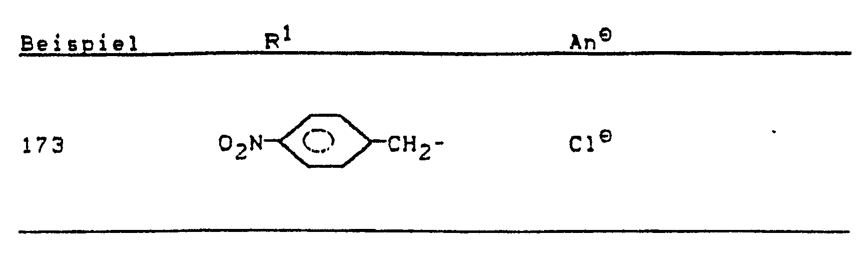

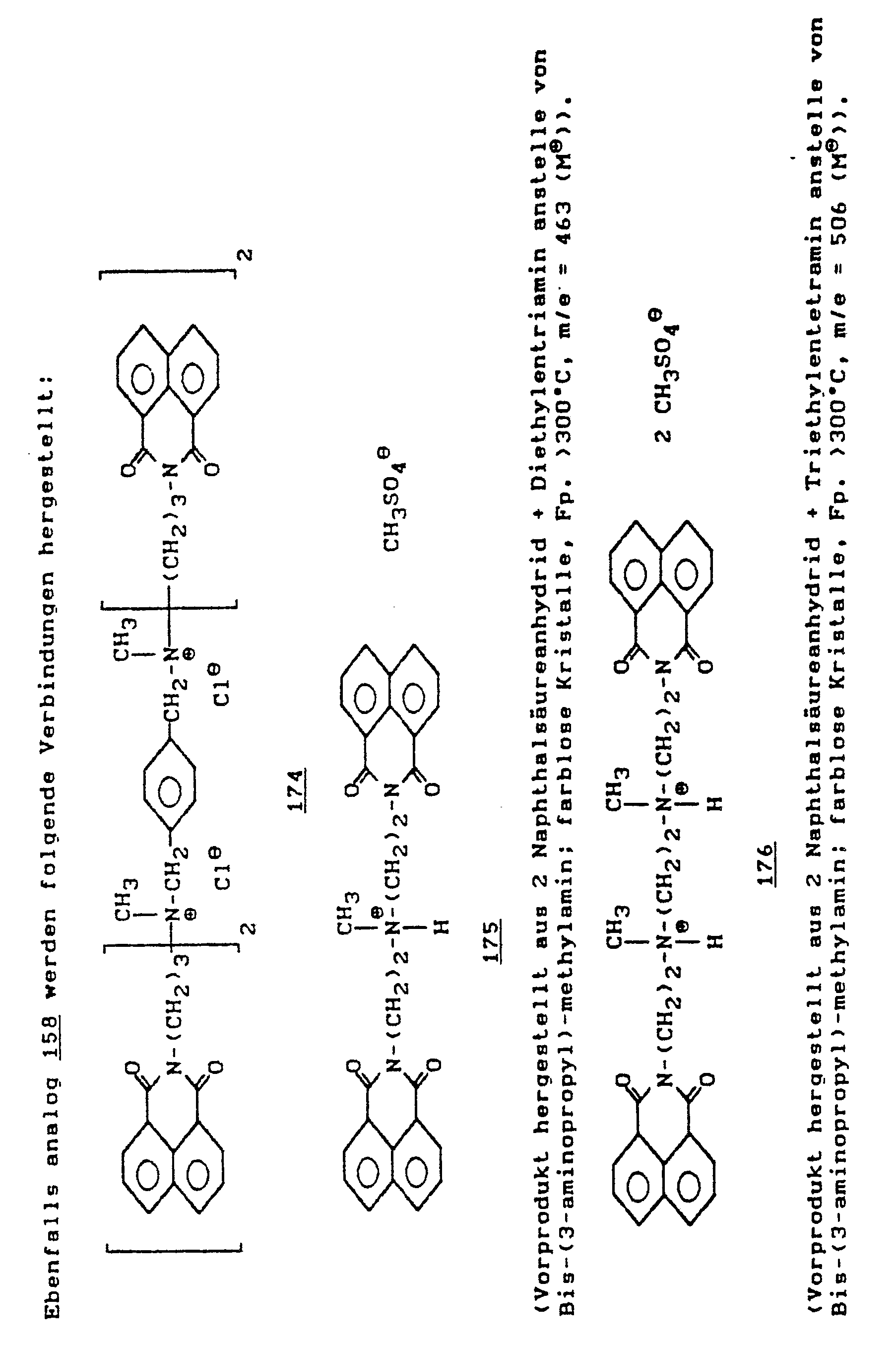

### Beispiel 177

50,6 g N,N'-Bis-(2-naphthalimido-N"-ethyl)-diamino-ethan (0,1 Mol, hergestellt aus 2 Naphthalsäureanhydrid + Triethylentetramin) und 15 g Acetiminoethylether-hydrochlorid (0,12 Mol) werden in 300 ml Acetonitril 2 Stunden zum Sieden erhitzt. Nach dem Abkühlen wird der kristalline Niederschlag abgesaugt, mit Aceton gewaschen und bei 50° C im Vakuum getrocknet. Man erhält 56 g (99 % der Theorie) Verbindung der Formel 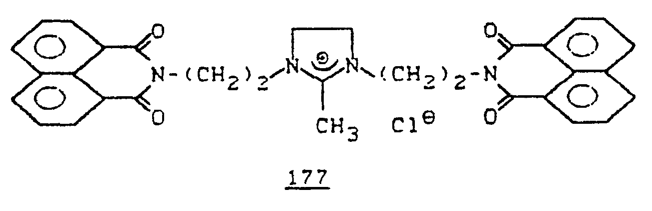 in Form fast farbloser, hochschmelzender Kristalle.

### Beispiel 178

0,1 Mol Verbindung der Formel 176 werden in Form der freien Base analog Beispiel 158, jedoch unter Zusatz von 0,25 Mol Triisopropanolamin als Protonenfänger mit 43 ml Dimethylsulfat in 2 1 Chlorbenzol bei 70 - 75° C umgesetzt (6 h). Nach Zugabe von 1,3 1 Aceton wird die farblose, kristalline Substanz isoliert. Ausbeute 93 % der Theorie. Sie entspricht der Formel 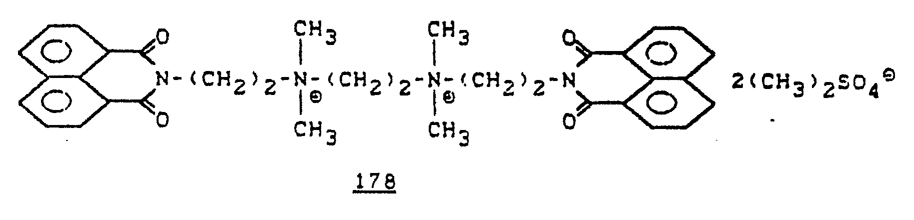 Farblose, gut wasserlösliche, hochschmelzende Kristalle.

### Beispiele 179 - 192

Analog Beispiel 130 , jedoch unter Einsatz der jeweiligen Ausgangskomponenten im Molverhältnis 1:1, werden folgende Verbindungen hergestellt:

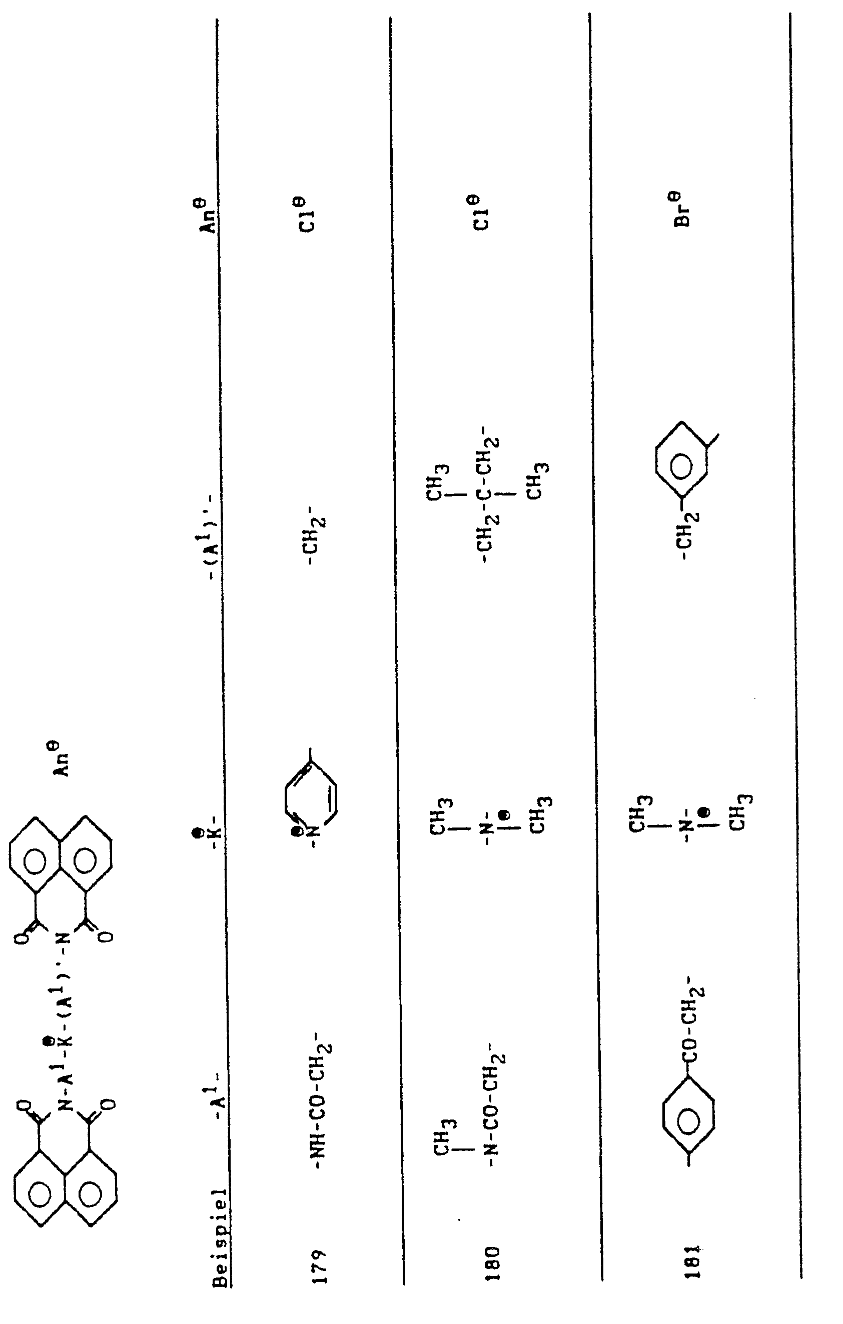

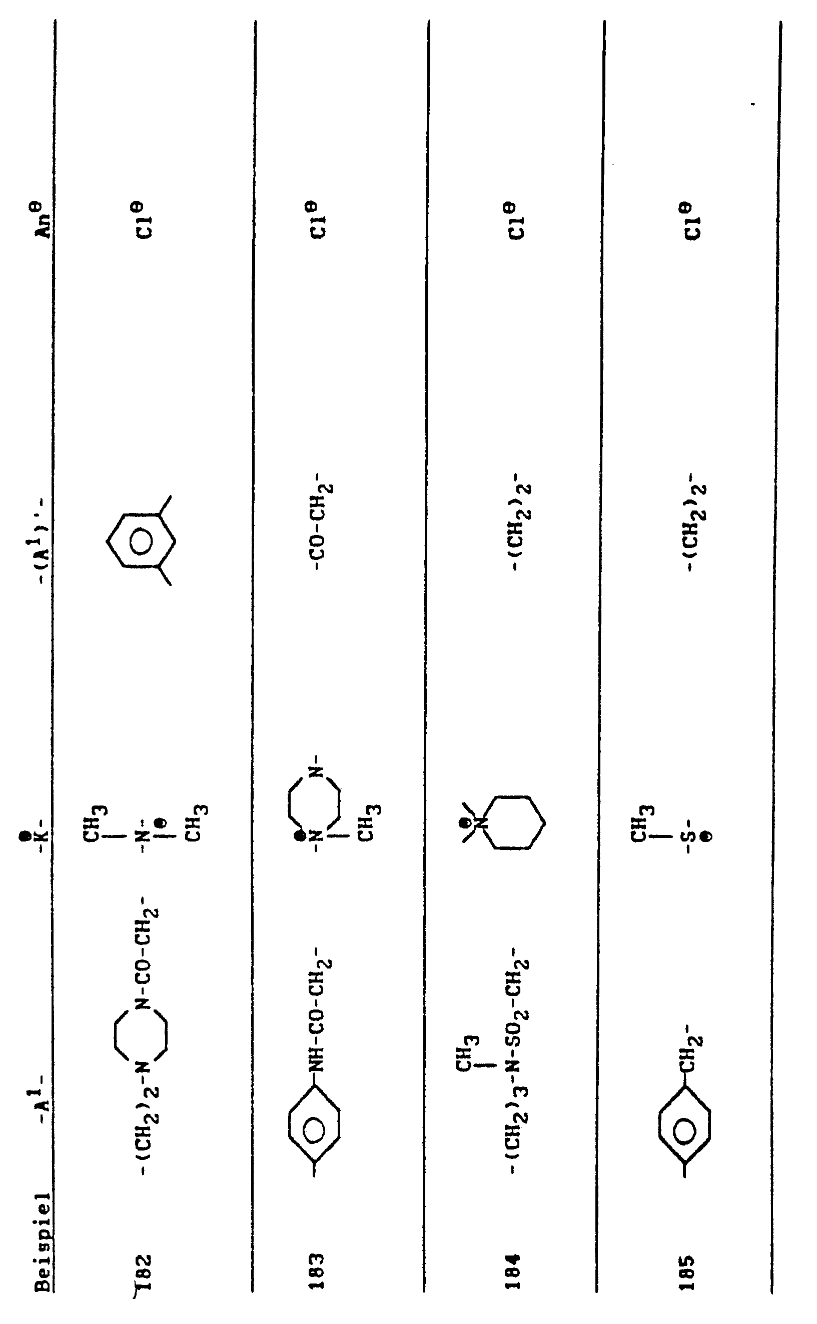

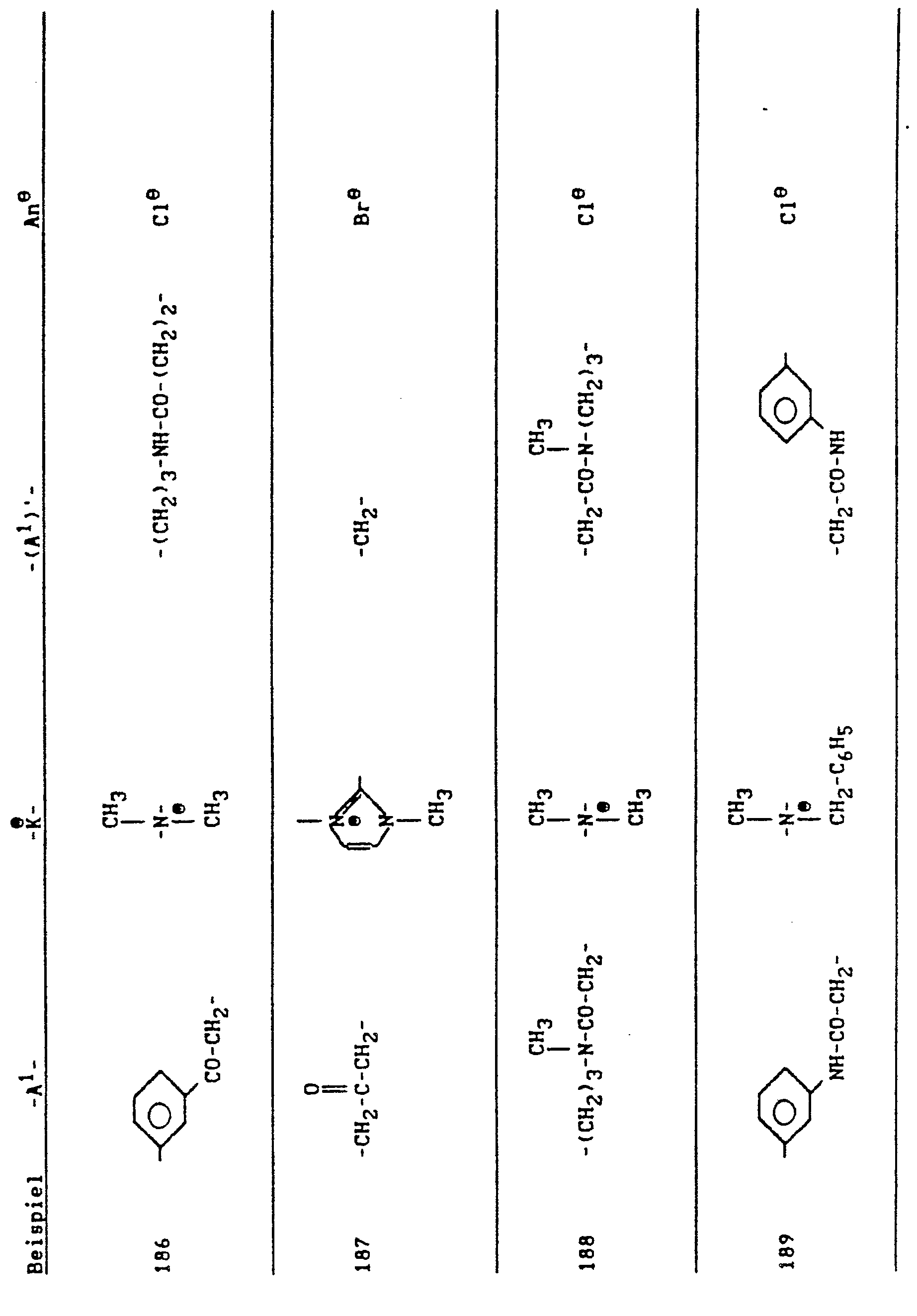

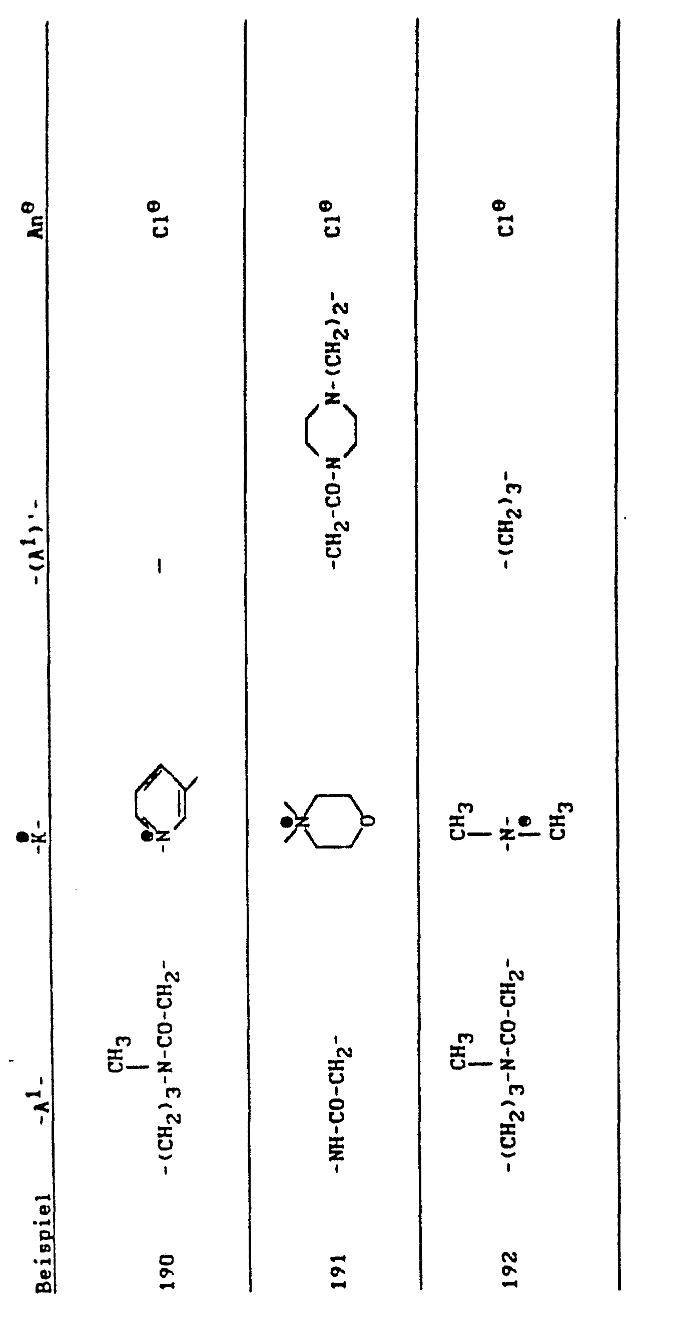

### Beispiel 193

43,6 g Naphthalin-1,4,5,8-tetracarbonsäure-N,N'-bis-(3-dimethylaminopropyl)-diimid (0,1 Mol) und 33 g 4-Chlor-benzylchlorid (ca. 0,2 Mol) werden in 1 I Acetonitril 20 h unter Rückfluß zum Sieden erhitzt. Nach dem Abkühlen wird der farblose kristalline Niederschlag abgesaugt, mit Acetonitril gewaschen und bei 50° C im Vakuum getrocknet. Man erhält 73,8 g (97 % der Theorie) Verbindung der Formel 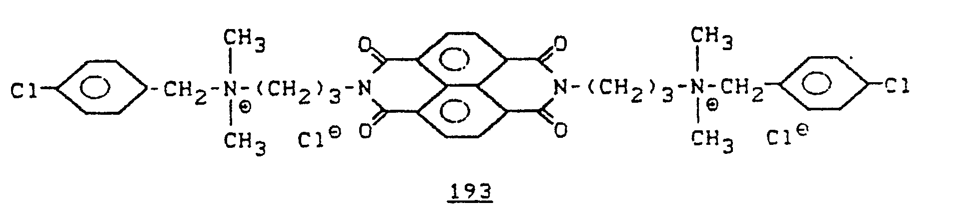 als annähernd farbloses Kristallpulver.

Die Substanz ist dünnschichtchromatographisch praktisch rein (Kieselgel, Laufmittel wie für Verbindung 1 ). RF-Wert: 0,22.

### Herstellung der Ausgangsverbindung

304 g Naphthalin-1,4,5,8-tetracarbonsäure werden in 1 1 Dimethylformamid 15 Min. unter Rückfluß zum Sieden erhitzt und abgekühlt. Der kristalline Niederschlag wird abgesaugt, mit Ethanol gewaschen und bei 50 C im Vakuum getrocknet. Man erhält 235 g (ca. 88 % der Theorie) reines Naphthalin-1,4,5,8-tetracarbonsäure-dianhydrid.
Schmelzpunkt: )300° C; m/e = 268 (M⁺).
53,6 g Naphthalin-1,4,5,8-tetracarbonsäure-dianhydrid (0,2 Mol) werden in 1 1 Toluol suspendiert, zunächst mit 2 g Eisessig, dann unter Rühren und Sieden am Wasserabscheider tropfenweise mit 41 g 1-Amino-3-dimethylaminopropan (0,4 Mol) versetzt und noch 2 h am Wasserabscheider zum Sieden erhitzt, wobei sich insgesamt etwa 7 ml Wasser abscheiden. Während der Reaktion geht das Umsetzungsprodukt bis auf geringe Verunreinigungen in Lösung. Man filtriert heiß und kühlt das Filtrat ab. Der kristalline Niederschlag wird abgesaugt, mit wenig Toluol gewaschen und bei 50° C im Vakuum getrocknet. Man erhält 72 g (ca. 83 % der Theorie) Naphthalin-1,4,5,8-tetracarbonsäure-N,N'-bis-(3-dimethylaminopropyl)-diimid als metallisch glänzendes goldgelbes Kristallpulver vom Schmelzpunkt 227° C: m/e = 436 (M^{⊕}).

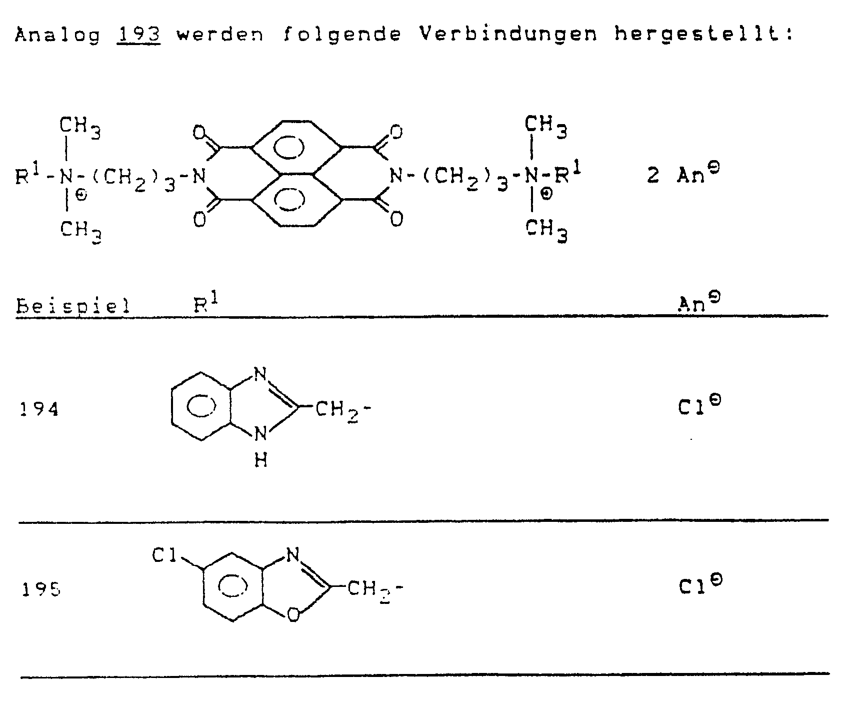

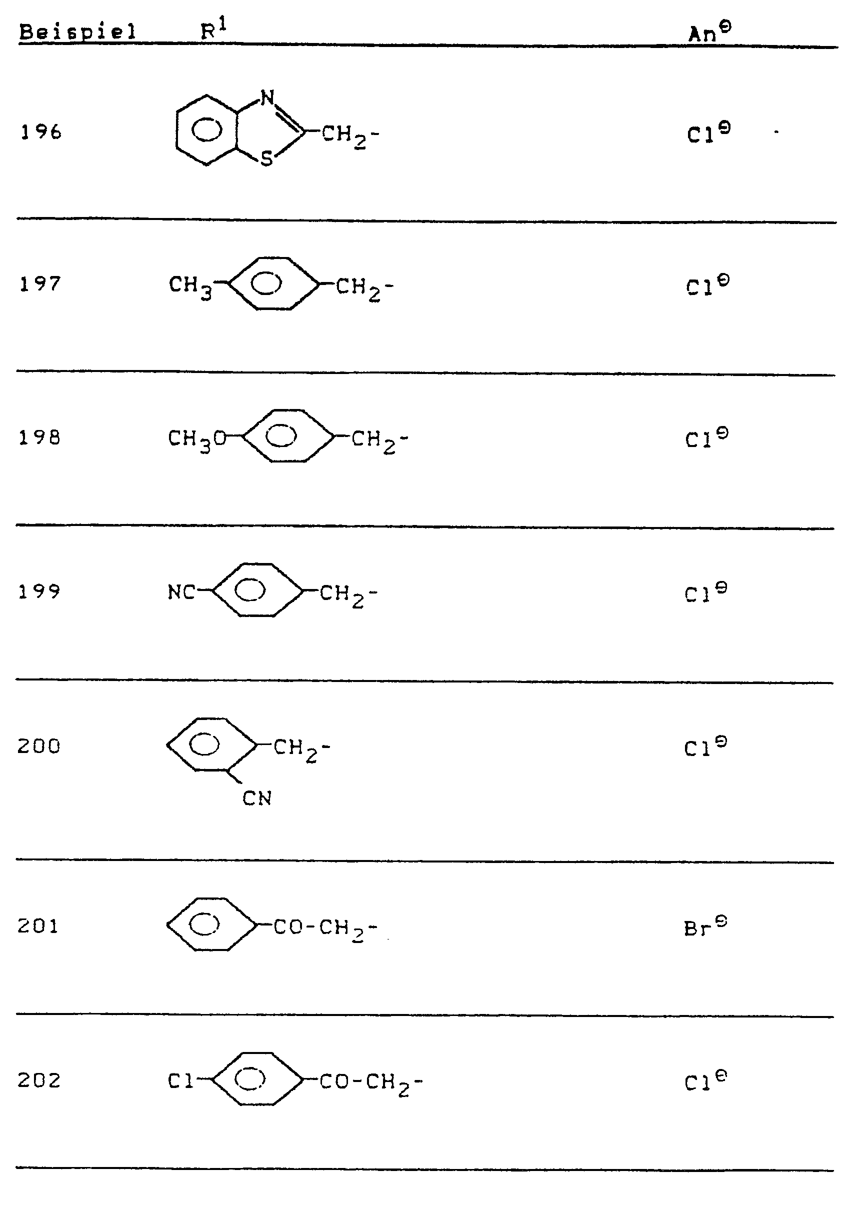

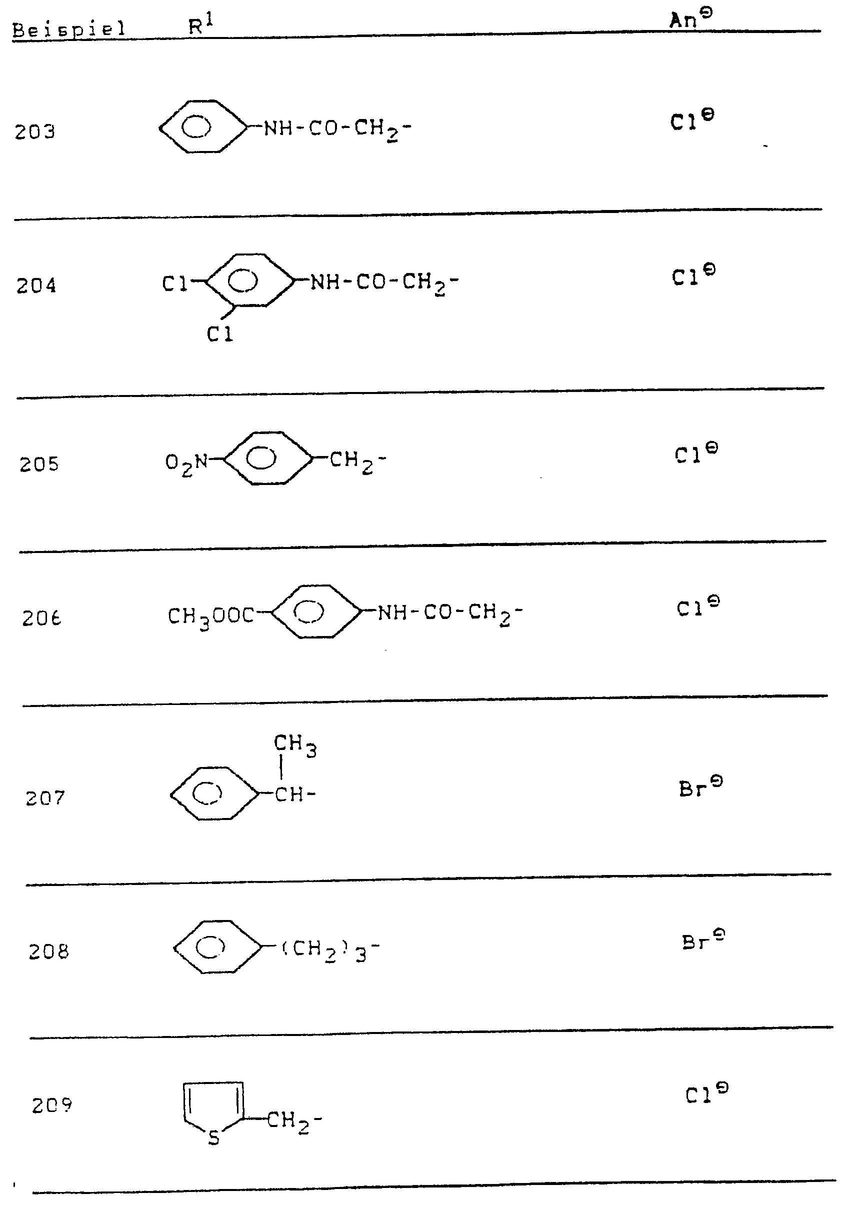

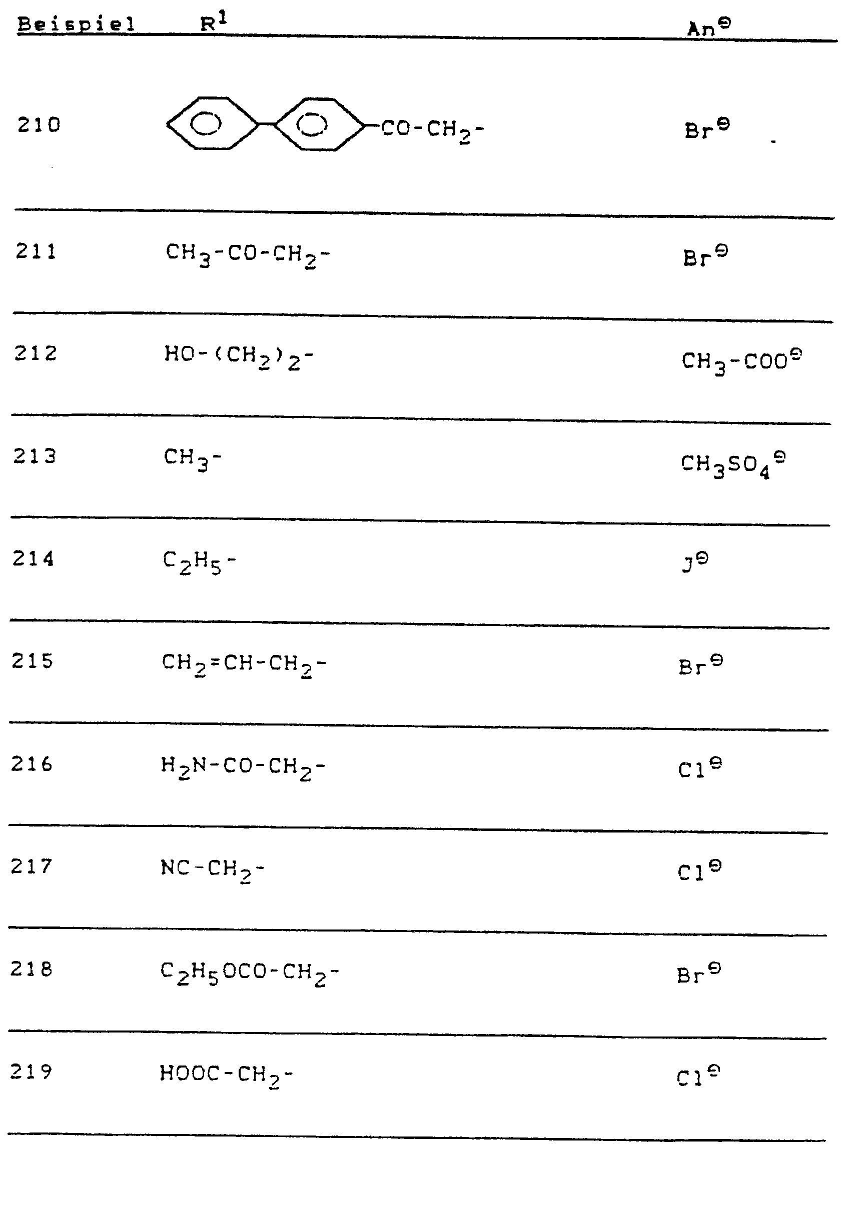

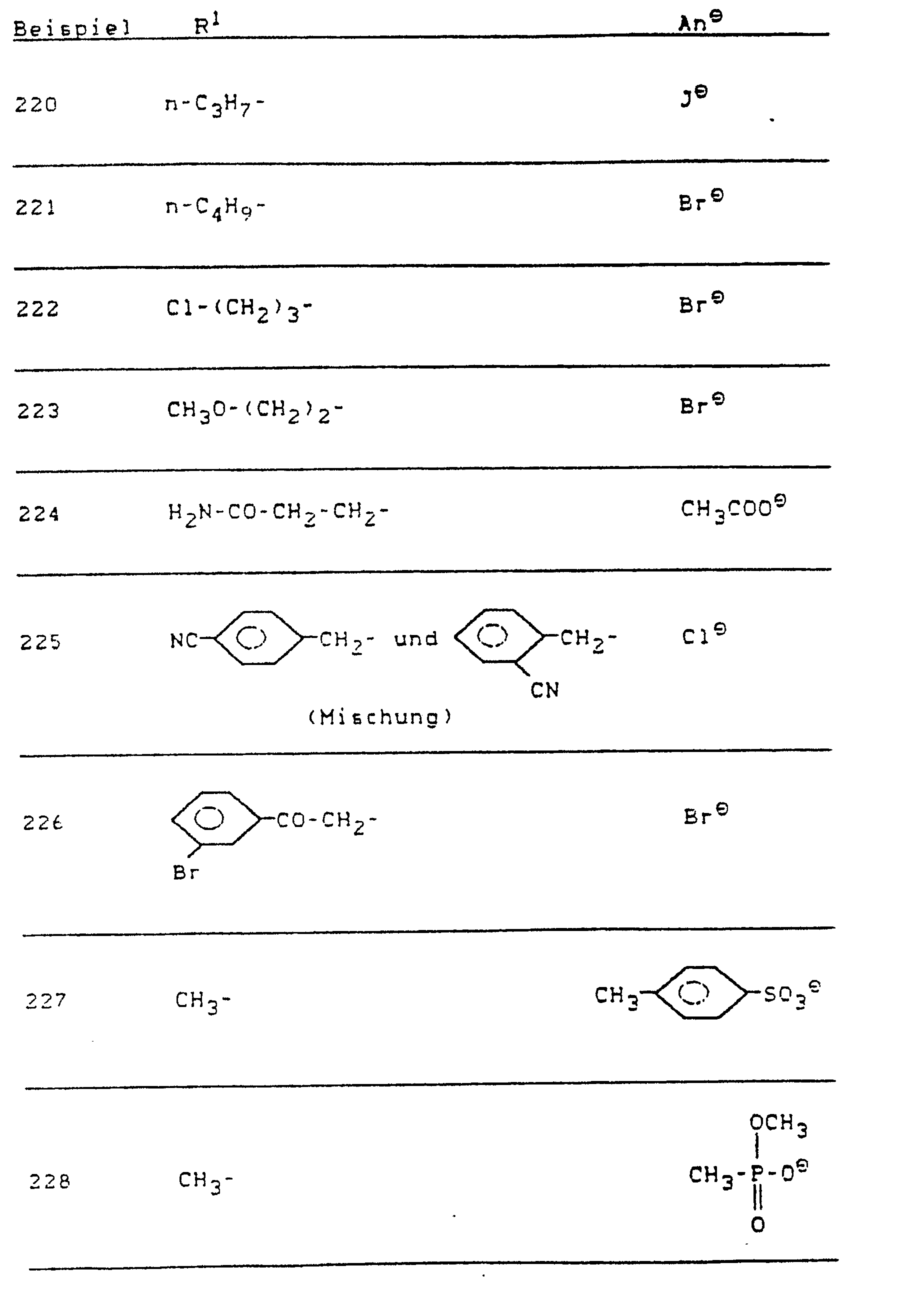

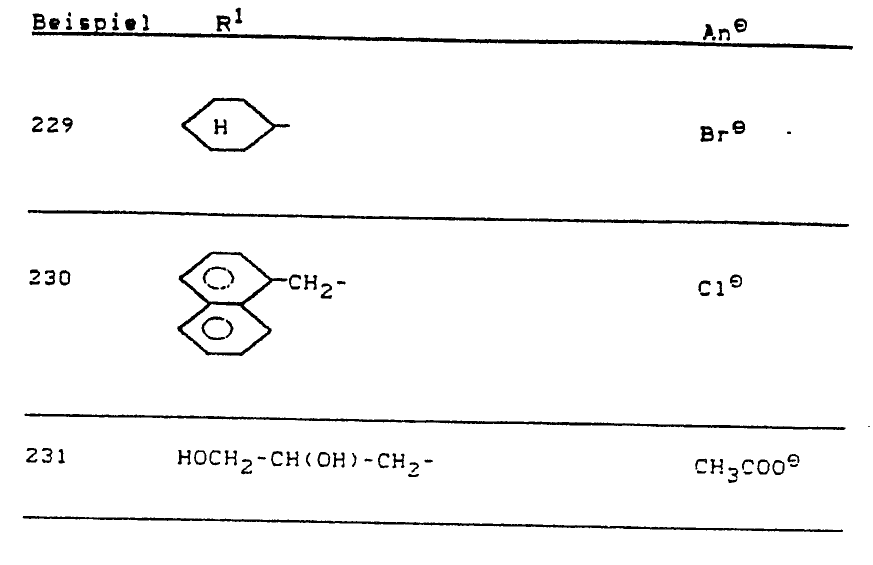

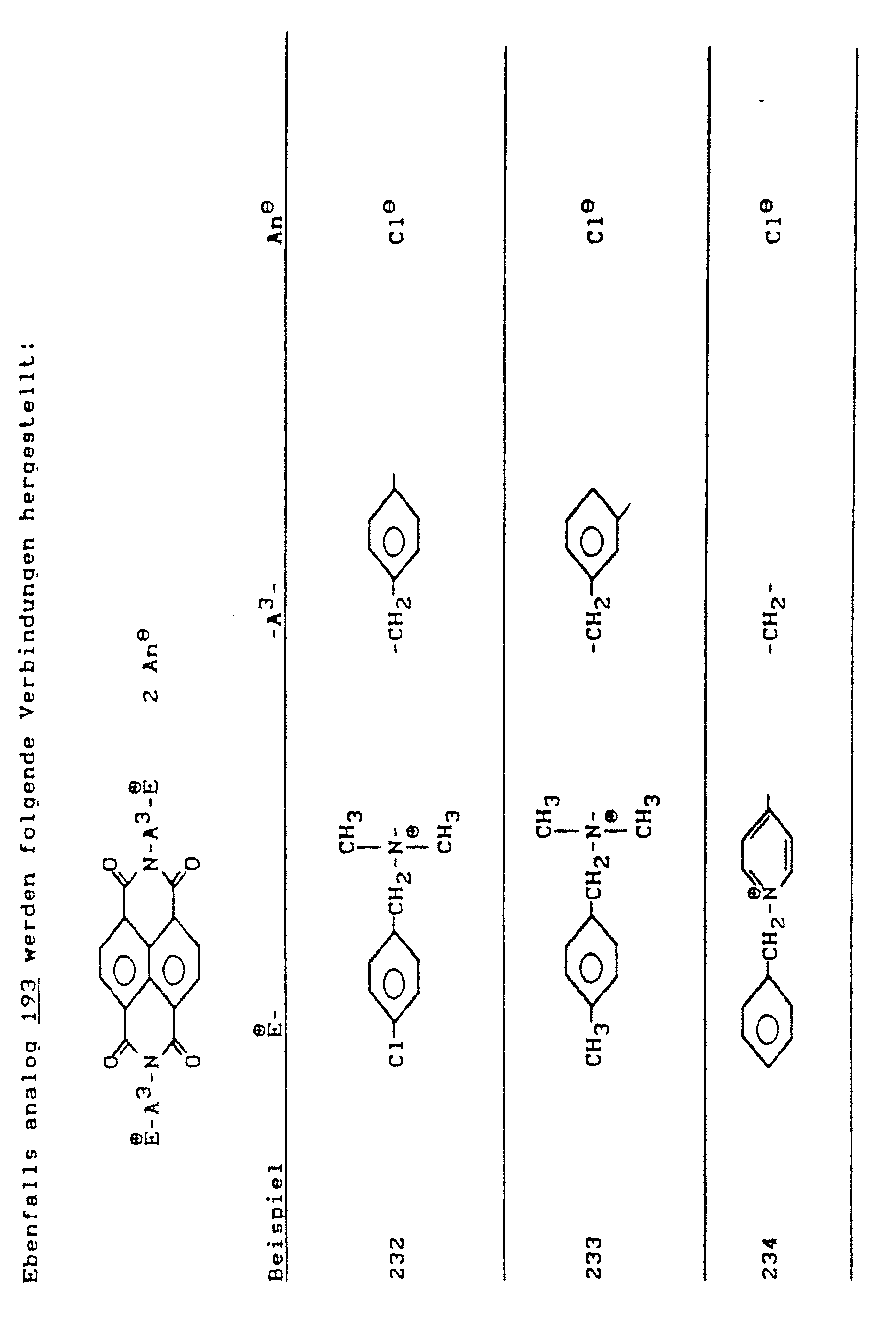

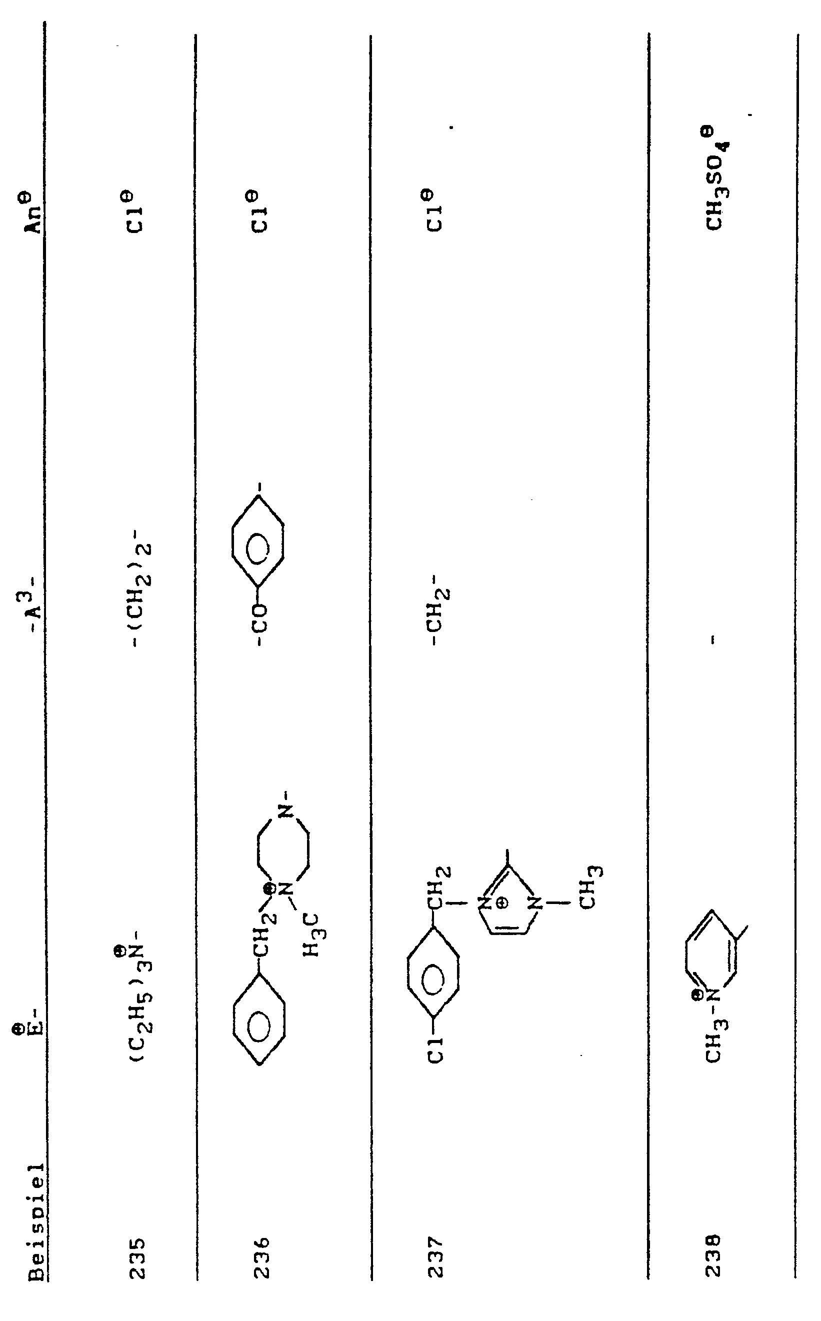

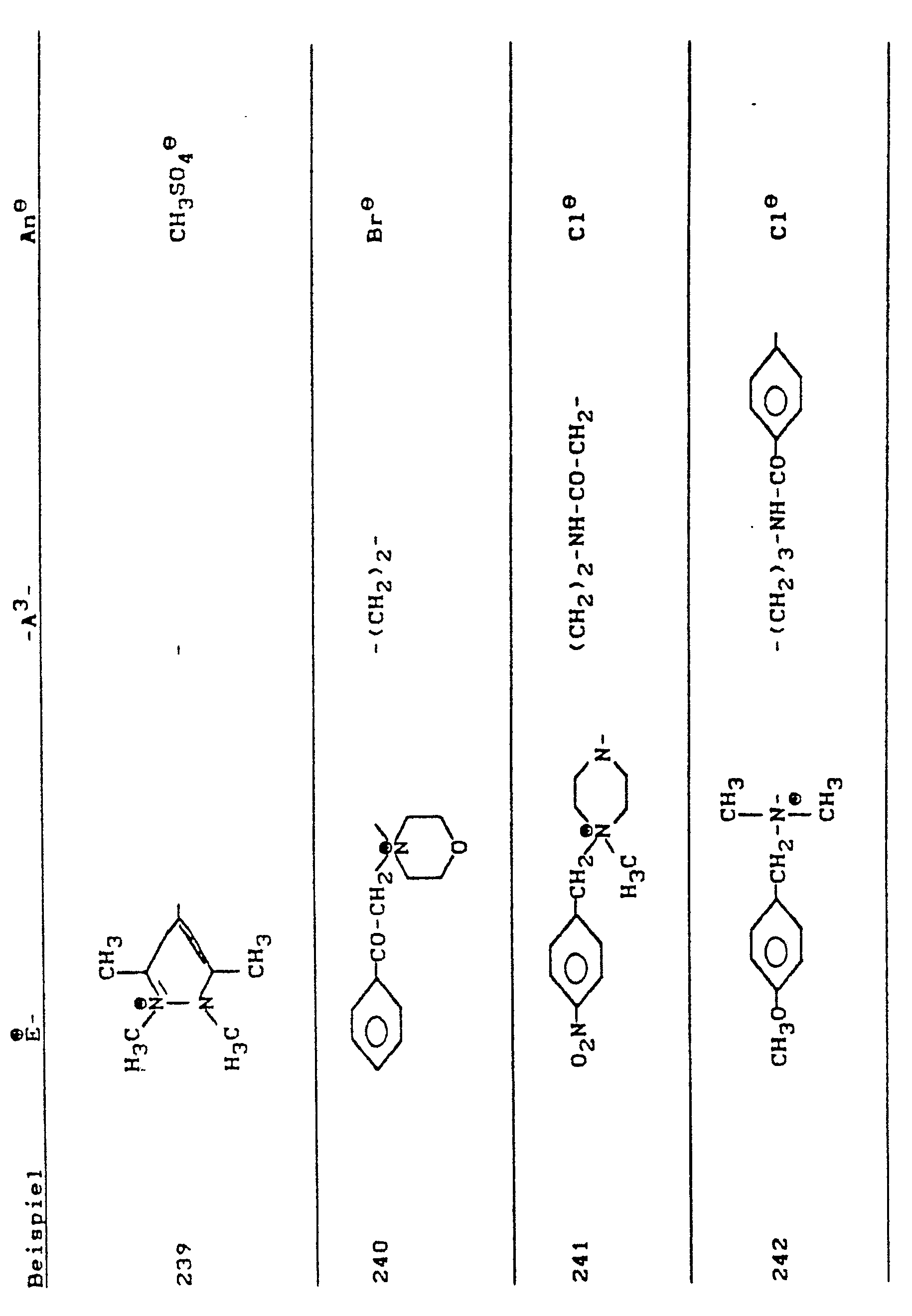

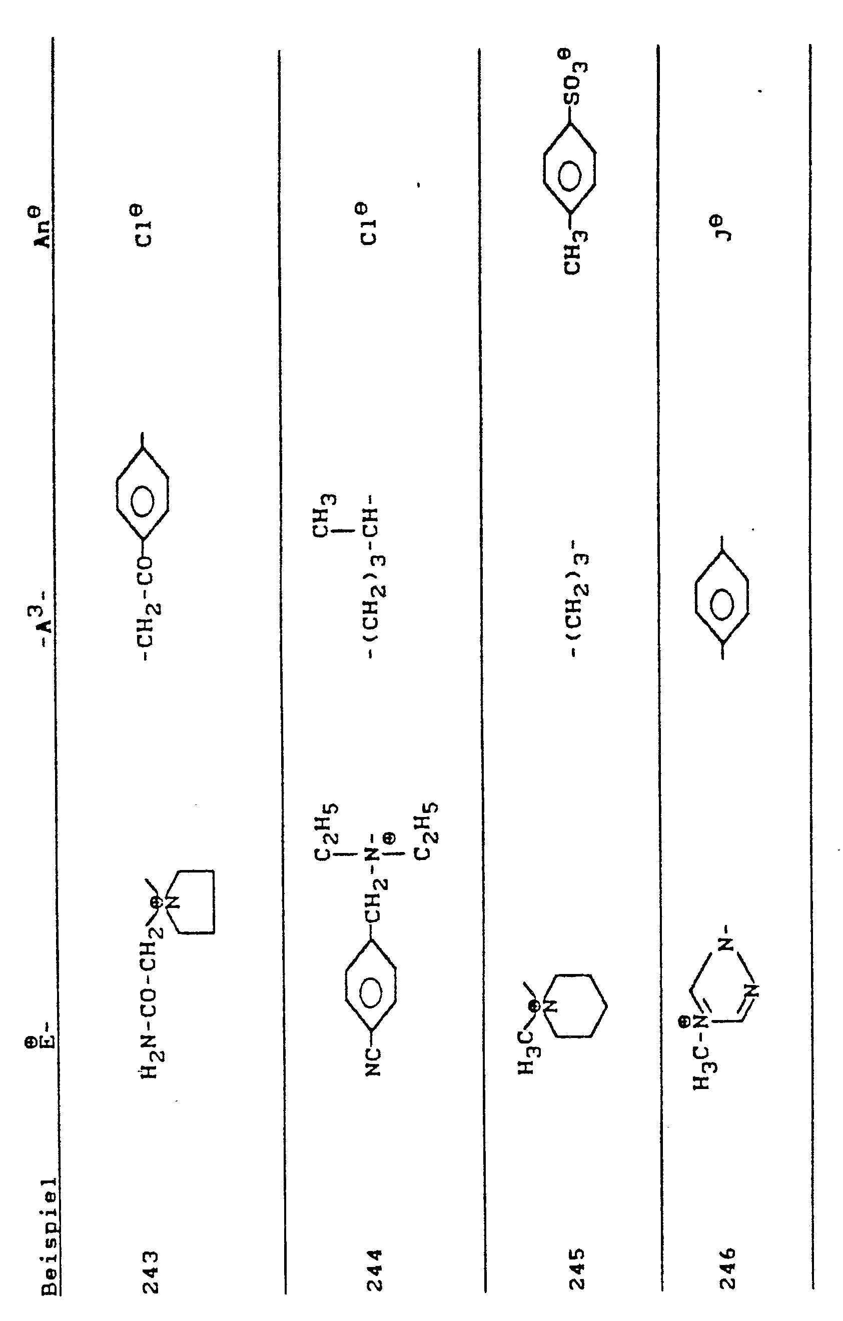

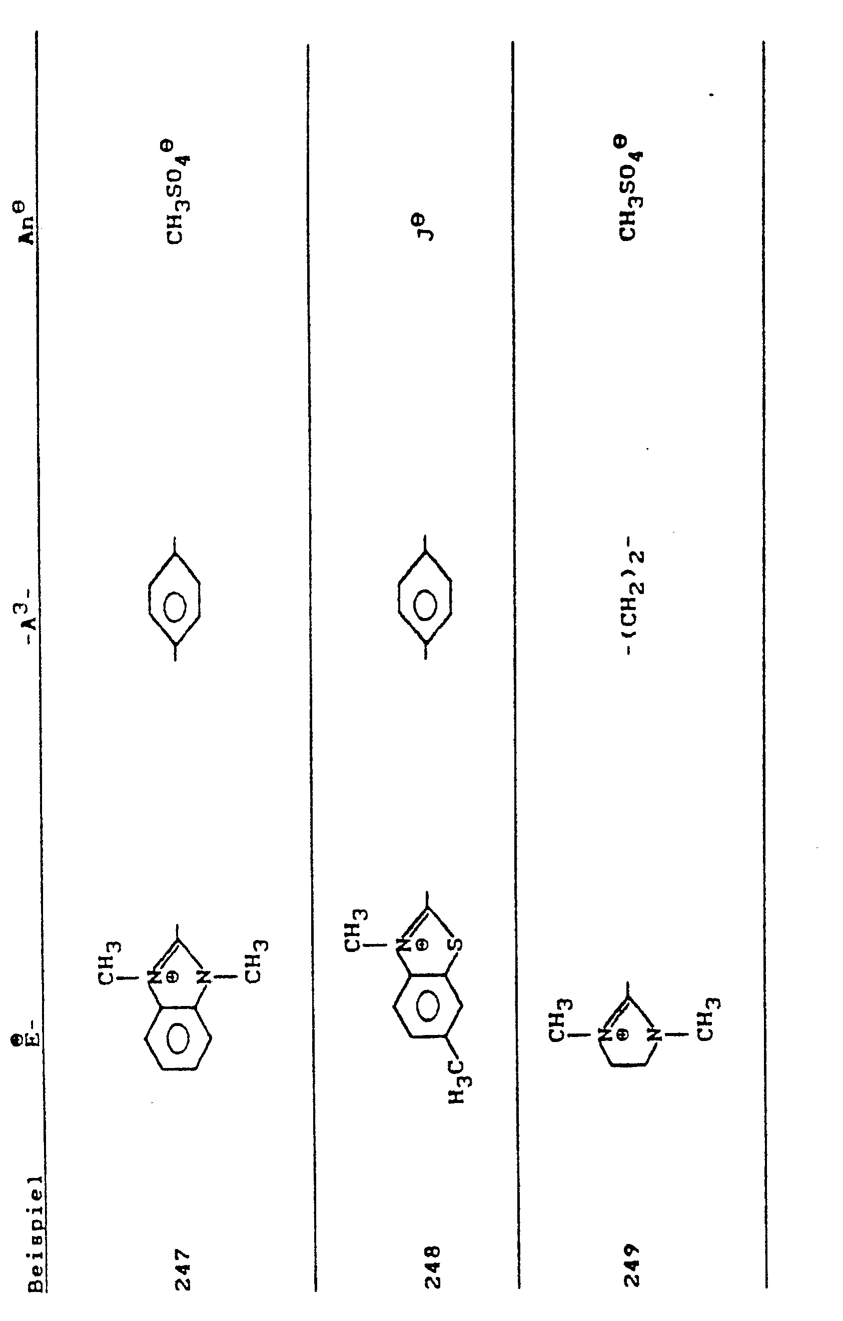

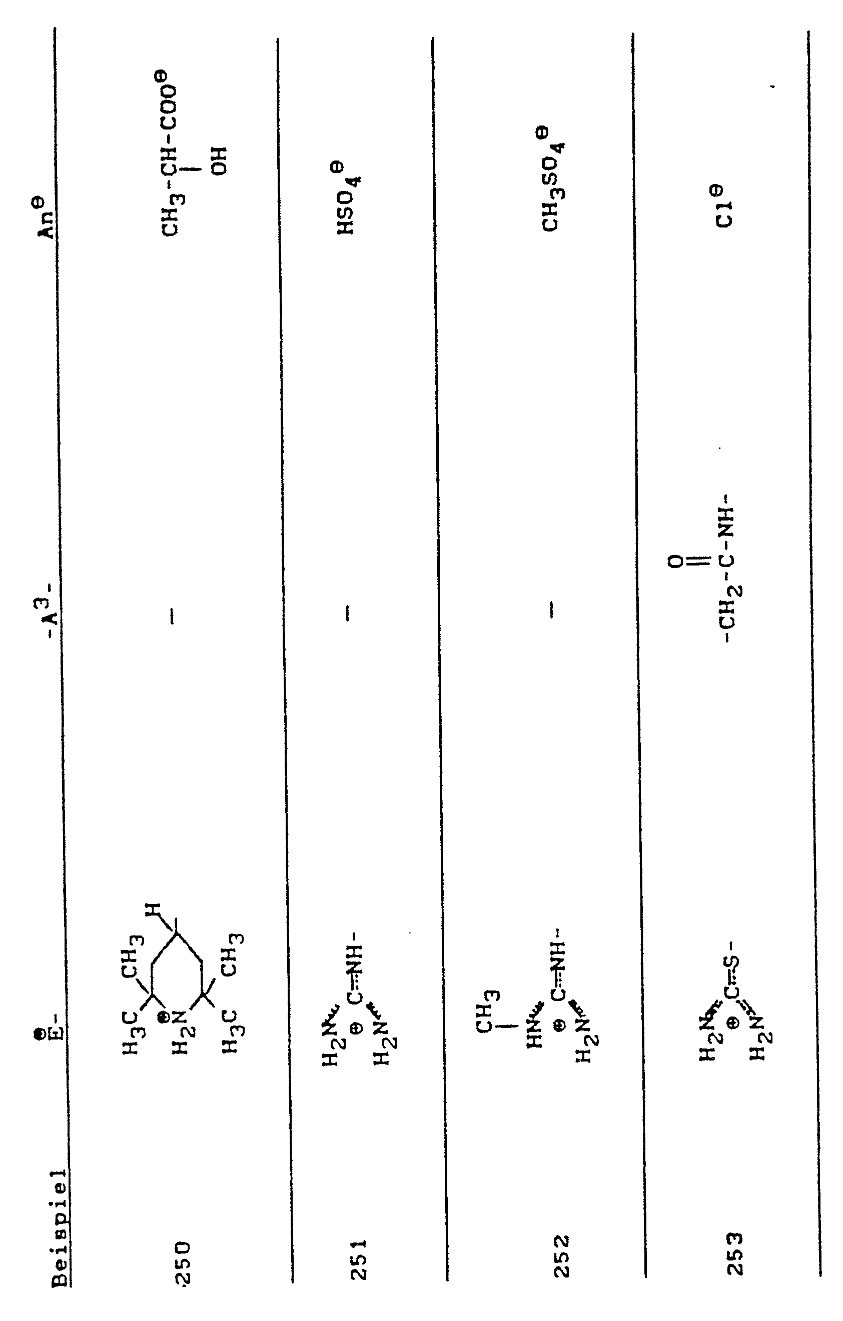

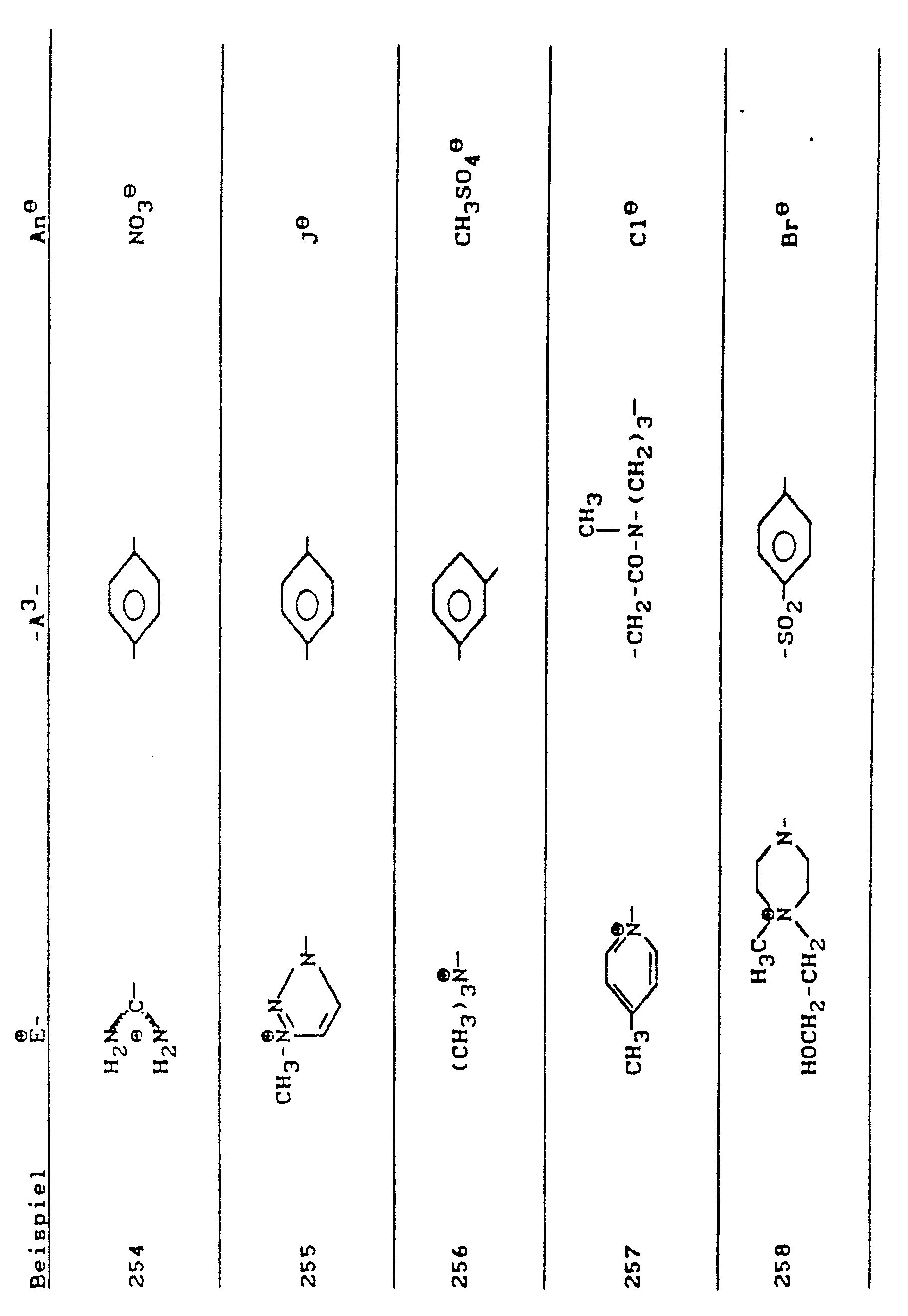

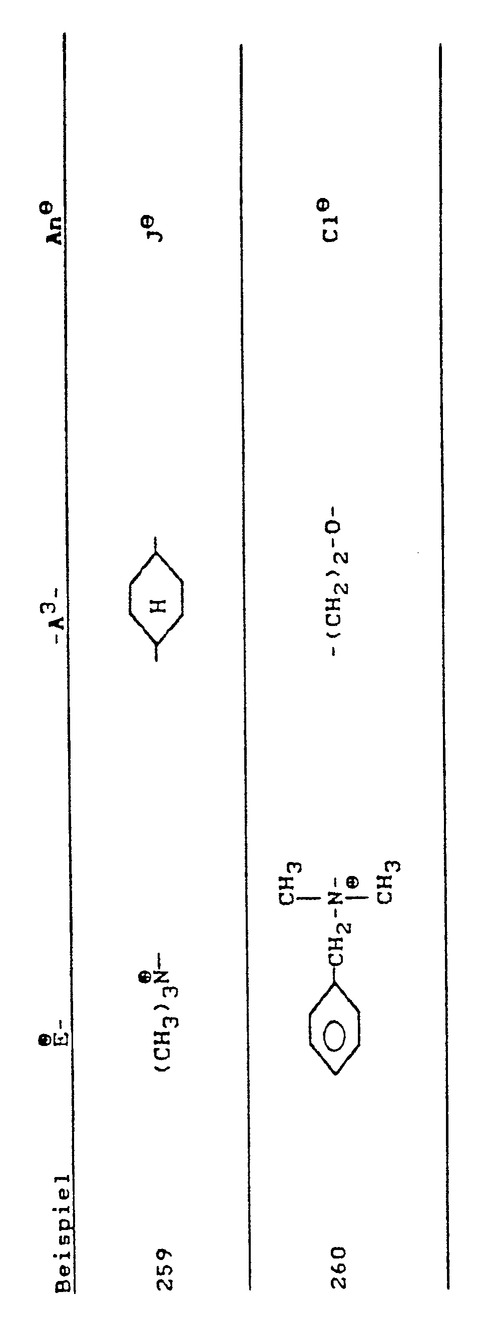

### Beispiel 261

43,6 g Naphthalin-1,4,5,8-tetracarbonsäure-N,N'-bis-(3-dimethylaminopropyl)-diimid (0,1 Mol) und 35 g 4,4'-Bis-(chloracetamidophenyl)-methan (0,1 Mol, Herst. s. Beispiel 1) werden in 1 I Acetonitril 20 h unter Rückfluß zum Sieden erhitzt. Nach dem Abkühlen wird der kristalline Niederschlag abgesaugt, mit Acetonitril gewaschen und bei 50 °C im Vakuum getrocknet. Man erhält 76 g (ca. 97 % der Theorie) einer hochmolekularen Verbindung mit wiederkehrenden Struktureinheiten der Formel 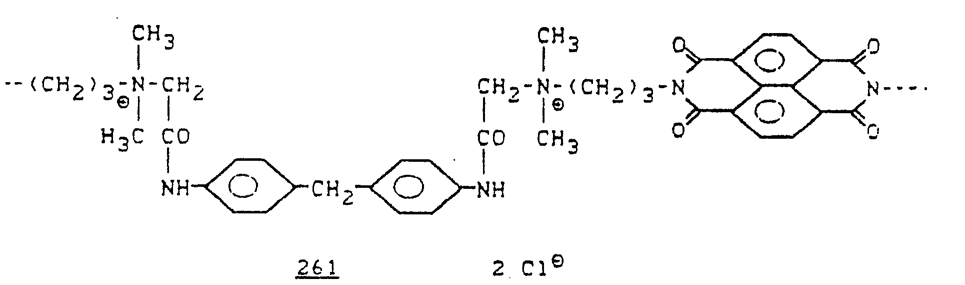 als hochschmelzendes, fast farbloses, wasserlösliches Kristallpulver. Die Substanz ist dünnschichtchromatographisch praktisch frei von Ausgangskomponenten (Kieselgel, Laufmittel wie für Verb. 1 ). RF-Wert ca. 0.

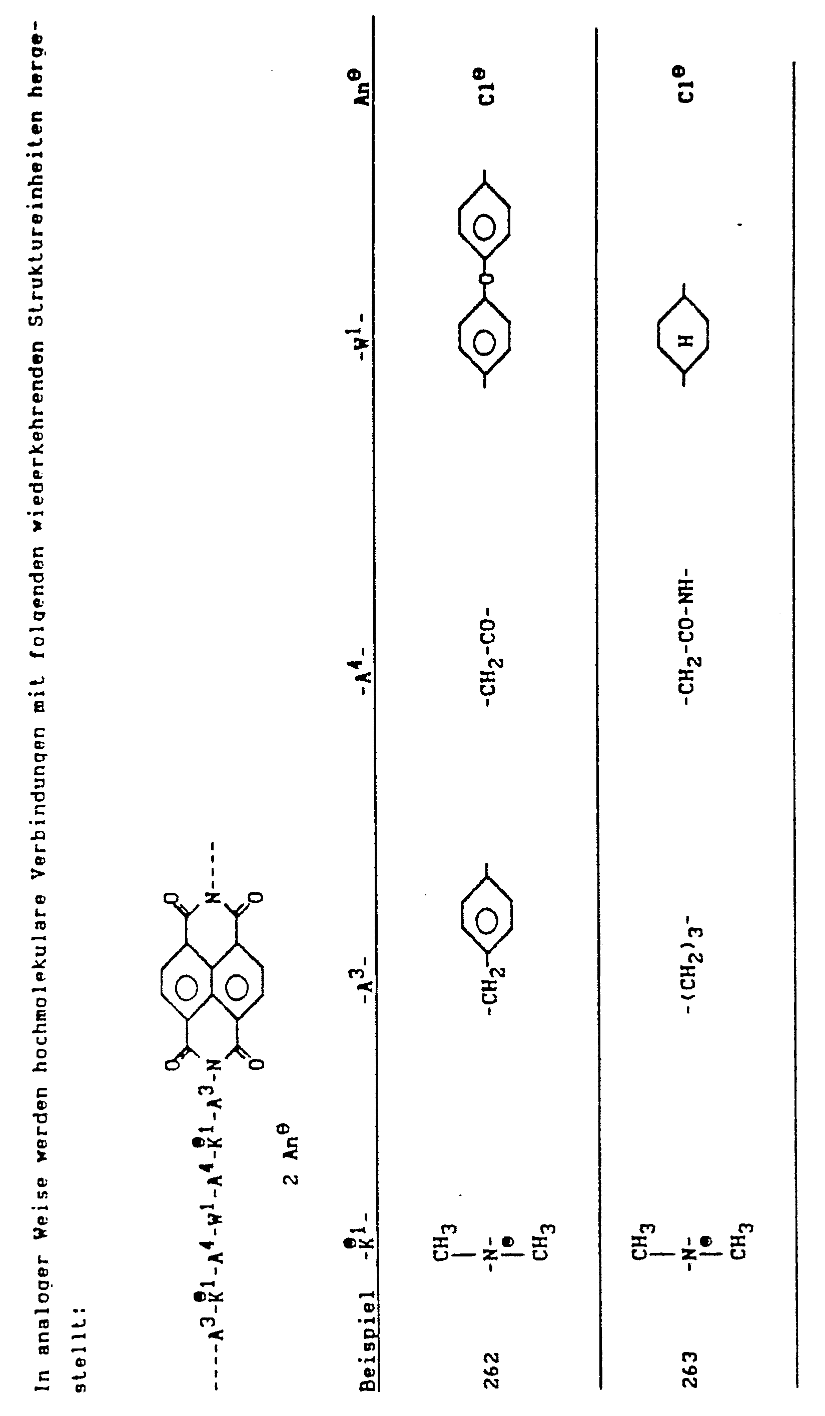

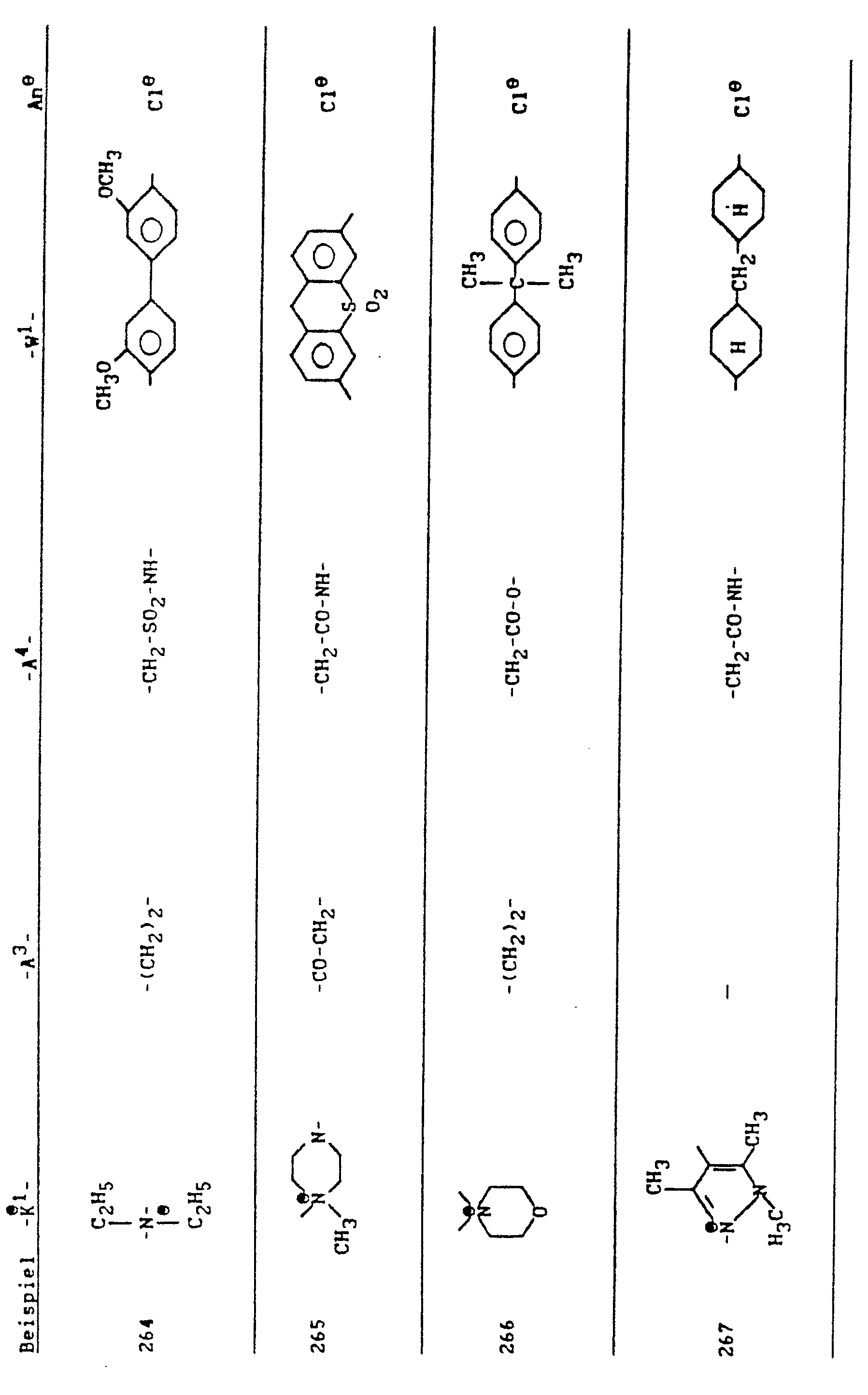

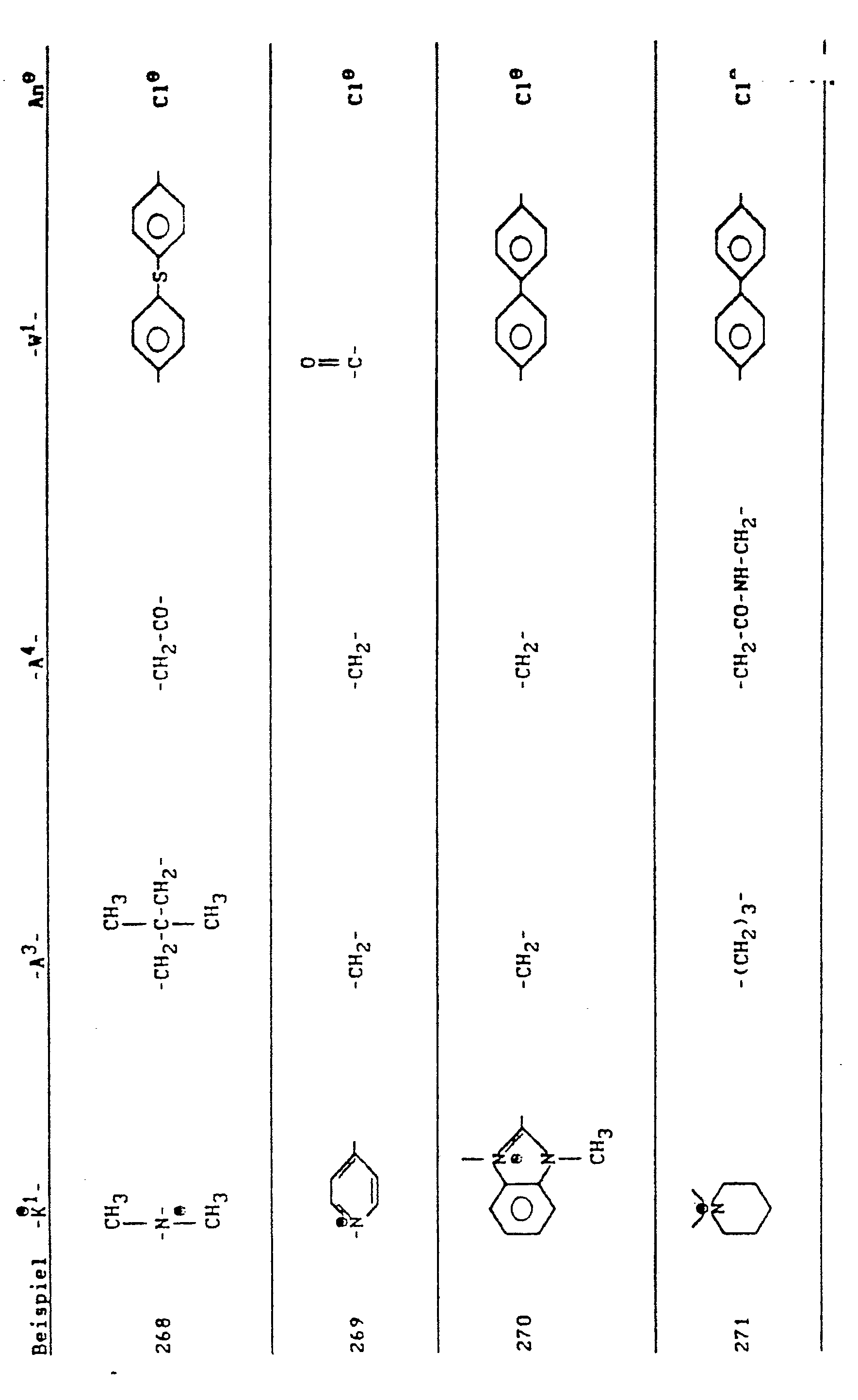

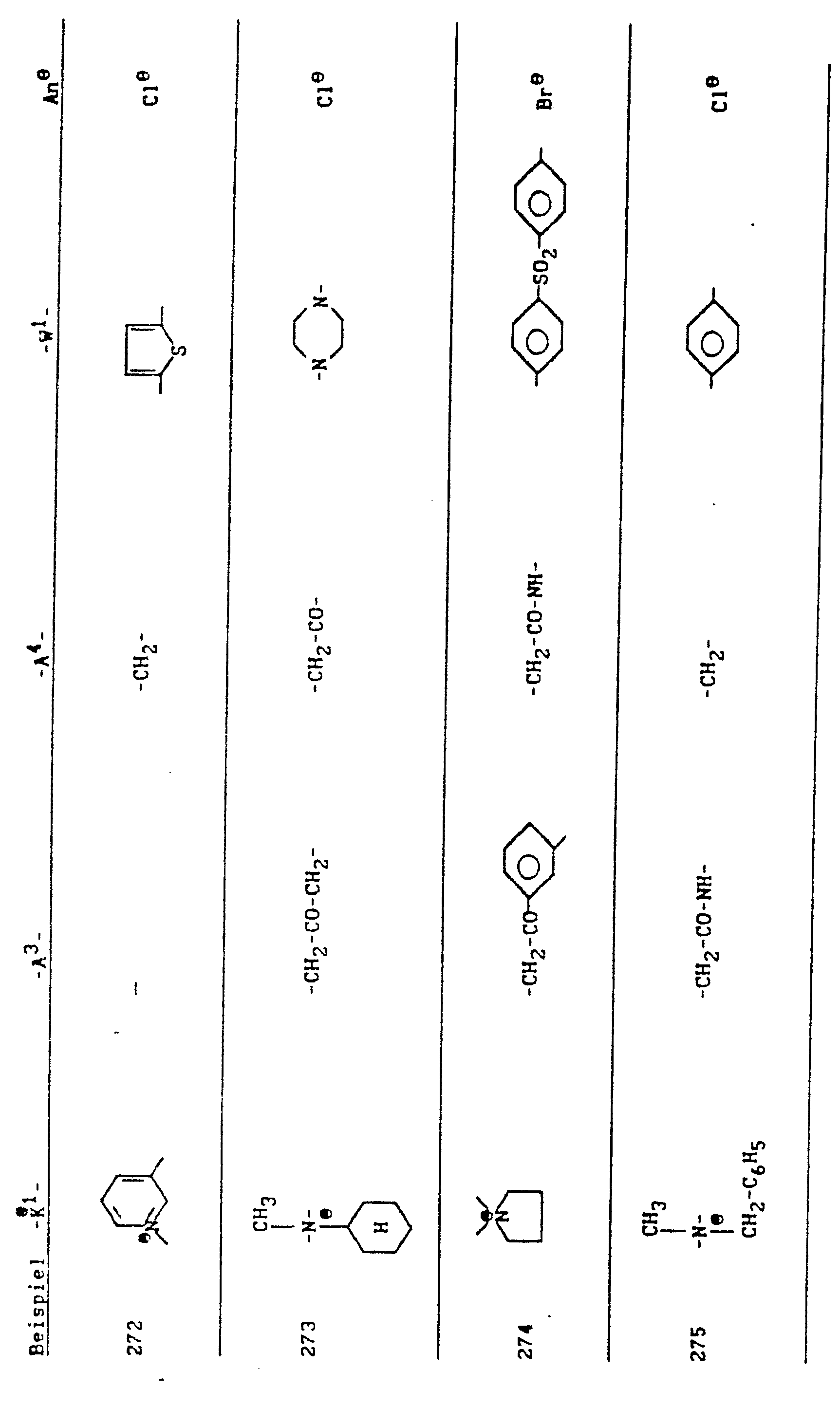

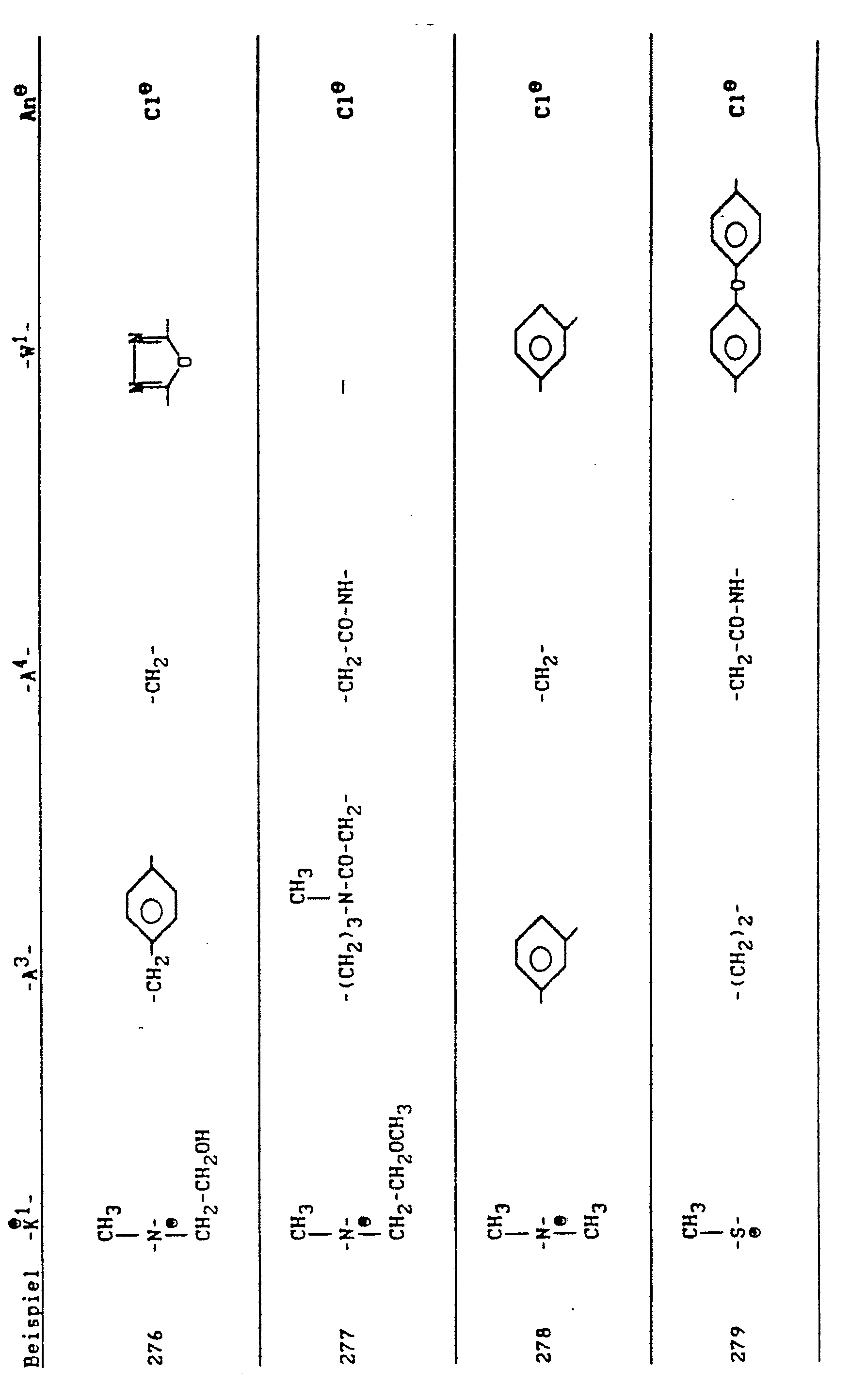

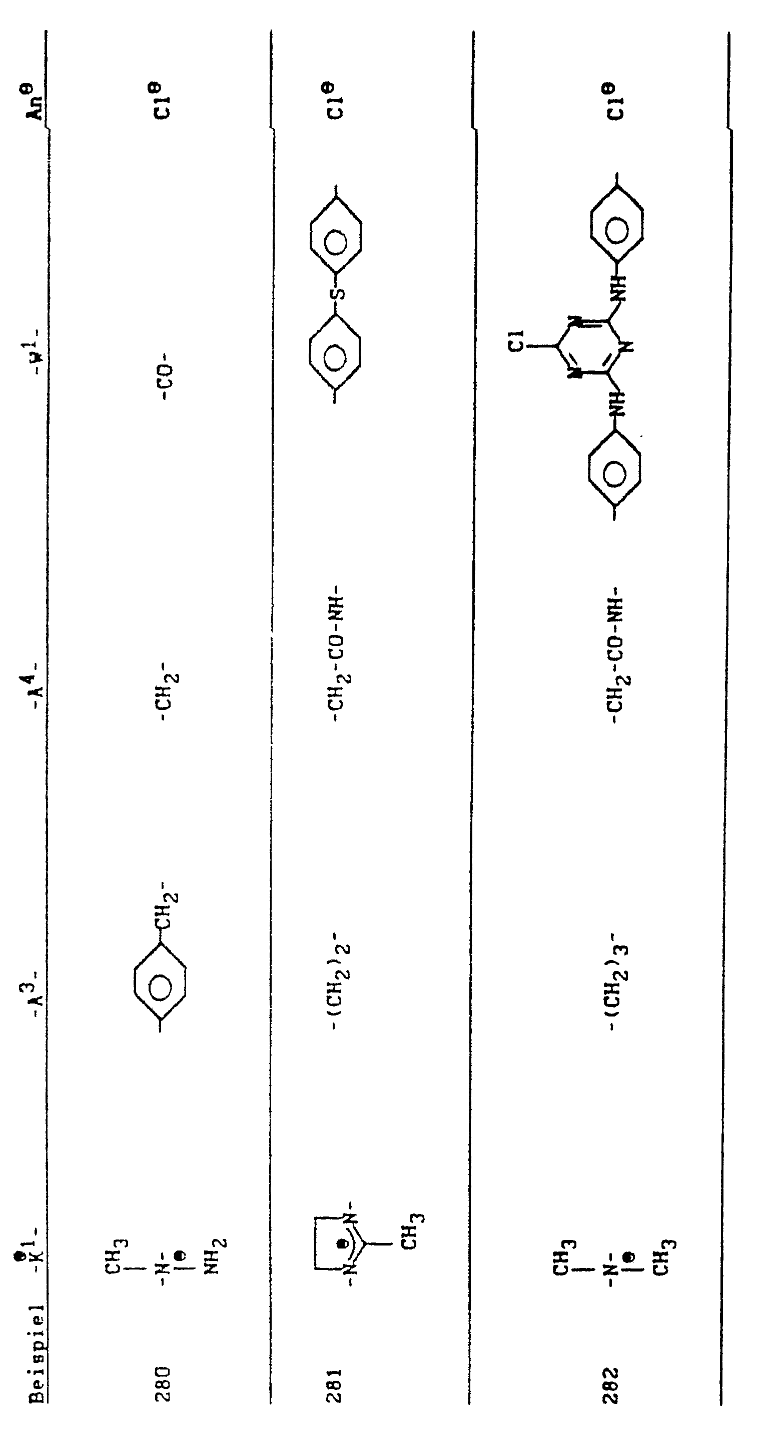

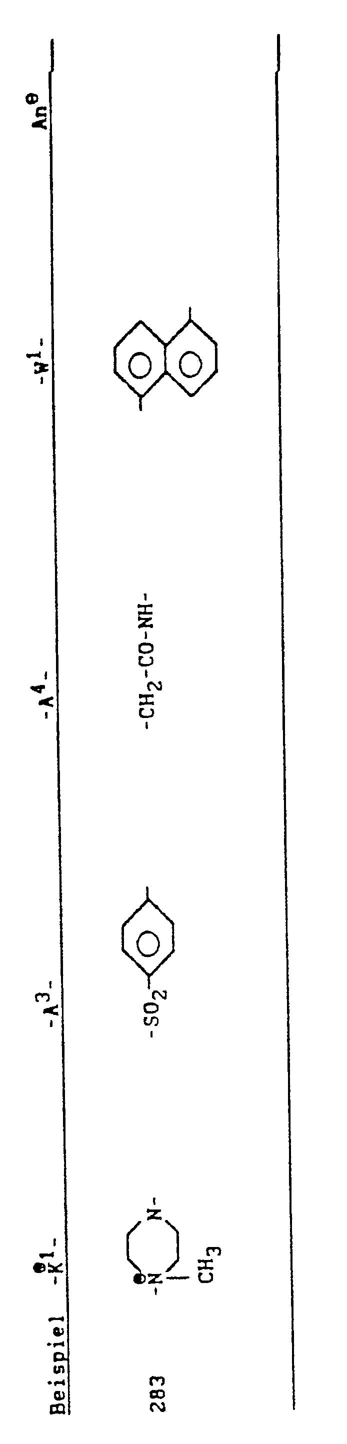

### Beispiel 284

53,6 g Naphthalin-1,4,5,8-tetracarbonsäure-dianhydrid (0,2 Mol) werden in 400 ml Eisessig suspendiert, unter Rühren tropfenweise mit 29 g Bis-(3-aminopropyl)-methylamin (0,2 Mol) versetzt, 5 h unter Abdestillieren von ca. 135 ml Essigsäure zum Sieden erhitzt, auf 60⁰ C abgekühlt und mit 650 ml Aceton verrührt. Der kristalline Niederschlag wird abgesaugt, mit Aceton gewaschen und bei 70 °C im Vakuum getrocknet. Man erhält 86,5 g (99 % der Theorie) einer hochmolekularen Verbindung mit wiederkehrenden Struktureinheiten der Formel 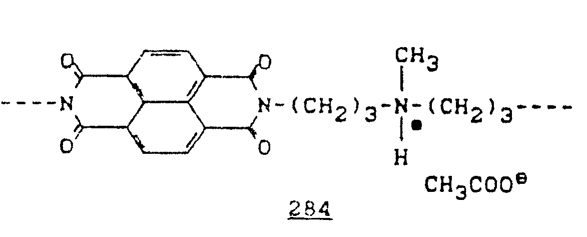 in Form annähernd farbloser, wasserlöslicher, hochschmelzender Kristalle, die dünnschichtchromstographisch praktisch frei von Ausgangskomponenten ist (Kieselgel, Laufmittel wie für Verbindung 1 ). RE-Wert ca. 0.

Durch Lösen in 2 I Wasser bei Raumtemperatur, Filtrieren von wenig Ungelöstem, Alkalischstellen des Filtrats mit 25 %igem wäßrigen Ammoniak, Absaugen des farblosen Niederschlages, Waschen mit Wasser und Trocknen bei 70° C im Vakuum wird Verbindung 284 in die Base überführt (92 % der Theorie).

### Beispiel 285

37,7 g der vorstehend genannten Base (0,1 Mol, bezogen auf die wiederkehrende Struktureinheit der hochmolekularen Verbindung) werden in 300 ml Dimethylformamid bei 70 - 75° C tropfenweise mit 14 g Dimethylsulfat versetzt und 6 h bei 70 C verrührt. Nach dem Abkühlen auf Raumtemperatur wird der kristalline Niederschlag abgesaugt, mit Aceton gewaschen und bei 70° C im Vakuum getrocknet. Man erhält 48,5 g (96 % der Theorie) einer hochmolekularen Verbindung mit wiederkehrenden Struktureinheiten der Formel 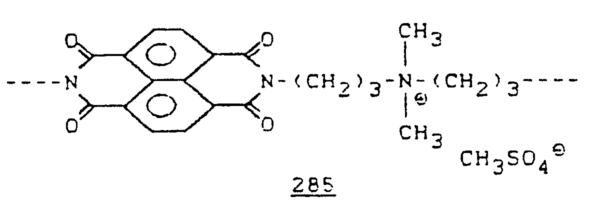 als farbloses, wasserlösliches, hochschmelzendes Kristallpulver.

Analog 285 werden hochmolekulare Verbindungen mit folgenden wiederkehrenden Struktureinheiten der Formel 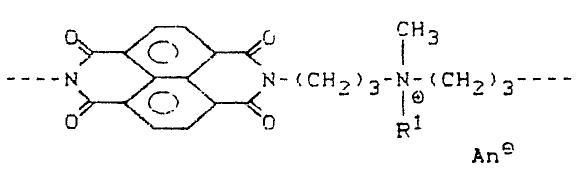 hergestellt:

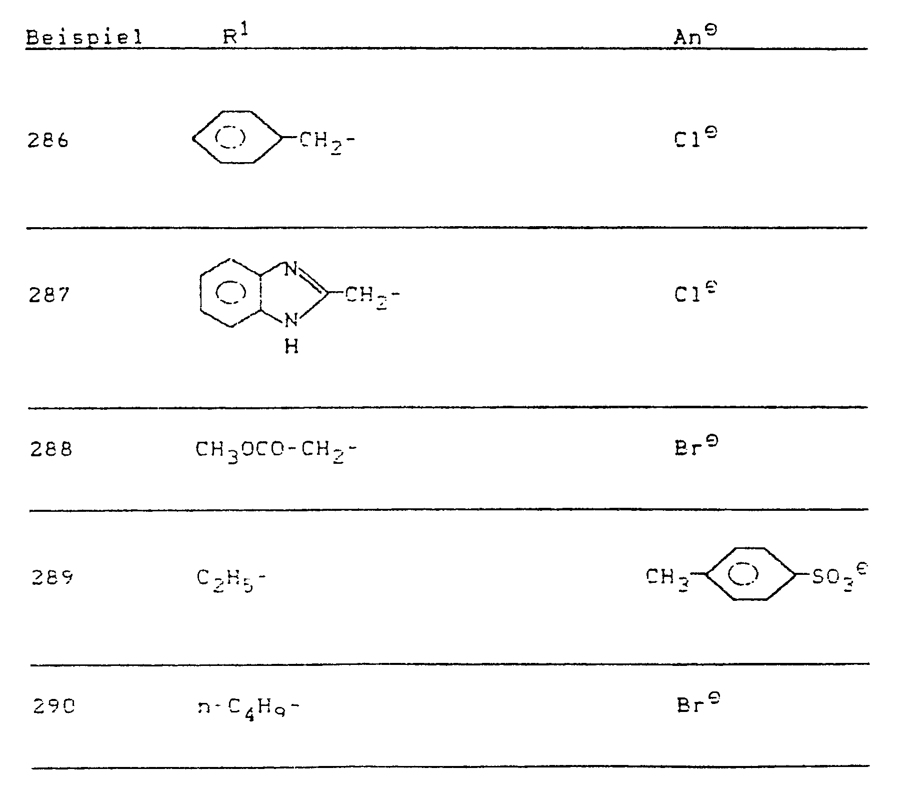

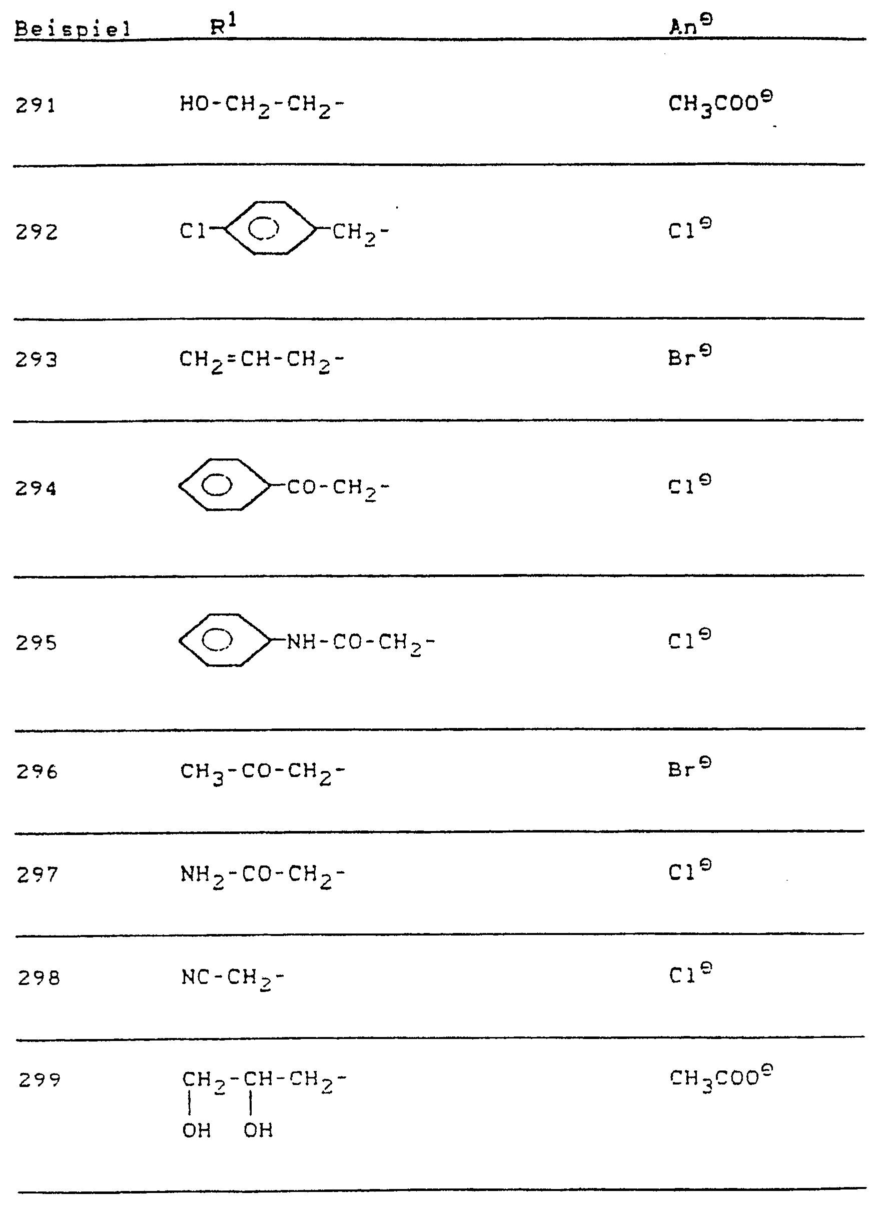

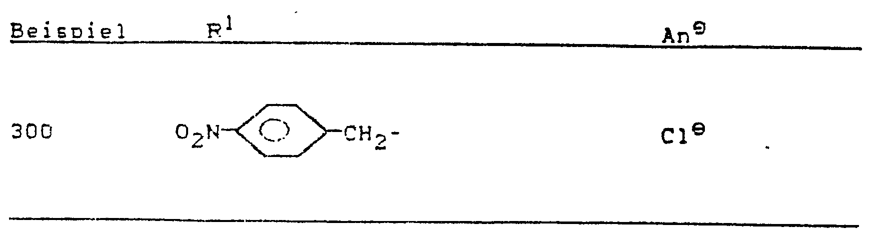

### Beispiel 301

42,7 g N-Phenyl-N-(3-dimethylaminopropyl)-naphthalin-1,4,5,8-tetracarbonsäure-diimid (0,1 Mol) und 18 g 4,4'-Bis-(chloracetamidophenyl)-methan (0,05 Mol) werden in 1 I Dimethylformamid 15 h auf 100°C erhitzt und abgekühlt. Der kristalline Niederschlag wird abgesaugt, mit Isopropanol gewaschen und bei 60° C im Vakuum getrocknet. Man erhält 45 g (74 % der Theorie) Verbindung der Formel 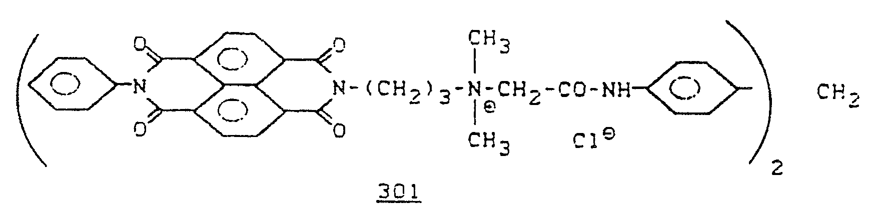 als Praktisch farbloses, hochschmelzendes, wasserlösliches Kristallpulver. Die Verbindung ist dünnschichtchromatographisch Praktisch rein (Kieselgel , Laufmittel wie bei Verbindung 1 ). RF-Wert: 0,14.

### Herstellung der Ausgangsverbindungen

### Naphthalin-1,4,5,8-tetracarbonsäure-monophenylimid

53,6 g Naphthalin-1,4,5,8-tetracarbonsäure-dianhydrid (0,2 Mol) oder 60,8 g Naphthalin-1,4,5,8-tetracarbonsäure (0,2 Mol) werden in 1 I Wasser unter Zusatz von 84 g Triethylamin (0,9 Mol) bei 90° C gelöst, unter Rühren mit 18,6 g Anilin (0,2 Mol) versetzt und bei 90 C durch tropfenweise Zugabe von ca. 20 ml Eisessig auf pH 6 gestellt. Man läßt ca. 8 h bei 90⁰ C nachrühren, wobei man den pH-Wert durch weiteres Zutropfen von Eisessig (insgesamt 6 ml) zwischen 7 und 6 hält. Man kühlt auf Raumtemperatur, filtriert von wenig Ungelöstem und stellt das Filtrat durch Zutropfen von ca. 160 ml konz. Salzsäure auf pH 1. Der kristalline Niederschlag wird abgesaugt, mit Wasser gewaschen, bis der Ablauf pH 5 zeigt und bei 70° C im Vakuum getrocknet. Man erhält 63 g (87 % der Theorie) Naphthalin-1,4,5,8-tetracarbonsäure-monophenylimid vom Schmelzpunkt >300° C als fast farbloses Kristallpulver.

### Naphthalin-1,4,5,8-tetracarbonsäure-N-phenyl-N'-(3-dime thylaminopropyl)-diimid

54,2 g Naphthalin-1,4,5,8-tetracarbonsäure-monophenylimid (0,15 Mol) werden in 1,3 I Toluol suspendiert, mit 1 g Eisessig unter Rühren und Sieden am Wasserabscheider tropfenweise mit 15,3 g 1-Amino-3-dimethylamino-propan (0,15 Mol) versetzt, weitere 10 h am Wasserabscheider zum Sieden erhitzt und abgekühlt. Der kristalline Niederschlag wird abgesaugt, mit Toluol gewaschen, aus 800 ml siedendem Dimethylformamid umkristallisiert, mit Ethanol gewaschen und bei 70° C im Vakuum getrocknet. Man erhält 54 g (84 % der Theorie) Naphthalin-1,4,5,8-tetracarbonsäure-N-phenyl-N'-(3-dimethylaminopropyl)-diimid als gelbliches Kristallpulver vom Schmelzpunkt 270° C.

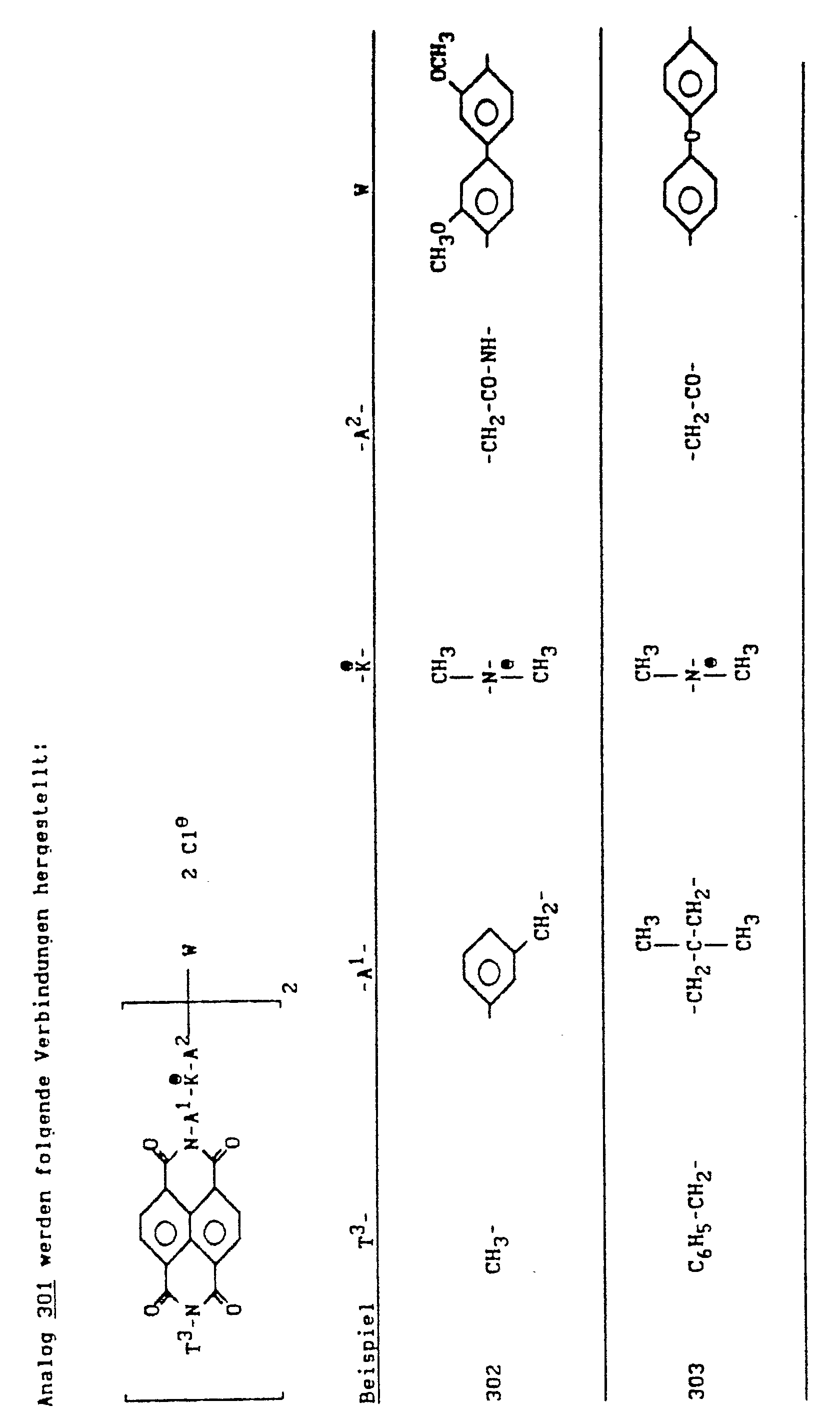

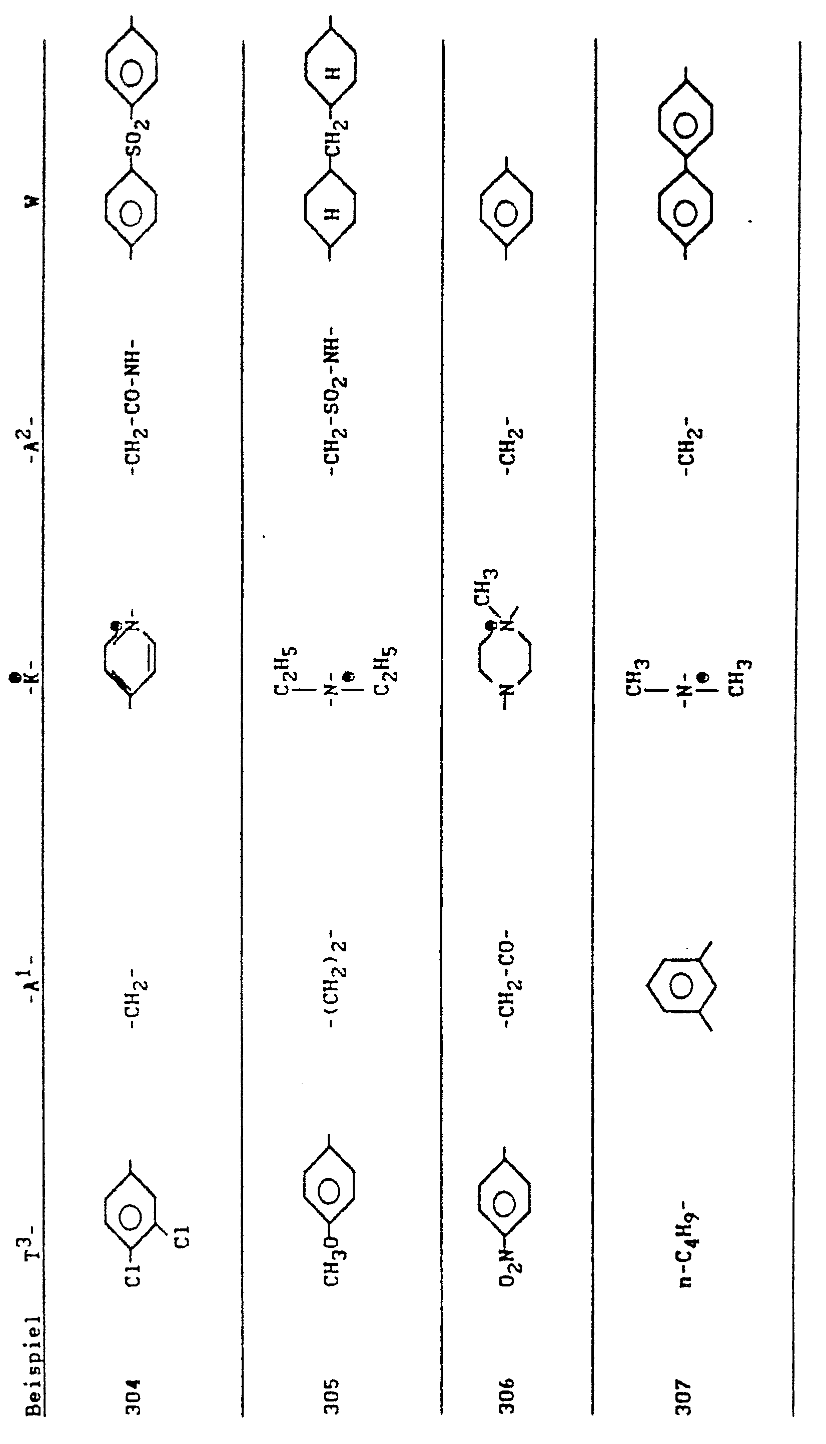

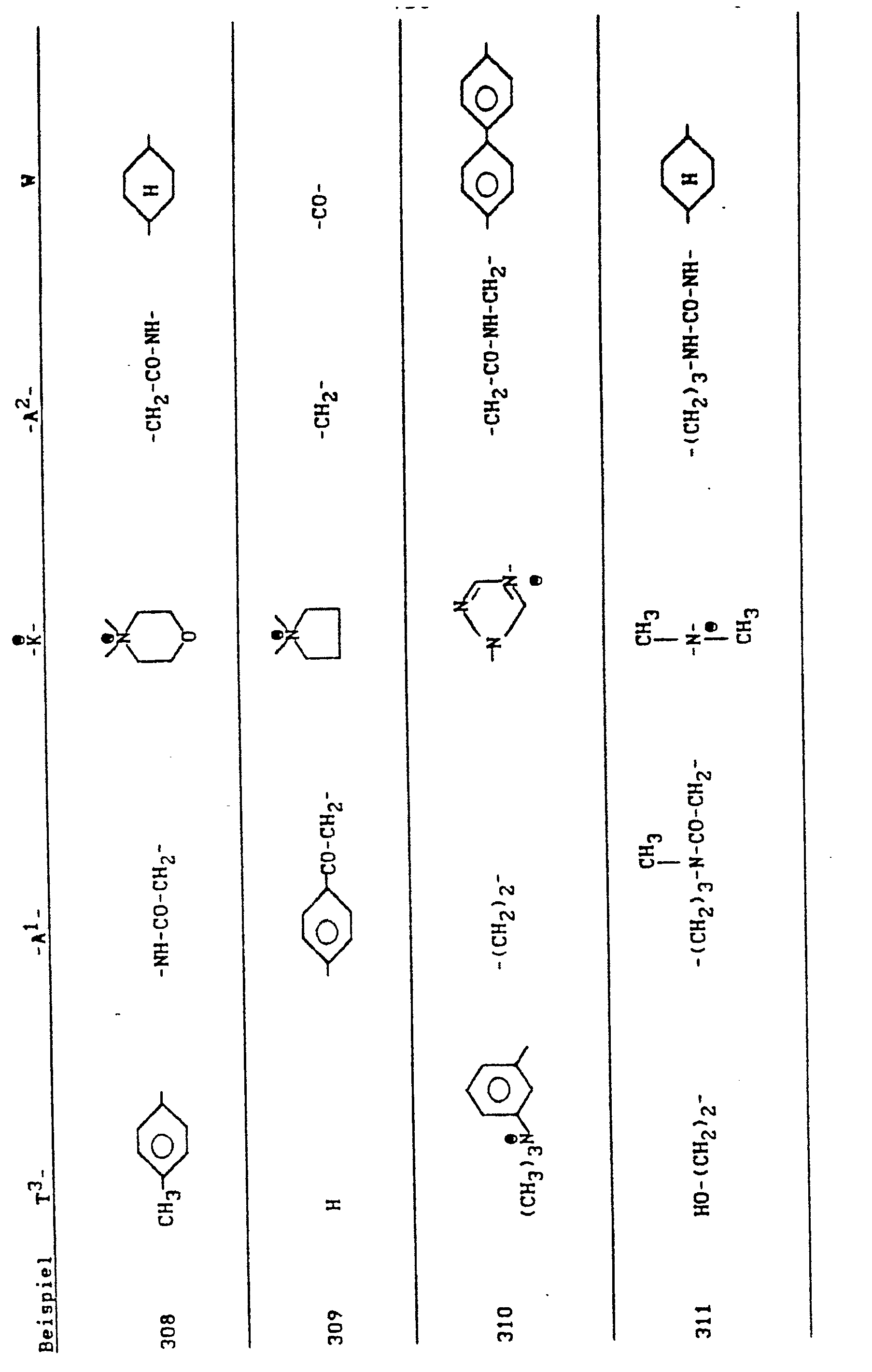

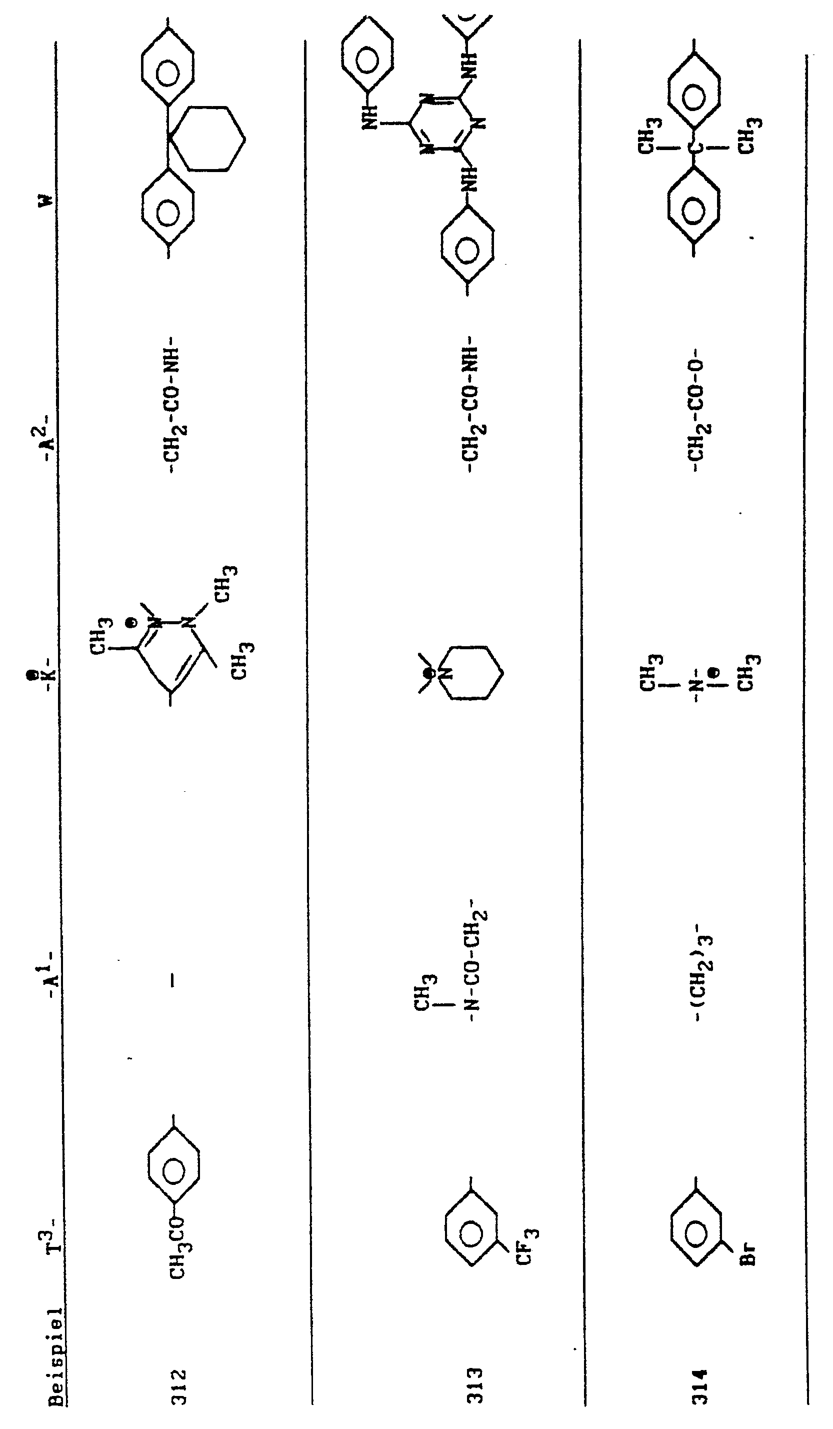

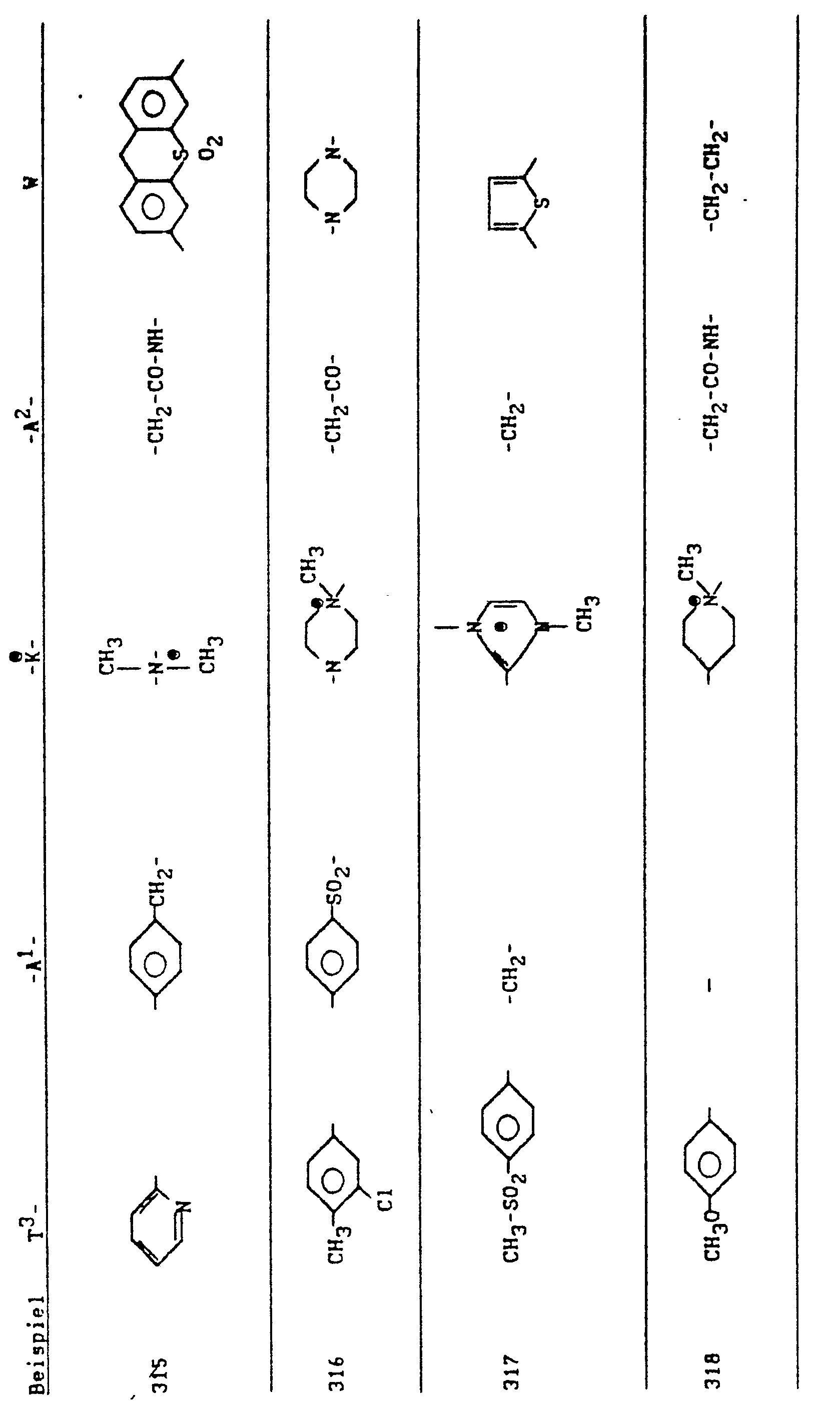

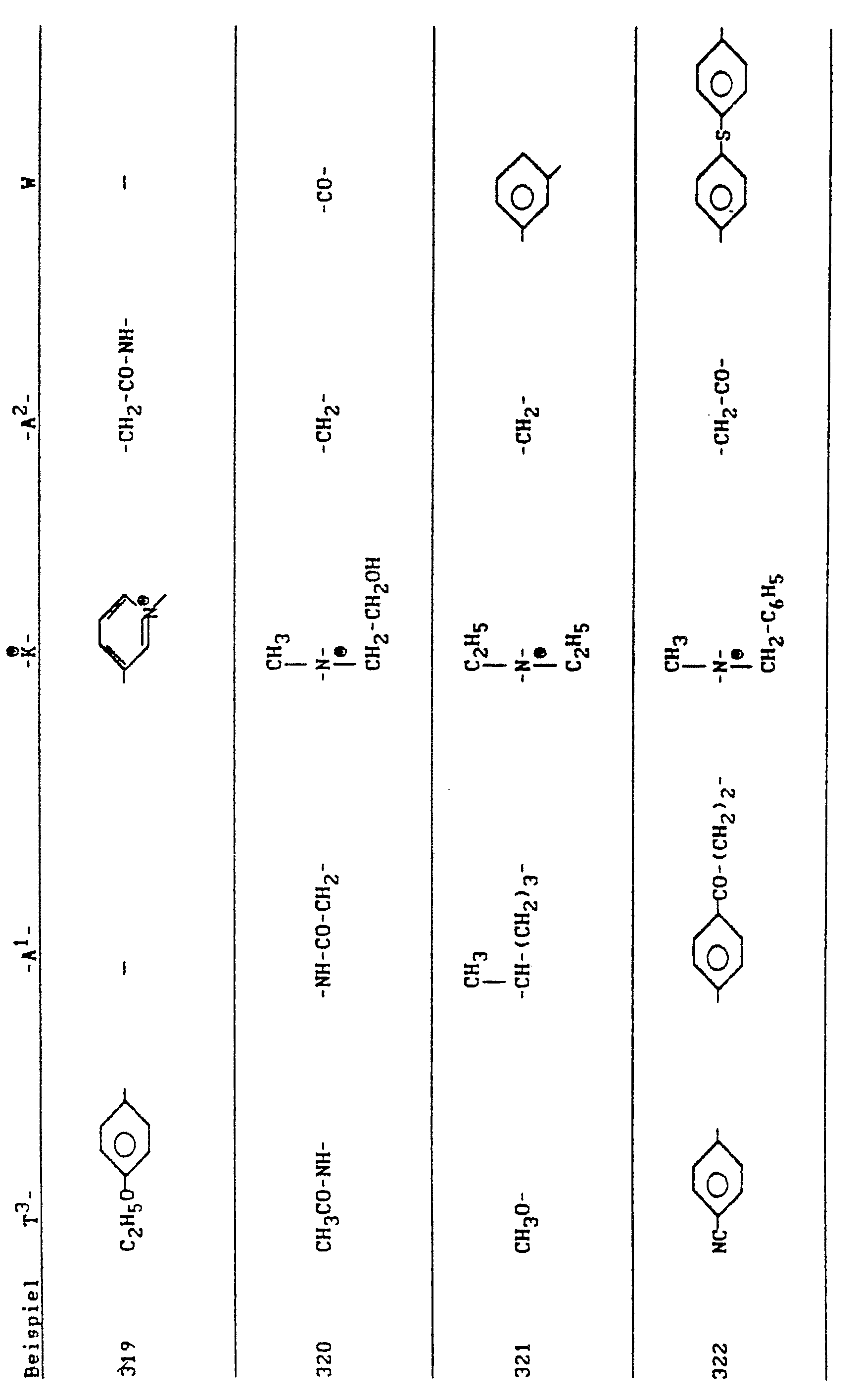

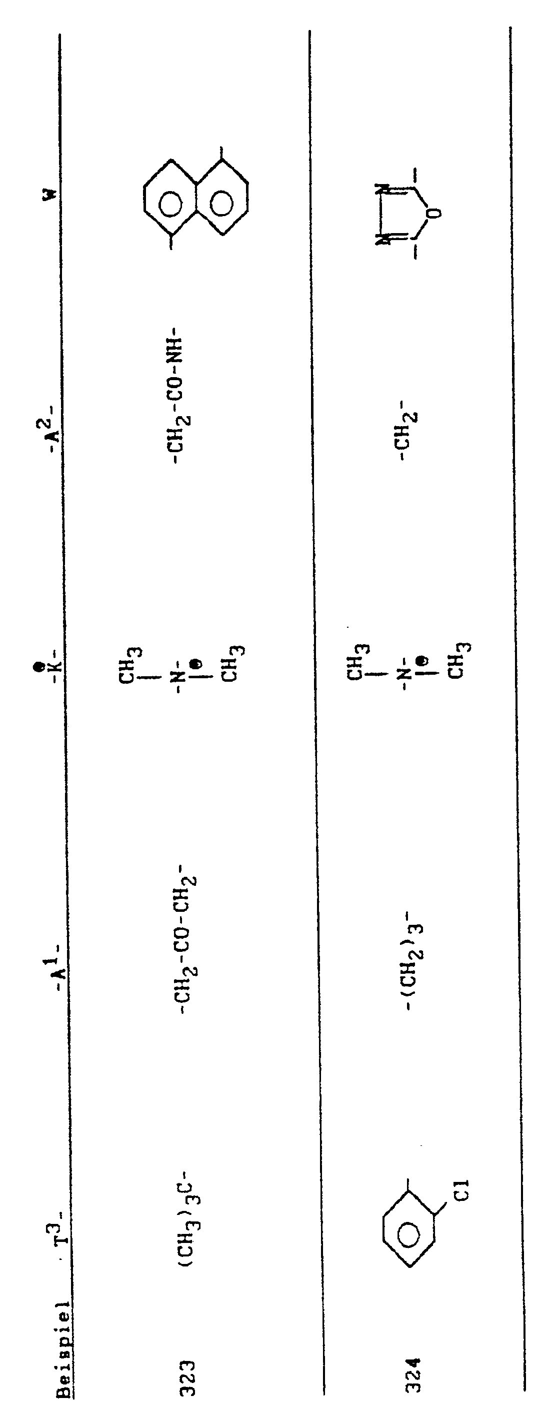

Ebenfalls analog 301 werden aus den Edukten 325E-329E hochmolekulare Verbindungen mit den wiederkehrenden Struktureinheiten 325-329 hergestellt:

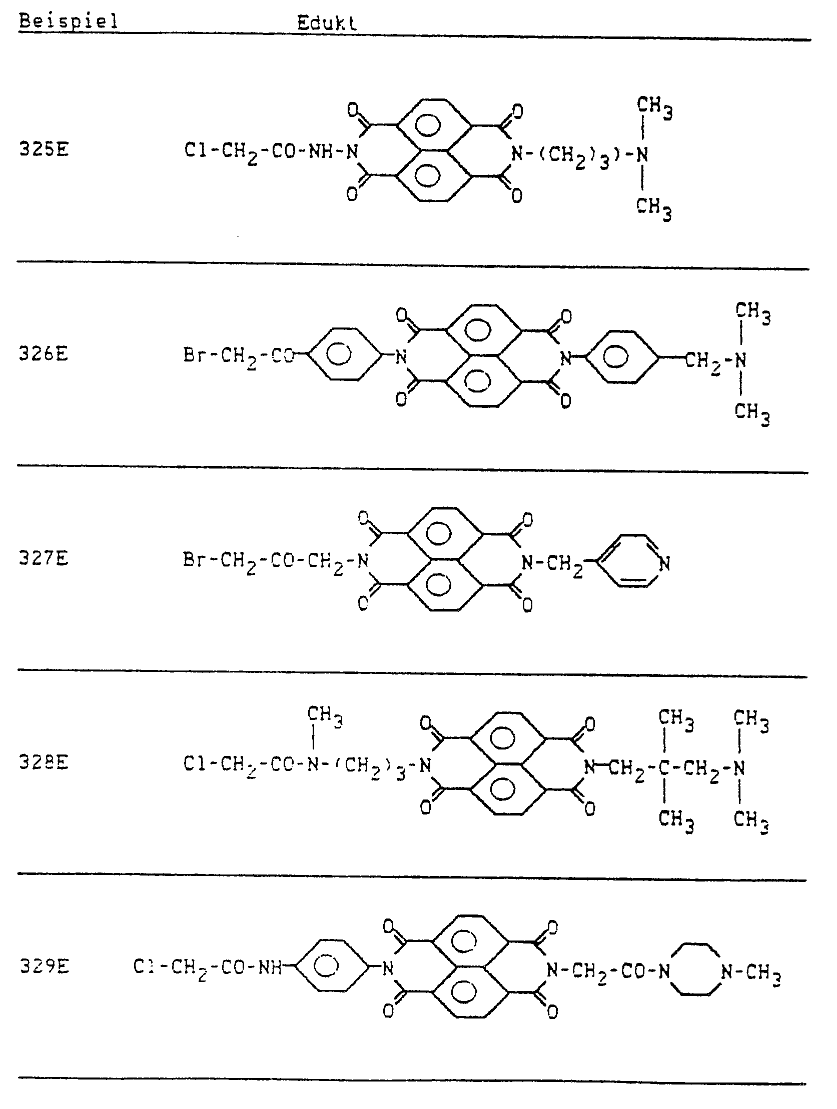

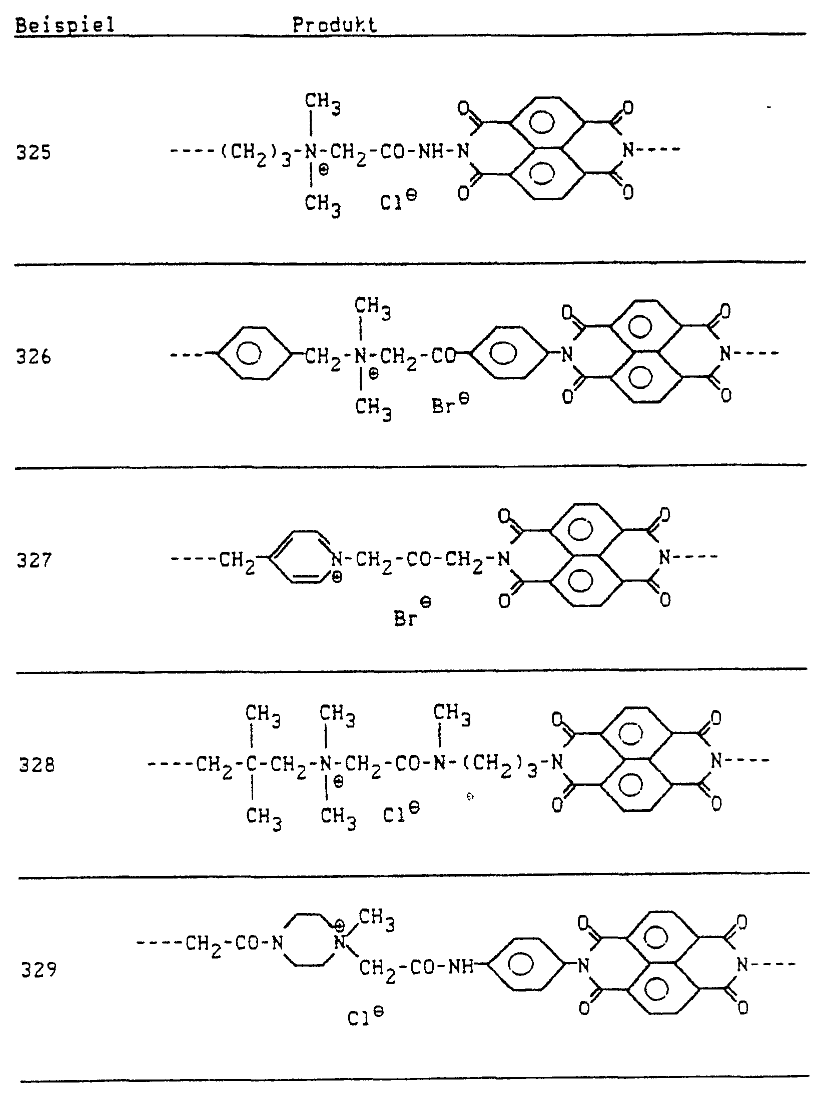

### Beispiel 330

41,8 g Naphthalin-1,4,5,8-tetracarbonsäure-N-(4-w-dimethylaminobenzyl)-monoimid (0,1 Mol), hergestellt analog dem in Beispiel 301 beschriebenen Monophenylimid, werden in 600 ml Toluol suspendiert und mit 1 g Eisessig versetzt. Anschließend tropft man unter Rühren und Sieden am Wasserabscheider 10,2 g 1-Amino-3-dimethylamino-propan (0,1 Mol) hinzu und läßt die Mischung noch 7 h am Wasserabscheider kochen, klärt heiß mit 2 g Tonsil, filtriert und dampft das Filtrat am Rotationsverdampfer zur Trockene ein. Man erhält als Rückstand 43 g (ca. 89 % der Theorie) Naphthalin-1,4,5,8-tetracarbonsäure-N-(4-ω-dimethy- laminobenzyl)-N'-(3-dimethylaminopropyl)-diimid als gelbes Kristallpulver vom Schmelzpunkt 90° C; m/e = 484 (M^{⊕}).

29 9 dieser Verbindung (0,06 Mol) und 13 g Amidosulfonsäure (ca. 0,13 Mol) werden in 400 ml Aceton 5 h unter Rückfluß zum Sieden erhitzt und abgekühlt. Man erhält 40 g (ca. 99 % der Theorie) Verbindung der Formel 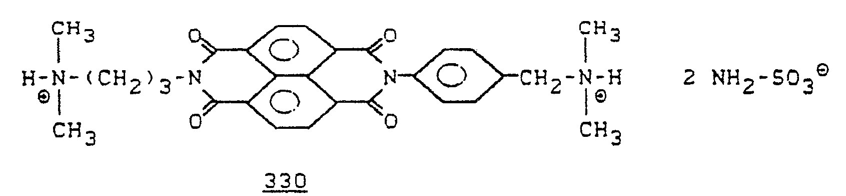 in Form farbloser, wasserlöslicher Kristalle.

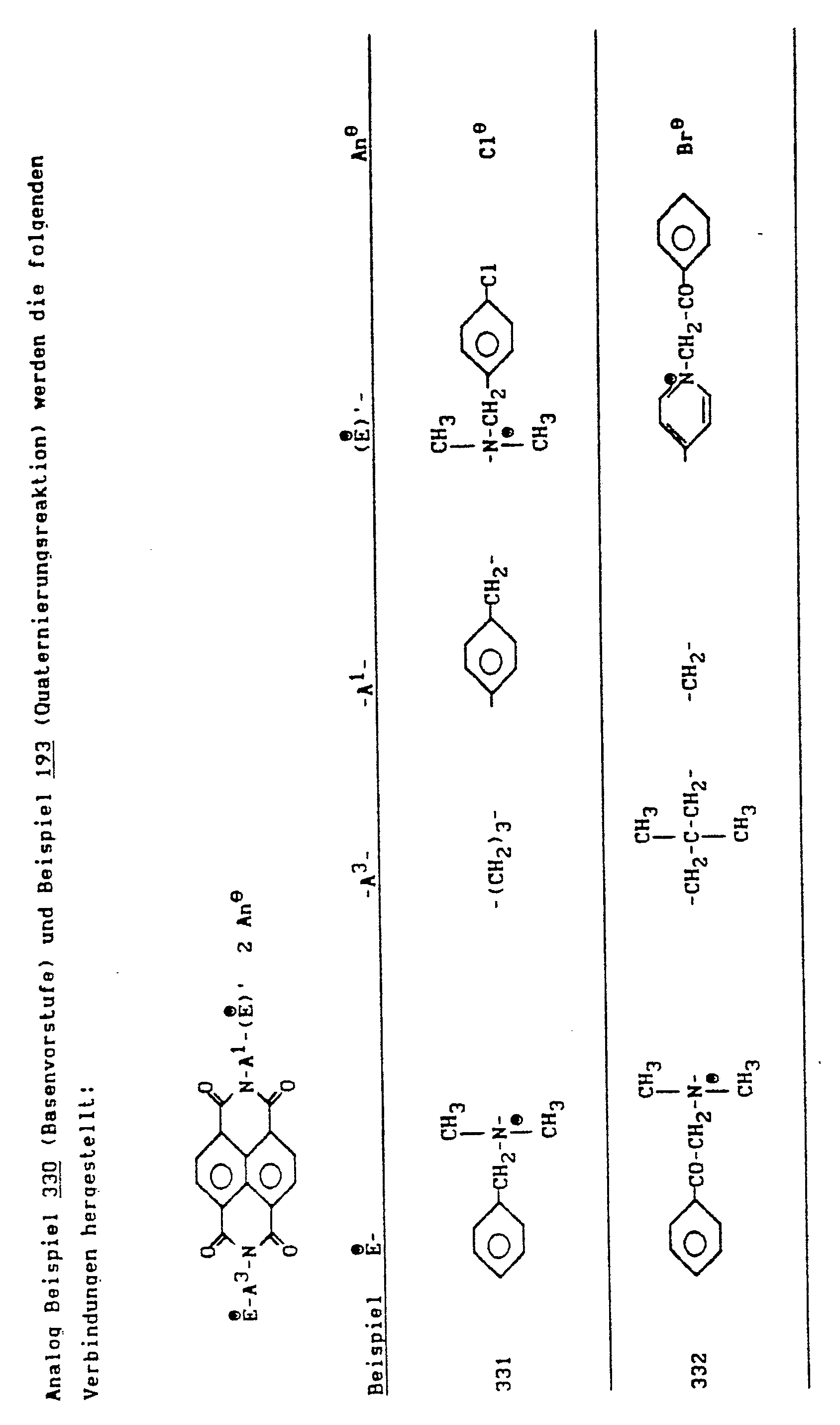

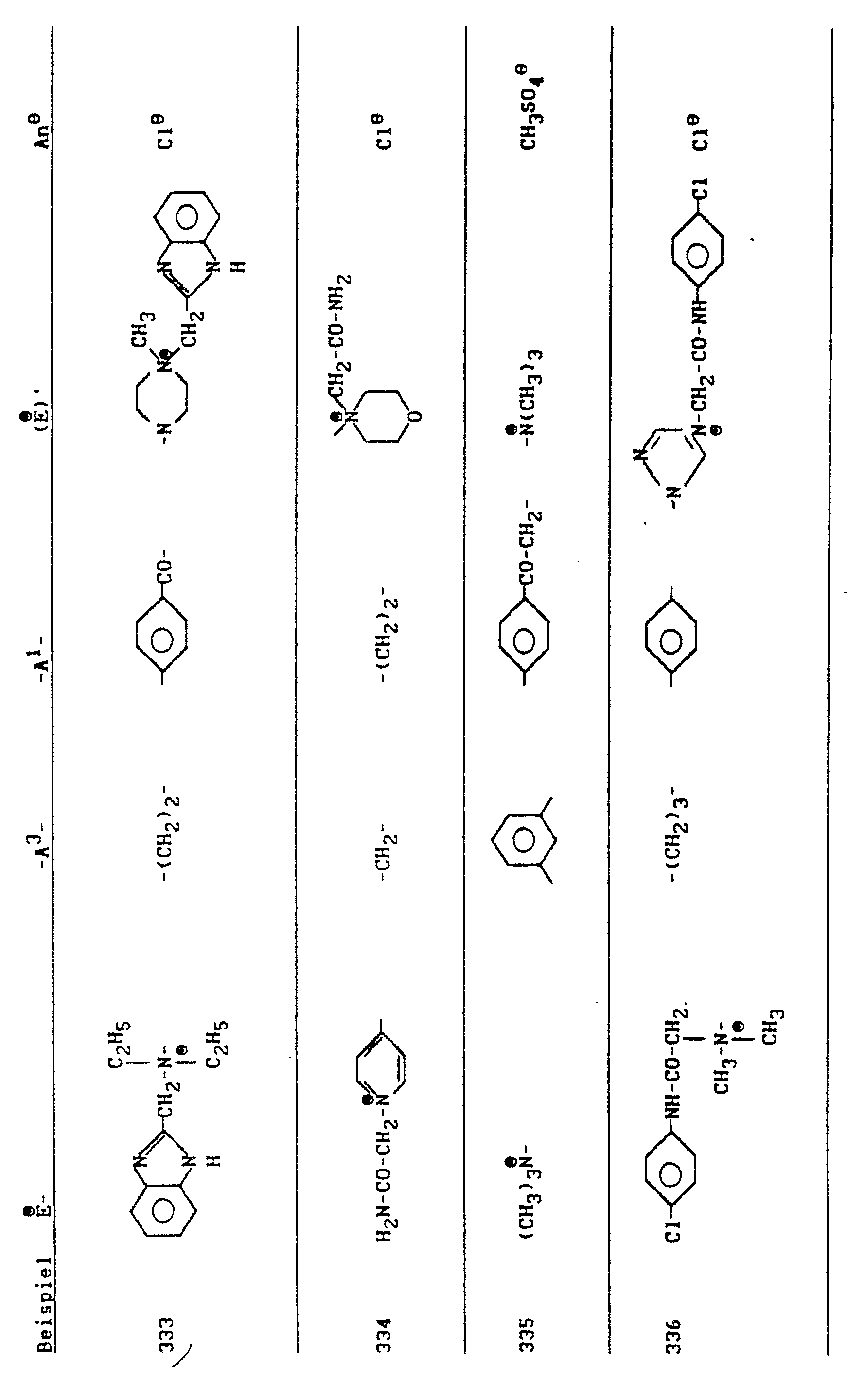

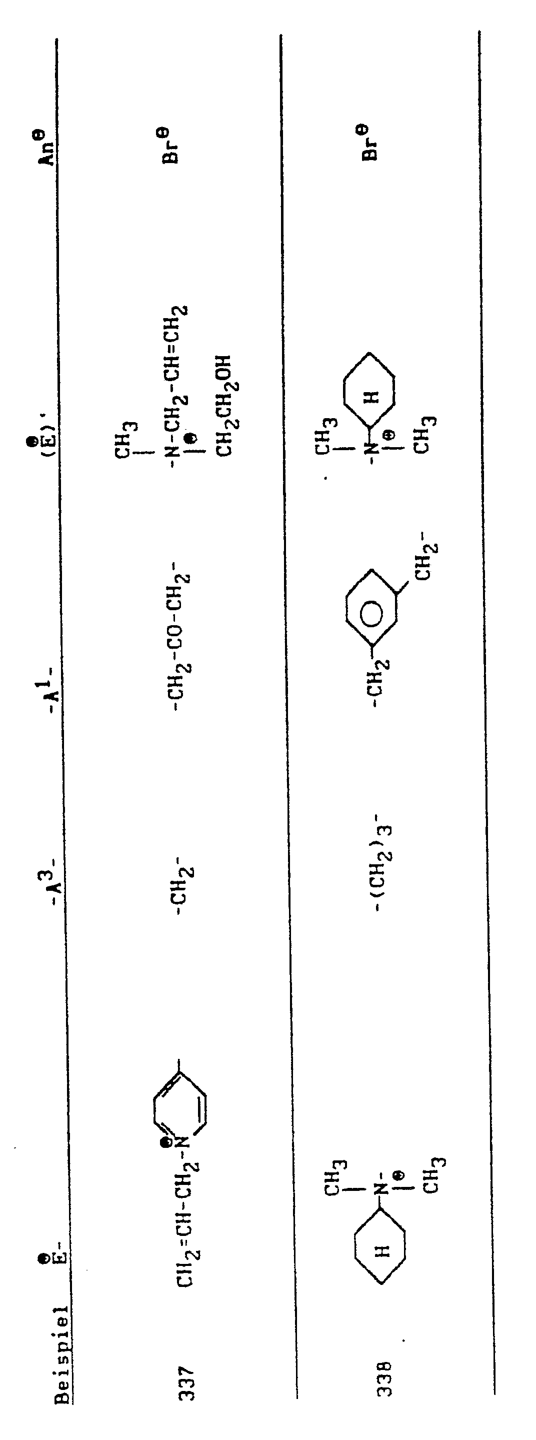

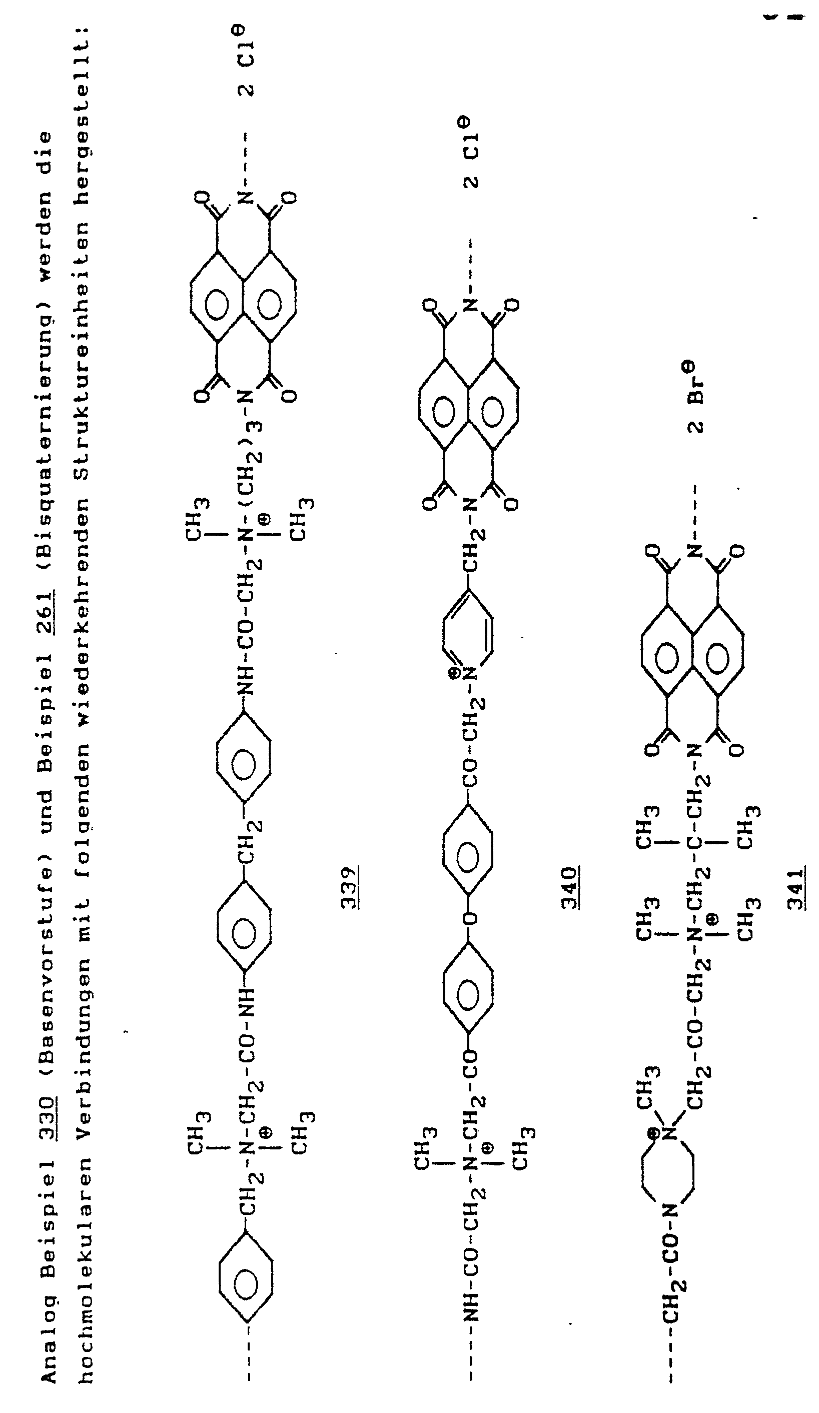

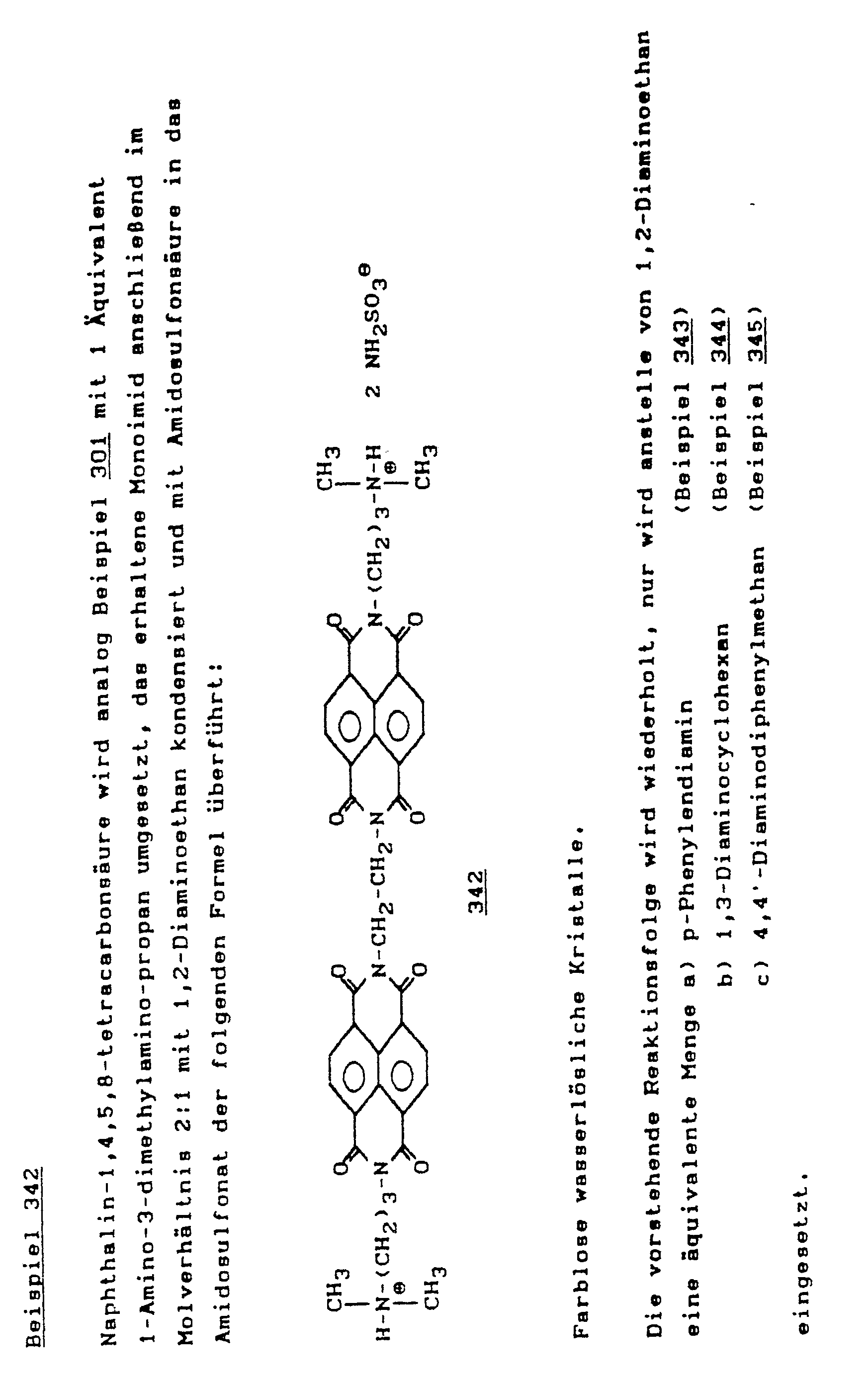

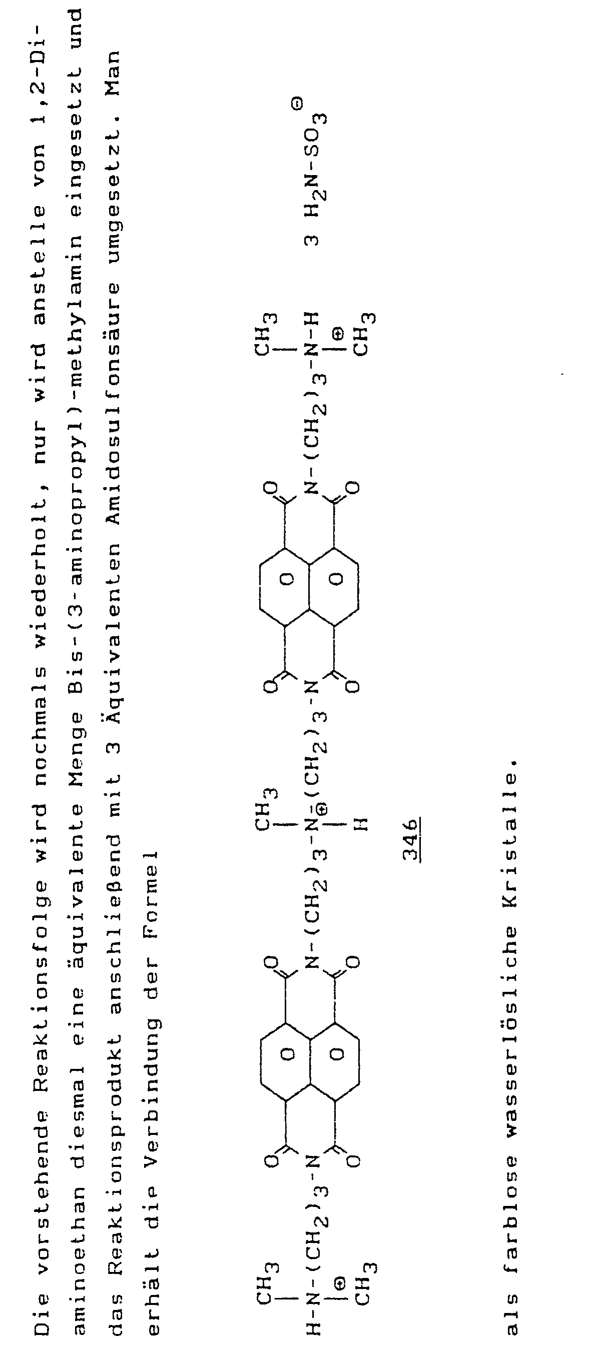

### Beispiel 347

Verbindung 342 wird in Form der freien Base analog Beispiel 193 mit 2 Äquivalenten 4-Chlorbenzylchlorid quaterniert. Man erhält die Verbindung der Formel 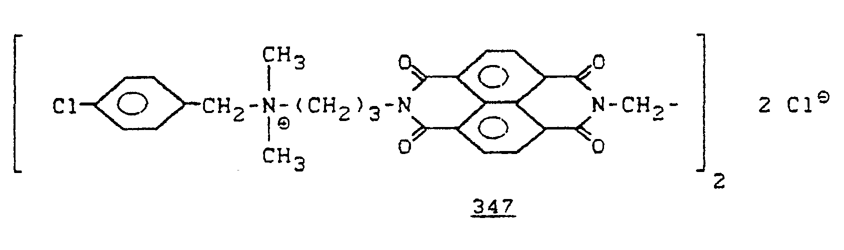 als farblose wasserlösliche Kristalle.

### Beispiel 348

Naphthalin-1,4,5,8-tetracarbonsäure-monophenylimid, dessen Herstellung im Beispiel 301 beschrieben ist, wird analog Beispiel 176 im Molverhältnis 2:1 mit Triethylentetramin kondensiert und die erhaltene Base anschließend analog Beispiel 158 mit 2 Äquivalenten Dimethylsulfat quaterniert. Man erhält die Verbindung der Formel 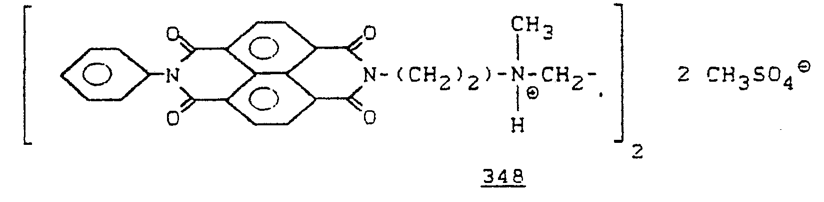 in Form fast farbloser, wasserlöslicher Kristalle.

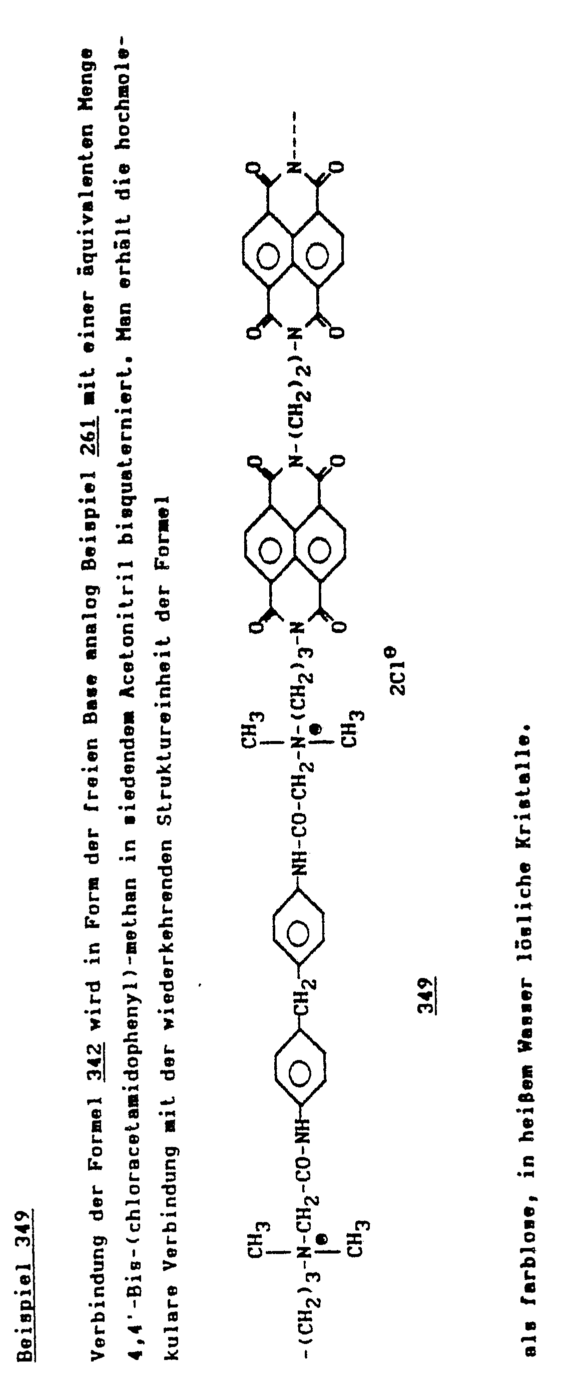

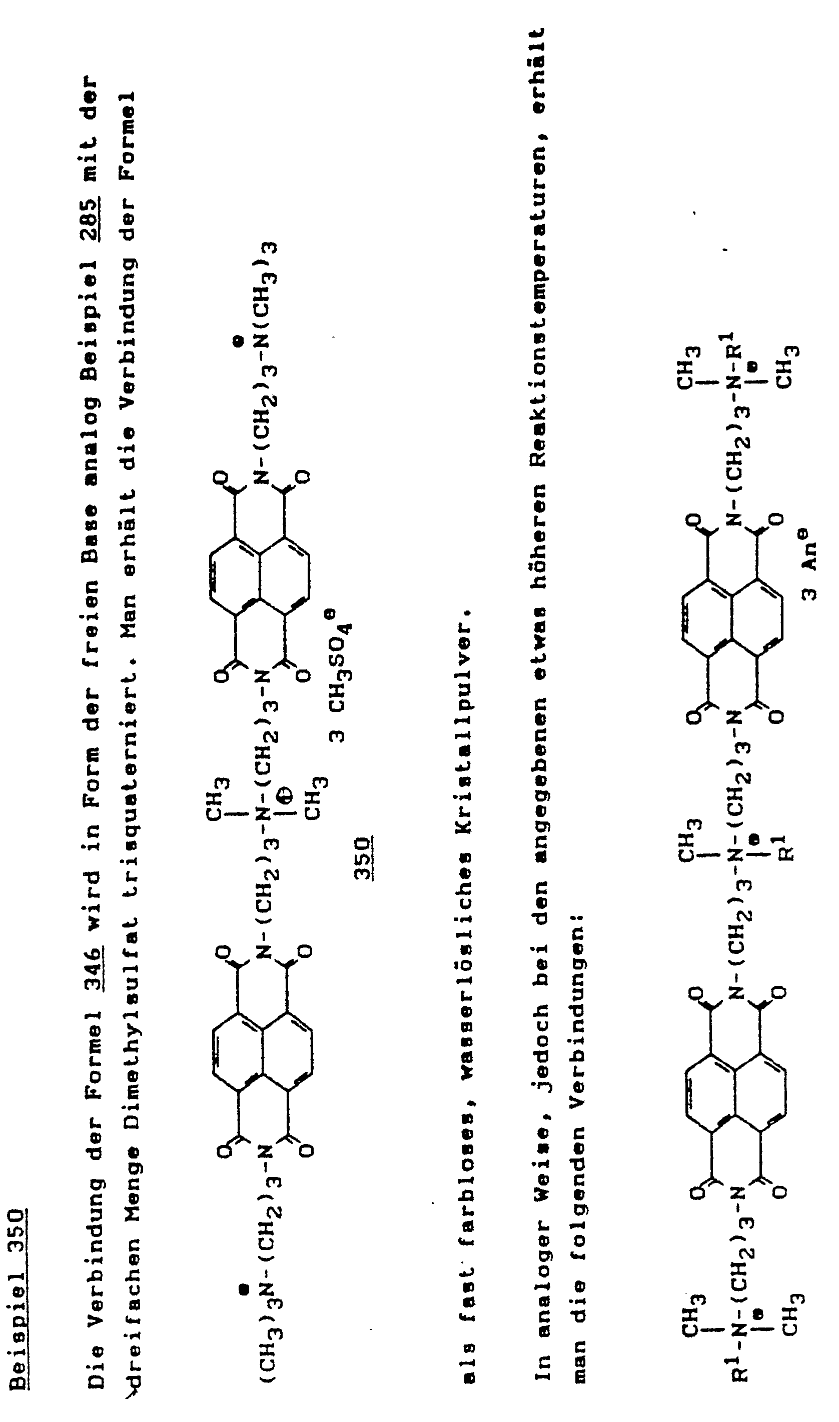

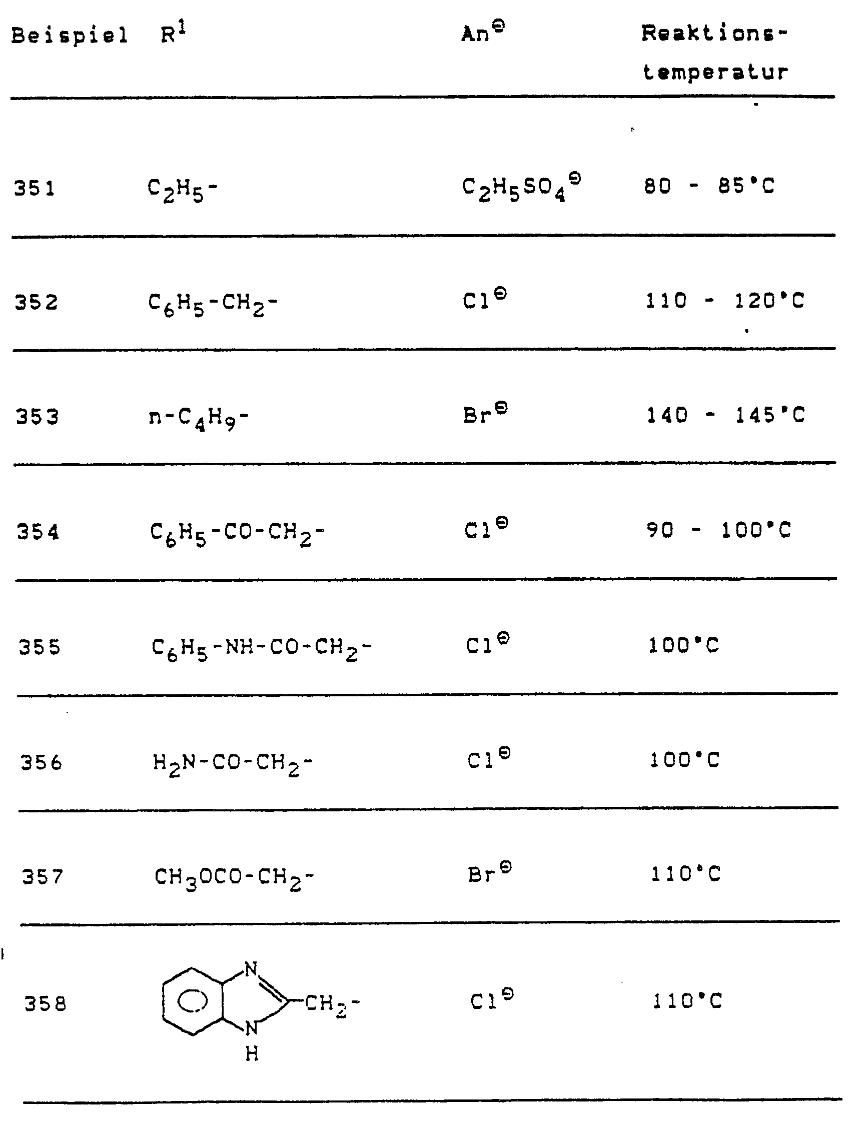

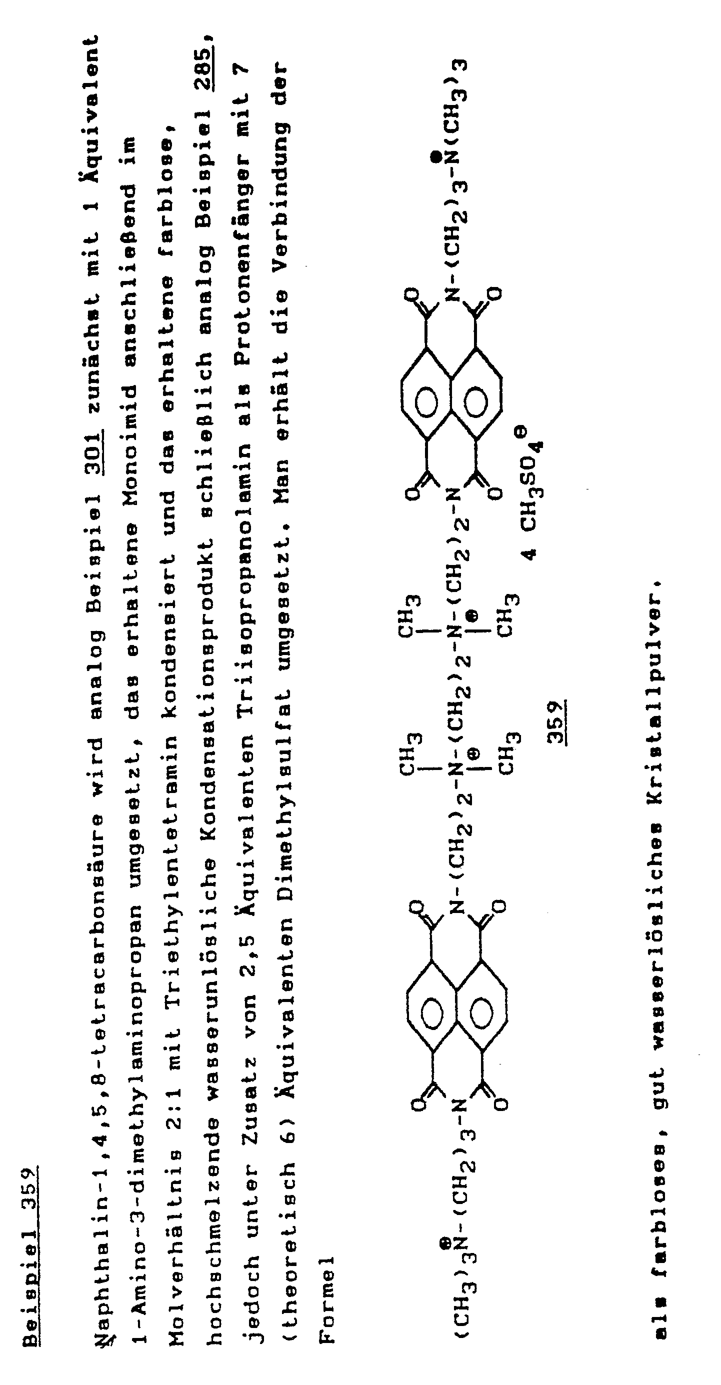

### Anwendungsbeispiel A

5000 Teile Papierbrei aus gebleichter Sulfitcellulose mit einem Mahlgrad von 40° Schopper-Riegler und einem Trockenstoffgehalt von 2 % (entsprechend 100 Teilen Sulfitcellulose) werden unter Rühren mit Natronlauge auf pH 7,5 gestellt, mit einer Lösung von 0,2 Teilen des optischen Aufhellers der Formel 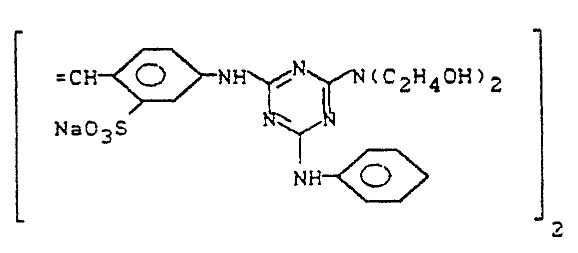 in 100 Teilen Wasser versetzt und 5 Minuten verrührt. Anschließend setzt man 100 Teile einer wäßrigen Lösung, die 1,0 Teile der im Herstellungsbeispiel 1 beschriebenen 20 %igen Lösung des Fluoreszenzlöschers der Formel 1 enthält, zu, verrührt 1 Minute und bildet das Papierblatt. Nach dem Trocknen wird ein Papier erhalten, das sich von nichtaufgehelltem Papier praktisch nicht unterscheidet.

Setzt man dagegen die gleiche Menge eines nach DE-A 1 912 647, Beispiel A oder B oder eines nach DE-A 2 448 293 Herstellungsbeispiel 1 hergestellten Fluoreszenzlöschers ein, wird ein Papier mit deutlich wahrnehmbarem Weißgrad erhalten.
Wiederholt man die Vergleichsversuche im pH-Bereich von 9, ist der Unterschied noch größer.

### Anwendungsbeispiel B

5000 Teile Papierbrei aus gebleichter Sulfitcellulose mit einem Mahlgrad von 40⁰ Schopper-Riegler und einem Trockenstoffgehalt von 2 % werden einige Minute mit 3 Teilen kristallisiertem Aluminiumsulfat verrührt, mit einer Lösung von 0,1 Teilen des optischen Aufhellers der Formel 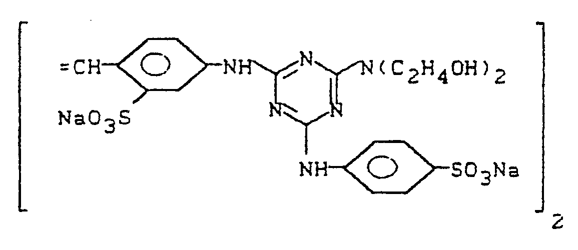 in 100 Teilen Wasser versetzt. Nach 15 Minuten werden 2 Teile Harzmilch zugegeben. Nach guter Durchmischung stellt man mit Schwefelsäure auf pH 4,5. Nun fügt man 100 Teile einer wäßrigen Lösung, die 1,0 Teile der im Beispiel 1 beschriebenen 20 %igen Lösung des Fluoreszenzlöschers der Formel 1 enthält, hinzu, verrührt 1 Minute, verdünnt mit Wasser auf 20 000 Teile und bildet das Papierblatt. Nach dem Trocknen wird ein geleimtes Papier erhelten, das sich von nichtaufgehelltem praktisch nicht unterscheidet.

Ähnlich starke Fluoreszenzlöscheffekte werden erhalten, wenn man einen der in den anderen Herstellungsbeispielen beschriebenen Fluoreszenzlöscher einsetzt.

### Anwendungsbeispiel C

Auf einer Langsiebmaschine wird unter Verwendung von aufgehelltem Altpapier als Rohstoff Papier mit einem Gewicht von 80 g/m² produziert.

Zum Löschen der Fluoreszenz der darin enthaltenen optischen Aufheller besprüht man die Papierbahn in der zweiten Hälfte der Siebpartie mit einer verdünnten wäßrigen Lösung der im Herstellungsbeispiel 1 beschriebenen Verbindungen derart, daß das trockene Papier 0,02 bis 0,1 % (je nach Konzentration des Aufhellers im Rohstoff) Fluoreszenzlöscher enthält. Das so fabrizierte Papier entspricht in den optischen Eigenschaften einer Papierqualität, welche keinen optischen Aufheller enthält.
Mit gleichem Erfolg werden die in den übrigen Herstellungsbeispielen beschriebenen Fluoreszenzlöscher eingesetzt.

### Anwendungsbeispiel D

10 g gebleichtes Nessel-Baumwollgewebe wird im Flottenverhältnis 1:20 30 Minuten bei 50° C mit einer Lösung von 0,1 % BLANKOPHOR@ BA 267 % behandelt. Nach dem Spülen und Trocknen wird ein Weißgrad nach Berger von 150 gemessen (Grundweiß ca. 80).
Das so weißgetönte Gewebe wird anschließend im Flottenverhältnis 1:20 30 Minuten bei 50 C mit 0,1 % des im Herstellungsbeispiel 1 beschriebenen Fluoreszenzlöschers behandelt. Nach dem Spülen und Trocknen wird wiederum der Weißgrad nach Berger bestimmt. Er liegt in Bereich von 100. Visuell ist nur noch ein geringer Aufhelleffekt wahrnehmbar.
Mit ähnlichem Erfolg können auch andere in den Herstellungsbeispielen beschriebene Verbindungen eingesetzt werden.

### Anwendungsbeispiel E

Die Möglichkeit, unerwünschte Restanteile von wäßrigen Lösungen anionischer Weißtöner in Färbeapparaturen, Färbemaschinen, Vorratsgefäßen und Zuleitungen unter Verwendung von Fluoreszenzlöschern der Beispiele 1 bis 359 unwirksam zu machen, sei an folgendem quantitativen Modellversuch gezeigt:
a) 0,03 mMol eines optischen Aufhellers der Formel 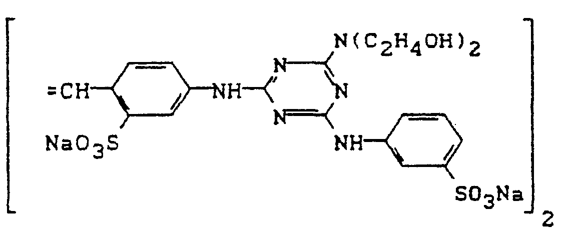 (42,2 mg 86,5 %ig = 36,5 mg 100 %ig) werden in 100 ml Wasser gelöst.
b) 0,1 mMol Fluoreszenzlöscher der Formel 1 (91,5 mg) werden in 100 ml Wasser gelöst.
c) 5 ml der unter a) hergestellten Lösung werden auf 100 ml aufgefüllt.
d) 5 ml der unter a) und x ml der unter b) hergestellten Lösung werden vermischt und auf 100 ml aufgefüllt (x = 1, 2, 3, 4).

In einem Zeiss-Spektralphotometer DMR 21 mit 1 ml Quarzküvette und Fluoreszenzzusatzgerät ZF M4 (450 W Xenonlampe) mißt man nacheinander unter Fluoreszenzanregung mit UV-Licht von 345 nm die Intensität der Fluoreszenzemission der folgenden Lösungen bei 436 nm:

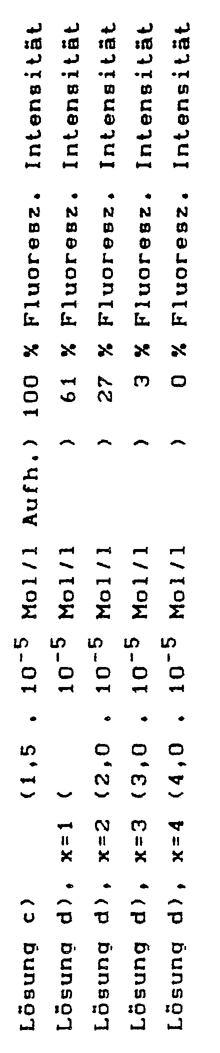

Der Versuch zeigt, daß Fluoreszenzlöscher den oben aufgeführten optischen Aufheller in verdünnter wäßriger Lösung bei Raumtemperatur bereits bei einem Molverhältnis von 2:1 weitgehend, bei einem Molverhältnis von 2,7:1 völlig unwirksam macht.

Setzt man anstelle von Verbindung 1 eine der in DE-A 1 912 647 oder 2 448 293 beschriebenen Verbindung ein, so wird eine völlige Fluoreszenzlöschung - auch bei noch höheren Anwendungskonzentrationen des Löschers - nicht erreicht.

## Patentansprüche

1. Verfahren zur Löschung der durch anionische optische Aufheller erzeugten Fluoreszenz durch Einwirkung praktisch farbloser, wasserlöslicher, kationischer Verbindungen auf die genannten Aufheller, dadurch gekennzeichnet, daß die Verbindungen die allgemeine Formel 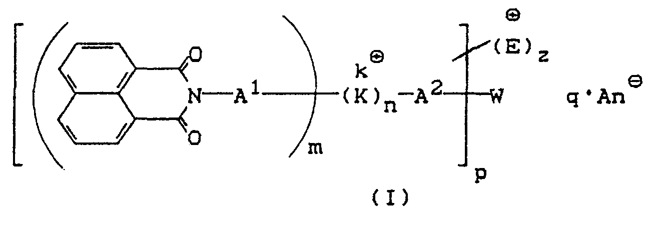 besitzen,
worin
A¹, A² und W für ein Brückenglied oder eine Einfachbindung,
W auch für Wasserstoff,
^{⊕}_{E} für eine endständige Ammonium- oder Sulfoniumgruppe,
^{⊕}_{K} für eine zweibindige Ammonium- oder Sulfoniumgruppe,
An^{⊖} für ein Anion,
m und k für 1 oder 2,
z für 0, 1, 2, 3 oder 4
n für 0 oder 1 und
p für 1, 2 oder 3 stehen,
q der Summe der freien kationischen Ladungen entspricht, wobei n + z ungleich 0 ist, worin
das Naphthalinringsystem in der zweiten peri-Stellung auch durch -CH₂-CH₂-, -CO-O-CO- oder -CO-NH-CO-substituiert sein kann,
das Naphthalinringsystem, A¹, A2, W, -E- -K- sowie das zweite peri-Dicarbonsäureimid-Stickstoffatom durch nichtionische Reste substituiert sein können, und die gesamte angegebene Struktur auch wiederkehrende Einheit einer hochmolekularen Verbindung sein kann.

2. Verfahren nach Anspruch 1, dadurch gekennzeichnet, daß die Verbindungen die allgemeine Formel 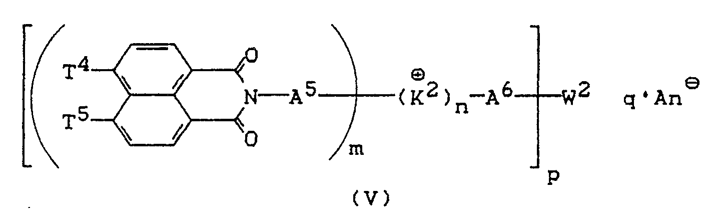 besitzen,
T⁴ und T⁵ für Wasserstoff, Chlor oder Brom, oder
T⁴ und T⁵ zusammen für eine -CO-N(T⁶)-CO-Gruppe,
T⁶ für Wasserstoff, OH, gegebenenfalls durch 1 bis 2 OH-Gruppen, Chlor, Ci-C₄-Alkoxy oder COOH substituiertes C₁-C₆-Alkyl, Allyl, Phenyl oder Benzyl, die durch 1 oder 2 C₁-C₄-Alkyl, Chlor, C₁-C₂-Alkoxy, Brom, Carboxy, Cyan, C₁-C₂-Alkoxycarbonyl, C₁-C₂-Alkylsulfonyl, Carbamoyl, Sulfamoyl substituiert sein können, ß-Aminoethylphenyl, Pyridyl-(2)- oder -(3)-, Benzthiazol-(2)-yl, 1,2,4-Triazol-3-yl, Cyclohexyl, -A⁵-^{⊕}_{E} ² oder im Falle m = 1, n = 0 und p = 1 für eine Gruppierung der Formel -A⁵ -^{⊕}_{K} 2- A⁶⁻ oder -A5-^{⊕}_{K} 2-A⁶-W²-A⁶-^{⊕}_{K} 2-A⁵-wobei A⁵ und A⁶ bzw. die beiden Reste A^{s} mit 2 verschiedenen 1,4,5,8-Naphthalintetracarbonsäurediimid-Molekülen verbunden sind und somit die gesamte angegebene Struktur zur wiederkehrenden Einheit einer hochmolekularen Verbindung machen,
A⁵ für eine Einfachbindung, C₁-C₅-Alkylen, m- oder p-C₆H₄-CH₂-,
A⁶ für eine Einfachbindung, -CH₂-, -CH₂-CO-0-, -CH₂-CO-N(R)-, -CH₂-S0₂-N(R)-, -(CH₂)₂-CO-N(R)-. -CH₂-CO-NH-CH₂-, C₁-C₅-Alkylen-N-(R)-CO-, C₁-C₅-Alkylen-N(R)-SO₂-, C₁-C₅-Alkylen-N(R)-CO-NH-, -CH₂-C₆H₄-NH-C0-, -CH₂-C₆H₄-NH-SO₂-, -CH₂-C₆H₄-NH-CO-NH-, -(CH₂)_{w}-CO-, -(CH₂)_{w}-CO-C₆H₄-NH-CO, -(CH₂)_{w} CO-C₆H₄-NH-SO₂-, -(CH₂)_{w}-CO-C₆H₄-NH-CO-NH-, außerdem: -S0₂-' -CO- oder -CONH-, wenn ^{⊕}_{K} = Piperazinium und -NH-CO-NH-, wenn ^{⊕}_{K} ² = Pyridinium, 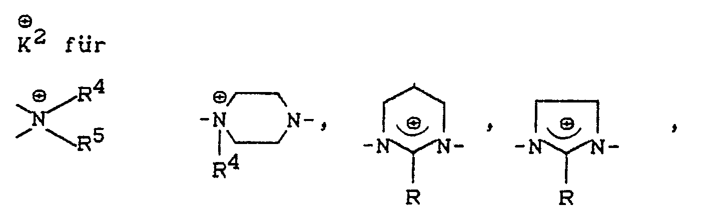 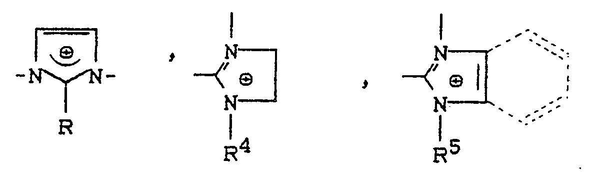 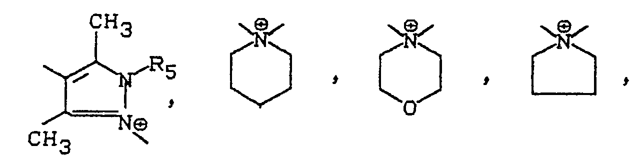 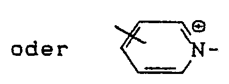 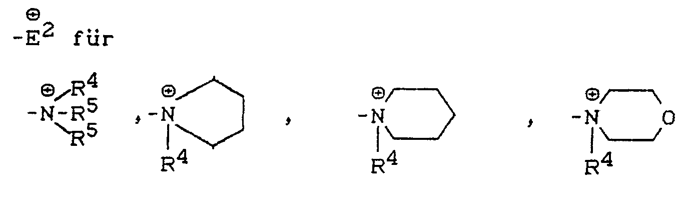 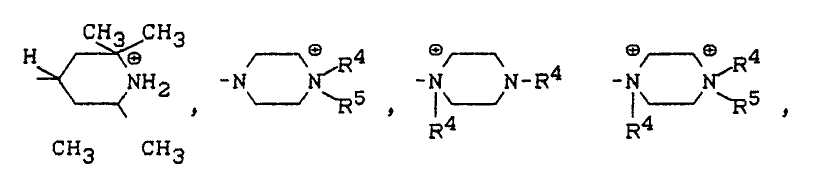 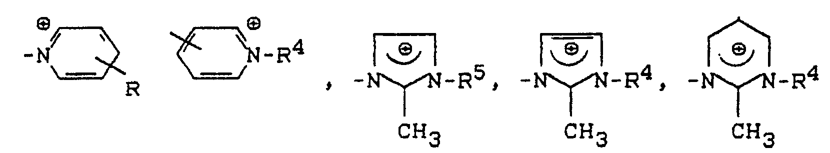 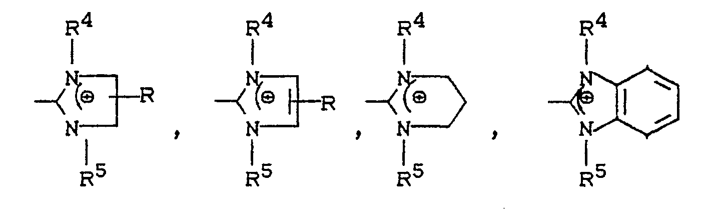 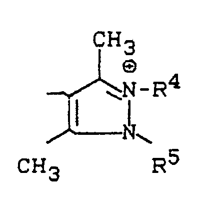
R für Wasserstoff oder 1-4 Methylgruppen,
R⁴ und R⁵ je für C₁-C₄-Alkyl, das durch OH, CONH_{z}, C₁-C₂-Alkoxycarbonyl oder C₁-C₂-Alkylcarbonyl substituiert sein kann, Allyl,
R⁴ außerdem für Wasserstoff, Cyclohexyl, Benzyl-, Benzoylmethyl-Reste, die durch 1 oder 2 Reste der Reihe Methyl, Chlor, Methoxy, Ethoxy, Cyan, C₁-C₂- Alkoxycarbonyl oder Nitro kernsubstituiert sein können, oder Benzimidazol-2-ylmethyl,
R⁵ außerdem für Benzyl oder je ein Rest R⁵ auch für Phenyl,
W² für -CO-, einen p-bindigen Benzol-, Naphthalin-, Cyclohexan-, Piperazin-, Thiophen-(2,5)-, 1,3,4-Oxadiazol-(2,5)-, s-Triazin-(2,4 oder 2,4,6)-Rest oder einen Rest der Formeln 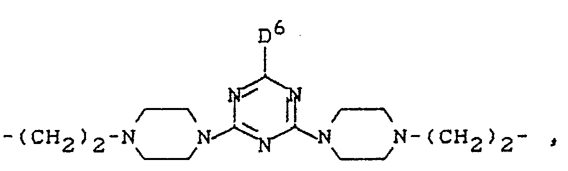 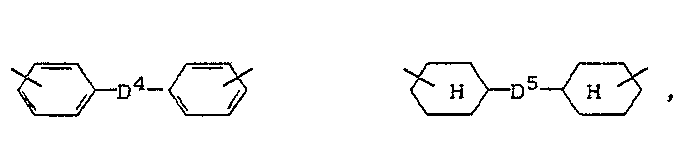 worin im Falle p = 1 eine der angegebenen freien Valenzen durch Wasserstoff abgesättigt ist,
D⁴ und D⁵ je für CH₂, einen gegebenenfalls durch -O-unterbrochenen C₂-C₅-Alkylenrest (insbesondere -CH₂-, -CH(CH₃)-, -C(CH₃)₂ und -CH₂-CH₂-), -O-, -H(R)-, -O-C₂-C₄-alkylen-O-oder einen Rest der Formel 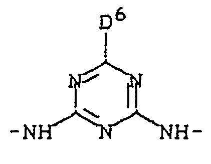 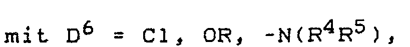 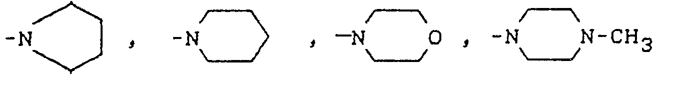 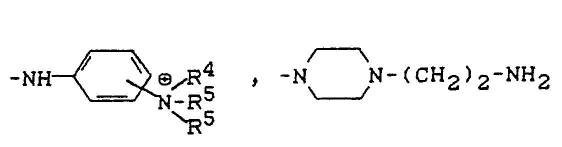 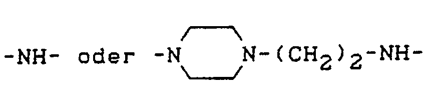 D⁴ außerdem für 1,1-Cyclohexylen, p-Phenylen, NH-, -CH(C₆H₅)-, -CH(C₆H₄-)-, -S-, S0₂-, -CO-, -NH-CO-NH-, -O-PO-O- oder eine Einfachbindung stehen, wobei die in D⁴, D⁵ und sonst noch in W² genannten Ringe durch CH₃, CH₃O oder CI substituiert sein können,
worin im Falle p = 2 die Gruppierung -^{⊕}_{K} 2-A⁶-W²-A⁶-^{⊕}_{K} 2- insgesamt auch für 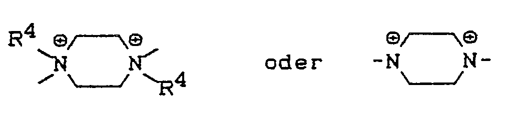 stehen kann,
worin die Indices m und p nur dann gleichzeitig 1 bedeuten, wenn T⁴ und T⁵ eine cyclische peri-Dicarbonsäureimid-Gruppierung bilden,
worin n, p, q, An^{s} und R die gleiche Bedeutung wie in Anspruch 1 besitzen,
und worin n nur dann Null sein kann, wenn T⁶ eine kationische Gruppe -^{⊕}_{K} 2- oder -^{⊕}_{E} ² enthält.

3. Verbindungen der allgemeinen Formel 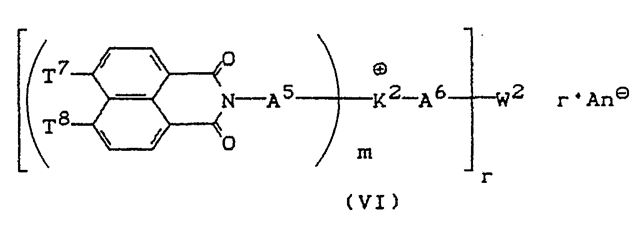
mit T⁷ und T⁸ = Wasserstoff, Chlor oder Brom,
r = 2 oder 3,
worin die Reste A⁶ und - wenn m = 2 - die Reste A⁵ voneinander verschieden sein können, und worin m, A⁵, K², A⁶, W² und An^{⊖} die gleiche Bedeutung wie in Anspruch 2 haben.

4. Verbindungen gemäß Anspruch 3 der allgemeinen Formel, 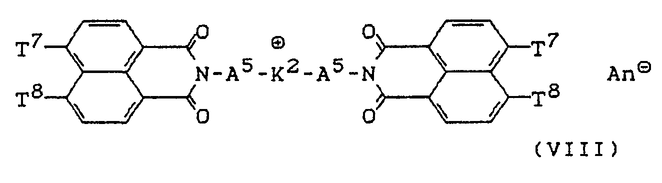 worin T⁷, T⁸, A⁵,^{⊕}_{K} ² und An⁹ die gleiche Bedeutung wie in Anspruch 3 haben.

5. Verfahren nach Anspruch 1, dadurch gekennzeichnet, daß die Verbindungen der Formel des Anspruchs 3 oder 4 entsprechen.

6. Verfahren nach Anspruch 1, dadurch gekennzeichnet, daß die Verbindungen der Formel 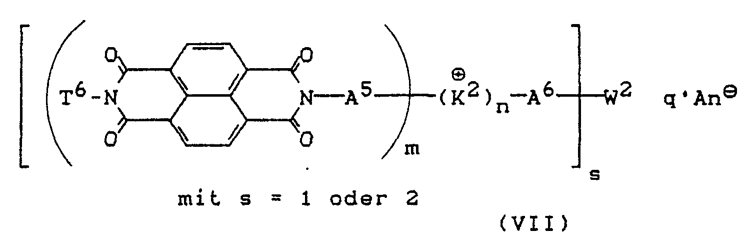 worin T⁶, A⁵, K², A⁶, W², An^{⊖}, m, n und q die gleiche Bedeutung wie in Anspruch 2 haben, entsprechen.

7. Verfahren zur Herstellung von Verbindungen des Anspruchs 3, dadurch gekennzeichnet, daß man
a) Verbindungen der Formel 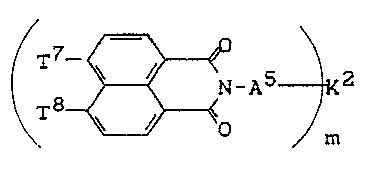 im Molverhältnis r:1 mit Verbindungen der Formel 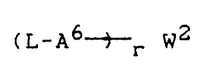 umsetzt, oder
b) Verbindungen der Formel 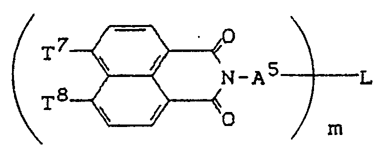 im Molverhältnis r:1 mit Verbindungen der Formel 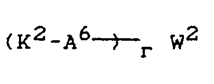 umsetzt, oder
c) die unter a) genannten Naphthalimid-Verbindungen im äquimolaren Verhältnis mit den unter b) genannten Naphthalimid-Verbindungen umsetzt, wobei
-K² die nichtkationische einbindige Basenvorstufe von -1 2- und
L eine als Anion abspaltbare Gruppe bedeuten, und die übrigen Symbole die gleiche Bedeutung wie in Anspruch 3 haben.

8. Verfahren zur Herstellung von Verbindungen des Anspruchs 3, dadurch gekennzeichnet, daß man Verbindungen der Formel 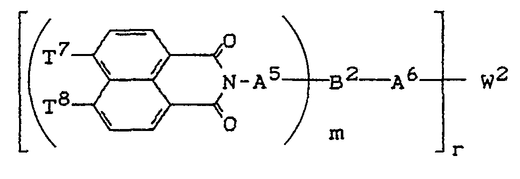 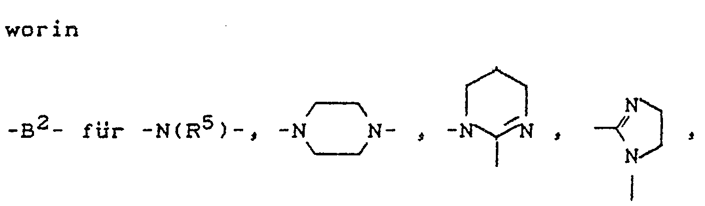 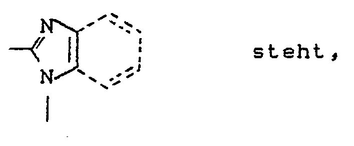 im äquimolaren Verhältnis mit Verbindungen der Formel
R4-L
worin
L für eine als Anion abspaltbare Gruppe steht, und die übrigen Symbole die gleiche Bedeutung wie in Anspruch 3 haben, umsetzt.

9. Mittel zur Löschung der durch anionische optische Aufheller erzeugten Fluoreszenz, enthaltend eine praktisch farblose, wasserlösliche, kationische Verbindung des Anspruchs 1.

## Claims

1. Process for the quenching of the fluorescence created by anionic optical brighteners by the action of virtually colourless, water-soluble, cationic compounds _on the brighteners mentioned, characterized in that the compounds have the general formula 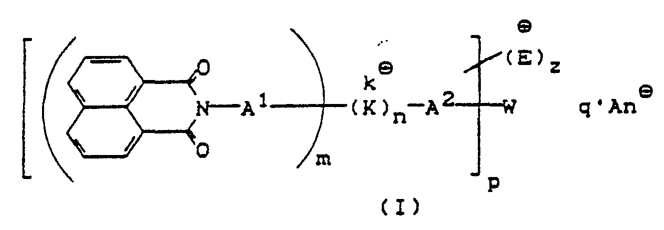 in which
A', A² and w represent a bridging member or a single bond,
W also represents hydrogen,
^{⊕}_{E} represents a terminal ammonium or sulphonium group,
^{⊕}_{K} represents a doubly bridging ammonium or sulphonium group,
An^{a} represents an anion,
m and k represent 1 or 2,
z represents 0, 1, 2, 3 or 4,
n represents 0 or 1 and
p represents 1, 2 or 3,
q corresponds to the sum of the free cationic charges, where n + z ≢ 0, in which
the naphthalene ring system can also be substituted in the second peri-position by -CH₂-CH₂-, -COO-CO- or -CO-NH-CO-, the naphthalene ring system, A¹, A², W, -^{⊕}_{E} -and -^{⊕}_{K} - and the second peridicarboxylic acid imide nitrogen atom can be substituted by non-ionic radicals, and the total structure specified can also be the repeating unit of a high molecular weight compound.

2. Process according to Claim 1, characterized in that the compounds have the general formula 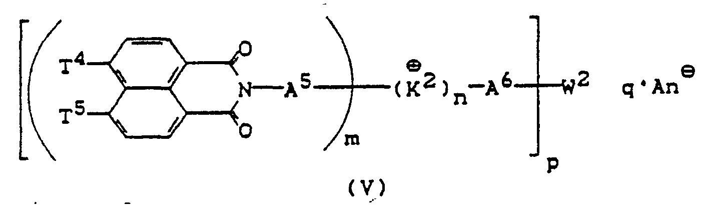
T⁴ and T⁵ represent hydrogen, chlorine or bromine, or T⁴ and T⁵ together represent a -CO-N(T⁶)-CO-group,
T⁶ represents hydrogen, OH, C₁-C₆-alkyl which is optionally substituted by 1 or 2 OH groups, chlorine, C₁-C₄-alkoxy or COOH, allyl, phenyl or benzyl which can be substituted by 1 or 2 C₁-C₄- alkyl, chlorine, C₁-C₂-alkoxy, bromine, carboxyl, cyano, C₁-C₂-alkoxycarbonyl, C₁-C₂-alkylsulphonyl, carbamoyl or sulphamoyl, β-aminoethylphenyl, pyridyl-(2)- or -(3)-, benzothiazol-(2)-yl, 1,2,4-triazol-3-yl, cyclohexyl, -A⁵-^{⊕}_{E} ² or, in the case where m = 1, n = 0 and p = 1, represents a grouping of the formula -A⁵-^{⊕}_{K} ²-A⁶- or -A⁵-^{⊕}_{K} ²-A⁶-W²-A⁶-^{⊕}_{K} 2-A⁵-
As and A⁶ or the two radicals A⁵ being bonded to 2 different 1,4,5,8-naphthalene tetracarboxylic acid diimide molecules and thus making the specified structure as a whole into the repeating unit of a high molecular weight compound,
A⁵ represents a single bond, C₁,-C₅-alkylene, m-or p-C₆H₄-CH₂-,
A⁶ represents a single bond, -CH₂-, -CH₂-CO-O-, -CH₂-C0-N(R)-, -CH₂-SO₂-N(R)-, -(CH₂)₂-CO-N(R)-, -CH₂-CO-NH-CH₂-, C,-Cₛ-alkylene-N(R)-CO-, C₁-C₅-alkylene-N(R)-SO₂-, C₁-Cₛ-alkylene-N(R)-CO-NH-, -CH₂-C₆H₄-NH-CO-, -CH₂-C₆H₄-NH-SO₂-, -CH₂-C₆H₄-NH-CO-NH-, -(CH₂)w-CO-, -(CH₂)_{w}-CO-C₆H₄-NH-CO-, -(CH₂),-CO-C₆H₄-NH-SO₂-,-(CH₂)_{w}-CO-C₆H₄,-NH-CO-NH-, in addition: -S0₂-, -CO- or -CONH-, when ^{⊕}_{K} ² = piperazinium, and -NH-CO-NH-, when^{⊕}ₖ ² = pyridinium,
² r epresents 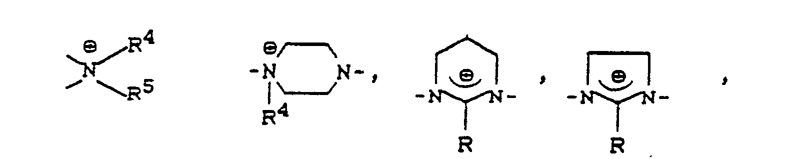 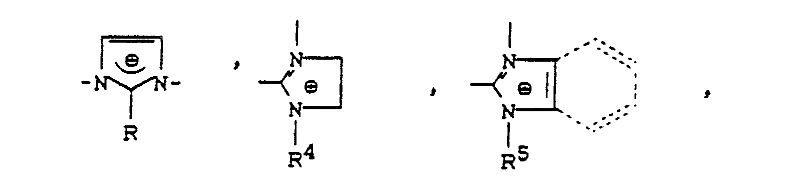 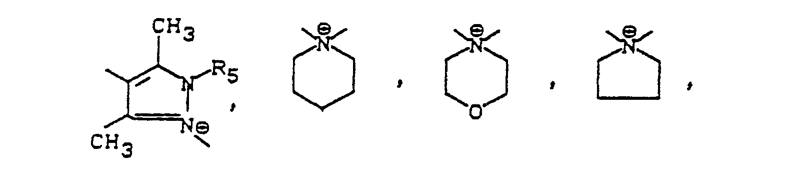 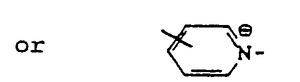 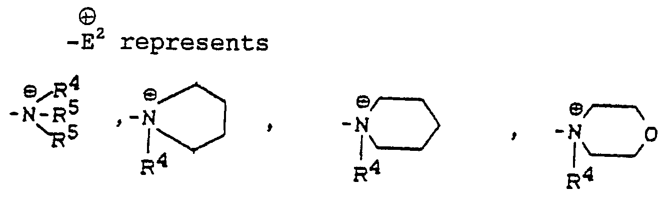 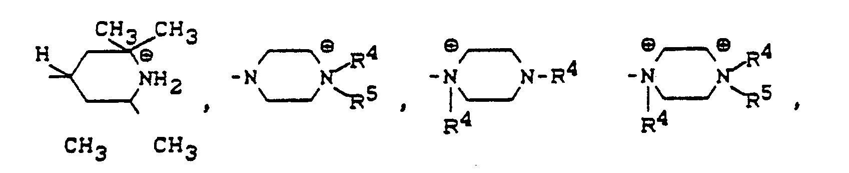 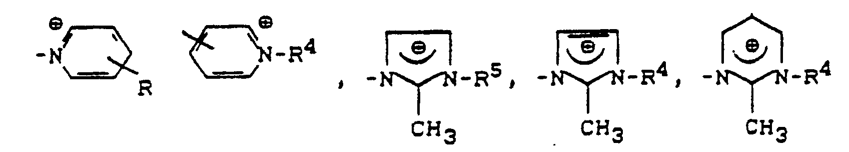 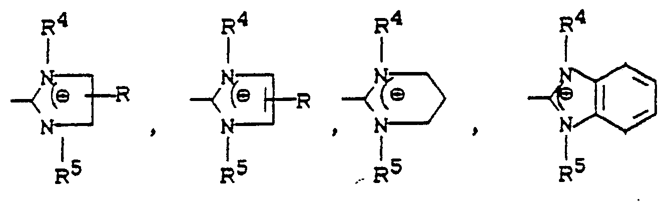 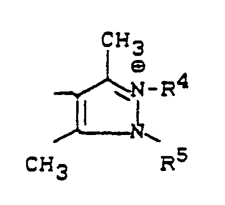
R represents hydrogen or 1-4 methyl groups, R⁴ and R⁵ each represent C₁-C₄-aklkyl, which can be substituted by OH, CONH_{z}, C₁-C₂-alkoxycarbonyl or C₁-C₂-alkylcarbonyl, or allyl,
R⁴, in addition, represents hydrogen, cyclohexyl, benzyl- or benzoylmethyl radicals, which can be substituted on the nucleus by 1 or 2 radicals from the series comprising methyl, chlorine, methoxy, ethoxy, cyano, C₁-C₂-alkoxycarbonyl or nitro, or benzimidazol-2-ylmethyl,
R⁵, in addition, represents benzyl or one radical
R⁵ in each case also represents phenyl,
W² represents -CO-, a p-bonded benzene, naphthalene, cyclohexane, piperazine, thiophene-(2,5)-, 1,3,4-oxadiazole-(2,5)-, s-triazine-(2,4 or 2,4,6) radical or a radical of the formulae 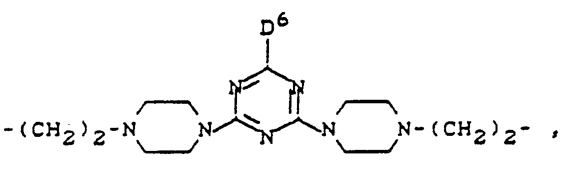 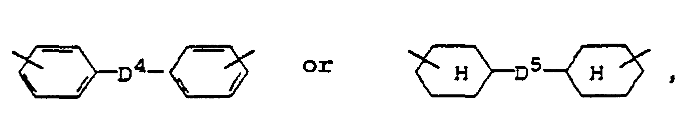 in which, in the case where p = 1, one of the specified free valences is saturated by hydrogen,
D4 and D⁵ each represent CH₂, a C₂-C₅-alkylene radical (particularly -CH₂-, -CH(CH₃)-, -C(CH₃)₂ and -CH₂-CH₂-) which is optionally interrupted by -0-, -0-, -N(R)-, -0-C₂-C₄-alkylene-0- or a radical of the formula 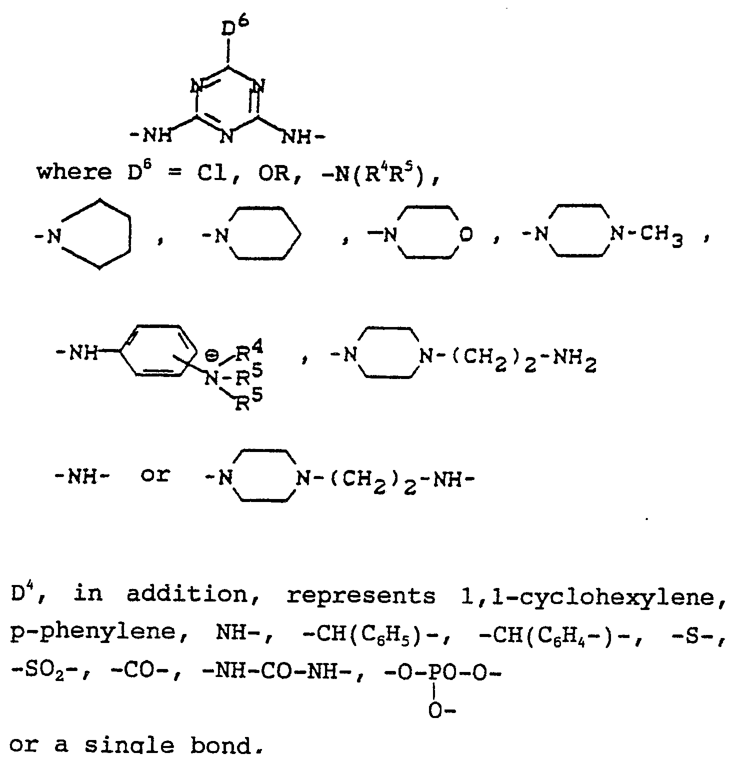 it being possible for the rings mentioned in D⁴, D⁵, and also W², to be substituted by CH₃, CH₃O or CI,
in which, in the case where p = 2, the grouping ⊕_{K} ²-A⁶-W²-A⁶-^{⊕}_{K} 2- as a whole can also represent 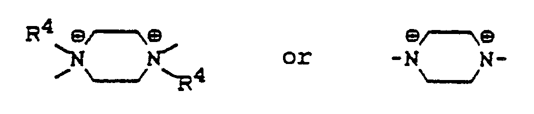 in which the indices m and p only denote 1 simultaneously when T⁴ and T⁵ form a cyclic peridicarboxylic acid imide grouping,
in which n, P, q, An^{e} and R have the same meaning as in Claim 1,
and in which n can only be zero when T⁶ contains a cationic group ^{⊕}_{K} 2- or -^{⊕}_{E} 2-.

3. Compounds of the general formula 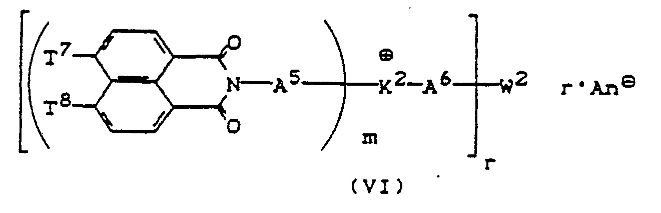 with T⁷ and T⁸ = hydrogen, chlorine or bromine,
r = 2 or 3,
in which the radicals A⁶ and - when m = 2 - the radicals A⁵ can be different from one another, and in which m, A⁵, K², A⁶, W² and An^{⊖} have the same meaning as in Claim 2.

4. Compounds according to Claim 3 of the general formula, 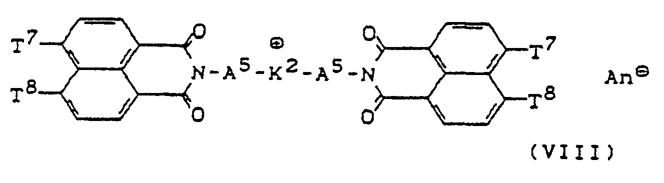 in which
T⁷, T⁸, A⁵, ^{⊕}_{K} 2 and An^{⊖} have the same meaning as in Claim 3.

5. Process according to Claim 1, characterized in that the compounds correspond to the formula of Claim 3 or 4.

6. Process according to Claim 1, characterized in that the compounds correspond to the formula 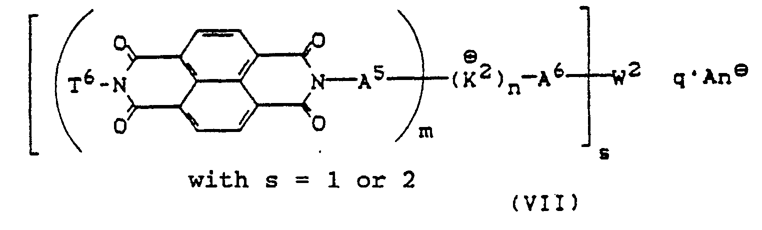 in which A⁵,^{⊕}K^{⊕}ₖ2,A⁶,W²,An^{⊖}m, n and q have the same meaning as in Claim 2.

7. Process for the preparation of compounds of Claim 3, characterized in that
a) compounds of the formula 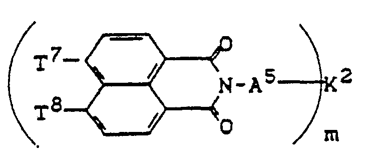 are reacted in the molar ratio r:1 with compounds of the formula 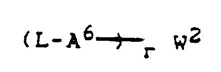 or
b) compounds of the formula 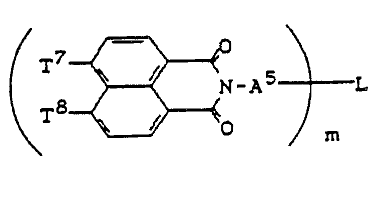 are reacted in the molar ratio r:1 with compounds of the formula 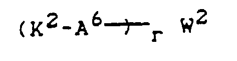 or
c) the naphthalimide compounds mentioned under a) are reacted in equimolar ratio with the naphthalimide compounds mentioned under b), where
-K² denotes the non-cationic terminal basic precursor of -1 2- and
L denotes a group which can be cleaved off as an anion, and the remaining symbols have the same meaning as in Claim 3.

8. Process for the preparation of compounds of Claim 3, characterized in that compounds of the formula 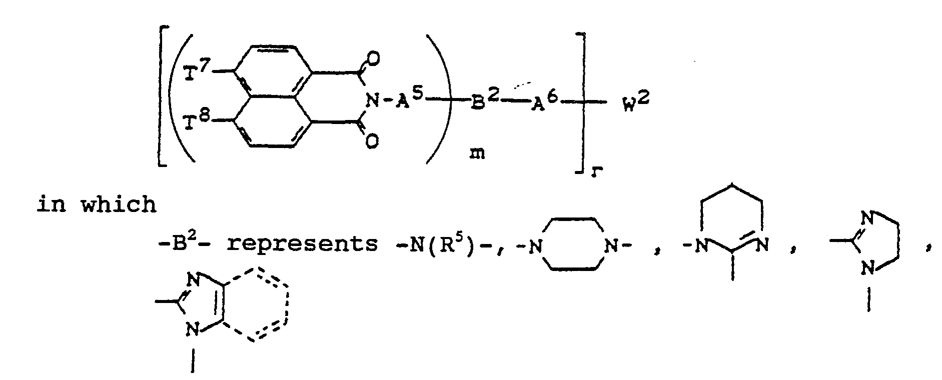 are reacted in equimolar ratio with compounds of the formula
R⁴⁻L
in which
L represents a group which can be cleaved off as an anion, and the remaining symbols have the same meaning as in Claim 3.

9. Means for quenching the fluorescence created by anionic optical brighteners, containing a virtually colourless, water-soluble, cationic compound of Claim 1.

## Revendications

1. Procédé pour éteindre la fluorescence produite par des agents anioniques d'éclaircissement optique, en faisant agir des composés cationiques, hydrosolubles et pratiquement incolores, sur l'agent précité d'éclaircissement optique, procédé caractérisé en ce que les composés possèdent la formule générale 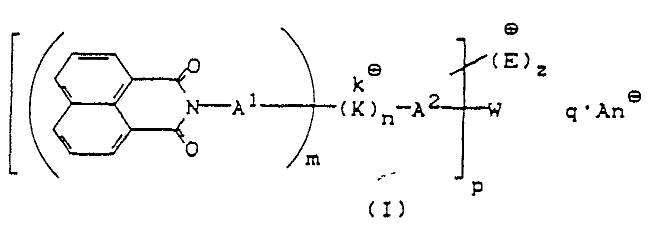 dans laquelle
A¹, A² et W représentent chacun un chaînon de pontage ou une liaison simple, W peut représenter aussi un atome d'hydrogène,
^{⊕}_{E} représente un groupe ammonium ou sulfonium en position terminale,
^{⊕}_{K} représente un groupe ammonium ou sulfonium bivalent,
An^{⊖} représente un anion,
m et k valent 1 ou 2,
z vaut 0, 1, 2, 3 ou 4,
n vaut 0 ou 1, et
p vaut 1, 2 ou 3,
q correspond à la somme des charges cationiques libres, la somme (n + z) étant différente de 0,
le système cyclique naptalénique pouvant également être substitué, sur la seconde position en péri, par -CH₂-CH₂-, -CO-O-CO- ou -CO-NH-CO-,
le système cyclique napthalénique A¹, A², W, -^{⊕}E -,-^{⊕}_{K} -ainsi que le second atome d'azote de fonction imide d'acide dicarboxylique en péri peuvent être substitués par des restes non ioniques, et la totalité de la structure indiquée peut aussi constituer un motif récurrent d'un composé à poids moléculaire élevé.

2. Procédé selon la revendication 1, caractérisé en ce que les composés possèdent la formule générale 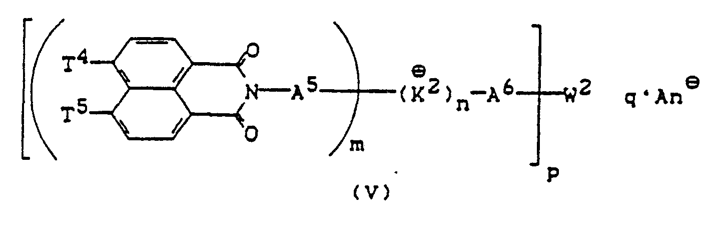 dans laquelle,
T⁴ et T⁵ représentent chacun un atome d'hydrogène, de chlore ou de brome, ou bien
T⁴ et T⁵ forment ensemble un groupe -CO-N(T⁶)-CO,
T⁶ représente un atome d'hydrogène, un groupe OH, un groupe alkyle en Ci-Ce (éventuellement substitué par 1 à 2 groupes OH, par du chlore, par un groupe alcoxy en C₁-C₄- ou par un groupe COOH), un groupe allyle, phényle ou benzyle, qui peuvent être substitués par 1 à 2 restes alkyles en C₁-C₄-, chloro, alcoxy en C₁-C₄-, bromo, carboxy, cyano, alcoxy (en C₁-C₂)carbonyle, alkylsulfony- le en C₁-C₂, carbamoyle, sulfamoyle, un groupe β-aminoéthylphényle, pyridyle-(2)- ou -(3)-, benzothiazole-(2)yle, 1,2,4-triazole-3-yle, cyclohexyle, -A⁵-^{⊕}_{E} 2 ou, si m vaut 1, n est nul et p vaut 1, T⁶ représente un groupement de formule -A⁵-^{⊕}_{K} 2- A⁶ ou -A⁵-^{⊕}_{K} ²-A⁶-W²-A⁶-^{⊕}_{K} ²-A⁵-, les restes A⁵ et A⁶ ou les deux restes A⁵, étant reliés à deux molécules différentes de diimide d'acide 1,4,5,8-naphtalènetétracarboxylique, et, ainsi, la totalité de la structure indiquée constituant un motif récurrent d'un composé à poids moléculaire élevé,
A^{S} représente une liaison simple, un groupe alkylène en C₁-C₅, un groupe m- ou p-C₆H₄-CH₂-,
A⁶ représente une liaison simple, -CH₂-, -CH₂-CO-O-, -CH₂-C0-N(R)-, -CH₂-SO₂-N(R)-, -(CH₂)₂-CON(R)-, -CH₂-CO--NH-CH₂-, un groupe alkylène (en C₁-C₅)-N-(R)-CO-, un groupe alkylène (en C₁-C₅)-N(R)-SO₂-, un groupe alkylène (en C₁-C₅)-N(R)-CO-NH-, -CH₂-C₆H₄NH-CO-, -CH₂-C₆H₄-NH-SO₂-, -CH₂-C₆H₄-NH-CO-NH-, -(CH₂)_{w}-CO-, -(CH₂)_{w} CO--C₆H₄-NH-C0, -(CH₂)_{w}-CO-C₆H₄-NH-SO₂-, --(CH₂)_{w}-CO-C₆H₄-NH-CO-NH-, et en outre -SO₂-, -CO- ou -CONH-, lorsque K^{Z} représente un groupe pipérazinium et -NH-CO-NH-, lorsque^{⊕}_{K} ² représente un groupe pyridinium ;
^{⊕}_{K} ² représente
^{⊕}_{K} ² représente 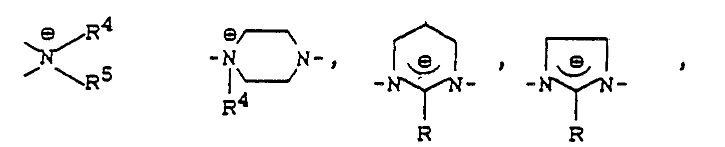 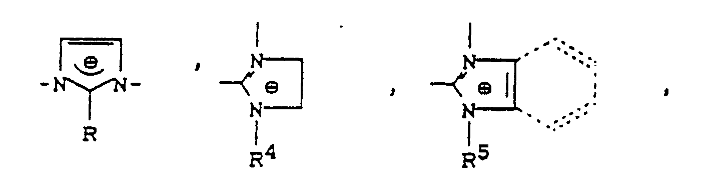 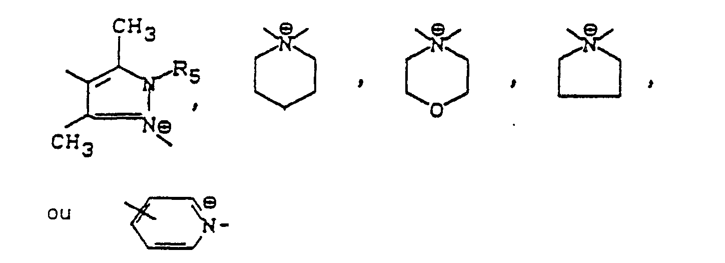 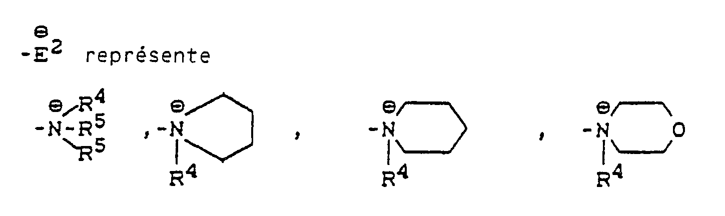 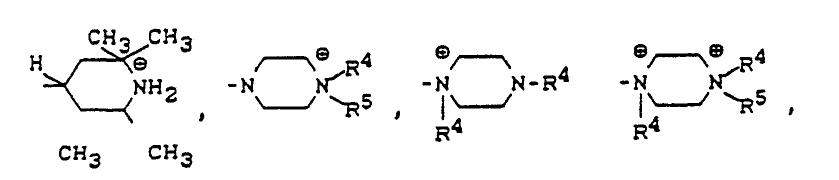 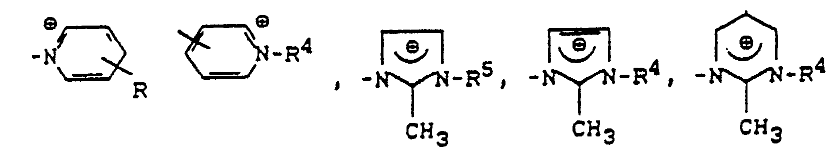 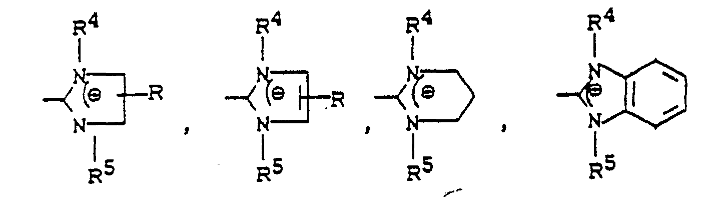 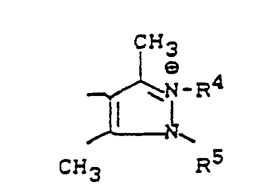
R représente un atome d'hydrogène ou bien 1 à 4 groupes méthyle,
R⁴ et R⁵ représentent chacun un groupe alkyle en C₁-C₄-, (qui peut être substitué par un reste OH, CONH₂, alcoxy (en C₁ -C₂)carbonyle ou alkyle (en C₁-C₂)carbonyle), un groupe allyle,
R⁴ pouvant en outre représenter un atome d'hydrogène, des restes cyclohexyle, benzyle, benzoyl- méthyle, (pouvant être substitués sur le noyau par un ou deux restes choisis parmi un reste méthyle, chloro, méthoxy, éthoxy, cyano, alcoxy (en C₁-C₂)carbonyle ou nitro) ou un groupe benzimidazole-2-ylméthyle,
R⁵ pouvant représenter en outre un groupe benzyle ou bien chaque reste R⁵ pouvant également représenter un groupe phényle,
w² représente -CO-, un reste de benzène, de naphtalène, de cyclohexane, de pipérazine, de thiophène-(2,5), de 1,3,4-oxadiazole-(2,5), de s-triazine-(2,4 ou 2,4,6) à pliaisons, ou un reste répondant aux formules 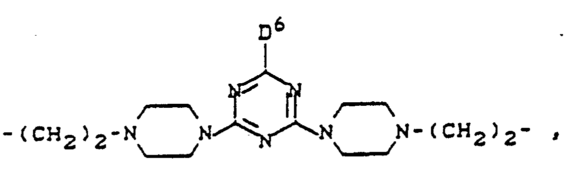 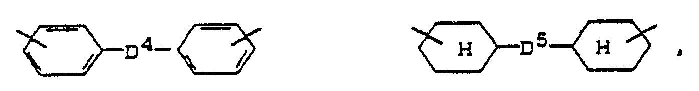 où, si p vaut 1, l'une des valences libres indiquées pouvant être saturée par de l'hydrogène,
D⁴ et D5 peuvent représenter chacun un groupe CH₂, un reste alkylène en Cz-Cs éventuellement interrompu par -O- (en particulier -CH₂-, -CH(CH₃)-, -C(CH₃)₂ et -CH₂-CH₂-), -O-, -N(R)-, -O-alkylène (en C₂-C₄)-O- ou un reste de formule 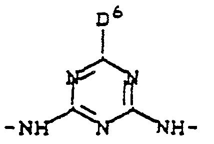 dans LaqueLLe D⁶ représente un reste = CL, OR, -N(R⁴R⁵), 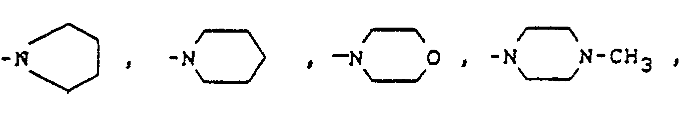 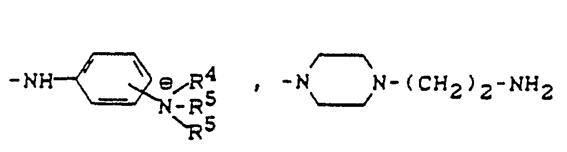 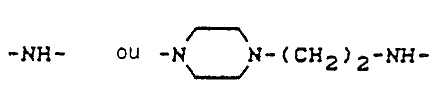
D⁴ pouvant représenter en outre un reste 1,1-cyclohexyléne, p-phénylène, NH-, -CH(C₆H₅)-, -CH-(C₆H₄-)-, -S-, -S0₂-, -CO-, -NH-CO-NH-, 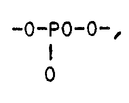 ou une liaison simple, les noyaux présents dans D⁴, D⁵ et par ailleurs encore des noyaux cités dans W² pouvant être substitués par si p vaut 2, le groupe 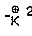 ²-A⁶-W²-A⁶-^{⊕}_{K} 2- pouvant dans son ensemble représenté aussi 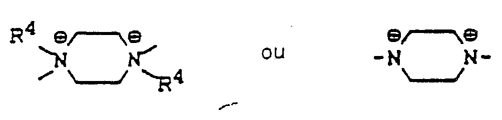 les indices m et p ne pouvant simultanément valoir 1 que lorsque T⁴ et T⁵ forment un groupement cyclique imide d'acide péri-dicarboxylique, les symboles n, p, q, An^{e} et R ont le même sens qu'à la revendication 1, et n ne peut être nul que lorsque T⁶ contient un groupe cationique 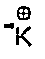 2- ou 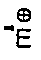 2.

Composés de formule générale 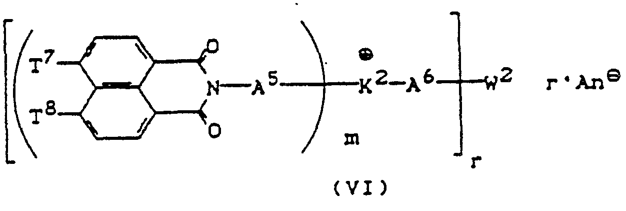 dans laquelle T⁷ et T⁸ représentent chacun un atome d'hydrogène, de chlore ou de brome, r vaut 2 ou 3.
les restes A⁶ et, lorsque m vaut 2, les restes A5 pouvant différer l'un de l'autre,
m, A^{S}, K², A⁶, W² et An^{⊖} ont le même sens qu'à la revendication 2.

4. Composés selon la revendication 3, de formule générale 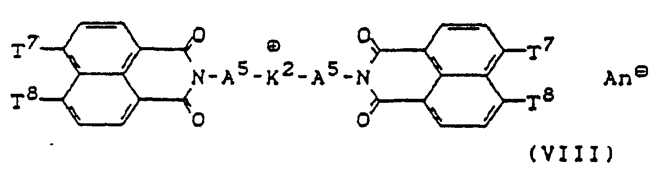 dans laquelle T⁷, T⁸, A5, 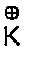 ² et An^{s} ont le même sens qu'à la revendication 3.

5. Procédé selon la revendication 1, caractérisé en ce que les composés répondent à la formule de la revendication 3 ou de la revendication 4.

6. Procédé selon la revendication 1, caractérisé en ce que les composés répondent à la formule 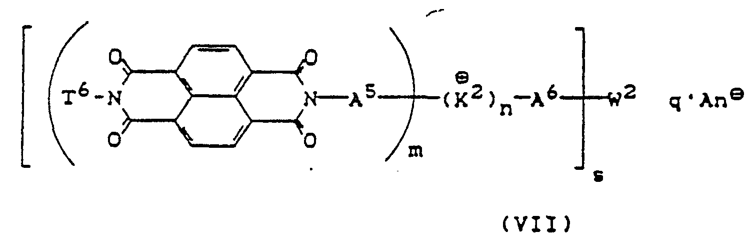 avec s valant 1 ou 2 les symboles T⁶, A⁵, 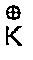 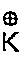 ², A⁶, w², An^{⊖}, m, n et q ont le même sens qu'à la revendication 2.

7. Procédé pour préparer des composés de la revendication 2, caractérisé en ce que :
a) on fait réagir des composés de formule 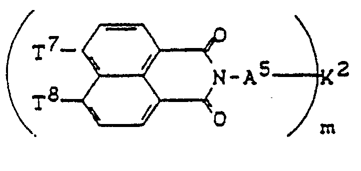 selon un rapport molaire de r:1, avec des composés de formule 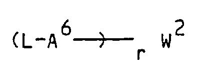 ou
b) on fait réagir des composés de formule 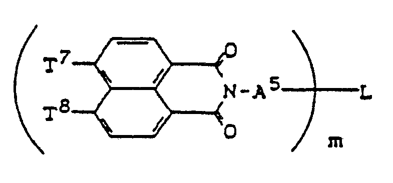 selon un rapport molaire de r:1, avec des composés de formule 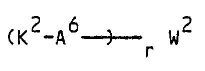 ou
c) on fait réagir les composés de type naphtalimide, cités en a), selon un rapport équimolaire avec les composés de type naphtalimide cités en b),
-K² représentant le précurseur basique monovalent non cationique de -K 2- et
L représentant un groupe pouvant être séparé sous forme d'un anion, les autres symboles ayant le même sens qu'à la revendication 3.

8. Procédé pour préparer des composés de la revendication 3, caractérisé en ce qu'on fait réagir des composés de formule 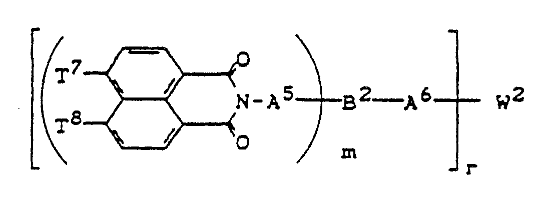 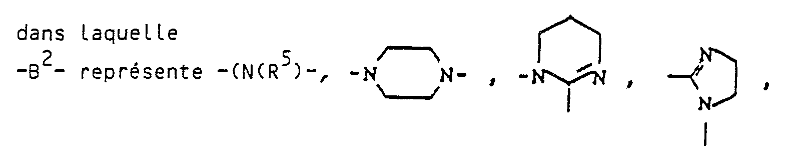 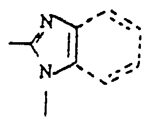 en un rapport équimolaire, avec des composés de formule dans laquelle
L représente un groupe pouvant être séparé sous forme d'anion, et les autres symboles ont le même sens qu'à la revendication 3.

9. Produit pour éteindre la fluorescence provoquée par un agent anionique d'éclaircissement optique, ce produit contenant un composé pratiquement incolore, hydrosoluble, cationique selon la revendication 1.
